# EUROPEAN PATENT SPECIFICATION

(11) **EP 1 005 446 B1**
(45) Date of publication and mention of the grant of the patent: **25.02.2004**
(21) Application number: 98946326.0
(22) Date of filing: 13.08.1998
(51) Int. Cl.: C07C 237/34, C07C 237/36, C07D 233/38, C07D 233/32, C07D 215/50, C07D 295/088, C07C 317/50, C07C 323/39, C07D 487/04, A61K 31/165, A61K 31/33

(54) **N-AROYLPHENYLALANINE DERIVATIVES**
N-AROYLPHENYLALANINDERIVATE
DERIVES DE LA N-AROYLPHENYLANANINE

(30) Priority: 22.08.1997 US 56929 P
(43) Date of publication of application: 07.06.2000
(73) Proprietor: F. HOFFMANN-LA ROCHE AG, 4070 Basel (CH)
(72) Inventor: CHEN, Li, Westfield, NJ 07090 (US); GUTHRIE, Robert, William, Saddle Brook, NJ 07663 (US); HUANG, Tai-Nang, Lexington, MA 02173 (US); HULL, Kenneth, G., Cambridge, Massachusetts 02138 (US); SIDDURI, Achytharao, Livingston, NJ 07039 (US); TILLEY, Jefferson, Wright, North Caldwell, NJ 07006 (US)
(74) Representative: Löschner, Thomas
(86) International application number: PCT/EP1998/005144
(87) International publication number: WO 1999/010313

(56) References cited:
- WO-A-97/36862
- DE-A- 19 548 709
- PATANI G A ET AL: "BIOISOSTERISM: A RATIONAL APPROACH IN DRUG DESIGN" CHEMICAL REVIEWS, vol. 96, no. 8, 1996, pages 3147-3176, XP000652176
- PATENT ABSTRACTS OF JAPAN vol. 013, no. 029 (C-562), 23 January 1989 & JP 63 233963 A (SHOWA DENKO KK;OTHERS: 01), 29 September 1988

## Description

Vascular cell adhesion molecule-1 (VCAM-1), a member of the immunoglobulin (Ig) supergene family, is expressed on activated, but not resting, endothelium. The integrin VLA-4 (a₄b₁), which is expressed on many cell types including circulating lymphocytes, eosinophils, basophils, and monocytes, but not neutrophils, is the principal receptor for VCAM-1. Antibodies to VCAM-1 or VLA-4 can block the adhesion of these mononuclear leukocytes, as well as melanoma cells, to activated endothelium *in vitro.* Antibodies to either protein have been effective at inhibiting leukocyte infiltration and preventing tissue damage in several animal models of inflammation. Anti-VLA-4 monoclonal antibodies have been shown to block T-cell emigration in adjuvant-induced arthritis, prevent eosinophil accumulation and bronchoconstriction in models of asthma, and reduce paralysis and inhibit monocyte and lymphocyte infiltration in experimental autoimmune encephalitis (EAE). Anti-VCAM-1 monoclonal antibodies have been shown to prolong the survival time of cardiac allografts. Recent studies have demonstrated that anti-VLA-4 mAbs can prevent insulitis and diabetes in non-obese diabetic mice, and significantly attenuate inflammation in the cotton-top tamarin model of colitis.

Thus, compounds which inhibit the interaction between α₄-containing integrins, such as VLA-4 and VCAM-1, will be useful as therapeutic agents for the treatment of chronic inflammatory diseases such as rheumatoid arthritis (RA), multiple sclerosis (MS), pulmonary inflammation (e.g., asthma), and inflammatory bowel disease (IBD). DE 195 48 709 describes Tyrosine derivatives as integrin inhibitors. The compounds of the present invention differ by having another substitution pattern.

Accordingly, the present invention relates to new compounds the formula: 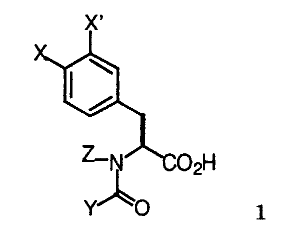 and the pharmaceutically acceptable salts and esters thereof wherein X, X', Z and Y are as defined below which inhibit the binding of VCAM-1 to VLA-4, methods for preparing such compounds, medicaments, a process for the production of such medicaments and the use of the new compounds in the treatment of illnesses, especially inflammatory diseases in which such binding acts to bring on the disease.

As used in this specification, the term "lower alkyl", alone or in combination, means a straight-chain or branched-chain alkyl group containing a maximum of six carbon atoms, such as methyl, ethyl, n-propyl, isopropyl, n-butyl, sec.butyl, isobutyl, tert.butyl, n-pentyl, n-hexyl and the like. Lower alkyl groups may be substituted by one or more groups selected independently from cycloalkyl, nitro, aryloxy, aryl, hydroxy, halogen, cyano, lower alkoxy, lower alkanoyl, lower alkylthio, lower alkyl sulfinyl, lower alkyl sulfonyl, and substituted amino, e.g., lower alkoxycarbonyl amino. Examples of substituted lower alkyl groups include 2-hydroxylethyl, 3-oxobutyl, cyanomethyl, and 2-nitropropyl

The term "cycloalkyl" means an unsubstituted or substituted 3- to 7-membered carbocyclic ring. Substitutents useful in accordance with the present invention are hydroxy, halogen, cyano, lower alkoxy, lower alkanoyl, lower alkyl, aroyl, lower alkylthio, lower alkyl sulfinyl, lower alkyl sulfonyl, aryl, heteroaryl and substituted amino.

The term "heterocycloalkyl" means an unsubstituted or substituted 5- to 6-membered carbacyclic ring in which one or two of the carbon atoms has been replaced by heteroatoms independently selected from O, S and N. Preferred heterocycloalkyl groups are pyrrolidinyl and morpholinyl.

The term "lower alkoxy" means a straight-chain or branched-chain alkoxy group containing a maximum of six carbon atoms, such as methoxy, ethoxy, n-propoxy, isopropoxy, n-butoxy, tert-butoxy and the like.

The term "lower alkylthio" means a lower alkyl group bonded through a divalent sulfur atom, for example, a methyl mercapto or a isopropyl mercapto group.

The term "aryl" means a mono- or bicylic aromatic group, such as phenyl or naphthyl, which is unsubstituted or substituted by conventional substituent groups. Preferred subsituents are lower alkyl, lower alkoxy, hydroxy lower alkyl, hydroxy, hydroxyalkoxy, halogen, lower alkylthio, lower alkylsulfinyl, lower alkylsulfonyl, cyano, nitro, perfluoroalkyl, alkanoyl, aroyl, aryl alkynyl, heteroaryl (especially tetrazolyl), lower alkynyl and lower alkanoylamino. Examples of aryl groups that may be used in accordance with this invention are unsubstituted phenyl, m- or o-nitrophenyl, p-tolyl, p-methoxyphenyl, p-chlorophenyl, m-methylthiophenyl, 2-methyl-5-nitrophenyl, 2,6-dichlorophenyl, m-perfluorophenyl, 1-naphthyl and the like.

The term "arylalkyl" means a lower alkyl group as hereinbefore defined in which one or more hydrogen atoms is/are replaced by an aryl or heteroaryl group as herein defined. Any conventional arylalkyl may be used in accordance with this invention, such as benzyl, phenyl ethyl, and the like.

The term "heteroaryl" means an unsubstituted or substituted 5- or 6-membered monocyclic hetereoaromatic ring or a 9- or 10-membered bicyclic hetereoaromatic ring containing 1, 2, 3 or 4 hetereoatoms which are independently N, S or O. Examples of hetereoaryl rings are pyridine, benzimidazole, indole, imidazole, thiophene, isoquinoline, quinzoline, tetrazole, and the like. Substitutents as defined above for "aryl" are included in the definition of heteroaryl. The wide variety of heteroaryl groups useful in accordance with the invention is illustrated by Examples 56, 57, 74, 364 and 381-386.

The term "lower alkoxycarbonyl" means a lower alkoxy group bonded via a carbonyl group. Examples of alkoxycarbonyl groups are ethoxycarbonyl and the like.

The term "lower alkylcarbonyloxy" means lower alkylcarbonyloxy groups bonded via an oxygen atom, for example an acetoxy group.
The term "lower alkanoyl" means lower alkyl groups bonded via a carbonyl group and embraces in the sense of the foregoing definition groups such as acetyl, propionyl and the like. Lower alkanoyl groups may be substituted by one or more groups selected from hydroxycarbonyl, aroyl or aryloxy, lower alkoxy, flouro, phenyl, cycloalkyl, lower alkoxy carbonyl, amino or lower alkoxycarbonyl amino.

The term "lower alkylcarbonylamino" means lower alkylcarbonyl groups bonded via a nitrogen atom, such as acetylamino.

The term "aroyl" means an mono- or bicyclic aryl or heteroaryl group bonded via a carbonyl group. Examples of aroyl groups are benzoyl, 3-cyanobenzoyl, 2-naphthyl and the like.

In the first aspect, the present invention relates to a compound of the formula: 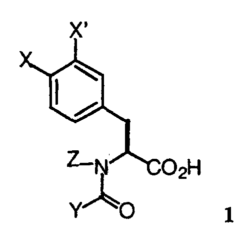 wherein X' is hydrogen, halogen, or C₁₋₆ alkyl and X is a group of the formula 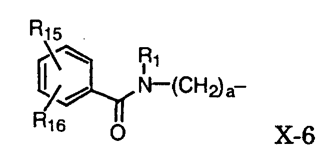 wherein:
R₁ is hydrogen or C₁₋₆ alkyl,
R₁₅ is hydrogen, halogen, nitro, C₁₋₆ alkyl sulfonyl, cyano, C₁₋₆ alkyl, C₁₋₆ alkoxy, C₁₋₆ alkoxycarbonyl, carboxy, C₁₋₆ alkyl aminosulfonyl, perfluoro C₁₋₆ alkyl, C₁₋₆ alkylthio, hydroxy C₁₋₆ alkyl, alkoxy C₁₋₆ alkyl, alkylthio C₁₋₆ alkyl, alkylsulfinyl C₁₋₆ alkyl, alkylsulfonyl C₁₋₆ alkyl, C₁₋₆ alkylsulfinyl, C₁₋₆ alkylcarbonyl, aryloxy, aroyl, aryl or a group of the formula R₁₇-C≡C-,
R₁₆ is H, halogen, nitro, cyano, C₁₋₆ alkyl, OH, perfluoro C₁₋₆ alkyl, or C₁₋₆ alkylthio,
R₁₇ is H, aryl, heteroaryl, or C₁₋₆ alkyl which is unsubstituted or substituted by OR, aryl, or heteroaryl, and
a is 0 or 1;
or X is a group of the formula: 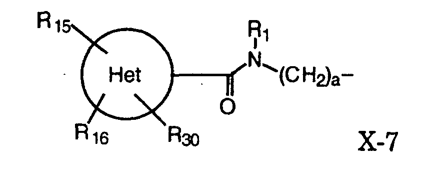
wherein Het is a 5- or 6-membered heteroaromatic ring containing 1, 2 or 3 heteroatoms selected from N,O, and S,
or
Het is a 9- or 10-membered bicyclic heteroaromatic ring containing 1, 2, 3 or 4 heteroatoms selected from O, S, and N;
a, R₁, R₁₅ and R₁₆ are as defined for X-6 and
R₃₀ is hydrogen or C₁₋₆ alkyl, or is absent;
or X is a group of the formula: 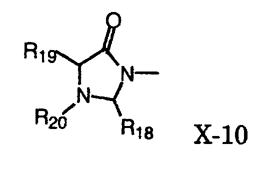 wherein:
R₁₈ is hydrogen, substituted or unsubstituted C₁₋₆ alkyl, aryl, heteroaryl, arylalkyl, heteroaryl alkyl,
R₁₉ is substituted or unsubstituted C₁₋₆ alkyl, aryl, heteroaryl, arylalkyl, heteroaryl alkyl, and
R₂₀ is substituted or unsubstituted C₁₋₆ alkyl, aroyl, C₁₋₆ alkylcarbonyl optionally substituted by carboxyl, aroyl or aryloxy;
and Y is a group of the formula: 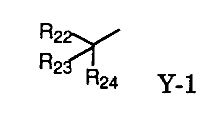
wherein:
R₂₂ and R₂₃ are independently aryl, heteroaryl or C₁₋₆ alkyl which is unsubstituted or substituted by one or more chloro, bromo, nitro, hydroxy, C₁₋₆ alkoxy, aryl, C₁₋₆ alkylcarbonyl, aroyl or cyano,
R₂₄ is aryl, cyano, alkylsulfonyl or C₁₋₆ alkyl or alkenyl up to C₆ unsubstituted or substituted by an aryl or heteroaryl ring, and when R₂₂ is aryl and R₂₃ is aryl or C₁₋₆ alkyl, R₂₄ is H, and the total number of carbon atoms in R₂₂, R₂₃ and R₂₄ is from 6 to 14; or
Y is a 3-7 membered ring of the formula: 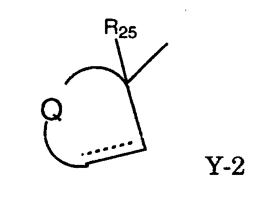
wherein:
R₂₅ is C₁₋₆ alkyl, unsubstituted or fluorine substituted alkenyl up to C₆, or a group of formula R₂₆―(CH₂)ₑ-,
R₂₆ is aryl, heteroaryl, azido, cyano, hydroxy, C₁₋₆ alkoxy, C₁₋₆ alkoxycarbonyl, C₁₋₆ alkylcarbonyl, C₁₋₆ alkylthio , C₁₋₆ alkyl sulfonyl, C₁₋₆ alkyl sulfinyl, perfluoro C₁₋₆ alkylcarbonyl, nitro, or R₂₆ is a group of formula -NR₂₈R₂₉, wherein:
R₂₈ H or C₁₋₆ alkyl,
R₂₉ is hydrogen, C₁₋₆ alkyl, C₁₋₆ alkoxycarbonyl, optionally substituted aminocarbonyl, optionally substituted C₁₋₆ alkylcarbonyl, aroyl, heteroaroyl, heterocycloalkylcarbonyl, C₁₋₆ alkyl sulfonyl, C₁₋₆ alkylaminothio-carbonyl,or R₂₈ and R₂₉ taken together with the nitrogen atom to which they are attached form a 4, 5 or 6-membered saturated heterocyclic ring containing one or two heteroatoms with the second heteroatom being O, S, or N-R₂₇;
Q is -(CH₂)_{f}O-, -(CH₂)_{f}S-, -(CH₂)_{f}-, or when f=0, a bond,
R₂₇ is H, C₁₋₆ alkyl, aryl, C₁₋₆ alkylcarbonyl, aroyl or C₁₋₆ alkoxycarbonyl,
the carbon atoms in the ring are unsubstituted or substituted by C₁₋₆ alkyl or halogen,
e is an integer from 0 to 4,
f is an integer from 0 to 3,
and the dotted line means a bond which may be absent or present;
Z is hydrogen or C₁₋₆ alkyl;
and the pharmaceutically acceptable salts and esters thereof;

the term "substituted C₁₋₆ alkyl", if not defined otherwise, means a C₁₋₆ alkyl group substituted by one or more groups selected independently from cycloalkyl, nitro, aryloxy, aryl, hydroxy, halogen, cyano, C₁₋₆ alkoxy, C₁₋₆ alkylcarbonyl, C₁₋₆ alkylthio, C₁₋₆ alkyl sulfinyl, C₁₋₆ alkyl sulfonyl and substituted amino;
the term "aryl" means a mono- or bicylic aromatic group which is unsubstituted or substituted by C₁₋₆ alkyl, C₁₋₆ alkoxy, hydroxy C₁₋₆ alkyl, hydroxy, hydroxyalkoxy, halogen, C₁₋₆ alkylthio, C₁₋₆ alkylsulfinyl, C₁₋₆ alkylsulfonyl, cyano, nitro, perfluoro-alkyl, alkanoyl, aroyl, aryl alkynyl, heteroaryl, lower alkynyl and C₁₋₆ alkylcarbonylamino;
the term "aryl C₁₋₆ alkyl" means a C₁₋₆ alkyl group in which one or more hydrogen atoms is/are replaced by an aryl or heteroaryl group;
the term "heteroaryl" means an unsubstituted or substituted 5- or 6-membered monocyclic hetereoaromatic ring or a 9- or 10-membered bicyclic hetereoaromatic ring containing 1, 2, 3 or 4 hetereoatoms which are independently N, S or O and which may be substituted with substitutents as defined above for "aryl";
the term "optionally substituted C₁₋₆ alkylcarbonyl" means C₁₋₆ alkyl groups bonded via a carbonyl group which alkyl group may be substituted by one or more groups selected from hydroxycarbonyl, aroyl or aryloxy, C₁₋₆ alkoxy, flouro, phenyl, cycloalkyl, C₁₋₆ alkoxy carbonyl, amino or C₁₋₆ alkoxycarbonyl amino;
the term "aroyl" means an mono- or bicyclic aryl or heteroaryl group as defined before bonded via a carbonyl group;
the term "cycloalkyl" means an unsubstituted or substituted 3- to 7-membered carbocyclic ring which may be substituted by hydroxy, halogen, cyano, C₁₋₆ alkoxy, C₁₋₆ alkylcarbonyl, C₁₋₆ alkyl, aroyl, C₁₋₆ alkylthio, C₁₋₆ alkyl sulfinyl, C₁₋₆ alkyl sulfonyl, aryl, heteroaryl and substituted amino
the term "heterocycloalkyl" means an unsubstituted or substituted 5- to 6-membered carbacyclic ring in which one or two of the carbon atoms has been replaced by heteroatoms independently selected from O, S and N. In a compound of formula 1 X' is preferably hydrogen. When Z is C₁₋₆ alkyl, methyl is preferred. Z is preferably hydrogen.

In a compound of formula 1, wherein the group is X-6, the groups R₁₅ and R₁₆ are preferably independently hydrogen, C₁₋₆ alkyl (especially methyl), nitro, halogen (especially chloro or fluoro), perfluoro C₁₋₆ alkyl (especially triflouromethyl), cyano or phenoxy or R₁₅ is phenoxy and R₁₆ is H. R₁ is preferably hydrogen and a is preferably 0.

The especially preferred groups X-6 are of the formula:

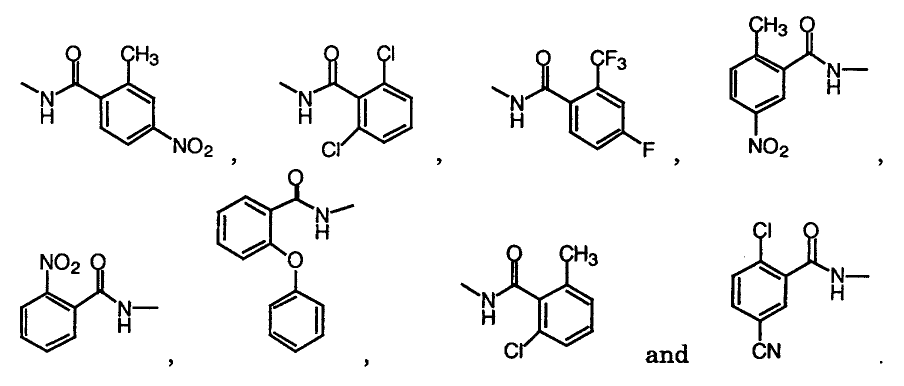

In the group X-7 Het is preferably a 5- or 6-membered monocyclic heteroaromatic ring containing 1, 2 or 3 nitrogens, or a nitrogen and a sulfur, or a nitrogen and an oxygen. More preferred the heteroaromatic ring alone is

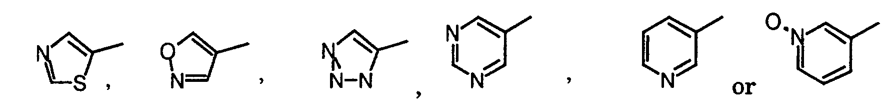

When in X-7 Het is a bicyclic heteroaromatic ring it preferably contains from 1 to 3 nitrogens as the heteroatoms. More preferred the bicyclic heteroaromatic ring is
4-quinolinyl, 1-isoquinolinyl or 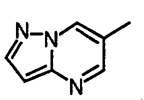 R₁₅ is preferably hydrogen, nitro, C₁₋₆ alkyl sulfonyl, cyano, C₁₋₆ alkyl, C₁₋₆ alkoxy, perfluoro C₁₋₆ alkyl, C₁₋₆ alkylthio, C₁₋₆ alkylcarbonyl, or aryl. More preferred R₁₅ is isopropyl, methyl or phenyl.
R₁₆ is preferably hydrogen, halogen, nitro, cyano, C₁₋₆ alkyl or perfluoro C₁₋₆ alkyl, in particular R₁₆ is methyl or triflouromethyl. R₃₀ in X-7 is preferably hydrogen or C₁₋₆ alkyl, especially methyl.

Most preferred the group X-7 is selected from the group consisting of 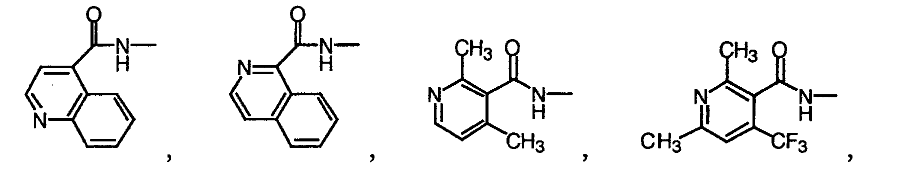 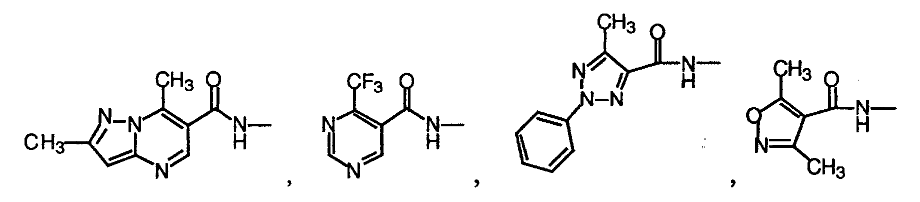 and

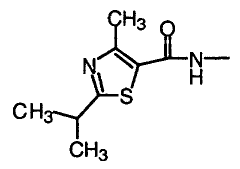

In a group of X-10 R₁₈ is preferably phenyl wherein the phenyl ring is unsubstituted or monosubstituted by halogen, or is phenyl C₁₋₆ alkyl with phenyl, chlorophenyl, especially 4-chlorphenyl, or phenylethyl being most prefered.

R₁₉ is preferably C₁₋₆ alkyl which is unsubstituted or substituted by pyridyl or phenyl wherein the phenyl ring is unsubstituted or monosubstituted by C₁₋₆ alkoxy or halogen. More preferred R₁₉ is methyl, isobutyl, benzyl, 4-chlorobenzyl, 4-methoxybenzyl or 2-pyridylmethyl.

R₂₀ is preferably optionally substituted C₁₋₆ alkylcarbonyl. More preferred R₂₀ is acetyl, butyryl, phenoxyacetyl, succinyl or glutaryl.

Most preferred X-10 groups are selected from the group consisting of:

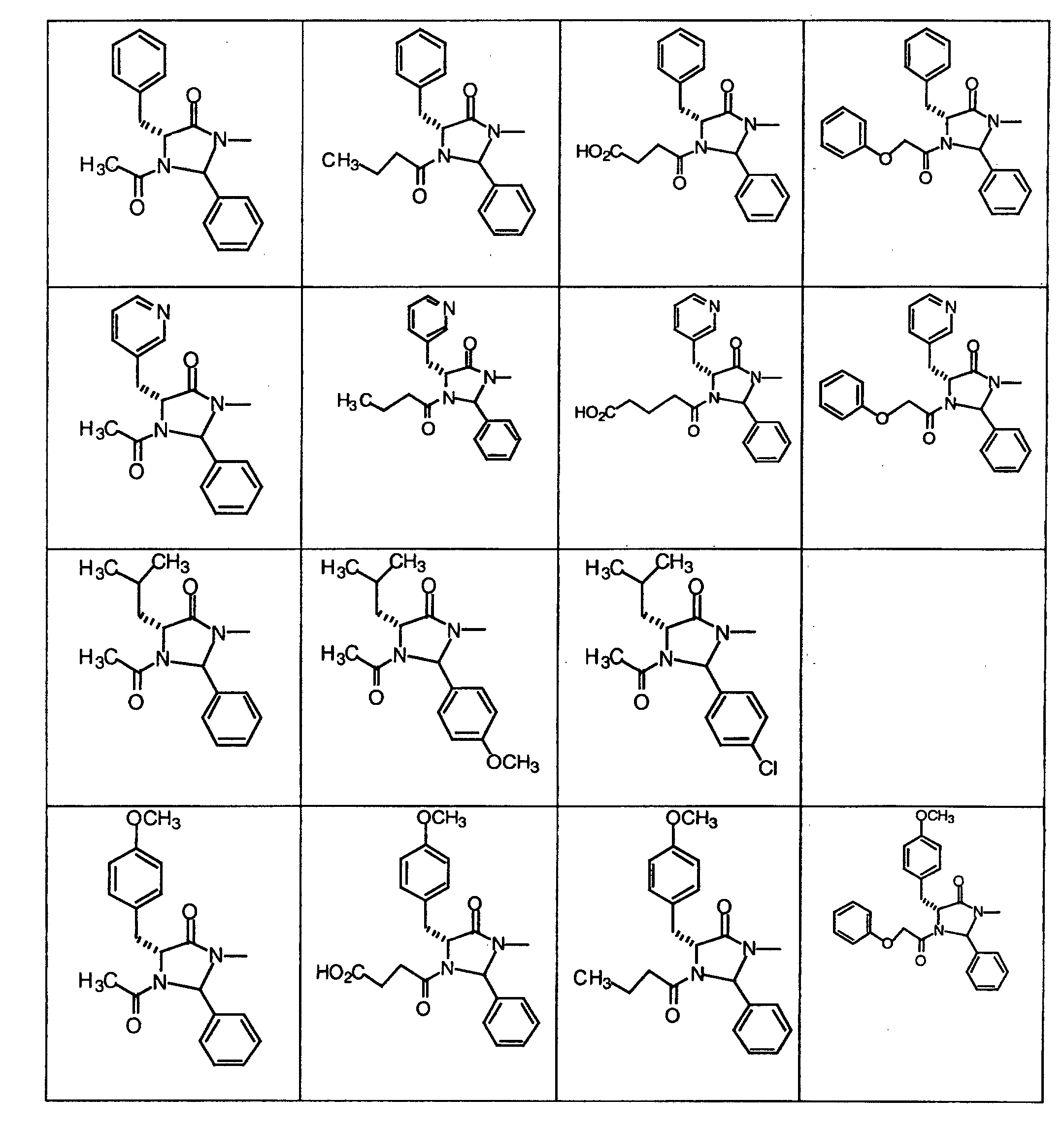

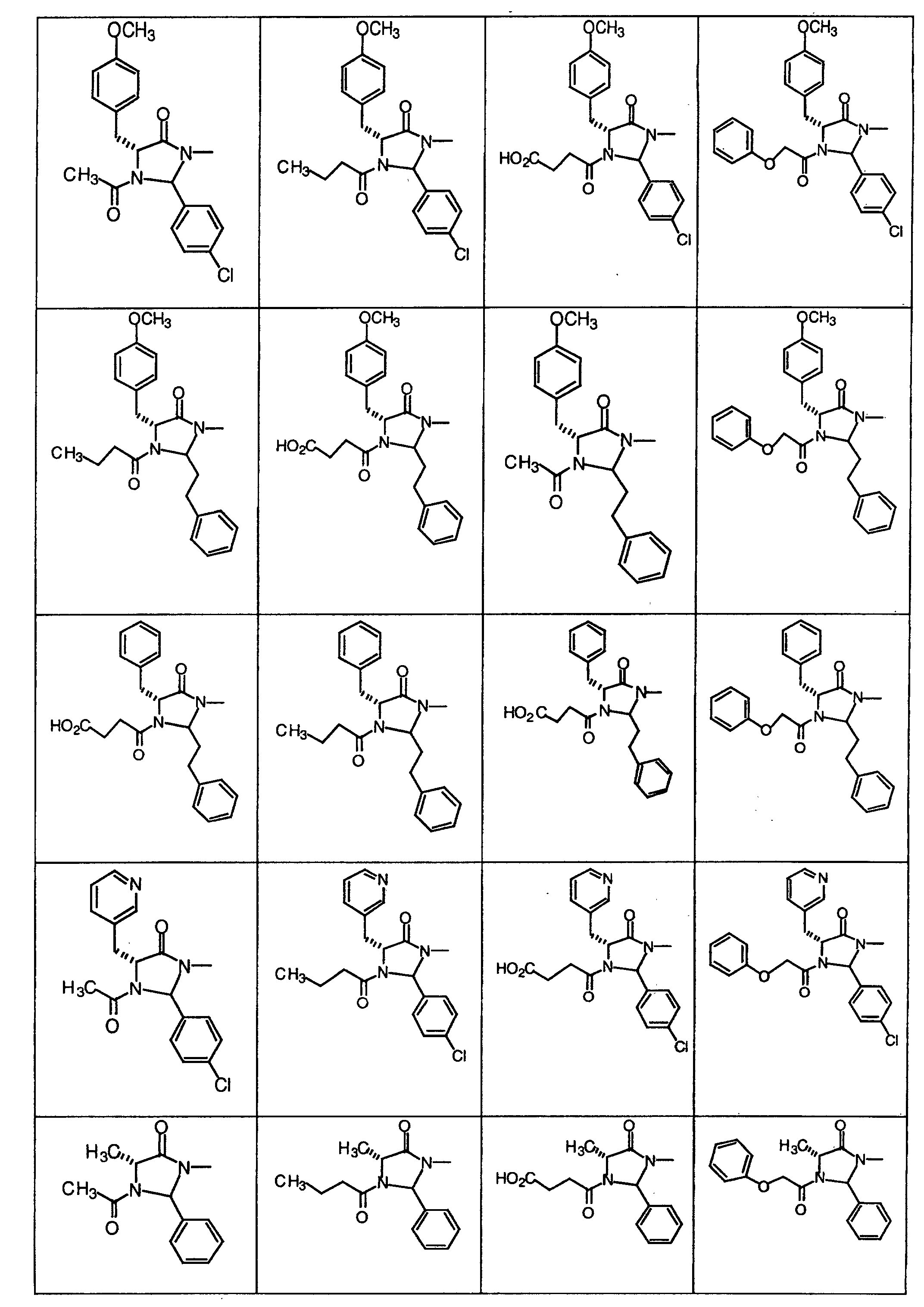

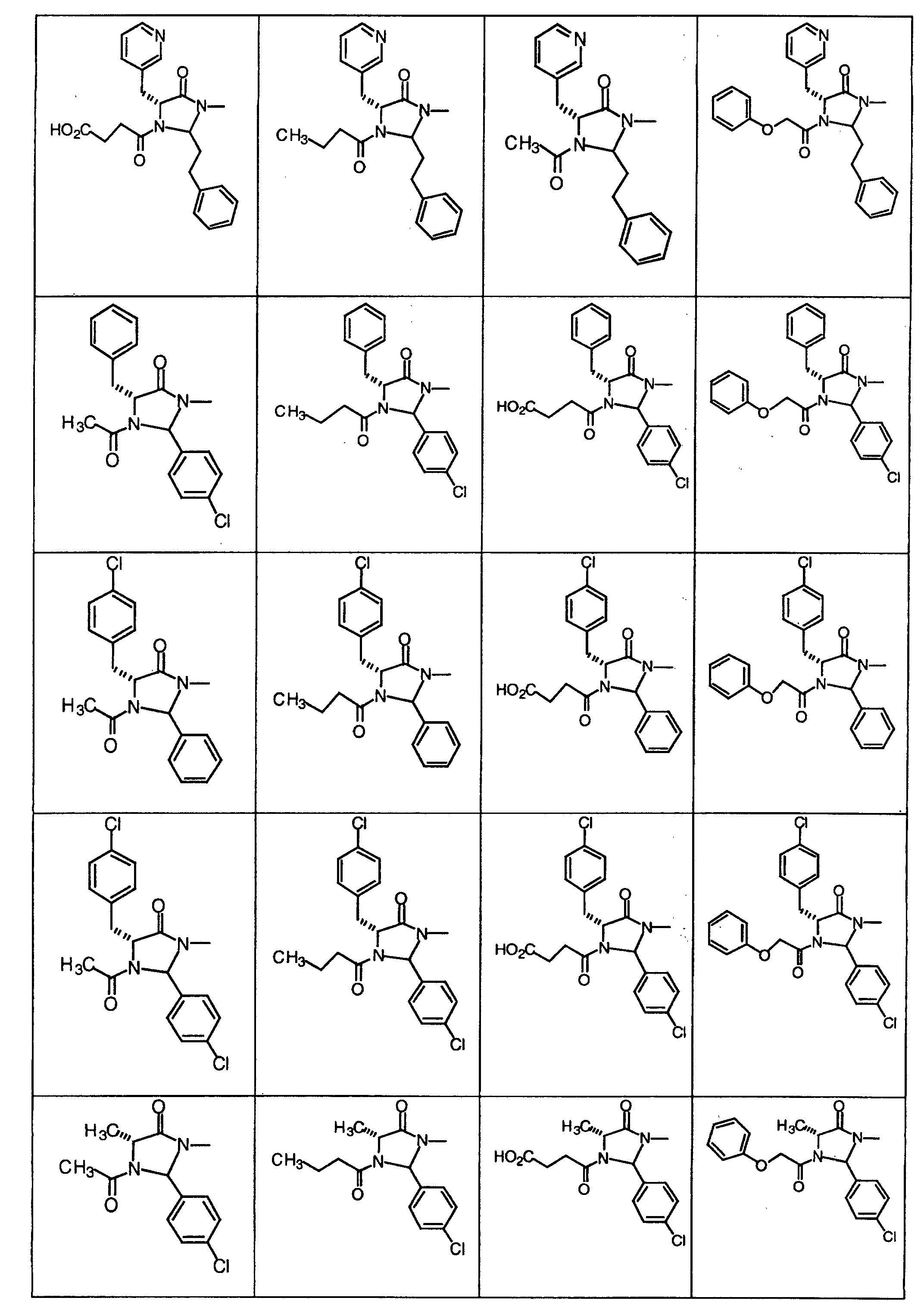

In the group Y-1, R₂₂ and R₂₃ are preferably C₁₋₆ alkyl or phenyl, and R₂₄ is preferably C₁₋₆ alkyl except when R₂₂ is aryl and R₂₃ is aryl or C₁₋₆ alkyl, then R₂₄ is preferably hydrogen.

More preferred Y-1 groups are

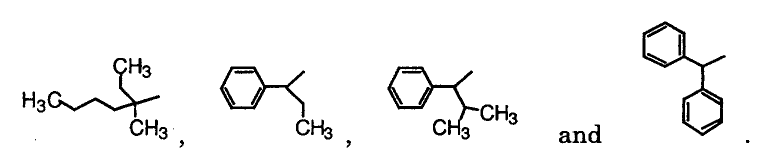

In the group Y-2, which is preferred over Y1, Q is preferably -(CH₂)_{f}- or, when f=0, a bond. Preferably f is 1,2, or 3. Preferably the additional bond is absent. R₂₅ is preferably C₁₋₆ alkyl, alkenyl up to C₆, preferably 3-buten-1yl, fluorosubstituted alkenyl up to C₆, preferably 3,3-diflouro-2-propen-1yl, or a group of formula R₂₆―(CH₂)ₑ- where e is an integer from 0 to 4. Preferably R₂₆ is aryl, heteroaryl, azido, cyano, hydroxy, C₁₋₆ alkoxy, C₁₋₆ alkoxycarbonyl, C₁₋₆ alkylcarbonyl, C₁₋₆ alkylthio, C₁₋₆ alkyl sulfonyl, C₁₋₆ alkyl sulfinyl, nitro, or R₂₆ is a group of formula -R₂₈R₂₉ wherein R₂₈ is H or C₁₋₆ alkyl, R₂₉ is preferably hydrogen, C₁₋₆ alkyl, C₁₋₆ alkoxycarbonyl, optionally substituted aminocarbonyl, optionally substituted C₁₋₆ alkylcarbonyl, aroyl, C₁₋₆ alkyl sulfonyl or R₂₈ and R₂₉ taken together with the nitrogen to which they are attached form a 4, 5 or 6-membered saturated heterocyclic ring which can contain one oxygen atom.

Preferably R₂₈ is H. Preferably R₂₆ is CH₃S(O)₂-, CH₃S-, CH₃SO-, CH₃O-, CH₃CO-, NC-, N₃-, or HO- and when R₂₆ is aryl it is preferably phenyl unsubstituted or mono-substituted by cyano, halogen, preferably chloro, C₁₋₆ alkoxy, preferably methoxy, C₁₋₆ alkyl, preferably methyl, or tetrazolyl which is unsubstituted or substituted by methyl or R₂₆ is phenyl disubstituted by C₁₋₆ alkoxy, preferably methoxy, most preferred 3,4-dimethoxyphenyl.

Preferably the alkyl group in the C₁₋₆ alkylcarbonyl group of R₂₉ is unsubstituted or substituted by C₁₋₆ alkoxy, flouro, phenyl, cycloalkyl, C₁₋₆ alkoxy carbonyl, amino or C₁₋₆ alkoxycarbonyl amino. More preferred the unsubstituted or substituted C₁₋₆ alkylcarbonyl group is CH₃CO-, (CH₃)₃CCO-, CH₃(CH₂)₃CHCH₃CO-, CH₃OCH₂CO-, CF₃CO-, C₆H₅CH₂CO-, CH₃OCO(CH₂)₂-CO-, cyclopentylCH₂CO-, H₂NCH₂CO or (CH₃)₃COCONH(CH₂)₂CO-.

In R₂₉ the aminocarbonyl group is preferably unsubstituted or substituted by C₁₋₆ alkoxy carbonyl, benzyl and preferably C₁₋₆ alkyl or monocyclic aryl. More preferred the unsubstituted or substituted aminocarbonyl group is H₂NCO-, CH₃NHCO-, CH₃OCONHCO-, C₆H₄NO₂NHCO- or C₆H₅CH₂NHCO-.

In other preferred embodiments R₂₉ is (CH₃)₃COCO-, methylaminothiocarbonyl, 4-methoxyphenylcarbonyl, 3-triflourmethyl phenylcarbonyl, 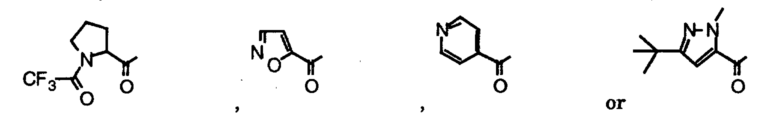 - NR₂₈R₂₉ is -NH₂ or -N(CH₃)₂ and R₂₈ and R₂₉ taken together is 4-morpholinyl.

Most preferred Y-2 is selected from a group represented by one of the following formulas:

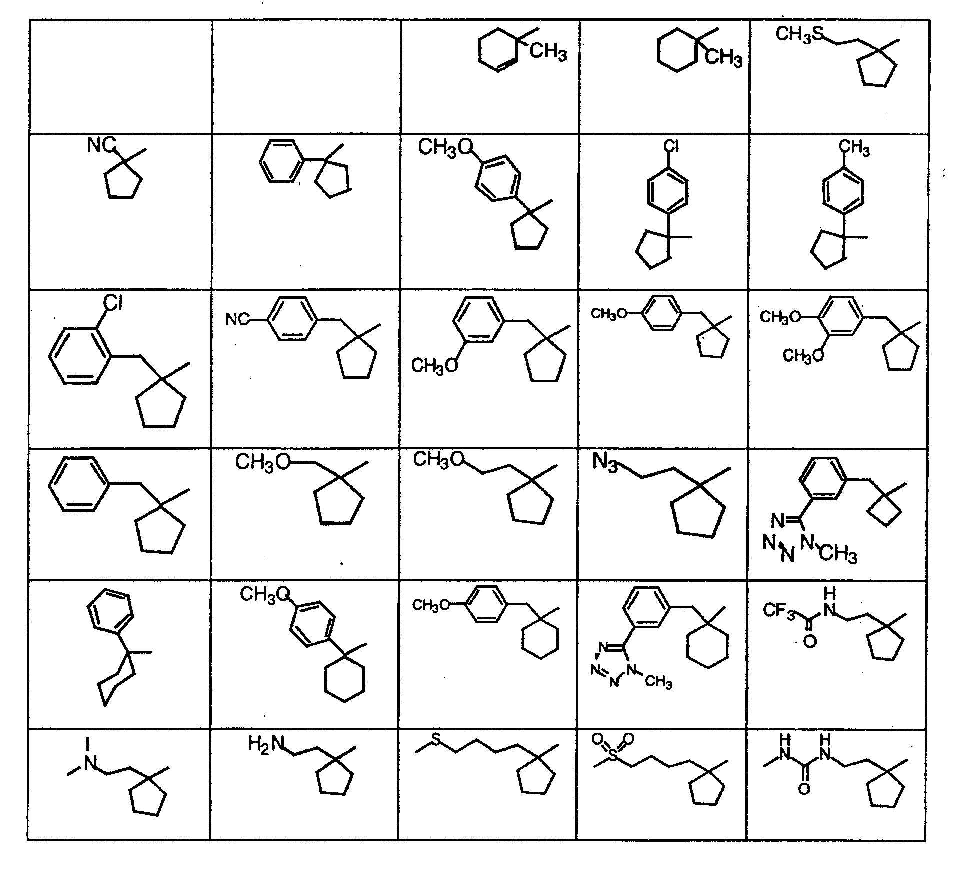

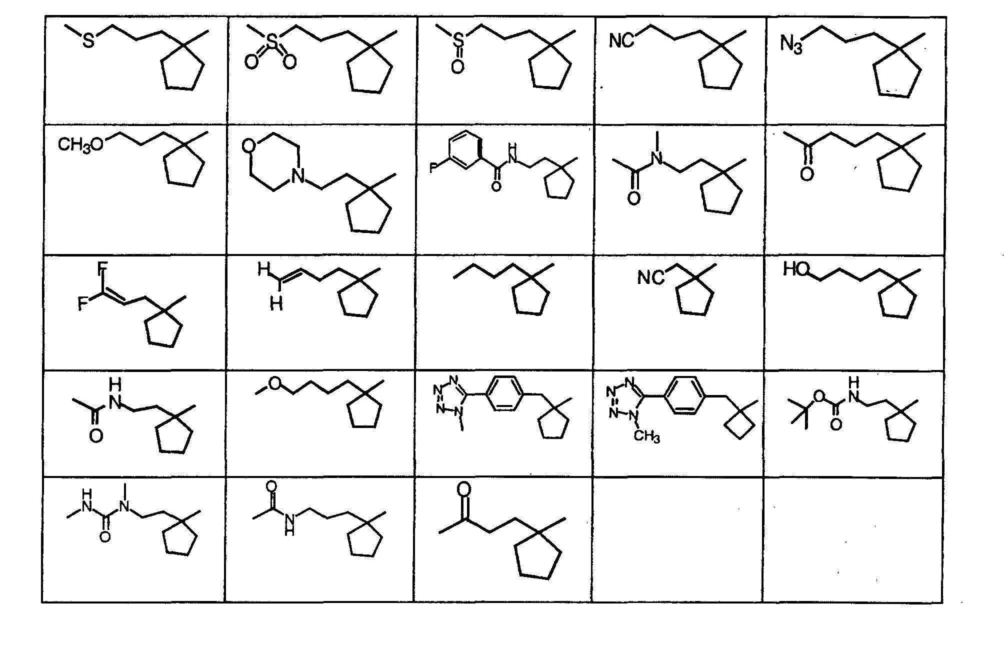

The compounds of the invention include the pharmaceutically acceptable salts and esters thereof. Certain prefered esters of the invention were discovered which are useful to improve bioavailabilty of compounds of this invention. These preferred esters are of the formula: 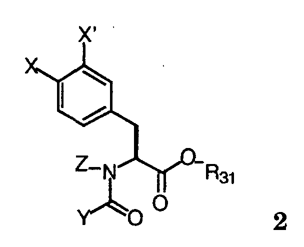 wherein X, X', Z and Y are as described above, and R₃₁ is C₁₋₆ alkyl, or R₃₁ is a group of formula P-1: 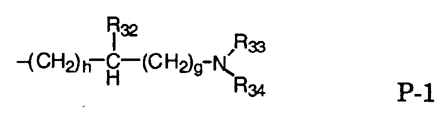 wherein:
R₃₂ is hydrogen or C₁₋₆ alkyl,
R₃₃ is hyrdrogen, C₁₋₆ alkyl, aryl,
R₃₄ is hydrogen or C₁₋₆ alkyl,
h is an integer from 0 to 2,
g is an integer from 0 to 2,
the sum of h and g is 1 to 3; or
R₃₁ is a group of formula P-2: 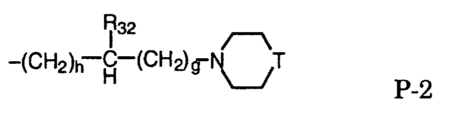
wherein:
R32, g, and h are as previously defined,
T is O, S, -(CH₂)ⱼ-, a bond (when j=0) or a group of the formula N-R₃₅,
R35 is hydrogen, C₁₋₆ alkyl, C₁₋₆ alkylcarbonyl, C₁₋₆ alkoxycarbonyl, and
j is 0, 1 or 2.

R₃₁ is preferably ethyl or 2-(4-morpholinyl)ethyl.

The compounds of the invention can exist as stereoisomers and diastereomers, all of which are encompassed within the scope of the present invention.

Preferred compounds of the invention are selected from the group consisting of:
4-[[(2,6-dichlorophenyl)carbonyl]amino]-N-[[1-[(4-methoxyphenyl) methyl]cyclopentyl]carbonyl]-L-phenylalanine,
N-[[1-[(4-methoxyphenyl)methyl]cyclopentyl]carbonyl]-4-[[(2-nitrophenyl) carbonyl] amino]-L-phenylalanine,
N-[[1-[(4-methoxyphenyl)methyl]cyclopentyl]carbonyl]-4-[[(2-methyl-5-nitrophenyl)carbonyl] amino] -L-phenylalanine,
N-[[1-[(4-methoxyphenyl)methyl]cyclopentyl]carbonyl]-4-[(4-quinolinylcarbonyl)amino]-L-phenylalanine,
N-[[1-(phenylmethyl)cyclopentyl)carbonyl]-4-[(4-quinolinylcarbonyl)amino)-L-phenylalanine,
4-[[(2,6-dichlorophenyl)carbonyl]amino]-N-[[1-(phenylmethyl) cyclopentyl]carbonyl]-L-phenylalanine,
4- [[(2-nitrophenyl)carbonyl] amino]-N-[[1-(phenylmethyl)cyclopentyl] carbonyl]-L-phenylalanine,
4-[[(2-methyl-5-nitrophenyl)carbonyl]amino]-N-[[1-(phenylmethyl) cyclopentyl] carbonyl]-L-phenylalanine,
4-[[(2,6-dichlorophenyl)carbonyl]amino]-N-[[1-[2-[[N-(1,1-dimethylethyl) carbonyl] amino] ethyl] cyclopentyl] carbonyl] -L-phenylalanine,
4-[[(2,6-dichlorophenyl)carbonyl]amino]-N-[[1-[2-[(trifluoroacetyl) amino] ethyl]cyclopentyl]carbonyl]-L-phenylalanine,
4- [ [(2,6-dichlorophenyl)carbonyl] amino] -N- [ [1- [2- [(2-amino-1-oxoethyl)amino] ethyl]yclopentyl]carbonyl]-L-phenylalanine,
4- [[(2,6-dichlorophenyl)carbonyl]amino] -N- [[1-[2- [ [2- [[(1,1-dimethylethoxy) carbonyl]amino]-1-oxoethyl]amino]ethyl] cyclopentyl]carbonyl]-L-phenylalanine,
4- [ [(2,6-dichlorophenyl)carbonyl] amino] -N- [ [2- [[(methoxy)carbonyl] amino]ethyl]cyclopentyl]carbonyl]-L-phenylalanine,
4-[[(2,6-dichlorophenyl)carbonyl]amino]-N-[[1-[2-[(methylsulfonyl) amino]ethyl]cyclopentyl]carbonyl]-L-phenylalanine,
4-[[(2,6-dichlorophenyl)carbonyl]amino]-N-[[1-[2-[(acetyl)(methyl) amino]ethyl]cyclopentyl]carbonyl]-L-phenylalanine,
4- [[(2,6-dichlorophenyl)carbonyl]amino] -N- [[1-[2- [ (methylamino)carbonyl] (methyl)amino]ethyl] cyclopentyl]carbonyl] -L-phenylalanine,
4-[[(2,6-dichlorophenyl)carbonyl]amino]-N-[[1-[2-[(methoxycarbonyl)-(methyl)amino]ethyl]cyclopentyl]carbonyl]-L-phenylalanine,
4-[[(2,6-dichlorophenyl)carbonyl]amino]-N-[[1-(2-methoxyethyl)cyclopentyl] carbonyl]-L-phenylalanine,
4- [[(2,6-dichlorophenyl)carbonyl] amino] -N- [[1-[(2- [(methylsulfonyl)ethyl) cyclopentyl]carbonyl]-L-phenylalanine,
4-[[(2,6-dichlorophenyl)carbonyl]amino]-N-[[1-[(2-(methylsulfinyl)ethyl] cyclopentyl]carbonyl]-L-phenylalanine,
4-[(2,6-Dimethyl-4-trifluoromethyl-3-pyridinyl)carbonyl]amino]-N-[[1-[4-(methylsulfonyl)butyl]cyclopentyl]carbonyl]-L-phenylalanine,
4-[[(2,4-dimethylpyridin-3-yl)carbonyl]amino]-N-[[1-[4-(methylsulfonyl) butyl]cyclopentyl]carbonyl]-L-phenylalanine, or
4- [[(2,6-dichlorophenyl)carbonyl]amino]-N-[[1-(4-methoxyphenylmethyl) cyclohexyl] carbonyl]-L-phenylalanine.

Other preferred compounds having the formula selected from the group consisting of

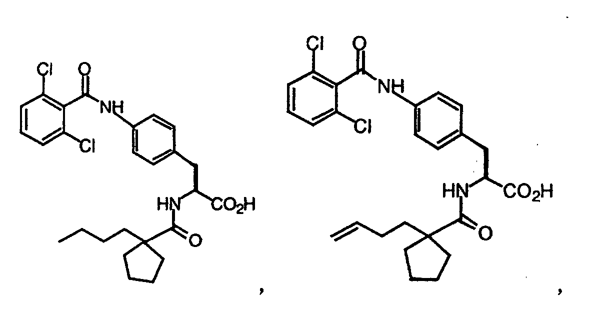

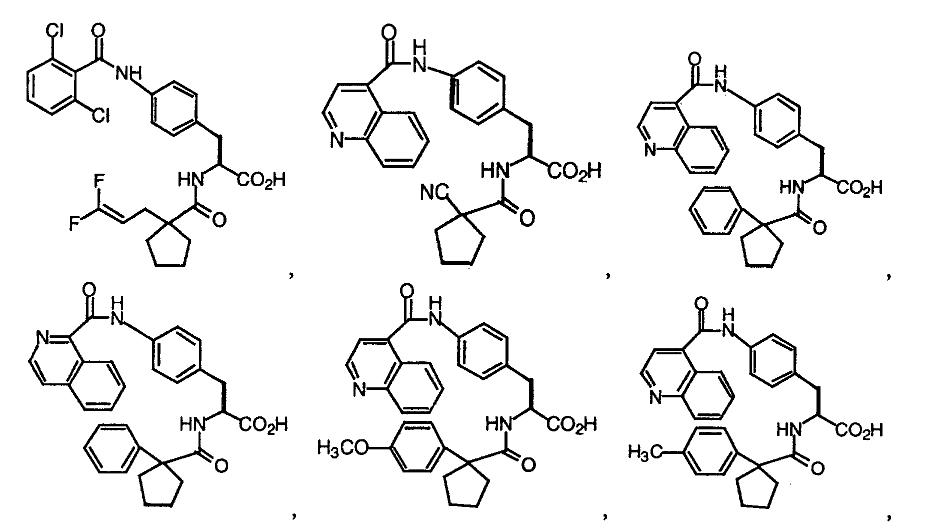

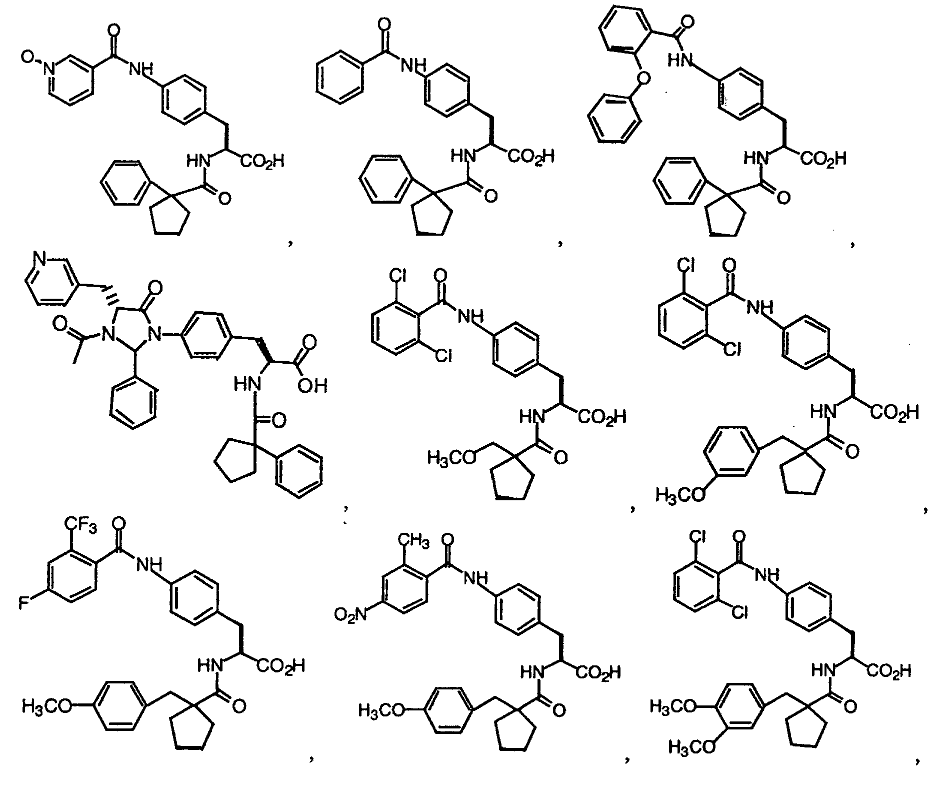

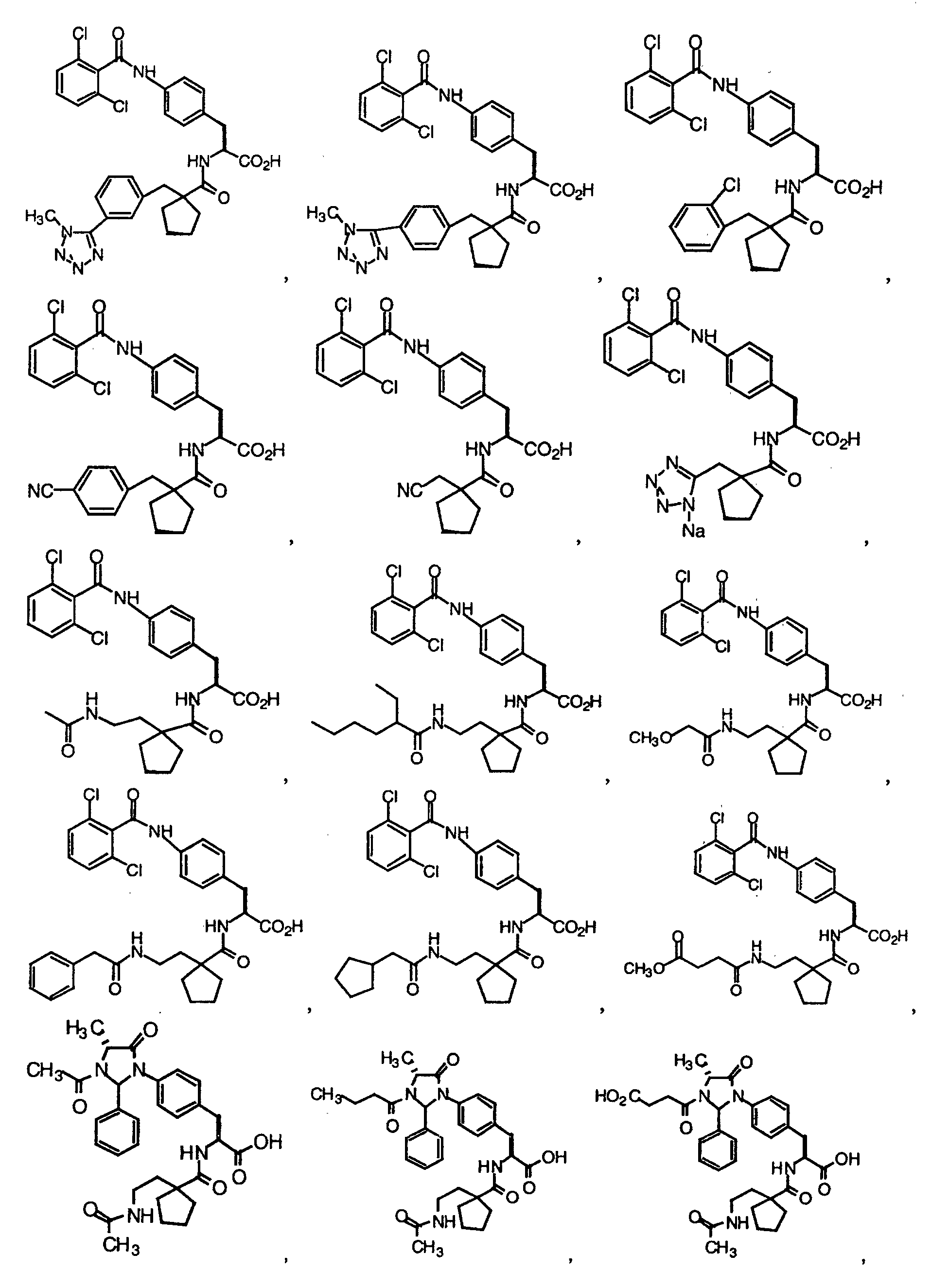

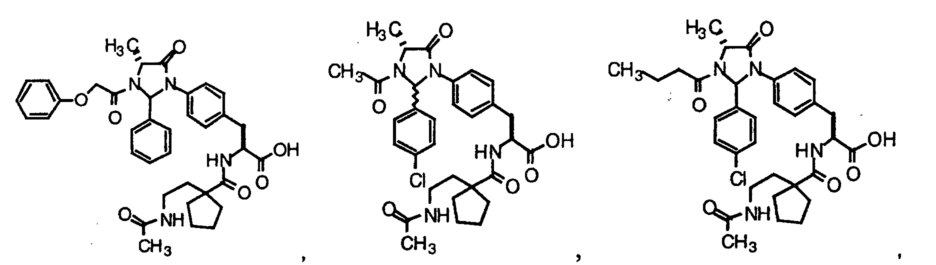

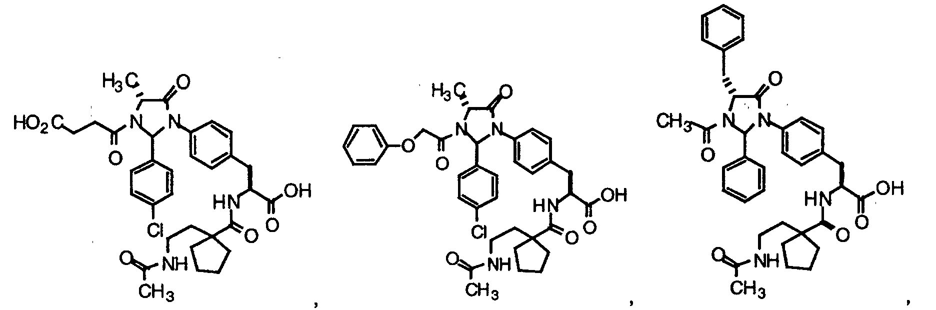

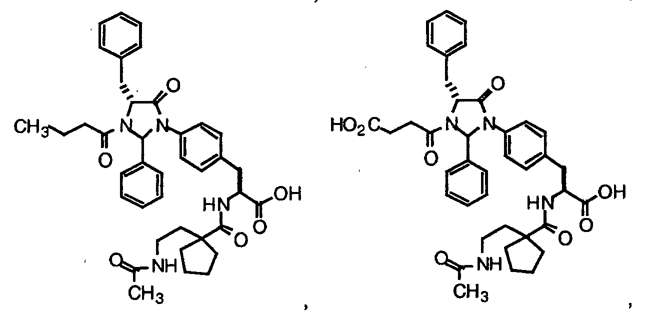

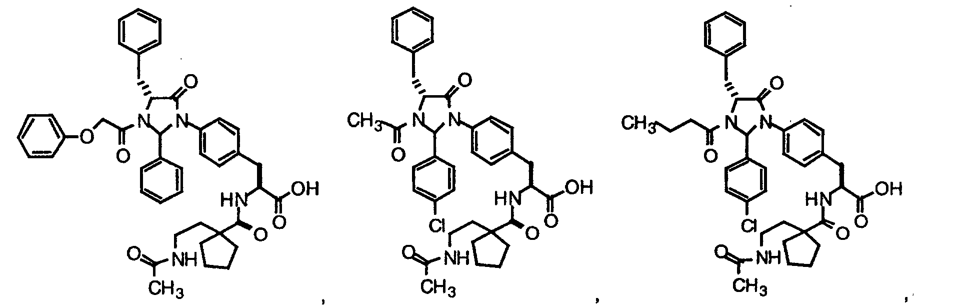

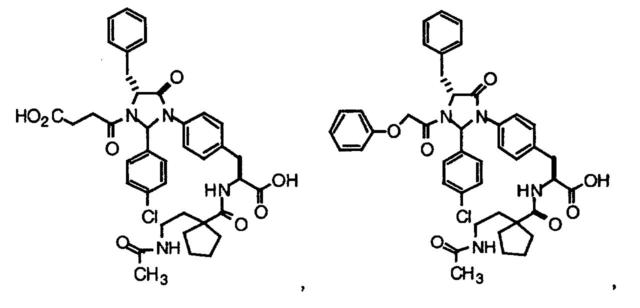

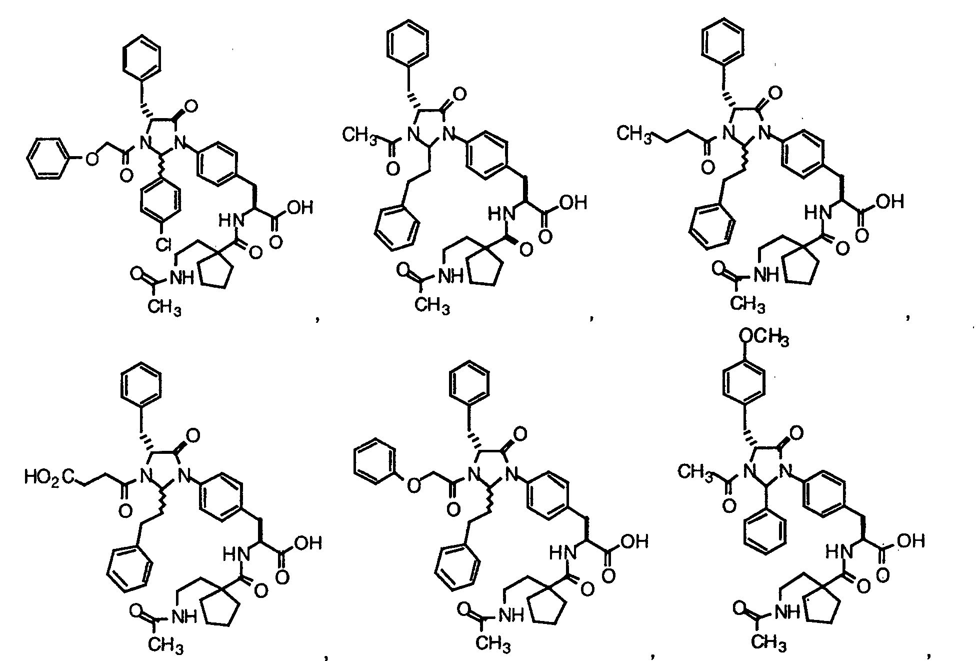

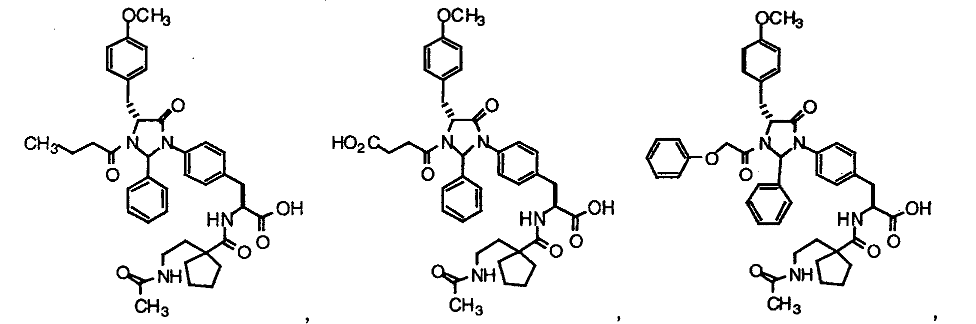

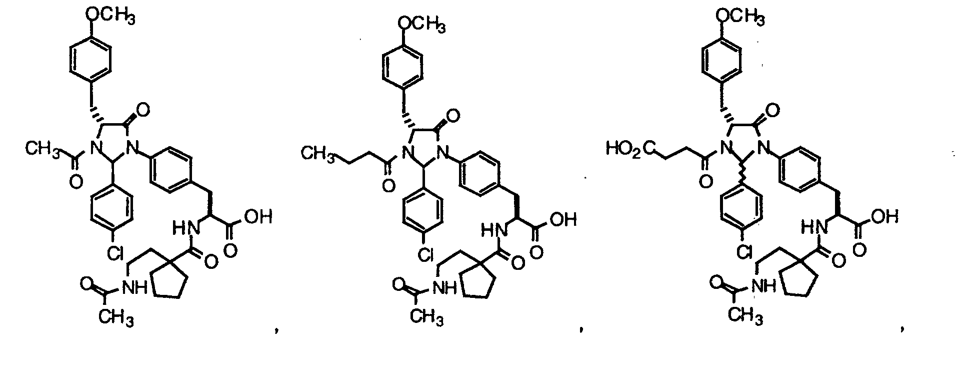

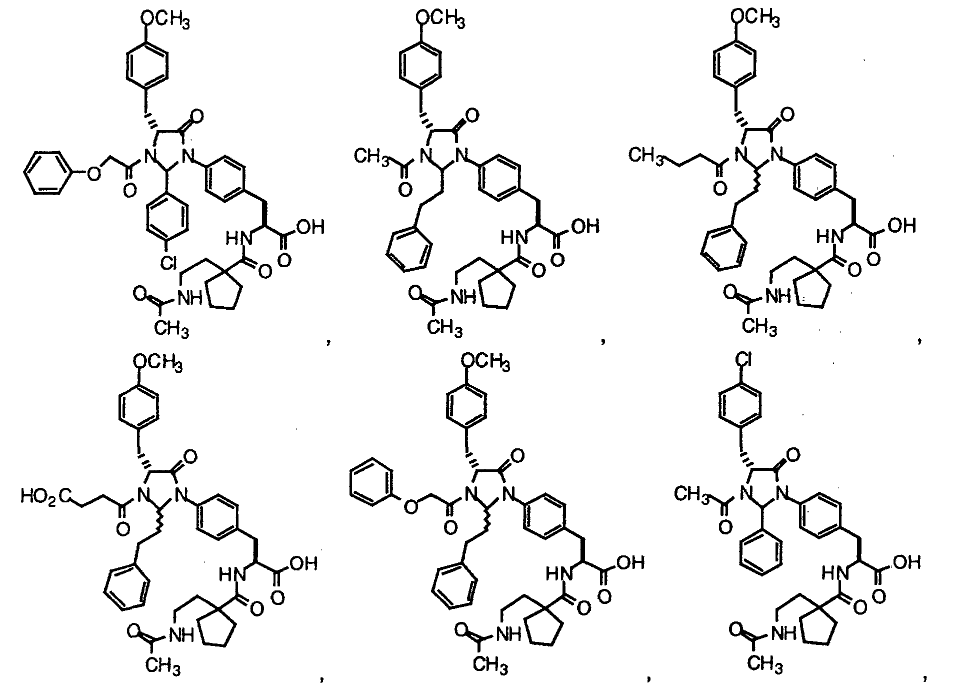

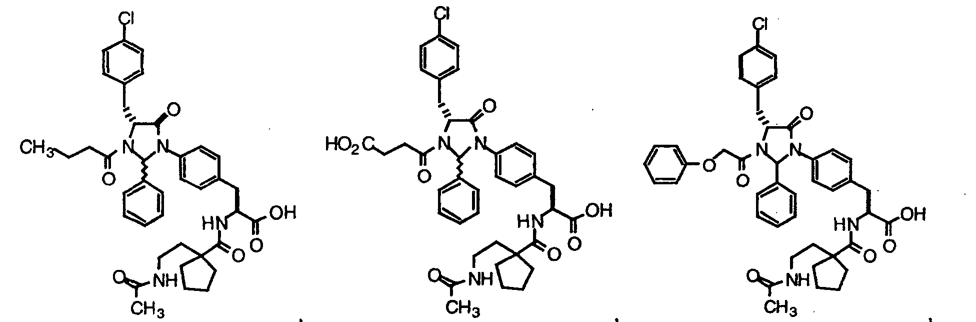

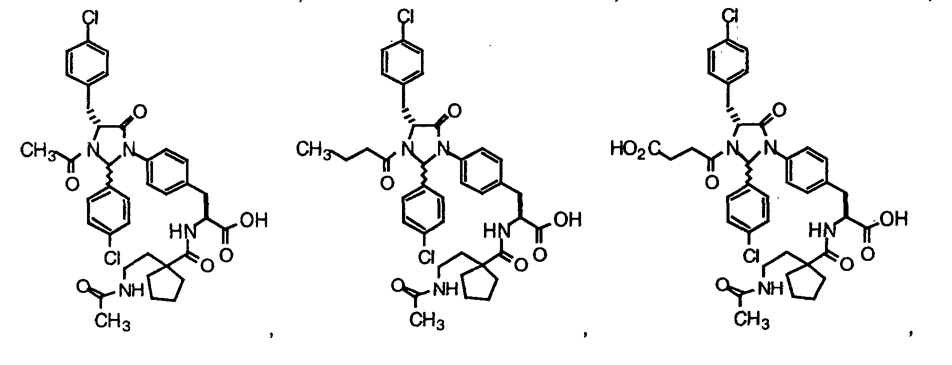

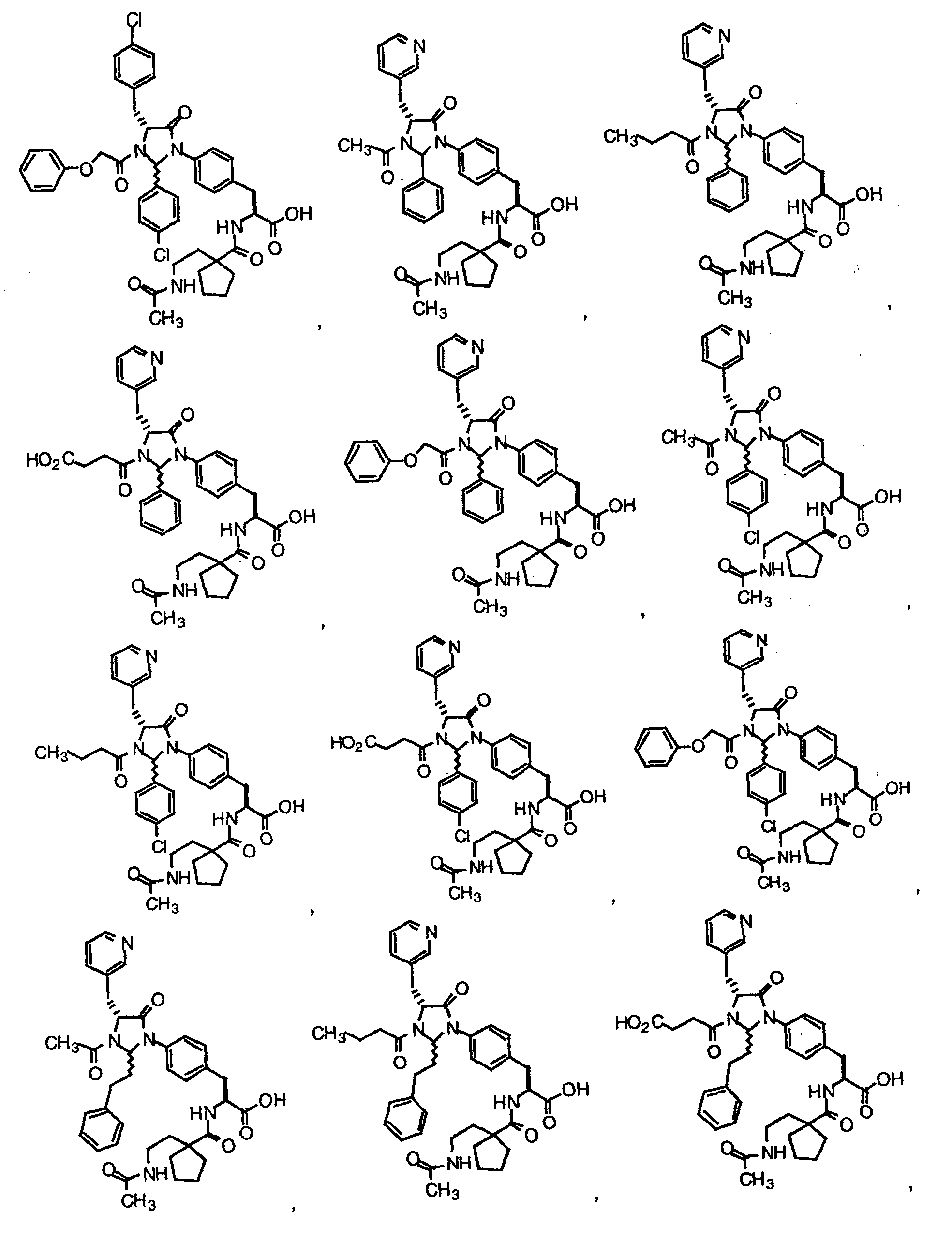

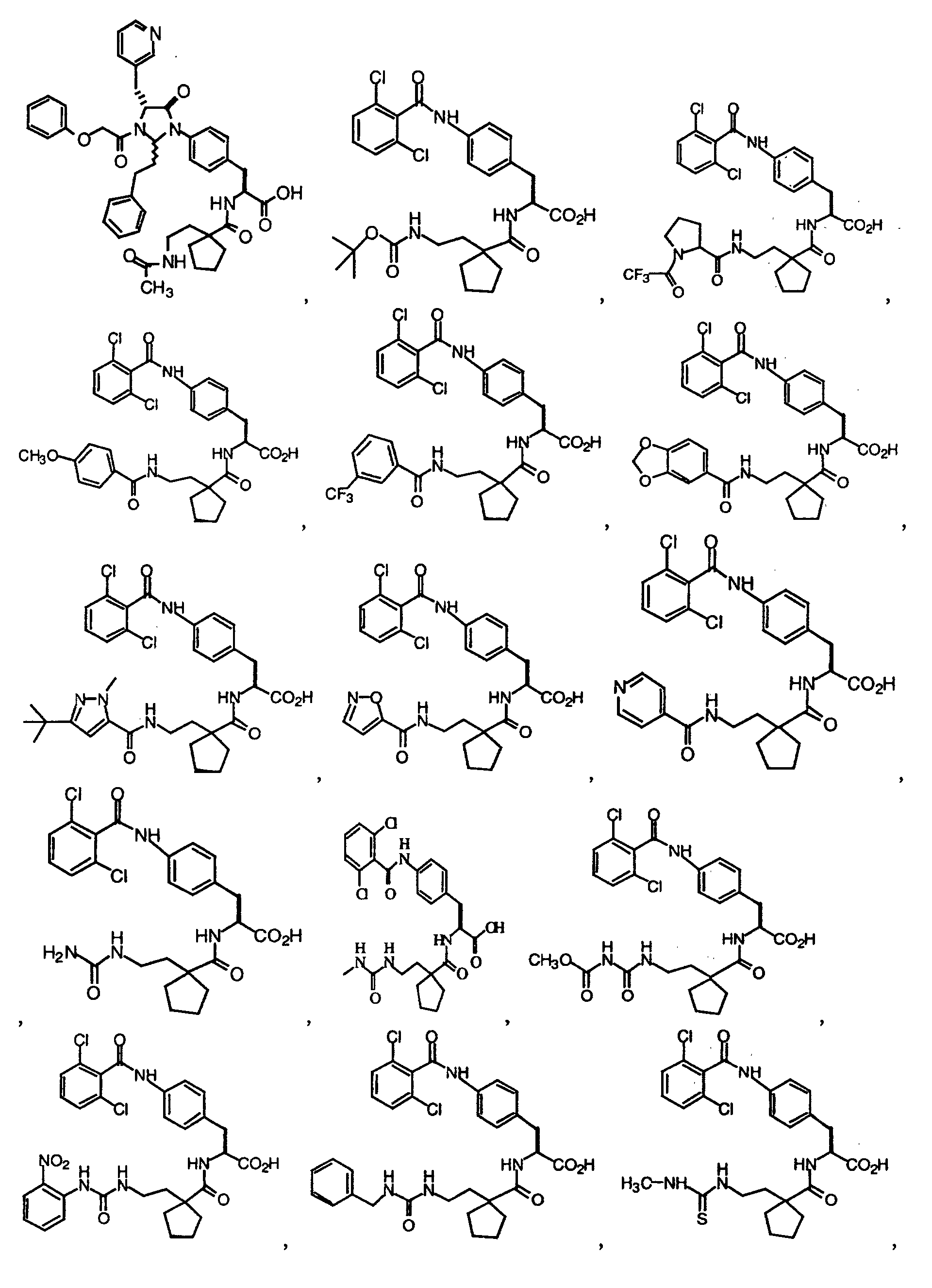

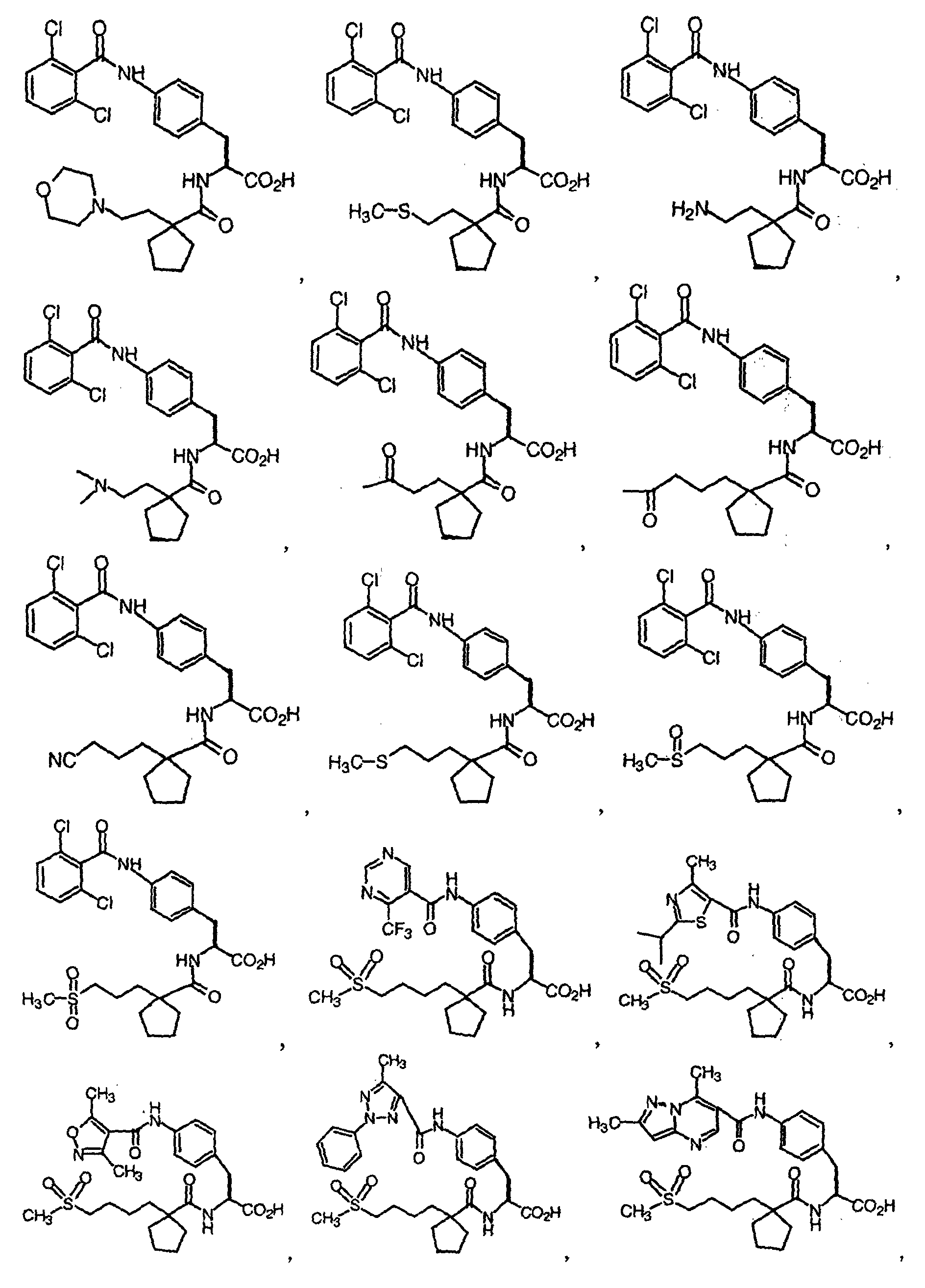

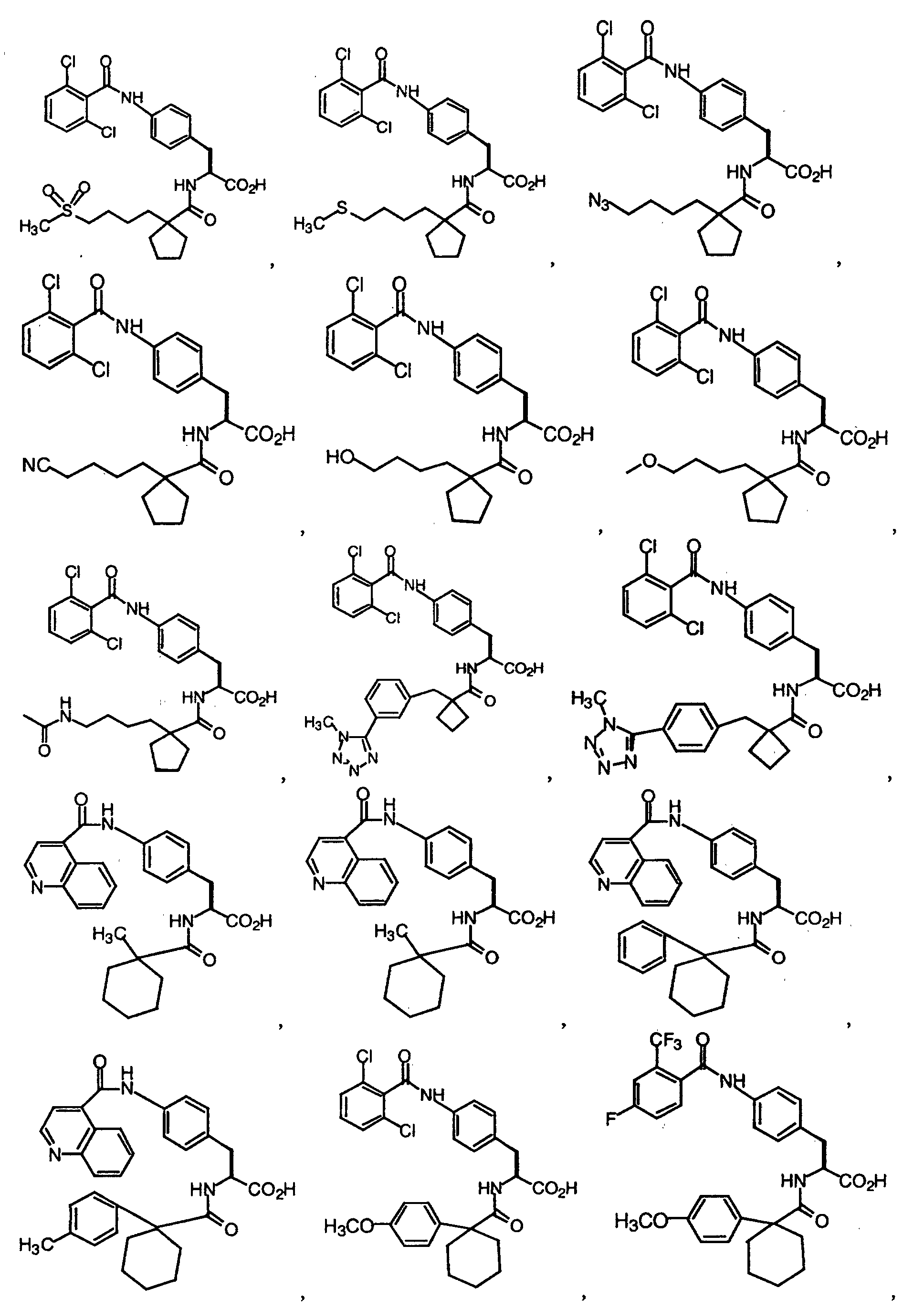

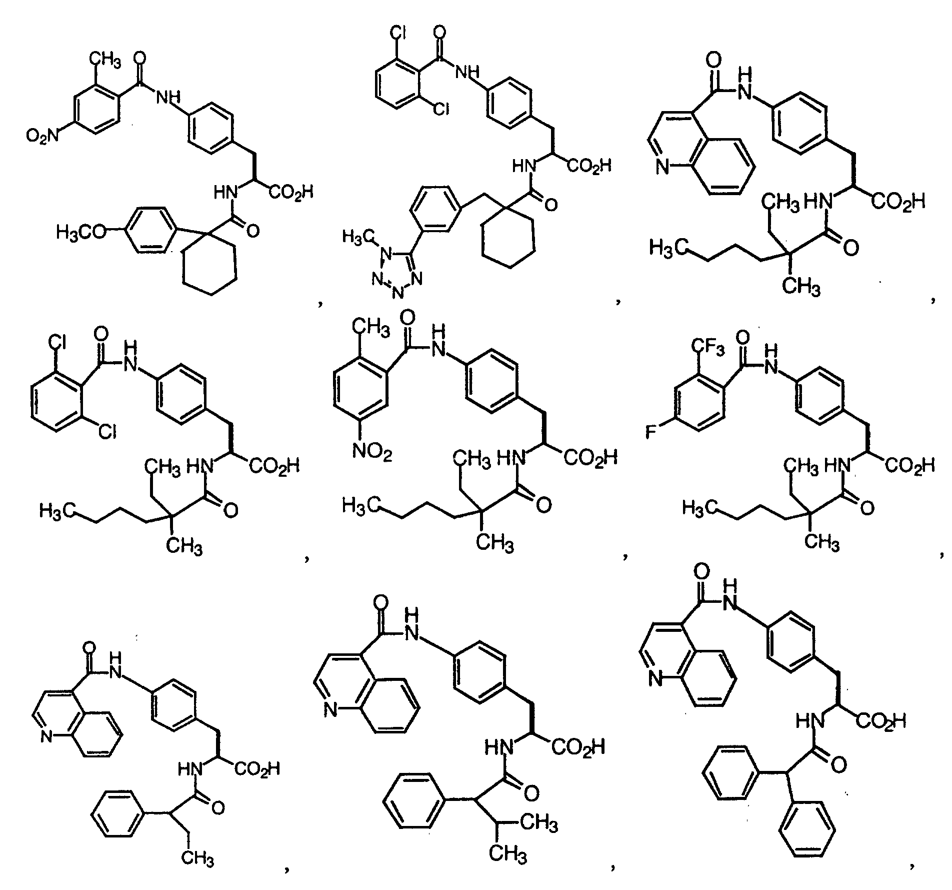

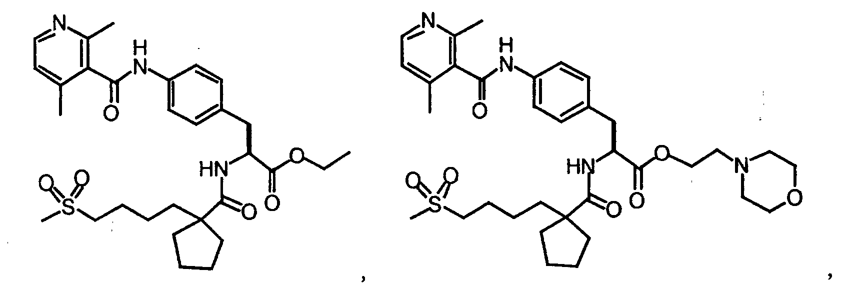

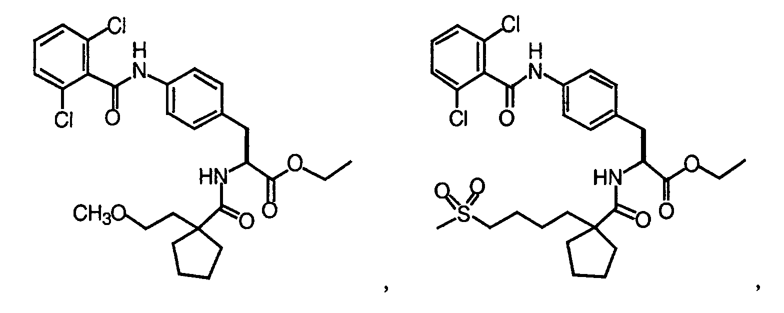

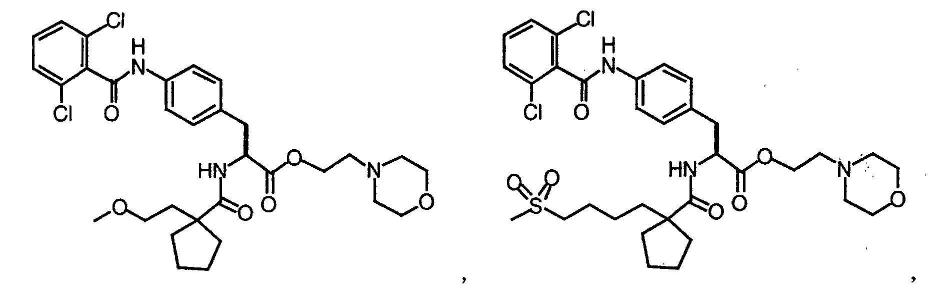

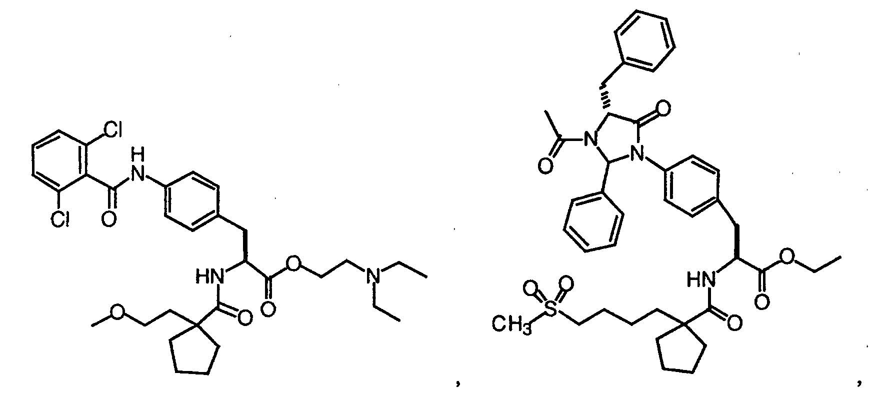

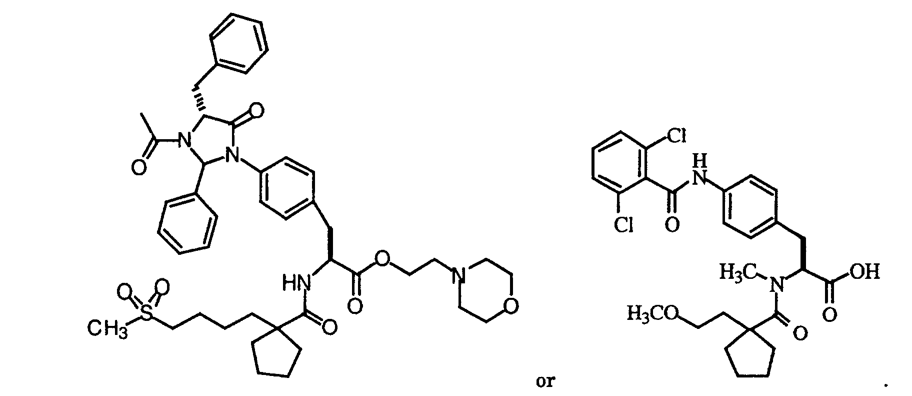

The compounds of the invention inhibit the binding of VCAM-1 and fibronectin to VLA-4 on circulating lymphocytes, eosinophils, basophils, and monocytes ("VLA-4-expressing cells"). The binding of VCAM-1 and fibronectin to VLA-4 on such cells is known to be implicated in certain disease states, such as rheumatoid arthritis, multiple sclerosis, inflammatory bowel disease, and particularly in the binding of eosinophils to pulmonary endothelium which is the cause of the pulmonary inflammation which occurs in asthma. Thus, the compounds of the present invention would be useful for the treatment of asthma.

In another aspect, on the basis of their capability of inhibiting binding of VCAM-1 and fibronectin to VLA-4 on circulating lymphocytes, eosinophils, basophils, and monocytes, the compounds of the invention can be used as medicament for the treatment of disorders which are known to be associated with such binding. Examples of such disorders are rheumatoid arthritis, multiple sclerosis, asthma, and inflammatory bowel disease. The compounds of the invention are preferably used in the treatment of diseases which involve pulmonary inflammation, such as asthma. The pulmonary inflammation which occurs in asthma is related to eosinophil infiltration into the lungs wherein the eosinophils bind to endothelium which has been activated by some asthma-triggering event or substance.

Furthermore, compounds of the invention also inhibit the binding of VCAM-1 and MadCAM to the cellular receptor alpha4-beta7, also known as LPAM, which is expressed on lymphocytes, eosinophiles and T-cells. While the precise role of alpha4-beta7 interaction with various ligands in inflammatory conditions such as asthma is not completely understood, compounds of the invention which inhibit both alpha4-beta1 and alpha4-beta7 receptor binding are particularly effective in animal models of asthma. Furthermore work with monoclonal antibodies to alpha4-beta7 indicate that compounds which inhibit alpha4-beta7 binding to MadCAM or VCAM are useful for the treatment of inflammatory bowel disease. They would also be useful in the treatment of other diseases in which such binding is implicated as a cause of disease damage or symptoms.

The compounds of the invention can be administered orally, rectally, or parentally, e.g., intravenously, intramuscularly, subcutaneously, intrathecally or transdermally; or sublingually, or as opthalmalogical preparations, or as an aerosol for the treatment of pulmonary inflammation. Capsules, tablets, suspensions or solutions for oral administration, suppositories, injection solutions, eye drops, salves or spray solutions are examples of administration forms.

Intravenous, intramuscular, oral or inhalation administration is a preferred form of administration. The dosages in which the compounds of the invention are administered in effective amounts depend on the nature of the specific active ingredient, the age and the requirements of the patient and the mode of administration. Dosages may be determined by any conventional means, e.g., by dose-limiting clinical trials. Thus, the invention further comprises a method of treating a host suffering from a disease in which VCAM-1 or fibronectin binding to VLA-4-expressing cells is a causative factor in the disease symptoms or damage by administering an amount of a compound of the invention sufficient to inhibit VCAM-1 or fibronectin binding to VLA-4-expressing cells so that said symptoms or said damage is reduced. In general, dosages of about 0.1-100 mg/kg body weight per day are preferred, with dosages of 1-25 mg/kg per day being particularly preferred, and dosages of 1-10 mg/kg body weight per day being espeically preferred.

The invention further relates to pharmaceutical compositions or medicaments which contain a pharmaceutically effective amount of a compound of the invention and a pharmaceutically and therapeutically acceptable carrier. Such compositions may be formulated by any conventional means by bringing a compound according to the present invention into a galenical administration form together with a therapeutically inert carrier material. If desired, one or more additional therapeutically active substances may be added.

Tablets or granulates can contain a series of binders, fillers, carriers or diluents. Liquid compositions can be, for example, in the form of a sterile water-miscible solution. Capsules can contain a filler or thickener in addition to the active ingredient. Furthermore, flavour-improving additives as well as substances usually used as preserving, stabilizing, moisture-retaining and emulsifying agents as well as salts for varying the osmotic pressure, buffers and other additives can also be present.

The previously mentioned carrier materials and diluents can comprise any conventional pharmaceutically acceptable organic or inorganic substances, e.g., water, gelatine, lactose, starch, magnesium stearate, talc, gum arabic, polyalkylene glycols and the like.

Oral unit dosage forms, such as tablets and capsules, preferably contain from 25 mg to 1000 mg of a compound of the invention.

The compounds of the present invention may be prepared by any conventional means.

In general the process for the preparation of a compound of formula 1 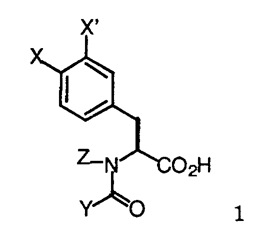 wherein X, X', Z and Y are as defined in claim 1,
is characterized in that in a compound of formula 1b 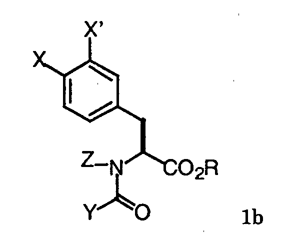 wherein X, X', Z and Y are as defined and R is a protecting group or a solid support,
the protecting group or the solid support is cleaved of and, if desired, converting a compound of formula 1 into a pharmaceutically acceptable salt.

The protecting group may be an alkyl group, e.g. methyl, ethyl or tertbutyl, and the solid support may be a resign used in solid phase synthesis, e.g. a Wang resin. The cleavage conditions depend on the substitution pattern of the final compound and the protecting group used are outlined in detail below in connection with the synthesis pathways for preparing compounds of formula 1 as well as their salts and esters thereof.

In reaction Scheme 1, a compound of formula 1 in which R₁ is H or lower alkyl, and which is a known compound or can be prepared by standard methodology, is treated with a reducing agent capable of selectively reducing a nitro group in the presence of a benzylic alcohol. This procedure is advantageously carried out in the presence of a derivatizing agent of the formula R₂-OCOX wherein X is a leaving group and R₂ is tert-alkyl, benzyl or the like so as to form a readily cleavable protecting group, thus leading directly to a compound of formula 2. For example, this procedure can be conveniently carried out by catalytic hydrogenation of 1 over Pd(C) in ethyl acetate in the presence of di-tert-butyl dicarbonate to give a derivative of 2 in which R₂ is tert-butyl.

Conversion to an aldehyde of formula 3 can be carried out using an one of a variety of oxidizing agents capable of oxidizing a benzylic alcohol to the corresponding aldehyde, for example activated manganese dioxide in a suitable solvent, for example dichloromethane. Reaction of 3 to give a dehydroamino acid of formula 5 can be effected by treatment with a Wittig reagent of formula 4 in which R₃ is lower alkyl and R₄ is an alkoxy group, for example benzyloxy- or tert-butoxy- or represents a portion of one of the acyl groups of the compounds of the invention, for example substituted lower alkyl or substituted cycloalkyl. For example treatment of 3 with (±)-N-(benzyloxycarbonyl)-α-phosphonoglycine trimethyl ester in the presence of a suitable base for example tetramethyl guanidine leads directly to a dehydroamino acid of formula 5, R₃ = methyl and R₄ = benzyloxy. Enantioselective reduction of 5 to the L-amino acid 6 can be effected by use of a number of reducing agents suitable for the purpose, for example, the recently described ethyl-DuPHOS rhodium reagent (Burk, M. J., Feaster, J. E.; Nugent, W. A.; Harlow, R. L. *J. Am. Chem. Soc.* **1993**, *115*, 10125) using essentially the literature procedure.

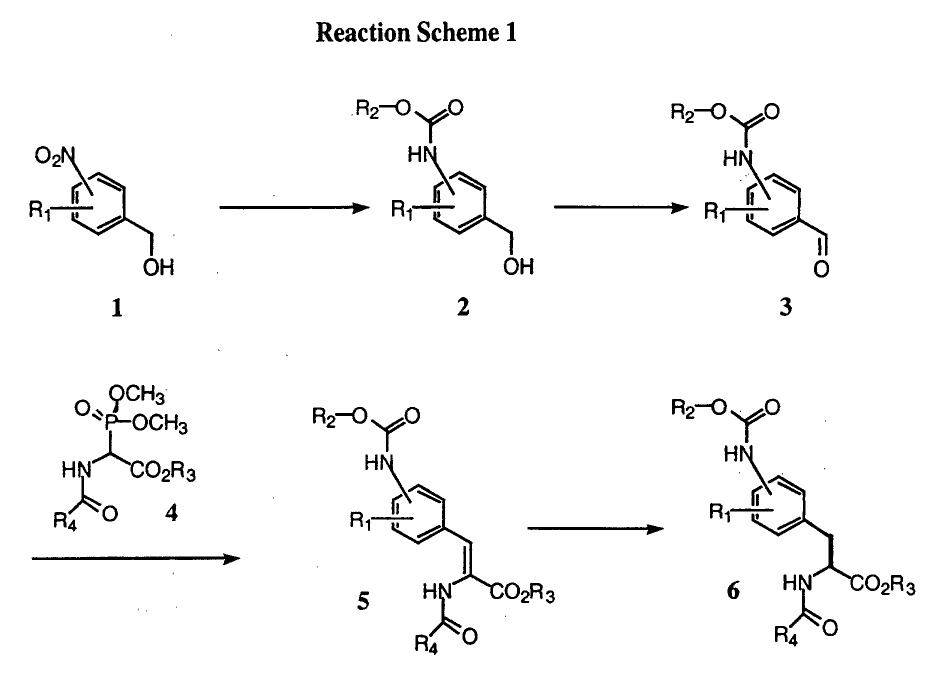

One process for the conversion of compounds of structure 6 into compounds of the invention is shown in Reaction Scheme 2. The protecting group incorporating R₂ can be removed under conditions dependent on the particular choice of R₂ as well as R₃ and R₄. The choice of these groups will be dependent on the particular target compound. A variety of common protecting groups and their use are described in "T. W. Green and P. G. M. Wuts, Protective Groups in Organic Synthesis, 2nd edition, Wiley Interscience, New York, 1991" For example when R₂ is a tert-butyl group and R₃ is lower alkyl and R₄ is either a benzyloxy group or represents a portion of one of the acyl groups of the compounds of the invention, for example substituted lower alkyl or substituted cycloalkyl, treatment with trifluoroacetic acid either neat or in dichloromethane solution in the presence of suitable scavengers, for example, triethylsilane or anisol leads to a compound of formula 7. This compound can be coupled with a carboxylic acid of formula 8 using standard peptide coupling conditions, for example HBTU in the presence of DIPEA in a polar, aprotic solvent such as DMF at a temperature between 0 °C and room temperature to give a compound of formula **9**. In the carboxylic acid of formula **8**, R₅ may represent a substituted alkyl group, a substituted aromatic ring, or a substituted heteroaromatic ring. R₅ may also incorporate suitably protected reactive functionalities to permit final conversion into compounds of the invention. The choice and use of such groups will be apparent to those skilled in the art.

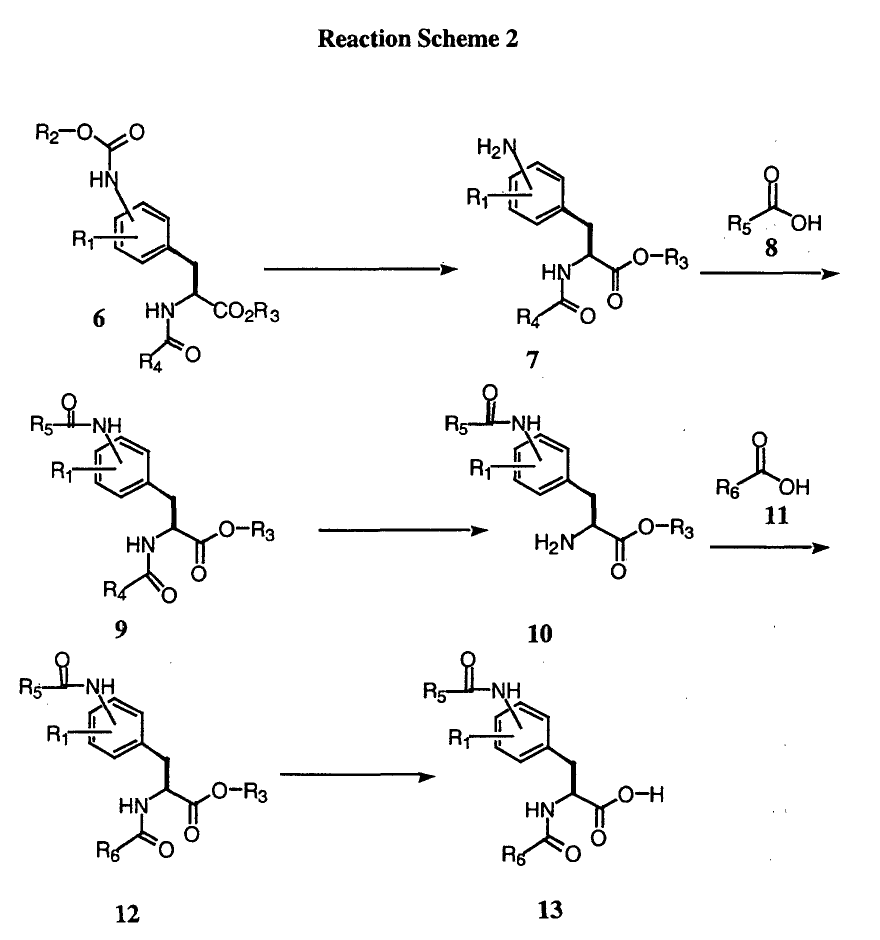

Depending on the choice of R₄ and whether an ester or acid is the final goal of the synthesis, compound 9 may be a compound of the invention or in the case that R₄ is a protecting group, for example, a benzyloxy group, it may be removed under appropriate conditions, for example by catalytic hydrogenation over Pd(C) in a suitable solvent such as a lower alcohol to give a compound of formula 10. This intermediate can be coupled with a carboxylic acid of formula 11 using standard peptide coupling conditions, for example HBTU in the presence of DIPEA in a polar, aprotic solvent such as DMF at a temperature between 0 °C and room temperature to give a compound of formula 12. In the carboxylic acid of formula 11, R₆ may represent a portion of a compound of the invention, for example, a substituted alkyl or substituted cycloalkyl. These compounds are known compounds or can be prepared by known methods. R₆ may also incorporate suitably protected reactive functionalities to permit final conversion into compounds of the invention. The choice and use of such groups will be apparent to those skilled in the art. General methods for the preparation of such compounds are illustrated in Reaction Scheme 2. If the acid 13 is the target compound, conversion of a compound of formula **12** can be effected using standard hydrolysis conditions appropriate for the particular choice of R₃ and any functional groups present as part of R₅ and R₆. In the case where R₃ is lower alkyl, treatment with an alkali metal hydroxide, for example lithium hydroxide in aqueous THF is generally effective.

In reaction Scheme 3, a compound of formula **14** in which R₇ is a lower alkyl group which may serve as a protecting group or a group suitable for use in a prodrug for example methyl, ethyl, tert-butyl or the like or represents a connection to a solid phase resin, for example a Wang resin, is coupled with a carboxylic acid of formula 11 using standard peptide coupling conditions, for example HBTU in the presence of DIPEA in a polar, aprotic solvent such as DMF at a temperature between 0 °C and room temperature to give a compound of formula 15. Reduction of the nitro group of 15 can be effected by catalytic hydrogenation for example using Pd(C) as a catalyst or by treatment with a standard reducing agent, for example SnCl₂. The resulting compound of structure 16 is useful as a key intermediate for several series of compounds. In the instance highlighted in Scheme 3, it can be coupled with an acid of formula 8 using standard peptide coupling conditions, for example HBTU in the presence of DIPEA in a polar, aprotic solvent such as DMF at a temperature between 0 °C and room temperature to give a compound of formula 17. Compound 17 may be a compound of the invention depending on the nature of R₇ or may be converted to a compound of the invention by an appropriate hydrolysis procedure, for example in the case where R₇ is lower alkyl, by hydrolysis by treatment with excess alkali metal hydroxide, such as lithium hydroxide in aqueous alcohol. When R₇ represents a resin suitable for solid phase synthesis, appropriate hydrolysis conditions will depend on the choice of resin. In the case of Wang resin, treatment with trifluoroacetic acid in the presence of appropriate scavengers will lead to an acid of formula 18.

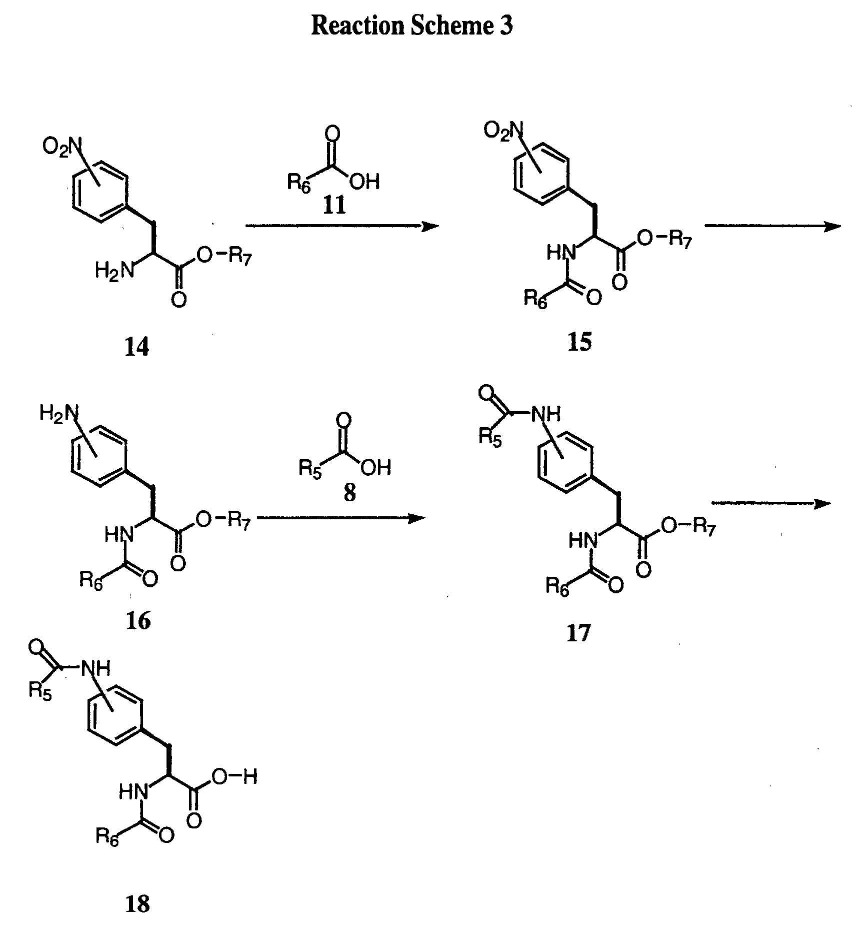

In a method particularly well suited for solid phase synthesis, an N'-Alloc-amino-N^{α}-Fmoc protected phenylalanine derivative of formula 19 can be coupled to a resin suitable for solid phase synthesis, for example, a Wang resin using standard coupling procedures, for example, by forming a mixed anhydride with 2,6-dichlorobenzoyl chloride and carrying out the coupling reaction in a polar, aprotic solvent such as N-methyl pyrrolidinone to give a compound of structure **20** in which R_{7'} represents the resin. The Alloc group may be removed by standard methods, for example by treatment with a reducing agent such as nBu₃SnH in the presence of a catalyst which is a source of Pd^{o}, for instance, Pd(Ph₃P)₂Cl₂ to give an amine derivative of structure **21.** This compound can be coupled with a carboxylic acid of formula 8 using standard peptide coupling conditions, for example HBTU in the presence of DIPEA in a polar, aprotic solvent such as DMF at a temperature between 0 °C and room temperature to give a compound of formula 22. The Fmoc protecting group may be removed from 22 using standard base treatment well known to those practicing peptide chemistry; for example with piperidine in DMF, to afford an amine of formula 23. The resulting compound 23 can be coupled with a carboxylic acid of formula 11 using standard peptide coupling conditions, for example HBTU in the presence of DIPEA in a polar, aprotic solvent such as DMF at a temperature between 0 °C and room temperature to give a compound of formula 24. Finally the compound of structure 24 can be cleaved from the resin under conditions dependent on the particular choice of resin. For example, in the case of a Wang resin, acid treatment with trifluoroacetic acid in dichloromethane in the presence of scavengers as necessary will afford a compound of formula 18.

Depending on the particular synthetic target, the order of removal of the protecting groups from 19 may be altered so that the Fmoc group is first removed, coupling of the resulting amine with an acid of formula 11 is carried out followed by removal of the Alloc group and coupling of the product with an acid of formula 8 and cleavage from the resin. Also the choice of protecting groups can be modified to reflect the reactivities of the resin and the nature of any functional groups incorporated into R₅ and R₆.

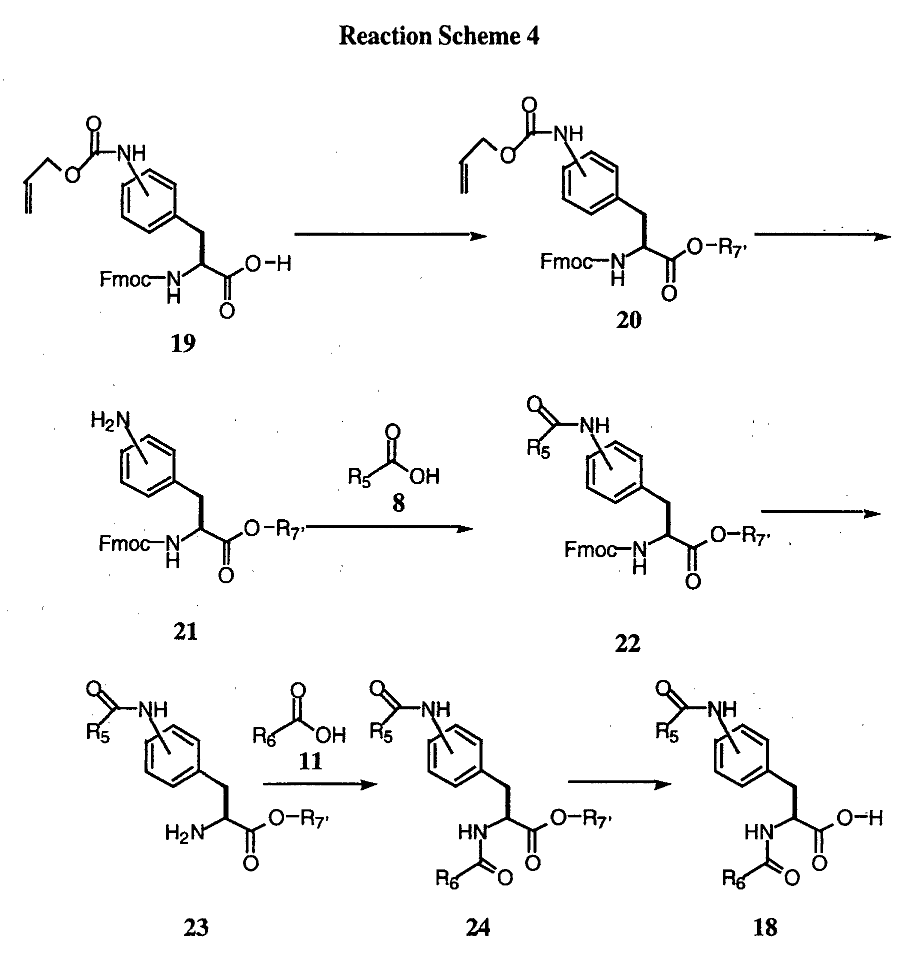

Compounds derived from 3- or 4-(alkylamino)phenylalanine derivatives can be prepared as outlined in Reaction Scheme 5. A compound of formula 16 or **7** may be treated with diazomethane in a suitable solvent, for example, ethyl ether to give products of formulas **25** and **26** respectively in which R₈ is methyl. Alternatively, the compound of structure **16** or **7** may be treated with an lower alkyl aldehyde or ketone, for example acetone, to give an intermediate Schiff's base which is in turn subjected to catalytic hydrogenation or reduction with sodium cyanoborohydride in the presence of an organic acid, for example acetic acid to give a compound of formula **25** or **26** in which R₈ is lower alkyl other than methyl. Conversion of compounds **25** or **26** to prodrug esters **27** or **28** or to the corresponding acids **29** or **30** respectively can be carried out as described above in Reaction Schemes 2 and 3.

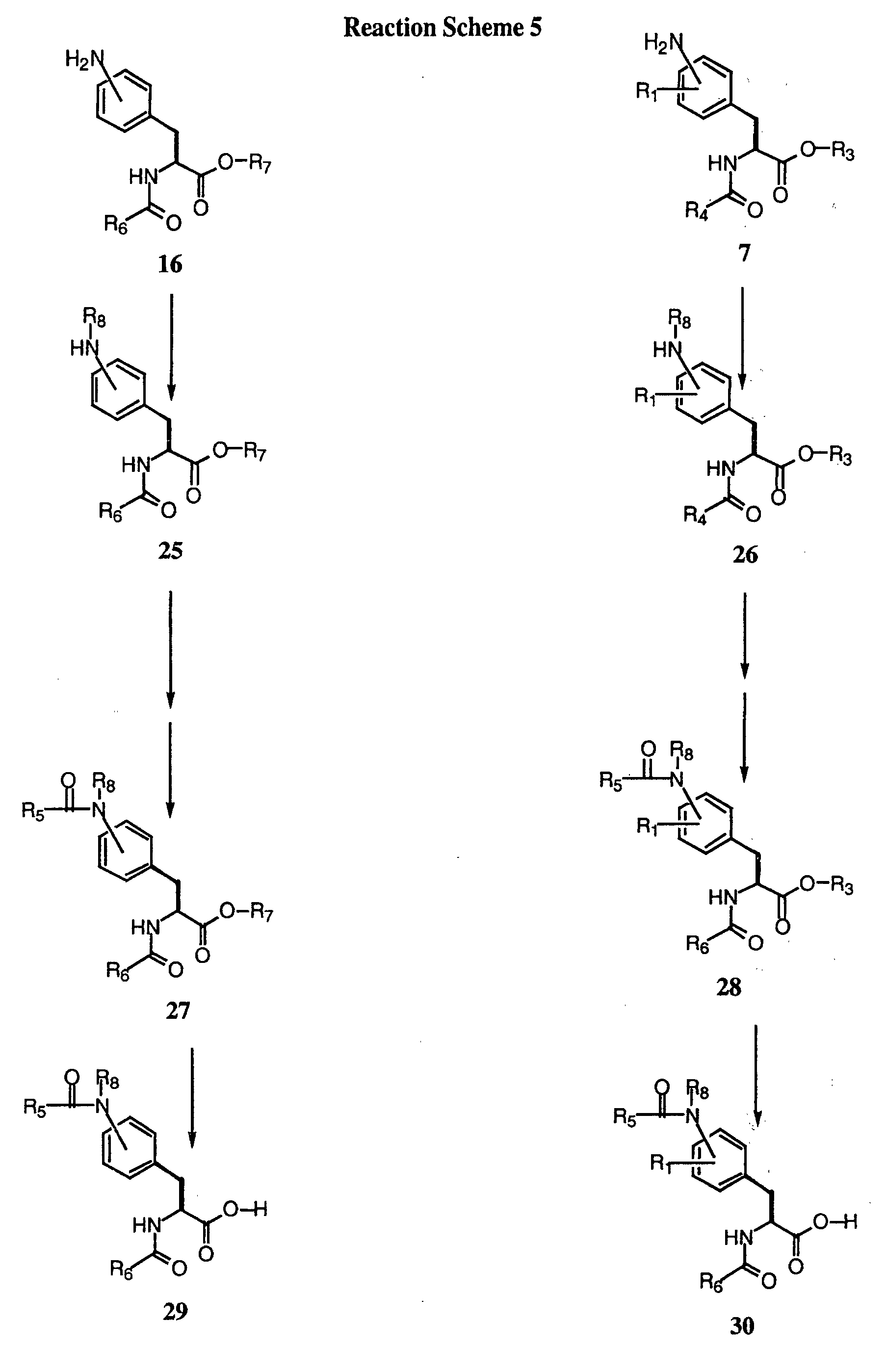

For the preparation of compounds derived from 3- or 4-aminomethylphenylalanine, the procedure shown in Reaction Scheme 7 may be employed. A 3- or 4-hydroxymethyl benzoate of formula **36** in which R₁₀ is lower alkyl, which are known compounds, or can be prepared by known methods, is treated with a silylating agent in which R₁₁-R₁₃ are lower alkyl or phenyl, for example tert-butyldimethylsilyl chloride in an inert solvent, for example dimethylformamide in the presence of imidazole at about 0 °C to give a silyl protected compound of formula **37**. Reduction of **37** may be carried out using a variety of suitable reducing agents, for example, lithium aluminum hydride in an inert solvent such as ether or tetrahydrofuran at a temperature of about 0 °C followed by an aqueous workup to give an intermediate alcohol which can be oxidized by any of several oxidizing agents suitable for oxidizing benzyl alcohols to the corresponding aldehydes, for example activated manganese dioxide, to give an aldehyde of formula **38**. Monosilyl protected diols are alternatively available from 3- or 4-hydroxymethylbenzylalcohols by monosilylation and separation of the side products. Alternatively, an ester of formula **37** may be reduced directly to an aldehyde of formula **38** using diisobutylaluminum hydride at low temperature, for example at -78 °C.

Reaction of **38** to give a dehydroamino acid of formula **39** can be effected by treatment with a Wittig reagent of formula **4** in which R₃ is lower alkyl and R₄ is an alkoxy group, for example benzyloxy- or tert-butoxy- or represents a portion of one of the acyl groups of the compounds of the invention, for example substituted lower alkyl or substituted cycloalkyl. For example treatment of **38** with (±)-N-(benzyloxycarbonyl)-α-phosphonoglycine trimethyl ester in the presence of a suitable base for example tetramethyl guanidine leads directly to a dehydroamino acid of formula **39**, R₃ = methyl and R₄ = benzyloxy. Enantioselective reduction of **39** to the L-amino acid **40** can be effected by use of one of a number of reducing agents suitable for the purpose, for example, the recently described ethyl-DuPHOS rhodium reagent. It will be readily apparent to those skilled in the art that the optimal procedure for the further conversion of **40** into compounds of the invention will depend on the choices of R₄ and R₃. For the case wherein R₃ is lower alkyl and R₄ is benzyloxy, conversion to an amine of formula **41** can be conveniently effected by catalytic transfer hydrogenation of **40** over Pd(C) in a suitable solvent, for example, methanol in the presence of ammonium formate as the reducing agent. Acylation of **41** with a carboxylic acid of formula 11 can be carried as described above in Reaction Scheme 2 to give a compound of formula **42**. Conditions for removal of the silyl protecting group will depend on the particular choice of R₁₁-R₁₃. In the case of R₁₁, R₁₂ = methyl and R₁₃ = tertbutyl, this group is readily removed by treatment with a strong acid, for example hydrochloric acid in an appropriate solvent for the choice of R₃, for example where R₃ is methyl, methanol.

The resulting benzylic alcohol of formula **43** can be converted to an amine of formula **45** using procedures well established for similar transformations. For example, the alcohol of formula **43** can be converted to a leaving group, for example a mesylate by treatment with methane sulfonyl chloride in the presence of a proton acceptor, for example pyridine, followed by displacement with an alkali metal azide, for example sodium azide in a polar aprotic solvent such as dimethylformamide. Alternatively, the transformation from **43** to an azide of formula 44 can be carried out directly by treatment with diphenyl phosphorazidate as described in: Thompson, A. S.; Humphrey, G R.; DeMarco, A. M.; Mathre, D. J.; Grabowski, E. J. J. J. *Org. Chem.* **1993,** *58,* 5886-5888. Reduction of the azide 44 to an amine of formula **45** can be carried out by a number of means suitable for the conversion of azides to amines, for example by treatment with a phosphine, for example triphenyl phosphine in an inert solvent such as dichloromethane or THF followed by an aqueous workup or by catalytic hydrogenation over an appropriate catalyst, for example Pd(C) in a solvent suitable for catalytic hydrogenations such as a lower alkanol or tetrahydrofuran. The resulting amine of formula **45** can be converted into the corresponding compounds of the invention using the procedures applicable to free amines described in the other reaction schemes. For example, coupling of **45** with a carboxylic acid of formula 8 under the conditions described in Reaction Scheme 2 leads to an amide of formula **46** which may be further converted to an acid of formula **47** if desired by base catalyzed hydrolysis as described in Reaction Scheme 2..

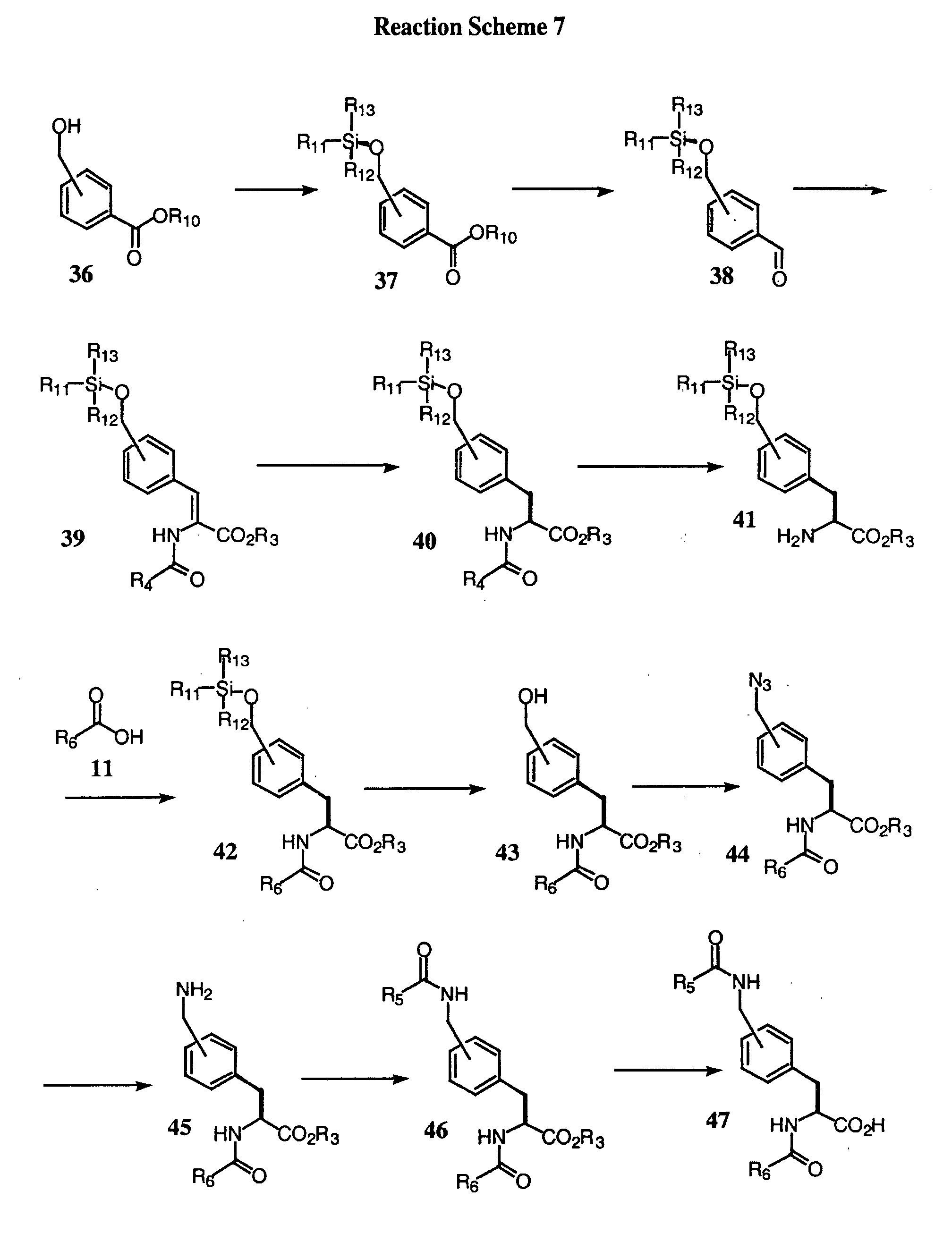

For the synthesis of compounds of the invention in which R₁ is halogen, preferably chloro, the appropriate halogen atom can be incorpoarted into the starting material or inserted at various points during the course of the synthesis depending on the nature of the additional functionality in the molecule. A chlorine atom can be incorporated into the compound of structure 1, shown in scheme 1 and carried through to the compounds of the invention by avoiding reagents which would be expected to react with a halogen atom For example a compound of formula **6** in which R₁ is hydrogen can be treated with a mild chlorinating agent, for example, N-chlorosuccinimide in the presence of a proton acceptor, for example, sodium acetate to give the corresponding compound of formula **6** in which R₁ is chloro. In the case where **6** is derived from 3-amino-L-phenylalanine, a mixture of regioisomers may ensue which may be separated at a convenient point in the overall synthesis. Other intermediates described in the above schemes may be more suitable starting materials for halogenation for a particular target molecule. The particular merits of individual candidate starting materials will be apparent to those skilled in the art..

For the synthesis of imidazolidinones of formula **67** shown in reaction scheme 11, an aminophenylalanine derivative of structure **16** in which R₆ and R₇ are as previously defined may be employed. Compound **16** can be readily obtained through the synthesis described in reaction scheme 3. This compound can be coupled with a N-protected α-amino acid of formula **63**, in which R₂₂ can be a lower alkyl or an aryl group, R₂₃ can be a natural or unnatural D- or L-α-amino acid side chain or R₂₂ and R₂₃ together can form a ring, for example a proline or pipicolinic acid ring and R₂₄ may be a standard amine protecting group suitable for the particular selection of R₆, R₇, R₂₂, and R₂₃ for example tert-butoxycarbonyl. The coupling reaction can be effected using standard peptide coupling conditions, for example HBTU in the presence of DIPEA in a polar, aprotic solvent such as DMF at a temperature between 0 °C and room temperature to give a compound of formula **64.** Depending on the nature of protecting group R₂₄, an appropriate deprotection method is employed to give a compound of formula **65.** In the event that the protecting group R₂₄ is a Boc group, the deprotection can be carried out by the reaction of **64** with HCl in dioxane at room temperature. Reaction of compound **65** with an aldehyde of formula **60,** in which the R₂₁ is as defined above, in the presence of a water scavenger such as 4Å molecular sieves at 60 to 80 °C in an appropriate solvent, for example THF, provides a compound of formula **66**. Compound **66** may be a compound of the invention depending on the nature of R₇ or may be converted to a compound of the invention by an appropriate hydrolysis procedure, for example in the case where R₇ is lower alkyl, by hydrolysis by treatment with an alkali metal hydroxide, such as sodium hydroxide in aqueous alcohol to give a carboxylic acid of formula 67.

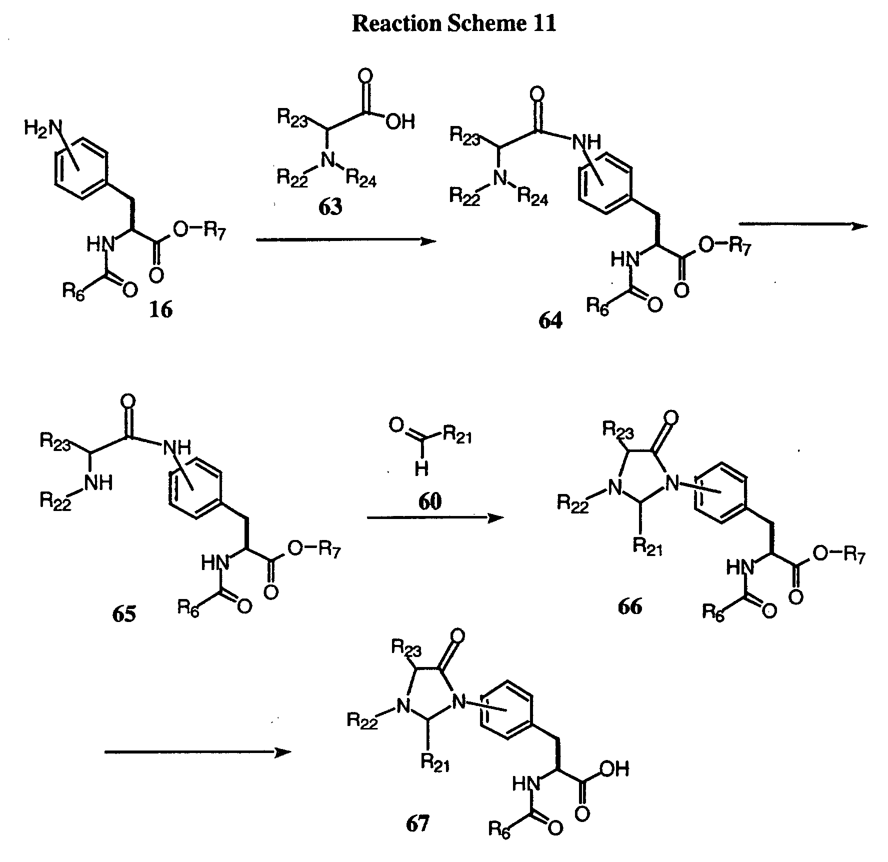

For the synthesis of imidazolidinones of formula **68** described in reaction scheme 12, an aminophenylalanine derivative of structure **16** in which R₆ and R₇ are as previously defined is employed. Compound **16** can be readily obtained through the synthesis described in reaction scheme 3 in the case of R₇ is lower alkyl. This compound can be coupled with a N-protected α-amino acid of formula **69,** in which R₂₅ can be a natural or unnatural, D- or L-α-amino acid side chain and R₂₆ is a nitrogen protecting group of the type conventionally used in peptide chemistry, for example, a Fmoc group, using standard peptide coupling conditions, for example HBTU in the presence of DIPEA in a polar, aprotic solvent such as DMF at a temperature between 0 °C and room temperature to give a compound of formula **70.** Depending on the nature of protecting group R₂₆, an appropriate deprotection method is employed to give compound of formula **71.** In the case of the protecting group R₂₆ is Fmoc group, it may be removed from **70** using standard base treatment well known to those practicing peptide chemistry, for example with piperidine in DMF, to afford an amine of formula **71.** The compound **71** can then react with an aldehyde **60,** in which R₂₁ is as previously defined, in the presence of a water scavenger such as 4Å molecular sieves in an appropriate solvent such as dichloromethane or THF at 25-80 °C (bath termperature) to give an imine of formula **72.** The imine **72** may then be treated with an acylating agent such as the acyl chloride of formula **74** in which R₂₇ can be an alkyl or aryl group in the presence of a base such DIPEA or DBU in an appropriate solvent such as dichloromethane or THF at 25-80 °C (bath temperature) to give an acyl imidazolidinone of formula **73**. Other acylating groups may be employed for example, acid anhydrides, and where appropriate, **74** may bear protected substituents which can later be removed at the neccessary point in the synthesis. Compound **73** may be a compound of the invention, or depending on the nature of R₇ may be converted to a compound of the invention by an appropriate hydrolysis procedure, for example in the case where R₇ is lower alkyl, by hydrolysis by treatment with an alkali metal hydroxide, for example sodium hydroxide in aqueous alcohol to give, after acidification, a carboxylic acid of formula **68**. The sequence may be initiated with related anilines, for example a compound of formula **7** in which R₁ is lower alkyl or halogen to give the corresponding 3-acyl imidazolidinones.

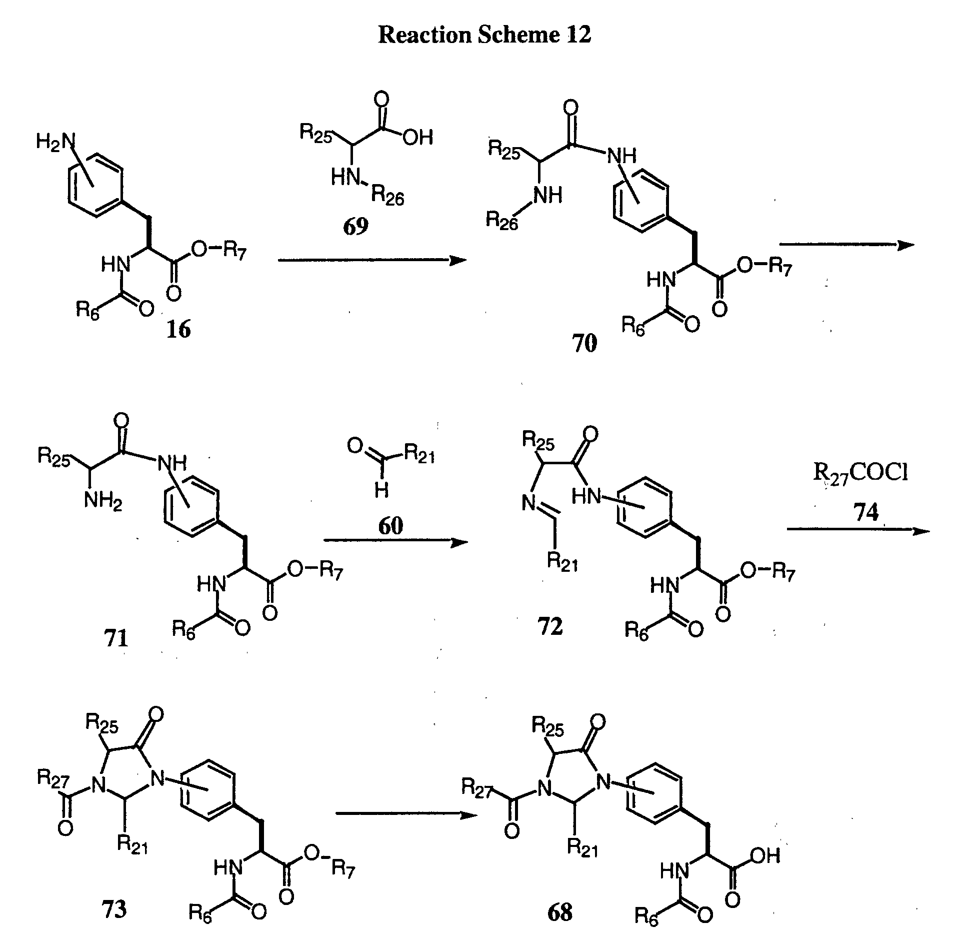

The acids of formula **11** are known compounds or can be prepared using standard methodologies. For the preparation of substituted alkyl- or cycloalkylcarboxylic acids, alkylation reactions can be employed using an alkali metal dianion of the acid or monoanion of the corresponding ester. For example, a cycloalkyl carboxylic acid ester of formula **75** can be treated with a strong base, for example, lithium diisopropylamide in an inert solvent, for example THF followed by addition of group R₂₈-Lv wherein R₂₈ represents a desired side chain, such as a substituted benzyl, lower alkyl, lower alkoxy alkyl, azidolower alkyl and the like and Lv represents a leaving group such as a bromide, iodide, mesylate or similar group known to participate in ester enolate alkylation reactions. The product ester **76** may be hydrolyzed to the acid **77** using alkali metal hydroxide in a suitable solvent, for example aqueous alcohol. Depending on the nature of R₂₈ and the eventual target, the compound **77** may be a coupled to an amine such as compound **23** and converted to the target directly or R₂₈ may be subject to further manipulation at a suitable point in the synthesis. For example, if R₂₈ is an azido lower alkyl moiety, the azide may be reduced using for example a trialkyl phosphine reagent followed by functionalization of the product amine by alkylation, acylation, sulfonylation and related procedures well known to those skilled in the art. If R₂₈ incorparates a leaving group, for example, a terminal bromine atom, this group may be displaced by an appropriate nucleophile, for example, sodium methyl mercaptide to give in this case, a thioether which may be the desired product or can be itself further manipulated, for example by oxidation to a sulfoxide or sulfone using standard reaction conditions. Other nucleophiles which may be employed to produce intermediates leading to compounds of this invention include: sodium cyanide, sodium methoxide, sodium azide, morpholine and others. When R₂₈ incorporates a ketal group, this group may be hydrolzyed at a convenient point in the synthesis to provide a keto group. This group in turn may be further manipulated, for example by reduction to an alcohol or conversion to derivative such as an oxime.

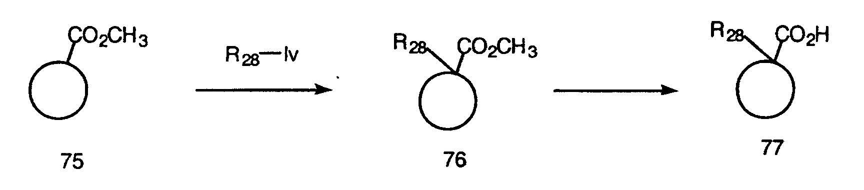

General Melting points were taken on a Thomas-Hoover apparatus and are uncorrected. Optical rotations were determined with a Perkin-Elmer model 241 polarimeter. ¹H-NMR spectra were recorded with Varian XL-200 and Unityplus 400 MHz spectrometers, using tetramethylsilane (TMS) as internal standard. Electron impact (EI, 70 ev) and fast atom bombardment (FAB) mass spectra were taken on VG Autospec or VG 70E-HF mass spectrometers. Silica gel used for column chromatography was Mallinkrodt SiliCar 230-400 mesh silica gel for flash chromatography; columns were run under a 0-5 psi head of nitrogen to assist flow. Thin layer chromatograms were run on glass thin layer plates coated with silica gel as supplied by E. Merck (E. Merck # 1.05719) and were visualized by viewing under 254 nm UV light in a view box, by exposure to I₂ vapor, or by spaying with either phosphomolybdic acid (PMA) in aqueous ethanol, or after exposure to Cl₂, with a 4,4'-tetramethyldiaminodiphenylmethane reagent prepared according to E. Von Arx, M. Faupel and M Brugger, *J. Chromatography,* **1976,** *120,* 224-228.

Reversed phase high pressure liquid chromatography (RP-HPLC)was carried out using either a Waters Delta Prep 4000 employing a 3 x 30 cm, Waters Delta Pak 15 µM C-18 column at a flow of 40 mL/min employing a gradient of acetonitrile:water (each containing 0.75% TFA) typically from 5 tp 95% acetonitrile over 35-40 min or a Rainin HPLC employing a 41.4 mm x 30 cm, 8 µM, Dynamax™ C-18 column at a flow of 49 mL/min and a similar gradient of acetonitrile:water as noted above.

Dichloromethane (CH₂Cl₂), 2-propanol, DMF, THF, toluene, hexane, ether, and methanol, were Fisher reagent grade and were used without additional purification except as noted, acetonitrile was Fisher hplc grade and was used as is.

### Definitions:

THF is tetrahydrofuran,
DMF is N,N-dimethylformamide,
HOBT is 1-hydroxybenzotriazole,
BOP is [(benzotriazole-1-yl)oxy]tris-(dimethylamino)phosphonium hexafluorophosphate,
HATU is O-(7-azabenzotriazol-1-yl)-1,1,3,3-tetramethyluronium hexafluorophosphate
HBTU is O-benzotriazole-N,N,N',N',-tetramethyluronium hexafluorophosphate,
DIPEA is diisopropylethylamine,
DMAP is 4-(N,N-dimethylamino)pyridine
DPPA is diphenylphosphoryl azide
DBU is 1,8-diazabicyclo[5.4.0]undec-7-ene
NaH is sodium hydride
brine is saturated aqueous sodium chloride solution
TLC is thin layer chromatography
LDA is lithium diisopropylamide
BOP-Cl is bis(2-oxo-3-oxazolidinyl)phosphinic chloride
NMP is N-methyl pyrrolidinone

### Examples

### Example 1. Synthesis of 1-Benzylcyclopentane carboxylic acid ethyl ester.

A solution of diisopropylamine (1.0 mL, 7.7 mmol) in 20 mL of dry THF was cooled to -78 °C in a dry ice-acetone bath under argon. n-Butyl lithium in hexanes (2.5 M, 3.6 mL, 7.7 mmol) was added all at once and the mixture was stirred for 0.5 hour and transferred to a precooled (-30 °C) solution of 1.0 g (7.0 mmol) of ethyl cyclopentane carboxylate in 10 mL of THF. After a further 45 min, benzyl bromide (0.92 mL, 7.7 mmol) was added and the mixture was allowed to warm to room temperature over night. The resulting mixture was concentrated, and the residue was taken up in 70 mL of ether, washed with water, 1 N HCl, water, saturated brine and was dried (MgSO₄). The residue obtained after filtration and evaporation was purified by flash chromatography over 100 g of silica gel, eluting with 3% ethyl acetate-hexane to give 1-benzylcyclopentane carboxylic acid ethyl ester (0.80 g, 45%) as a colorless oil.

### Example 2. Synthesis of 1-benzylcyclopentane carboxylic acid.

A solution of 1-benzylcyclopentane carboxylic acid ethyl ester (0.40 g, 1.7 mmol) in 10 mL of THF and 5 mL of methanol was treated with a solution of 200 mg of lithium hydroxide hydrate in 5 mL of water and the mixture was stirred for 3 days at room temperature and at 40 °C for 3 days. The mixture was diluted with water, washed with ether and acidified with excess 6 N HCl. The aqueous layer was extracted with ether and the combined extracts were washed with water and brine and were dried (MgSO₄). Evaporation gave 0.34 g (96%) of 1-benzylcyclopentane carboxylic acid as a thick yellow oil.

### Example 3. Synthesis of 1-[(4-methoxyphenyl)methyl]cyclopentane carboxylic acid.

A solution of diisopropylamine (58 mL, 0.44 mol) in THF 400 mL was cooled to below 0 °C and n-butyl lithium in hexane (170 mL, 2.5 N, 0.43 mol) was added dropwise maintaining the temperature below 0 °C. Upon completion of the addition, ethyl cyclopentylcarboxylate (55 g, 0.39 mol) in THF (175 mL) was added dropwise maintaining the internal temperature between -60 and -70°C. Upon completion of the addition, the internal temperature was allowed to rise to -40 °C and was held there for 20 min and lowered back to -70 °C. A solution of 4-methoxybenzyl bromide (75 g, 0.48 mol) in 175 mL of THF was added dropwise and the mixture was allowed to warm to room temperature overnight. A solution of 20% ammonium chloride in water (225 mL) was added followed by 450 mL of ethyl acetate, the layers were separated, the aqueous layer was extracted with ethyl acetate (450 mL) and the combined organic layers were washed with saturated brine (2 x 450 mL) and were dried (MgSO₄). The crude product was chromatographed on silica gel, eluting with 1-5% ether in hexane to give 1-[(4-methoxyphenyl)methyl)cyclopentanecarboxylic acid ethyl ester (80.8 g, 79%) as an oil.

The material obtained above was dissolved in methanol (620 mL) and 2 N sodium hydroxide (350 mL) and the mixture was heated to reflux overnight. The mixture was allowed to cool and was concentrated. The yellow, basic residue was washed with ether (2 x 500 mL) and was acidified with excess 6 N hydrochloric acid to pH <2. This solution was extracted with dichloromethane (2 x 500 mL), the extracts were combined, dried (MgSO₄) and evaporated to a white solid (69.6 g, 89%), mp 63.5-64.5 °C.

### Example 4. Synthesis of 1-(2-azidoethyl)cyclopentane carboxylic acid.

To an ice cold solution of diisopropylamine (56 mL, 0.396 mol) in THF 85 (mL) was added n-butyl lithium in hexane solution (240 mL, 1.6 M, 0.393 mol) over 20 min. The mixture was stirred at 0 °C for 30 min, cooled to a bath temperature of -65 °C and ethyl cyclopentane carboxylate (37.4 g, 0.263 mol) in THF (50 mL) was added over 20 min. After 1 hr, a solution of 1,2-dibromoethane (47 mL, 0.545 mol) in THF (50 mL) was added, the mixture was held at -65 °C for 3 hr and allowed to warm to room temperature overnight. The reaction was quenched by addition of saturated ammonium chloride solution (200 mL), the layers were separated and the aqueous layer was extracted with ethyl acetate (100 mL). The combined extracts were washed with 1:1 brine:water (250 mL) and were dried (Na₂SO₄). The solution was filtered and concentrated, diluted with toluene (100 mL) and concentrated. The dilution and concentration was repeated twice to give ethyl 1-(2-bromoethyl)cyclopentane carboxylate (52.5 g).

A solution of the above bromide (52.5 g, 0.211 mol) and sodium azide (54 g, 0.831 mol) in DMF (200 mL) was stirred at 50 °C for 5 hr under a nitrogen atmosphere and was filtered. The filtrate was concentrated to near dryness, diluted with ethyl acetate (500 mL), filtered and concentrated to give crude ethyl 1-(2-azidoethyl)cyclopentane carboxylate (40.9 g) as a brown oil. This material was combined with product from a previous run (total 63.5 g) and was purified by chromatography over 250 g of silica gel, eluting with 5% ethyl acetate in hexane to give 50.3 g of product as a light brown oil.

The oil from above (50.3 g, 0.238 mol) was dissolved in THF (750 mL) and methanol (375 mL) and a solution of LiOH hydrate (15 g, 0.357 mol) in water (300 mL) was added. The resulting solution was stirred at 40 °C overnight and concentrated. The residue was dissolved in 2 L of water containing 40 mL of 1N NaOH and was washed with hexane (1 L). The aqueous layer was acidified with 1 N HCl (375 mL) and was extracted with ether (2 x 1 L). The combined extracts were dried (Na₂SO₄) and concentrated to give 1-(2-azidoethyl)cyclopentane carboxylic acid (37.5 g) as an amber liquid.

### Example 5. Synthesis of 4-(chloromethyl)-N-methylbenzamide.

To a solution of 4-chloromethyl benzoic acid (17.05 g, 100 mmol) in toluene (dried over molecular sieves 4A) was added thionyl chloride (11 mL, 150 mmol). The mixture was heated to 80 °C and stirred overnight and then 3 h at 105 °C. The reaction mixture was cooled to room temperature and excess thionyl chloride and toluene were removed under vacuum. The resulting oily residue was azeotroped with toluene (50 mL), then dried under high vacuum for 45 min to give the crude acid chloride.

To the crude acid chloride in dichloromethane (200 mL, dried over molecular sieves 4A) was added methylamine hydrochloride (7.5 g, 110 mmol) at -10 °C in one portion. To the mixture diisopropylamine (35 mL, 201 mmol) was added dropwise over 15 min while maintaining the temperature of the reaction mixture below 2 °C. After addition, the suspension was allowed to warm to room temperature and stirred for 30 min. Then, the reaction mixture was diluted with water (125 mL) and the layers were separated. The aqueous layer was extracted with dichloromethane (2 X 60 mL) and the combined extracts were washed successively with water (150 mL) and brine solution (150 mL). After drying over anhydrous magnesium sulfate, the solution was concentrated to 50 mL. The precipitated white solid was collected by nitration and washed with dichloromethane and hexane to obtain 4-(chloromethyl)-N-methylbenzamide (12.02 g) as a white solid. A second crop material (3.05 g) was obtained from mother liquor by concetration and dilution with hexane to give a total of 15.07 g, 82%, mp 138-139.5 °C.

### Example 6. Synthesis of 4-(1-methyltetrazol-5-yl)benzyl chloride.

To a suspension of 4-(chloromethyl)-N-methylbenzamide (12 g, 65.3 mmol) in toluene (dried over molecular sieves 4A) was added thionyl chloride (7.15 mL, 98 mmol). The mixture was heated to reflux (-90 °C) and the resulting light yellow solution was stirred at reflux overnight. The reaction mixture was cooled to room temperature and excess thionyl chloride and toluene were removed under vacuum. The resulting oily residue was azeotroped with toluene (50 mL), then dried under high vacuum for 1.5 h to give the crude imidoyl chloride.

To a suspension of sodium azide (5.1 g, 78.5 mmol) in acetonitrile (62 mL) was added chlorotrimethylsilane (10.5 mL, 82.5 mmol) and the mixture was stirred for 1.5 h at room temperature. After cooling to 0 °C, a solution of the crude imidoyl chloride prepared above in acetonitrile (20 mL) was added. This mixture was stirred for 1.5 h at 0 °C, then allowed to warm to room temperature and stirred for 18 h. TLC analysis indicated the presence of traces of starting amide. Then, the reaction mixture was diluted with water (70 mL) and ethyl acetate (70 mL) and was poured into a mixture of saturated ammonium chloride (70 mL) and ethyl acetate (70 mL). The two layers were separated and the aqueous layer was extracted with ethyl acetate (2 x 50 mL). The combined extracts were washed successively with water (90 mL) and brine solution (90 mL). After drying over anhydrous magnesium sulfate, the solution was concentrated to 40 mL to afford a white precipitate. The solid was collected by filtration washing with hexane. Attempted purification by crystallization in various solvents were unsuccessful. Thus, it was purified by preparative HPLC using ethyl acetate and hexane in 1:2 ratio as eluent to afford 4-(1-methyltetrazol-5-yl)benzyl chloride (11.35 g, 83%) as a white solid; mp 90-92 °C.

### Example 7. Synthesis of 1-[[4-(1-methyltetrazol-5-yl)phenyl)methyl]cyclobutane carboxylic acid.

A solution of diisopropylamine (1.05 mL, 7.5 mmol) in THF (5 mL) was colled to -10 °C and a solution of n-butyl lithium (2.9 mL, 7.25 mmol) in hexanes was added dropwise while maintaining the temperature below 0 °C. After addition, the solution was stirred for 30 min at 0 °C. The solution was cooled to -70 °C and a solution of methyl cyclobutane carboxylate (0.57 g, 5 mmol) in THF (2 mL) was added dropwise maintaining the internal temperature between -60 to - 70 °C. After addition, the reaction mixture was stirred for 30 min at -50 to -60 °C. Then, a solution of 4-(1-methyltetrazol-5-yl)benzyl chloride (0.94 g, 4.5 mmol) in THF (5 mL) was added dropwise and the reaction mixture was stirred for 1 h at -60 to -70 °C. Then, it was allowed to warm to room temperature and stirred overnight at which point TLC analysis indicated the absence of 4-(1-methyltetrazol-5-yl)benzyl chloride (Note: the product and 4-(1-methyltetrazol-5-yl)benzyl chloride has the same Rf value, however they were differentiated by spraying with PMA). The mixture was poured into a mixture of water (70 mL) and ethyl acetate (70 mL). An emulsion formed and was filtered through celite. The resulting two layers were separated and the aqueous layer was extracted with ethyl acetate (2 x 50 mL). The combined extracts were washed with saturated brine and dried over anhydrous magnesium sulfate. After filtration, the solution was concentrated under vacuum and the residue was purified by silica gel chromatography eluting with 1:2 ethyl acetate:hexane to give methyl 1-[[4-(1-methyltetrazol-5-yl)phenyl]methyl]cyclobutane carboxylate (0.42 g, 32%) as a syrup. HR MS: obs. mass, 301.1668. Calcd mass, 301.1664.

Example 8 - 14. Using the procedure described in example 7, the cyclopentane carboxylate derivatives shown below were prepared.

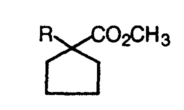

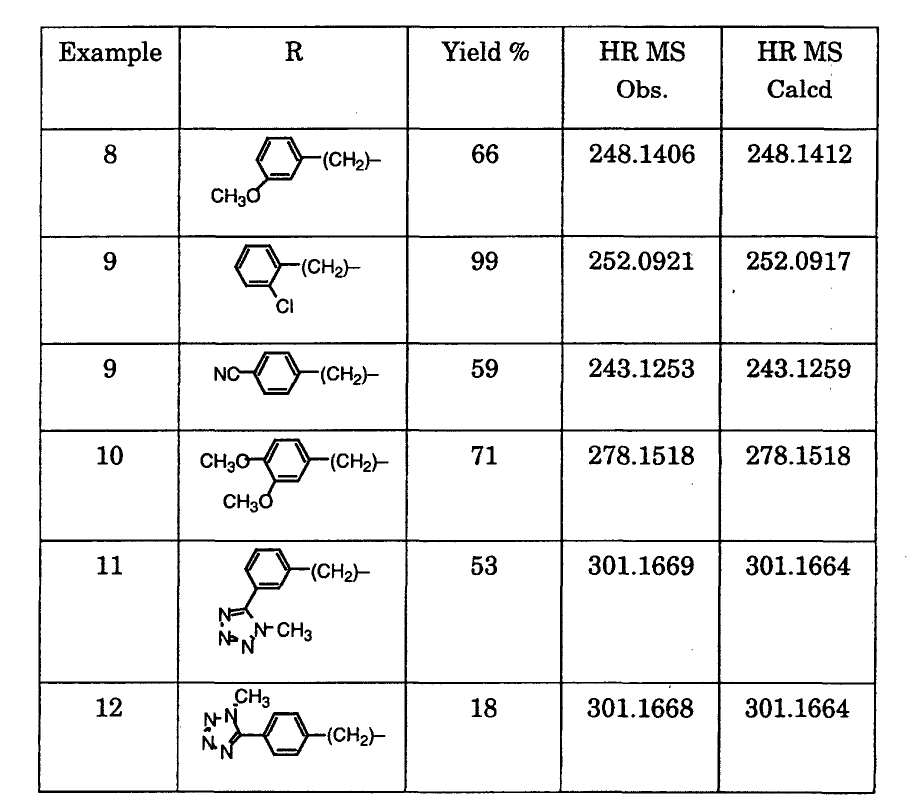

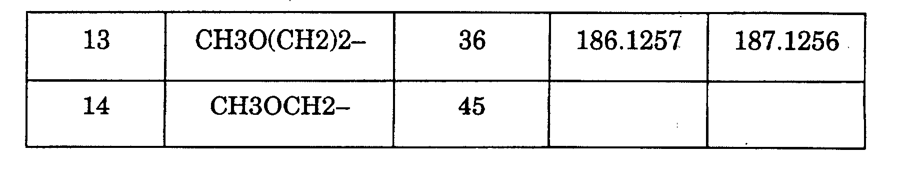

### Example 15. Synthesis of 1-[[4-(1-methyl-5-tetrazolyl)phenyl]methyl]cyclobutane carboxylic acid.

To a solution of methyl 1-[[4-(1-methyltetrazol-5-yl)phenyl]methyl]cyclobutane. carboxylate (0.33 g, 1.15 mmol) in a mixture of THF (7 mL) and methanol (7 mL) was added 1 N sodium hydroxide (7 mL). The mixture was heated to 55 °C and stirred for 4 h at which point TLC analysis indicated the absence of starting material. After cooling to room temperature, the solvent was removed under vacuum and the residue was diluted with water and extracted with ethyl acetate to remove any neutral impurities. Then, the aqueous layer was neutralized with 1 N hydrochloric acid and the product was extracted with ethyl acetate (2 x 50 mL). The combined extracts were washed with saturated brine and dried over sodium sulfate. After filtration, the solution was concentrated under vacuum and the residue was dried under high vacuum to afford 1-[[4-(1-methyl-5-tetrazolyl)phenyl]methyl]cyclobutane carboxylic acid (180 mg, 57%) as a light yellow syrup.

Examples 16 - 23. Using the procedure described in example 15, the following cyclopentane carboxylic acids were prepared from the corresponding methyl esters:

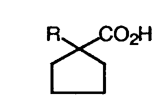

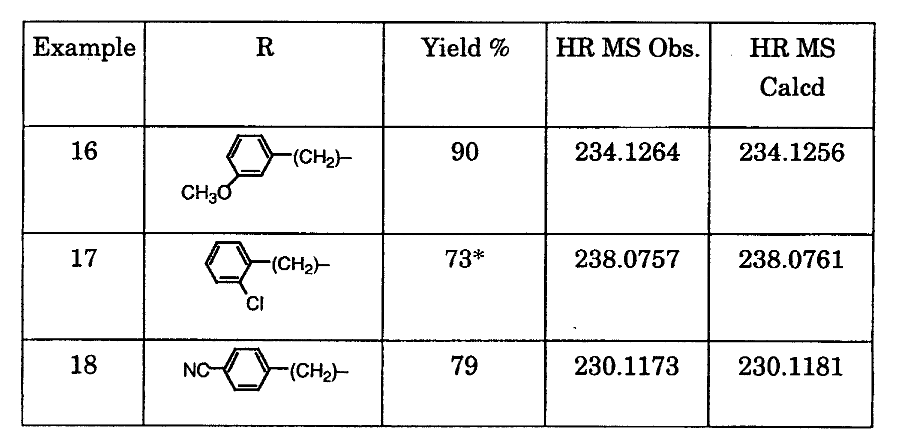

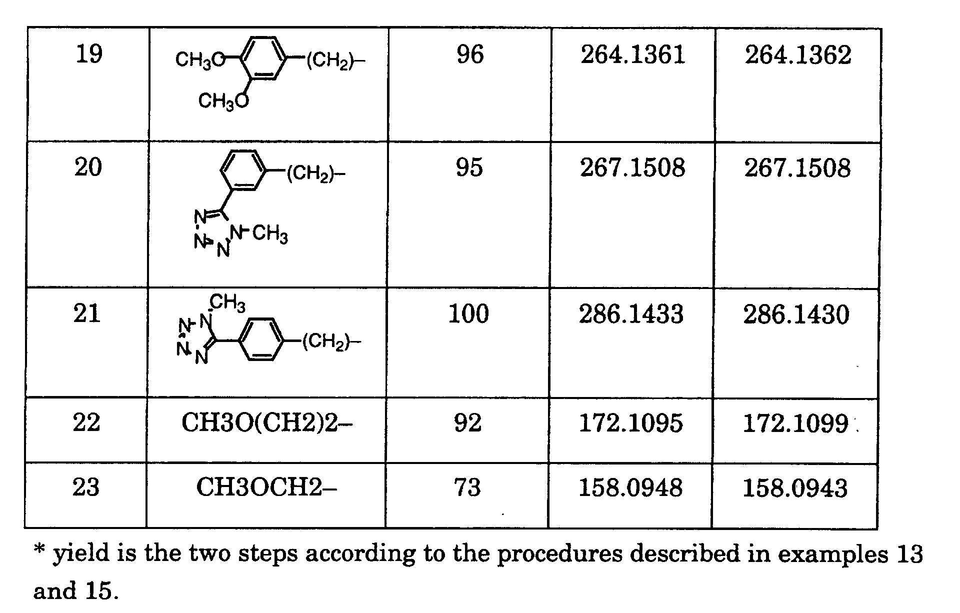

### Example 24. Synthesis of 1-[(4-methoxyphenyl)methyl]cyclohexane carboxylic acid

Using the procedures described in examples 7 and 15, starting with 4-methoxybenzyl chloride, 1-[(4-methoxyphenyl)methyl]cyclohexane, carboxylic acid was prepared in 23% overall yield. HRMS: obs. mass, 248.1426. Calcd. mass, 248.1412 (M+).

### Example 25. Synthesis of 1-[3-(1-methyl-5-tetrazolyl)phenyl)methyl]cyclohexane carboxylic acid

Using the procedures described in examples 7 and 15, starting with 1-[3-(1-methyl-5-tetrazolyl)benzyl chloride, 1-[3-(1-methyl-5-tetrazolyl)phenyl]methyl]cyclohexane carboxylic acid was prepared in 77% overall yield. HRMS: obs. mass, 301.1667. Calcd. mass, 301.1664 (M+H).

### Example 26. Synthesis of N-[(1-phenylcyclopentyl)carbonyl]-4-amino-L-phenylalanine methyl ester

To a solution of 4-nitrophenylalanine hydrochloride methyl ester (3.90 g, 15 mmol) and 1-phenylcyclopentane carboxylic acid (3.4 g, 18 mmol) in 30 mL of DMF was added HBTU (6.8 g, 18 mmol) and diisopropylethyl amine (6.4 mL, 30 mmol) at room temperature. The mixture was then stirred at this temperature for 8 hr. The reaction was then diluted with 250 mL of ethyl acetate and was washed with 0.5 N HCl (40 mL), saturated NaHCO3 (2 x 40 mL) and saturated brine (2 x 40 mL). After removal of the solvent, the residue was purified on a silica gel column eluting with ethyl acetate:hexane (1:3) to give N-[(1-phenylcyclopentyl)carbonyl]-4-nitro-L-phenylalanine methyl ester (4.5 g, 75.7%).

A suspension of N-[(1-phenylcyclopentyl)carbonyl]-4-nitro-L-phenylalanine methyl ester (3.5 g, 8.88 mmol) and stannous chloride (10 g, 44 mmol) in 60 mL of ethanol was refluxed for 50 min under argon. The ethanol was then removed under reduced pressure and the residue was treated with 50 mL of saturated NaHCO3 followed by sodium carbonate to adjust pH above 9. The white slurry was extracted with ethyl acetate (3 x 300 mL). The combined extracts were washed with water (100 mL) and brine (100 mL) and were dried (MgSO4). Removal of the solvent afforded 4-amino-N-[(1-phenylcyclopentyl)carbonyl]-L-phenylalanine methyl ester (2.9 g, 89%).

### Example 27. Synthesis of N-[(1-phenylcyclopentyl)carbonyl]-4-[(4-quinolinylcarbonyl)amino]-L-phenylalanine sodium salt

To a solution of 4-amino-N-[(1-phenylcyclopentyl)carbonyl]-L-phenylalanine methyl ester (81 mg, 0.2 mmol) and 4-quinolinecarboxylic acid (43.3 mg, 0.25 mmol) in 1 mL of DMF was added HBTU (95 mg, 0.25 mmol) and diisopropylethylamine (65 µL, 0.5 mmol) at room temperature. The mixture was then stirred at this temperature for overnight. The reaction was then diluted with 15 mL of ethyl acetate and was washed with water (2 mL), saturated NaHCO3 (2 x 2 mL) and saturated brine (2 x 2 mL). The solution was dried (MgSO4) and concentrated. The residue was hydrolyzed with 0.5 mL of 1N NaOH in 5 mL of ethanol at 25 °C overnight. The crude product was purified by passing through an open C-18 column eluting with water (200 mL), 30% methanol in water (200 mL), 40% methanol in water (200 mL) and pure methanol (200 mL). The fractions containing product were concentrated and lyophilized to give N-[(1-phenylcyclopentyl)carbonyl]-4-[(4-quinolinylcarbonyl)amino]-L-phenylalanine sodium salt (79.5 mg, 75%), HR-FABMS: obs. mass, 530.2056. Calcd. mass, 530.2058.

Examples 28 - 31. Using the general method described in Example 27, the following analogs were prepared starting with the product from example 26 and the appropriate benzoic or hetereoaromatic carboxylic acids:

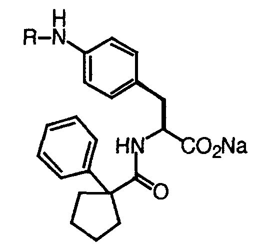

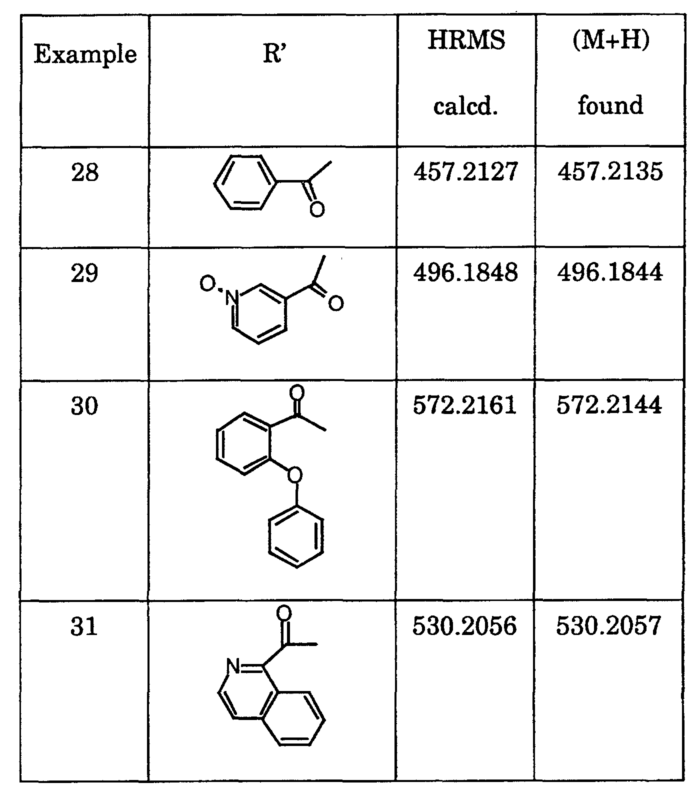

### Example 32. Synthesis of 4-nitro-N-[[1-(phenylmethyl)cyclopentyl]carbonyl]-L-phenylalanine methyl ester

A solution of 1-benzylcyclopentane carboxylic acid (0.135 g, 0.66 mmol), 4-nitro-L-phenylalanine methyl ester (0.187 g, 0.72 mmol), and HBTU (0.272 g, 0.72 mmol) in 2 mL of DMF was treated with diisopropylethylamine (0.35 mL, 2 mmol). The mixture was stirred over night, concentrated, diluted with ethyl acetate, washed with water, 1 N HCl, water, saturated NaHCO3 and dried (MgSO4). The residue obtained after evaporation was purified by chromatography over 30 g of silica gel, eluting with 40% ethyl acetate:hexane to give 4-nitro-N-[[1-(phenylmethyl)cyclopentyl]carbonyl]-L-phenylalanine methyl ester (194 mg, 71%), as a white foam.

### Example 33. Synthesis of 4-amino-N-[[1-(phenylmethyl)cyclopentyl]carbonyl]-L-phenylalanine methyl ester

A solution of 4-nitro-N-[[1-(phenylmethyl)cyclopentyl]carbonyl]-L-phenylalanine methyl ester (185 mg, 0.45 mmol) in 10 mL of ethanol was hydrogenated at atmospheric pressure over 47 mg of 10% Pd(C) for 3 hours. The reaction mixture was filtered through a pad of celite and evaporated to dryness to give 4-amino-N-[[1-(phenylmethyl)cyclopentyl]carbonyl]-L-phenylalanine methyl ester (170 mg, 99%) of as a white solid suitable for use in the next step.

### Example 34. Synthesis of N-[[1-(phenylmethyl)cyclopentyl]carbonyl]-4-[(4-quinolinylcarbonyl)amino)-L-phenylalanine

A solution of 4-amino-N-[[1-(phenylmethyl)cyclopentyl]carbonyl]-L-phenylalanine methyl ester (29.5 mg, 0.078 mmol), quinoline-4-carboxylic acid (17 mg, 0.10 mmol), and HBTU (38 mg, 0.10 mmol) in 1 mL of DMF was treated with diisopropylethylamine (30 µL, 0.17 mmol). The mixture was stirred over night and was diluted with 15 mL of ethyl acetate and 10 mL of ether and was washed with portions of water (2 x 10 mL), saturated NaHCO3 (10 mL) and was dried (MgSO4). Concentration gave 48 mg which was dissolved in 3 mL of methanol. Sodium hydroxide solution (0.10 mL, 4 N, 0.4 mmol) was added and the mixture was stirred for 2 hr. The excess base was quenched by addition of 0.1 mL of acetic acid, the solution was filtered through a 0.2 µ nylon filter and the filtrate was purified by RP-HPLC on a 4 x 30 cm Rainin C-18 column using a gradient of 5 to 95% acetonitrile:water containing 0.75% trifluoroacetic acid at a flow of 49 mL/min over 30 min. The peak eluting at 74.5% acetonitrile was the acid N-[[1-(phenylmethyl)cyclopentyl]carbonyl]-4-[(4-quinolinylcarbonyl)amino)-L-phenylalanine (15 mg), HR-FAB-MS: obs. mass 522.2393. Calcd. mass 522.2393 (M+H), the peak eluting at 83% acetonitrile was recovered N-[[1-(phenylmethyl)cyclopentyl]carbonyl]-4-[(4-quinolinylcarbonyl)amino]-L-phenylalanine methyl ester (15 mg), HR-FAB-MS: obs. mass 536.2556. Calcd. mass 536.2549 (M+H).

### Example 35. Synthesis of 4-[[(2-nitrophenyl)carbonyl]amino]-N-[[1-(phenylmethyl)cyclopentyl]carbonyl]-L-phenylalanine

4-Amino-N-[[1-(phenylmethyl)cyclopentyl]carbonyl]-L-phenylalanine methyl ester (31.7 mg, 0.083 mmol) and 2-nitrobenzoic acid (38 mg, 0.10 mmol) were reacted as described in example 34 to give 26.7 mg of 4-[[(2-nitrophenyl)carbonyl]amino]-N-[[1-(phenylmethyl)cyclopentyl] carbonyl]-L-phenylalanine, HR-FAB-MS: obs. mass 516.2113. Calcd. mass 516.2134 (M+H).

### Example 36. Synthesis of 4-[[(2-methyl-5-nitrophenyl)carbonyl]amino]-N-[[1-(phenylmethyl)cyclopentyl]carbonyl]-L-phenylalanine.

4-Amino-N-[[1-(phenylmethyl)cyclopentyl]carbonyl]-L-phenylalanine methyl ester (35.3 mg, 0.093 mmol) and 2-methyl-5-nitrobenzoic acid 20 mg, 0.11 mmol) were reacted as described in example 34 to give 4-[[(2-methyl-5-nitrophenyl)carbonyl)amino]-N-[[1-(phenylmethyl)cyclo-pentyl)carbonyl]-L-phenylalanine (34 mg, 69%), HR-FAB-MS: obs. mass 530.2298. Calcd. mass 530.2291 (M+H).

### Example 37. Synthesis of 4-[[(2,6-dichlorophenyl)carbonyl]amino]-N-[[1-(phenylmethyl)cyclopentyl] carbonyl]-L-phenylalanine

4-Amino-N-[[1-(phenylmethyl)cyclopentyl]carbonyl]-L-phenylalanine methyl ester (67 mg, 0.176 mmol) and 2,6-dichlorobenzoyl chloride (50 mg, 0.23 mmol) were dissolved in 5 mL of dichloromethane and 2,6-lutidine (50 µL, 0.43 mmol) were added. After 4 hours, the mixture was diluted with ether and dichloromethane and washed with 1 N HCl, water, and saturated NaHCO3 and was dried (MgSO4). The crude product was dissolved in 4 mL of methanol and treated with 4 N NaOH (0.1 mL). After 2 hours, the excess base was quenched with 0.1 mL of acetic acid, the solution was filtered through a 0.2 µ nylon filter and the filtrate was purified by RP-HPLC on a 4 x 30 cm Rainin C-18 column using a gradient of 5 to 95% acetonitrile:water containing 0.75% trifluoroacetic acid at a flow of 49 mL/min over 30 min. The peak eluting at 87 % acetonitrile was concentrated and lyophilized to give 4-[[(2,6-dichlorophenyl)carbonyl]amino]-N-[[1-(phenylmethyl)cyclopentyl]carbonyl]-L-phenylalanine (46 mg), LR(+)LSIMS : m/z 539 (M+H) (2 Cl)

### Example 38. Synthesis of N-[[1-[(4-methoxyphenyl)methyl]cyclopentyl]carbonyl]-4-nitro-L-phenylalanine methyl ester.

A solution of 1-(4-methoxy)benzylcyclopentane carboxylic acid (0.0.20 g, 0.86 mmol), 4-nitro-L-phenylalanine methyl ester (0.24 g, 0.94 mmol), and HBTU (0.36 g, 0.94 mmol) in 3 mL of DMF was treated with 0.52 mL (3 mmol) of diisopropylethylamine. The mixture was stirred over night, concentrated, diluted with ethyl acetate, washed with water, 1 N HCl, water, saturated NaHCO3 and dried (MgSO4). The residue obtained after evaporation was purified by chromatography over 30 g of silica gel, eluting with 40% ethyl acetate:hexane to give N-[[1-[(4-methoxyphenyl)methyl]cyclopentyl]carbonyl]-4-nitro-L-phenylalanine methyl ester (256 mg, 68%), as a white foam, HR-FAB-MS: Obs mass, 441.2027. Calcd mass, 441.2025 (M+H).

### Example 39. Synthesis of 4-amino-N-[[1-[(4-methoxyphenyl)methyl]cyclopentyl]carbonyl]-L-phenylalanine methyl ester

A solution of N-[[1-[(4-methoxyphenyl)methyl]cyclopentyl]carbonyl]-4-nitro-L-phenylalanine methyl ester (253 mg, 0.575 mmol) in 10 mL of ethanol was hydrogenated at atmospheric pressure over 45 mg of 10% Pd(C) for 3 hours. The reaction mixture was filtered through a pad of celite and evaporated to dryness to give 4-amino-N-[[1-[(4-methoxyphenyl)methyl]cyclopentyl]carbonyl]-L-phenylalanine methyl ester (225 mg, 95%) as a white solid suitable for use in the next step, HR-FAB-MS: Obs mass, 410.2196. Calcd mass, 410.2200 (M+).

### Example 40. Synthesis of N-[[1-[(4-methoxyphenyl)methyl] cyclopentyl]carbonyl]-4-[(4-quinolinylcarbonyl)amino]-L-phenylalanine.

A solution of 4-amino-N-[[1-[(4-methoxyphenyl)methyl)cyclopentyl]carbonyl]-L-phenylalanine methyl ester (30.7 mg, 0.075 mmol), quinoline-4-carboxylic acid (17 mg, 0.10 mmol), and HBTU (38 mg, 0.10 mmol) in 1 mL of DMF was treated with diisopropylethylamine (30 µL, 0.17 mmol). The mixture was stirred over night and was diluted with 15 mL of ethyl acetate and 10 mL of ether and was washed with water (2 x 10 mL), saturated NaHCO3 (1 x 10 mL) and was dried (MgSO4). Concentration gave 42 mg which was dissolved in 3 mL of methanol. Sodium hydroxide (0.10 mL, 4 N, 0.4 mmol) was added and the mixture was stirred for 2 hours. The excess base was quenched by addition of acetic acid (0.1 mL), the solution was filtered through a 0.2 µ nylon filter and the filtrate was purified by RP-HPLC on a 4 x 30 cm Rainin C-18 column using a gradient of 5 to 95% acetonitrile:water containing 0.75% trifluoroacetic acid at a flow of 49 mL/min over 30 min. The peak eluting at 74% acetonitrile was concentrated and lyophilized to give N-[[1-[(4-methoxyphenyl)methyl]cyclopentyl]carbonyl]-4-[(4-quinolinylcarbonyl) amino]-L-phenylalanine (22.8 mg), HR-FAB-MS: obs. mass 552.2482. Calcd. mass 552.2498 (M+H). The peak eluting at 82.6 % acetonitrile was recovered N-[[1-[(4-methoxyphenyl) methyl]cyclopentyl]carbonyl]-4-[(4-quinolinylcarbonyl)amino]-L-phenylalanine methyl ester (11.2 mg), HR-FAB-MS: obs. mass 566.2675. Calcd. mass 566.2655 (M+H).

### Example 41. Synthesis of N-[[1-[(4-methoxyphenyl)methyl]cyclopentyl]carbonyl]-4-[[(2-nitrophenyl)carbonyl]amino]-L-phenylalanine.

4-Amino-N-[[1-[(4-methoxyphenyl)methyl]cyclopentyl)carbonyl]-L-phenylalanine methyl ester (32.3 mg, 0.079 mmol) and 2-nitrobenzoic acid (17 mg, 0.10 mmol) were reacted as described in example 40 to give 22 mg of N-[[1-[(4-methoxyphenyl)methyl]cyclopentyl]carbonyl]-4-[[(2-nitrophenyl)carbonyl]amino]-L-phenylalanine, HR-FAB-MS: obs. mass 546.2235. Calcd. mass 546.2240 (M+H).

### Example 42. Synthesis of N-[[1-[(4-methoxyphenyl)methyl]cyclopentyl]carbonyl]-4-[[(2-methyl-5-nitrophenyl)carbonyl] amino]-L-phenylalanine.

4-Amino-N-[[1-[(4-methoxyphenyl)methyl]cyclopentyl]carbonyl]-L-phenylalanine methyl ester (23.4 mg, 0.057 mmol) and 2-methyl-5-nitrobenzoic acid 13 mg, 0.07 mmol) were reacted as described in example 40 to give 23 mg (69%) of N-[[1-[(4-methoxyphenyl)methyl]cyclopentyl]carbonyl]-4-[[(2-methyl-5-nitrophenyl)carbonyl]amino]-L-phenylalanine, HR-FAB-MS: obs. mass 560.2413. Calcd. mass 560.2397 (M+H).

### Example 43. Synthesis of 4-[[(2,6-dichlorophenyl)carbonyl]amino]-N-[[1-[(4-methoxyphenyl)methyl]cyclopentyl]carbonyl]-L-phenylalanine.

4-Amino-N-[[1-[(4-methoxyphenyl)methyl]cyclopentyl]carbonyl]-L-phenylalanine methyl ester (66 mg, 0.17 mmol) and 2,6-dichlorobenzoyl chloride (50 mg, 0.23 mmol) were dissolved in 5 mL of dichloromethane and 2,6-lutidine (50 µL, 0.43 mmol) were added. After 4 hours, the mixture was diluted with ether and dichloromethane and washed with 1 N HCl, water, and saturated NaHCO3 and was dried (MgSO4). The crude product was dissolved in methanol (4 mL) and treated with 4 N NaOH (0.1 mL). After 2 hours, the excess base was quenched with acetic acid (0.1 mL), the solution was filtered through a 0.2 µ nylon filter and the filtrate was purified by RP-HPLC on a 4 x 30 cm Rainin C-18 column using a gradient of 5 to 95% acetonitrile:water containing 0.75% trifluoroacetic acid at a flow of 49 mL/min over 35 min. The peak eluting at 79 % acetonitrile was concentrated and lyophilized to give 4-[[(2,6-dichlorophenyl)carbonyl]amino]-N-[[1-[(4-methoxyphenyl)methyl)cyclopentyl]carbonyl)-L-phenylalanine (28.2 mg), HR-FABMS: obs. mass 591.1445. Calcd. mass 591.1430 (M+Na).

### Example 44. Synthesis of 4-[[(2,6-dichlorophenyl)carbonyl]amino]-N-[(1,1-dimethylethoxy)carbonyl]-L-phenylalanine methyl ester

To a solution of 4-(amino)-N-[(1,1-dimethylethoxy)carbonyl]-L-phenylalanine methyl ester (2.6 g, 8.6 mmol) in dichloromethane (20 mL) were added diisopropylethylamine (2.3 mL, 13 mmol) followed by 2,6-dichlorobenzoyl chloride (1.99 g, 9.5 mmol) at room temperature. The mixture was stirred for 15 h at which time a white precipitate was formed. The mixture was diluted with 30 mL of dichloromethane and 50 mL of water. The layers were separated and the aqueous layer was extracted with 100 mL (2 × 50 mL) of dichloromethane. The combined organic extracts were washed with brine and dried over anhydrous magnesium sulfate. Filtration and concentration of the solvent gave 4.03 g (quantitative) of 4-[[(2,6-dichlorophenyl)carbonyl]amino]-N-[(1,1-dimethylethoxy)carbonyl]-L-phenylalanine methyl ester as a white solid, mp 148-151 °C.

### Example 45. Synthesis of 4-[[(2,6-dichlorophenyl)carbonyl]amino]-L-phenylalanine methyl ester hydrochloride salt

4- [[(2,6-Dichlorophenyl)carbonyl] amino] -N- [(1,1-dimethylethoxy)carbonyl]-L-phenylalanine methyl ester (1.86 g, 4.0 mmol) was treated with 4 N hydrochloric acid in dioxane (10 mL) at room temperature. After 5 minutes, the solids went into solution and the mixture was stirred for 1 h. Then, 25 mL of ethyl ether was added to precipitate the product. The solids were collected by filtration and washed with hexane. The resulting solid was very hydroscopic and became gummy. This material was dissolved in 50 mL of methanol and concentrated. After drying under high vacuum, 4-[[(2,6-dichlorophenyl)carbonyl]amino]-L-phenylalanine methyl ester hydrochloride salt. (1.64 g, 97%) was obtained as a light yellow solid: mp 158-161 °C.

### Example 46. Synthesis of 4-[[(2,6-dichlorophenyl)carbonyl]amino]-N-[[1-(4-methoxyphenyl methyl)cyclohexyl]carbonyl]-L-phenylalanine methyl ester.

To a solution of 4-[[(2,6-dichlorophenyl)carbonyl]amino]-L-phenylalanine methyl ester hydrochloride salt (0.2 g, 0.5 mmol) and 1-(4-methoxyphenylmethyl)cyclohexanecarboxylic acid (0.15 g, 0.60 mmol) in DMF (2 mL) were added HBTU (0.23 g, 0.60 mmol) and diisopropylethylamine (0.20 mL, 1.2 mmol) at room temperature. The mixture was stirred overnight and diluted with 25 mL of ethyl acetate. The ethyl acetate layer was washed successively with 0.5N hydrochloric acid (2 x 20 mL), saturated sodium bicarbonate solution (2 x 20 mL), brine (1 x 20 mL) and dried over anhydrous magnesium sulfate. Filtration and concentration gave 350 mg of white solid which was purified by column chromatography over 15 g of silica gel, eluting with 20-30% ethyl acetate in hexane to give 4-[[(2,6-dichlorophenyl)carbonyl]amino]-N- [[1-(4-methoxyphenylmethyl)cyclohexyl]carbonyl]-L-phenylalanine methyl ester (0.25 g, 84% ) as a white solid, mp 85-87 °C. HR MS: Obs mass, 597.1913. Calcd mass, 597.1923 (M+H).

### Example 47. Synthesis of 4-[[(2,6-dichlorophenyl)carbonyl]amino]-N-[[1-(4-methoxyphenylmethyl)cyclohexyl]carbonyl)-L-phenylalanine.

To a suspension of 4-[[(2,6-dichlorophenyl)carbonyl]amino]-N-[[1-(4-methoxyphenyl methyl)cyclohexyl]carbonyl]-L-phenylalanine methyl ester (0.15 g, 0.25 mmol) in ethanol (2 mL) was added aqueous 1.0 N sodium hydroxide (1.5 mL, 3 mmol) at room temperature. The mixture was heated to 50 °C and the resulting clear solution was stirred overnight. The mixture was concentrated, the residue was diluted with 5 mL of water and extracted with 25 mL of ether to remove any neutral impurities. The aqueous layer was acidified with 1 N HCl and the precipitated white solid was collected by filtration and washed with water (20 mL) and hexane (20 mL). After air-drying, 4-[[(2,6-dichlorophenyl)carbonyl]amino]-N-[[1-(4-methoxyphenylmethyl)cyclohexyl]carbonyl]-L-phenylalanine (0.12 g, 82%) was obtained as a white solid, mp 136-140 °C. HR MS: Obs mass, 583.1763. Calcd mass, 583.1766 (M+H).

Examples 48 to 60. The compounds shown below were prepared according to the procedures given in examples 46 and 47.

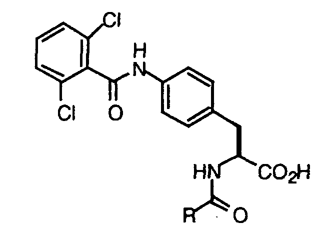

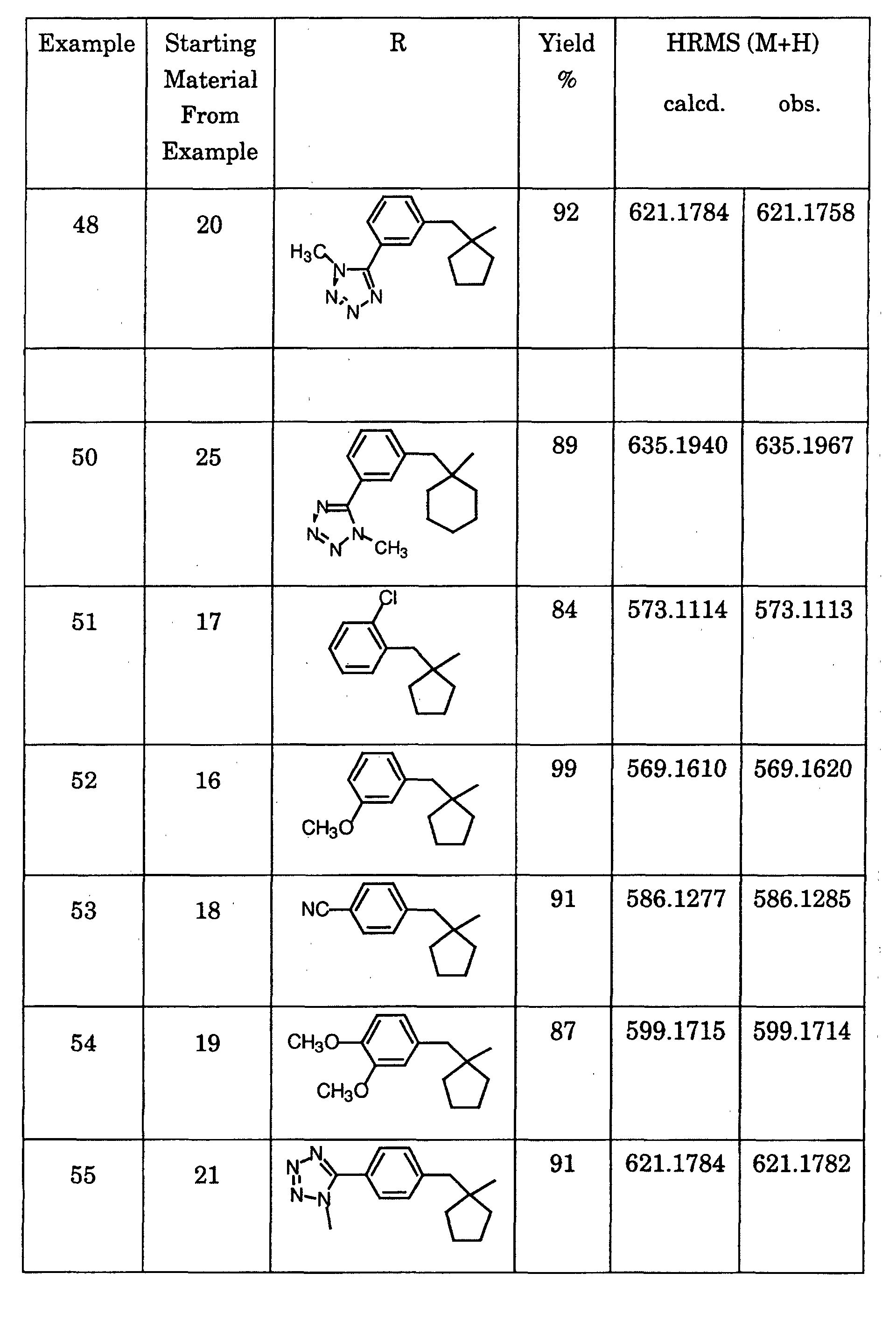

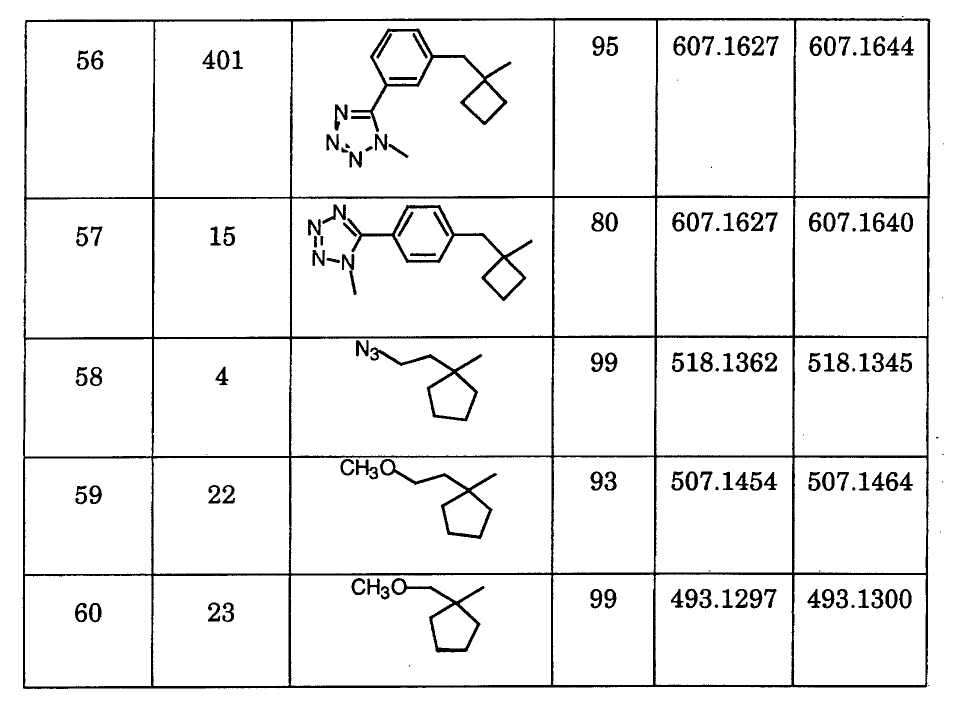

### Example 63. Synthesis of 4-[(4-quinolinylcarbonyl)amino]-L-phenylalanine on Wang resin

A 250 mL cylindrical glass vessel equipped with a coarse glass frit was charged with 4-amino-N-[(9H-fluoren-9-ylmethoxy)carbonyl]-L-phenylalanine (10 g) obtained in Example 62 and a solution prepared from quinoline-4-carboxylic acid (5.2 g, 30 mmol), BOP (13.75 g, 30 mmol) and diisopropylethylamine (6.8 mL) in 70 mL of N-methylpyrrolidinone. The slurry was agitated for 4 hr. The mixture was filtered and washed with dichloromethane (2x100 mL), methanol (2x100 mL) and dimethylformamide (2x100 mL). To the washed resin was added a solution of 25% piperidine in N-methylpyrrolidinone (80 mL), the mixture was agitated at room temperature for 20 minutes and filtered. The process was repeated and the resulting slurry was filtered and washed with dichloromethane (2x100 mL), methanol (2x100 mL) and dimethylformamide (2x100 mL). Filtration afforded 4-[(4-quinolinylcarbonyl)amino]-L-phenylalanine on Wang resin suitable for use in the next step.

### Example 64. Synthesis of N-[(2,2-dichloro-1-methylcyclopropyl)carbonyl]-4-[(4-quinolinylcarbonyl)amino]-L-phenylalanine

4-[(4-Quinolinylcarbonyl)amino]-L-phenylalanine on Wang resin (300 mg) obtained from Example 63 was washed with dichloromethane (2x10 mL), methanol (2x10 mL) and dimethylformamide (2x10 mL). To the resin was added a solution prepared from 2,2-dichloro-1-methylcyclopropylcarboxylic acid (180 mg, 1.02 mmol), BOP (450 mg, 1.02 mmol) and diisopropylethylamine (0.23 mL) in 4 mL of N-methylpyrrolidinone at room temperature. The resulting mixture was agitated for 2 hours. The reaction mixture was then filtered and washed with dichloromethane (2x10 mL), methanol (2x10 mL) and dichloromethane (2x10 mL). Cleavage was effected with 90% trifluoroacetic acid (TFA) in dichloromethane for 5 minutes. The mixture was filtered and the TFA was removed under high vacuum. Addition of ether (25 mL) effected precipitation of N-[(2,2-dichloro-1-methylcyclopropyl)carbonyl]-4-[(4-quinolinylcarbonyl)amino]-L-phenylalanine. The compound was purified by reverse phase HPLC (DuPont, Rx C18, 7 µM, 2.12 cm x 25 cm) using acetonitrile and water as the mobile phase with a linear gradient from 20-50% of acetonitrile over 180 minutes. LRMS (M+H) obs. mass, 486.5. Calcd. mass 486.3.

Examples 65 - 81. Using the procedure described in Example 64, the compounds shown below were prepared.

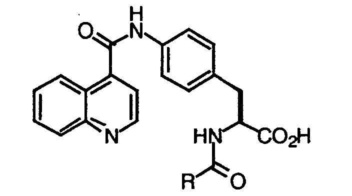

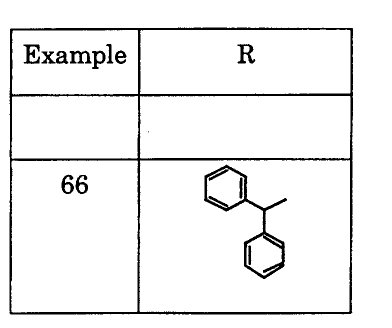

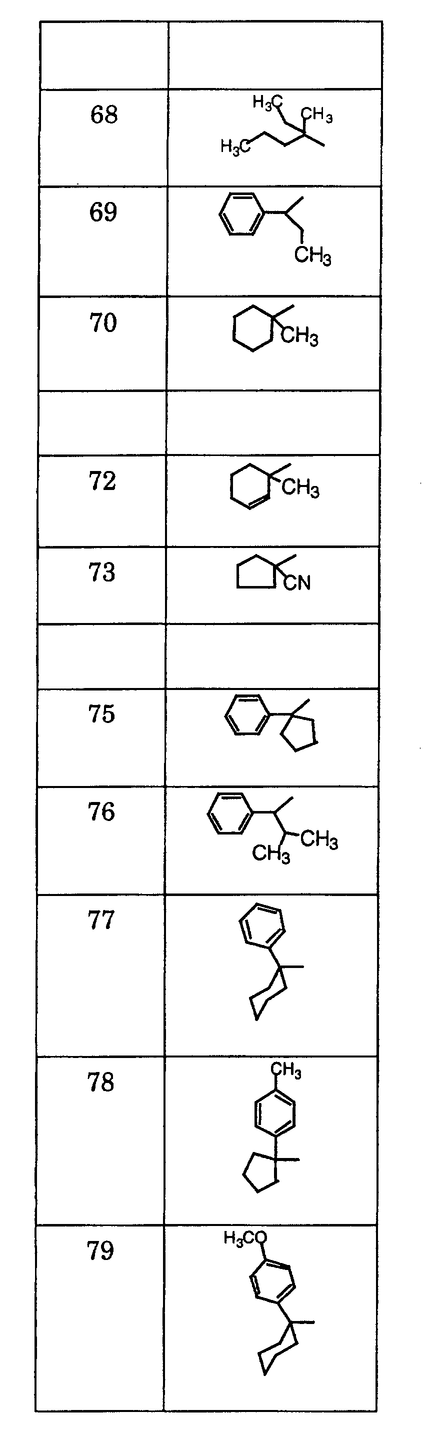

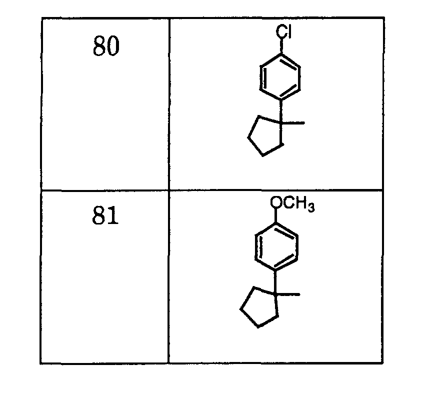

Examples 82 to 106. Using the method described in examples 63 and 64, the following derivatives were prepared:

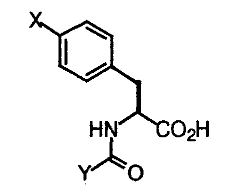

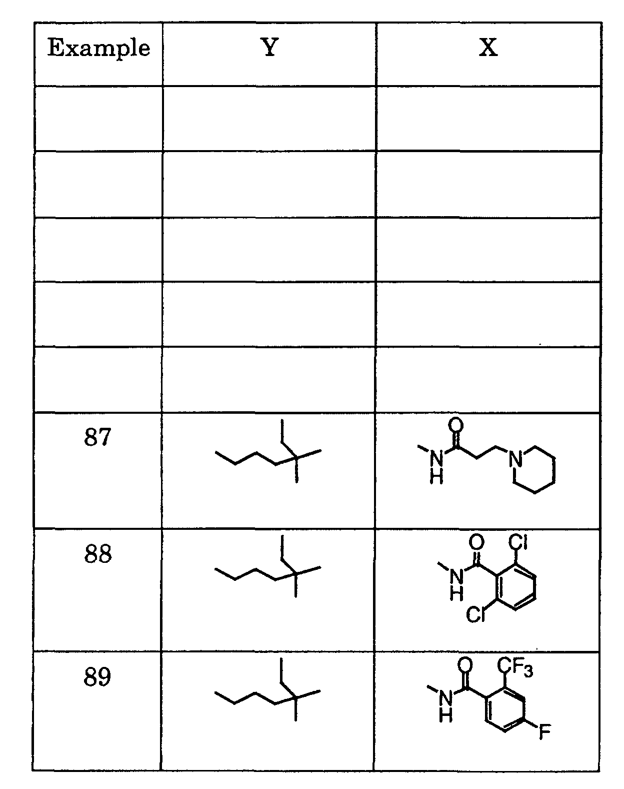

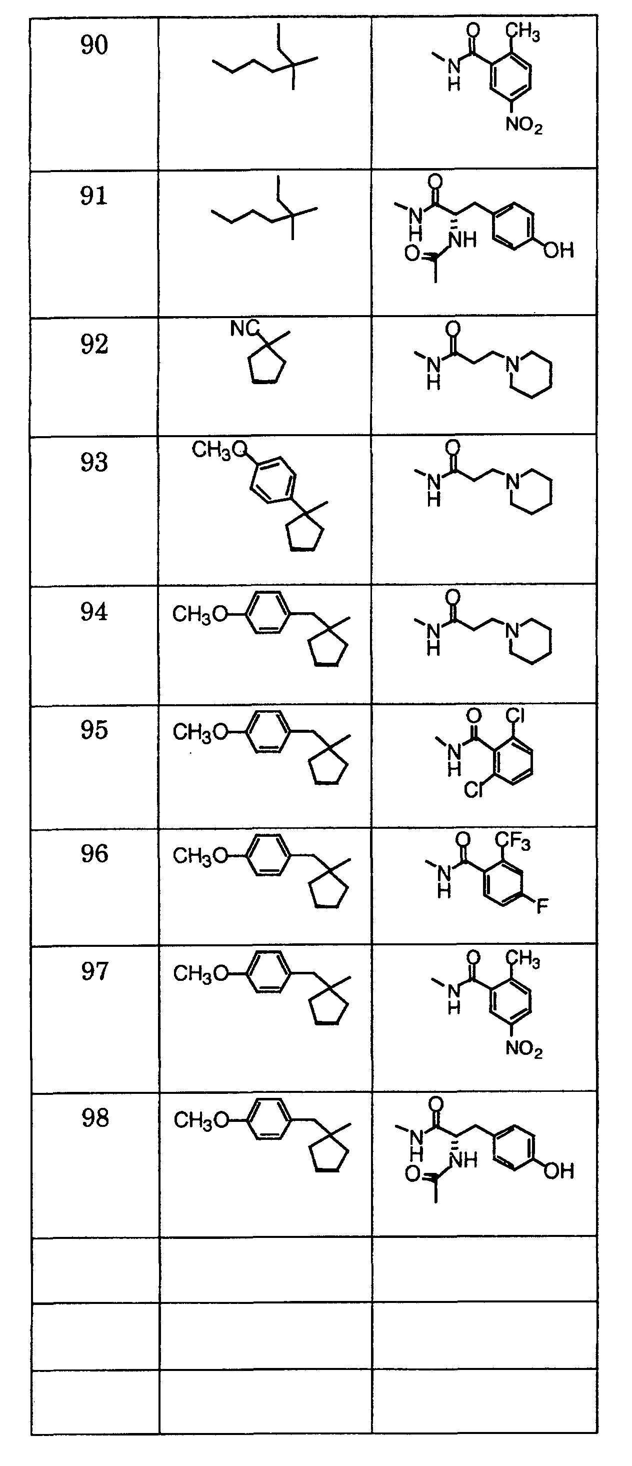

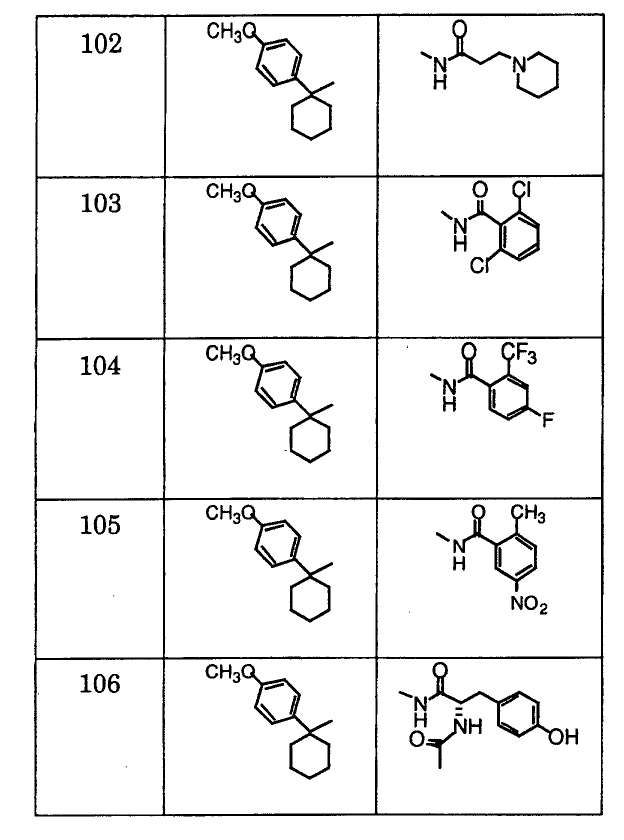

### Example 107. Synthesis of N-[(1,1-dimethylethoxy)carbonyl]-4-[[(9H-fluoren-9-ylmethoxy)carbonyl]amino]-L-phenylalanine phenylmethyl ester

N-[(1,1-Dimethylethoxy)carbonyl]-4-[[(9H-fluoren-9-ylmethoxy)carbonyl]amino]-L-phenylalanine (5.02 g, 10 mmol) and benzyl bromide (3.5 mL, 29 mmol) were stirred in DMF (25 mL) over KHCO3 (1.75 g, 17.5 mmol). After 18 hr, a white precipitate had formed. Most of the DMF was evaporated under reduced pressure, the residue was taken up in 100 mL of dichloromethane and was washed with water (2 x 50 mL). Most of the dichloromethane was evaporated and ether (100 mL) was added to precipitate the product. Filtration, washing with ether afforded N-[(1,1-dimethylethoxy)carbonyl]-4-[[(9H-fluoren-9-ylmethoxy)carbonyl]amino]-L-phenylalanine phenylmethyl ester (5.3 g), mp 186-187 °C.

### Example 108. Synthesis of 4-amino-N-[[1-[(4-methoxyphenyl)methyl]cyclopentyl]carbonyl]-L-phenylalanine

4-Amino-N-[[1-[(4-methoxyphenyl)methyl]cyclopentyl]carbonyl]-L-phenylalanine methyl ester (280 mg, 0.68 mmol) in THF (12 mL) was treated with a solution of lithium hydroxide hydrate (100 mg, 2.4 mmol) in water (2 mL) and the mixture was stirred for 3 hr. The mixture was concentrated and the residue was acidified with 6 H HCl to give a white sticky solid. Trituration with water and drying under high vacuum afforded crude 4-amino-N-[[1-[(4-methoxyphenyl) methyl]cyclopentyl]carbonyl]-L-phenylalanine (200 mg) suitable for use in the next step.

### Example 109. Synthesis of 4-(2,3-dihydro-1,3-dioxo-1H-pyrrolo[3,4-c]pyridin-2-yl)-N-[[1-[(4-methoxyphenyl)methyl]cyclopentyl]carbonyl]-L-phenylalanine

A solution of 4-amino-N-[[1-[(4-methoxyphenyl)methyl]cyclopentyl]carbonyl]-L-phenylalanine (98 mg, 0.23 mmol), DIPEA (30 µL, 0.23 mmol) and 3,4-pyridinedicarboxylic acid anhydride (114 mg, 0.77 mmol) in dichloromethane (10 mL) was stirred 18 hr at room temperature. The mixture was concentrated to remove most of the dichloromethane and the residue was taken up in DMF (3 mL). Carbonyl diimidazole (103 mg, 0.64 mmol) was added to the resulting solution and the reaction was allowed to proceed for 18 hr. The resulting mixture was purified directly by RP-HPLC on a 4 x 30 cm Rainin C-18 column using a gradient of 5 to 95% acetonitrile:water containing 0.75% trifluoroacetic acid at a flow of 49 mL/min over 35 min. The peak eluting at 45.6 % acetonitrile was concentrated and lyophilized to give 4-(2,3-dihydro-1,3-dioxo-1H-pyrrolo[3,4-c]pyridin-2-yl)-N-[[1-[(4-methoxyphenyl)methyl]cyclopentyl]carbonyl]-L-phenylalanine (28 mg) HR FABMS: obs. mass 528.2146. Calcd. mass 528.2134 (M+H).

### Example 110: Synthes1s of 4-(1,3-dioxo-2H-isoindol-2-yl)-N-[[1-[(4-methoxyphenyl)methyl]cyclopentyl]carbonyl]-L-prolyl]-L-phenylalanine

Using the procedure described in example 109, starting with phthalic anhydride, 4-(1,3-dioxo-2H-isoindol-2-yl)-N-[[1-[(4-methoxyphenyl)methyl]cyclopentyl]carbonyl]-L-prolyl]-L-phenylalanine was obtained, HR FABMS: obs. mass 527.2172. Calcd. mass 527.2182 (M+H).

### Example 111. Synthesis of 4-[(RS)-2,3,5,6,7,7a-hexahydro-1,3-dioxo-1H-pyrrolo[3,4-c]pyridin-2-yl]-N-[[1-[(4-methoxyphenyl)methyl]cyclopentyl]carbonyl]-L-phenylalanine and N-[[1-[(4-methoxyphenyl)methyl]cyclopentyl]carbonyl]-4-[(3aRS,7aRS)-(octahydro-1,3-dioxo-1H-pyrrolo [3,4-c]pyridin-2-yl)]-L-phenylalanine

A solution of 4-(2,3-dihydro-1,3-dioxo-1H-pyrrolo[3,4-c]pyridin-2-yl)-N-[[1-[(4-methoxy-phenyl)methyl]cyclopentyl]carbonyl]-L-phenylalanine (30 mg, 0.057 mmol) in ethanol (4 mL) containing a few drops of TFA was hydrogenated over 10% Pd(C) (6 mg) for 18 hours. The mixture was filtered and evaporated and the residue was purified by RP-HPLC on a 4 x 30 cm Rainin C-18 column using a gradient of 5 to 95% acetonitrile:water containing 0.75% trifluoroacetic acid at a flow of 49 mL/min over 35 min. The peak eluting at 59.5 % acetonitrile was concentrated and mixture was lyophilized to give N-[[1-[(4-methoxyphenyl)methyl] cyclopentyl]carbonyl]-4-[(3aRS,7aRS)-(octahydro-1,3-dioxo-1H-pyrrolo[3,4-c]pyridin-2-yl)]-L-phenylalanine (4.2 mg), HR FABMS: obs. mass 534.2602. Calcd. mass 534.2604 (M+H). The peak eluting at 62% acetonitrile was concentrated and the mixture lyophilzed to give 4-[(RS)-2,3,5,6,7,7a-hexahydro-1,3-dioxo-1H-pyrrolo[3,4-c]pyridin-2-yl]-N-[[1-[(4-methoxyphenyl) methyl]cyclopentyl]carbonyl]-L-phenylalanine (4.9 mg), HR FABMS: obs. mass 532.2452. Calcd. mass 532.2447 (M+H).

### Example 112. Synthesis of 4-[[(2,4,6-trimethylphenyl)sulfonyl]amino]-L-phenylalanine on Wang resin

4-Amino-N-[(9H-fluoren-9-ylmethoxy)carbonyl]-L-phenylalanine on Wang resin (3.0 g, 2.28 mmol) obtained from Example 62 was suspended in pyridine (15 mL), the slurry was cooled to 0 °C and 2,4,6-trimethylphenylsulfonyl chloride (2.49 g, 11.4 mmol) was added. The resulting mixture was agitated for 2 hr. The mixture was filtered and washed with dichloromethane and methanol. The coupling procedure was repeated. The resulting resin was treated with 25% piperidine in N-methylpyrrolidinone (2 x 15 min) and was washed with dichloromethane and methanol to give 4-[[(2,4,6-trimethylphenyl)sulfonyl]amino]-L-phenylalanine on Wang resin.

### Example 123. General Procedure for the preparation of ethyl esters from 4-substituted-N-acyl-L-phenylalanine derivatives.

To a suspension of N-(acyl)-4-[(aroyl)amino]-L-phenylalanine (10 mmol) and powdered sodium bicarbonate (4.2 g, 50 mmol) in DMF (75 mL) was added excess iodoethane (7.8 g, 50 mmol) at room temperature. The resulting suspension was stirred until TLC analysis of the mixture indicated the absence of staring material, typically 20 h. The excess iodoethane and some DMF was removed on a rotary evaporator under vaccum. The residue was diluted with 100 mL of ethyl acetate and washed successively with water (2 x 70 mL), brine solution (70 mL) and dried over MgSO4. Filtration of the drying agent and removal of the solvent afforded a residue which was purified by crystallization or silica gel chromatography.

### Example 124. Wang resin linked N-[[1-(2-methoxyethyl)cyclopentyl]carbonyl]-4-nitro-L-phenylalanine

A mixture of Wang resin linked N-Fmoc-4-nitro-L-phenylalanine (2.30 g, 1.35 mmol) in 27 mL of 25% piperidine in NMP (N-methylpyrrolidinone) was shaken for 30 minutes at room temperature. Solvent was filtered through sintered funnel. With resin still on the funnel, another 27 mL of 25% piperidine in NMP was added and the suspension was allowed to stand at room temperature for 30 minutes. After removal of the solvent by filtration, the resin was then washed with dichloromethane, DMF, isopropyl alcohol, dichloromethane sequentially and was dried under vaccume.

The above resin was placed into a 50 mL round bottom flask containing 13 mL of NMP. To it was added, 1-(2-methoxyethyl)cyclopentane carboxylic acid (930 mg, 5.4 mmol), diisopropylethylamine (DIEA, 1.3 mL, 7.4 mmol), and BOP reagent (2.4 g, 5.4 mmol). Reaction was shaken overnight. A small aliquot was removed and analyzed by the Kaiser test which showed negative for amine. Resin was collected by filtration and was washed with dichloromethane, DMF, isopropyl alcohol, dichloromethane and dried under reduced pressure to give 1.97 g of Wang resin linked N-[[1-(2-methoxyethyl)cyclopentyl]carbonyl]-4-nitro-L-phenylalanine.

### Example 125. Synthesis of Wang resin linked 4-amino-N-[[1-(2-methoxyethyl) cyclopentyl]carbonyl]-L-phenylalanine.

In a 20 mL scintillation vial was placed Wang resin linked N-[[1-(2-methoxyethyl)cyclopentyl]carbonyl]-4-nitro-L-phenylalanine (1.91 g, 1.12 mmol) and a 2 M solution of SnCl2.2H2O in DMF (8 mL). The reaction mixture was shaken overnight at room temperature. The resin was collected by filtration and was washed with DMF, isopropyl alcohol, dichloromethane, and Et2O to give Wang resin linked 4-amino-N-[[1-(2-methoxyethyl)cyclopentyl]carbonyl]-L-phenylalanine (2.21 g). A small sample was cleaved from the resin with 50% TFA/dichloromethane and was analyzed by ESPMS which showed presence of product but no starting material, m/z 335 (M+H).

### Example 126. Synthesis of Wang resin liked 4-[((2R)-2-amino-4-methyl-1-oxopentyl)amino]-N-[[1-(2-methoxyethyl)cyclopentyl]carbonyl]-L-phenylalanine.

In a 20 mL scintillation vial was placed Wang resin linked 4-amino-N-[[1-(2-methoxyethyl)cyclopentyl]carbonyl]-L-phenylalanine (200 mg, 0.118 mmol), Fmoc-D-Leu-OH (201 mg, 0.571 mmol), DIEA (164 uL, 0.95 mmol), HBTU (360 mg, 0.95 mmol) in DMF (5 mL). The reaction mixture was shaken overnight at room temperature. The resin was filtered and washed with DMF, isopropyl alcohol, dichloromethane. A Kaiser test was negative for amine.

The resin obtained above was treated with 5 mL of 25% piperidine in NMP for 45 minutes at room temperature. After it was filtered through sintered funnel and washed with DMF, the resin was re-suspended in 5 mL of 25% piperidine/NMP in the funnel and was allowed to stand for 15 minutes at room temperature. This process was repeated once more and the resin was then washed with DMF, isopropyl alcohol, dichloromethane. Wang resin linked 4-[((2R)-2-amino-4-methyl-1-oxopentyl)amino]-N-[[1-(2-methoxyethyl)cyclopentyl]carbonyl]-L-phenylalanine (215 mg) was obtained.

### Example 127. Synthesis of Wang resin linked 4-[(2S,4R)-3-acetyl-4-(2-methylpropyl)-5-oxo-2-phenyl-1-imidazolidinyl]-N-[[1-(2-methoxyethyl)cyclopentyl]carbonyl]-L-phenylalanine and Wang resin linked 4-[(2R,4R)-3-acetyl-4-(2-methylpropyl)-5-oxo-2-phenyl-1-imidazolidinyl]-N-[[1-(2-methoxyethyl)cyclopentyl]carbonyl]-L-phenylalanine.

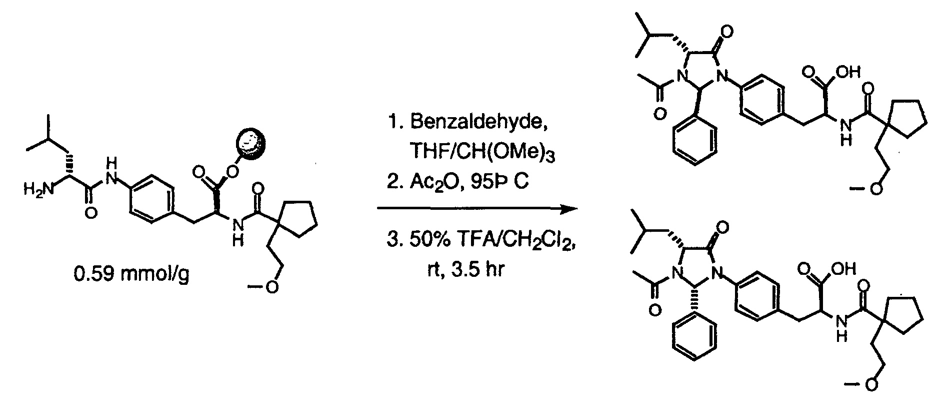

The resin obtained in Example 126 ( 0.59 mmol/g, 0.202 g, 0.11 mmol) was reacted with benzaldehyde (1.78 mmol, 182 µL) in 4 mL of a mixed solvent THF/trimethylorthoformate (1/1) at room temperature and was shaken for 4 days. To the above suspension was then added 3 mL of acetic anhydride and the mixture was stirred at 95° C overnight. After the suspension was cooled to room temperature, the solvent was removed by filtration and the resin was washed with dichloromethane, THF and then dichloromethane.

Treatment of resulting resin with 6 mL of TFA/dichloromethane (1/1) at room temperature for 3.5 hr resulted in a crude mixture containing products. The crude products gave the correct mass by ESPMS (M+H) = 578. RP-HPLC (41.4mm x 30 mm Dynamax C18 column, 5:95 to 95:5 acetonitrile:water gradient over 30 min monitoring the effluent at 214 Å) separation gave two diastereomeric products tentatively assigned as: 4-[(2S,4R)-3-acetyl-4-(2-methylpropyl)-5-oxo-2-phenyl-1-imidazolidinyl)-N-[[1-(2-methoxyethyl)cyclopentyl]carbonyl]-L-phenylalanine rention time 25.06 min, 25% yield and 4-[(2R,4R)-3-acetyl-4-(2-methylpropyl)-5-oxo-2-phenyl-1-imidazolidinyl]-N-[[1-(2-methoxyethyl)cyclopentyl]carbonyl]-L-phenylalanine, retention time 26.98 min, 33% yield.

### Example 128 - 139

Using procedure described in Example 126-127, the compounds shown below were prepared

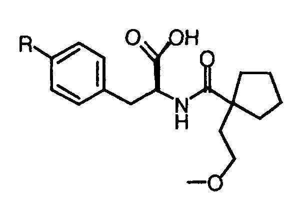

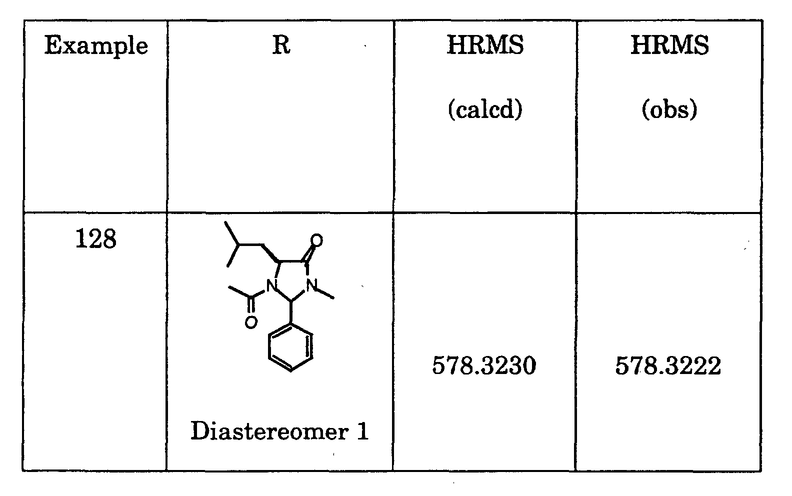

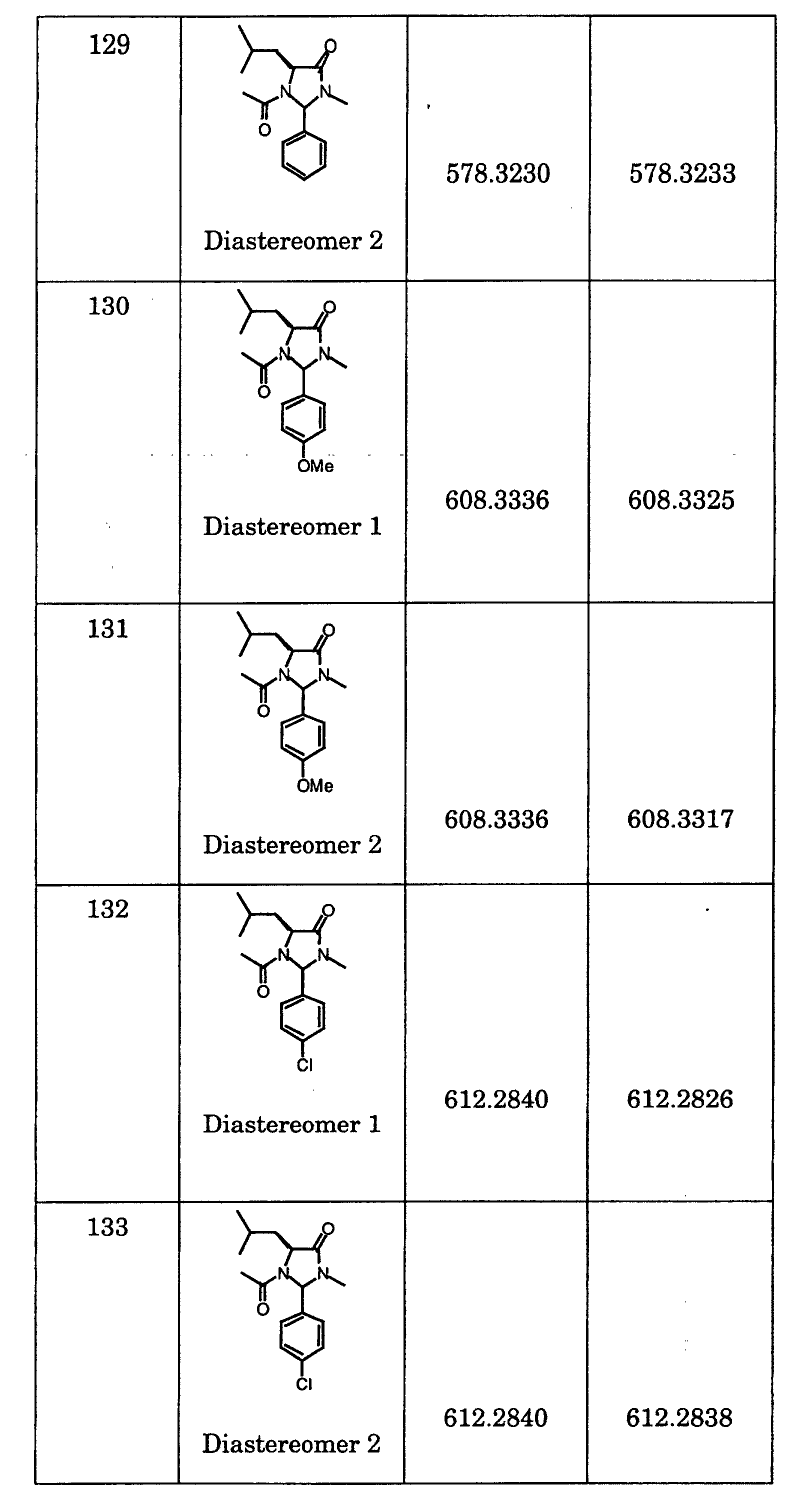

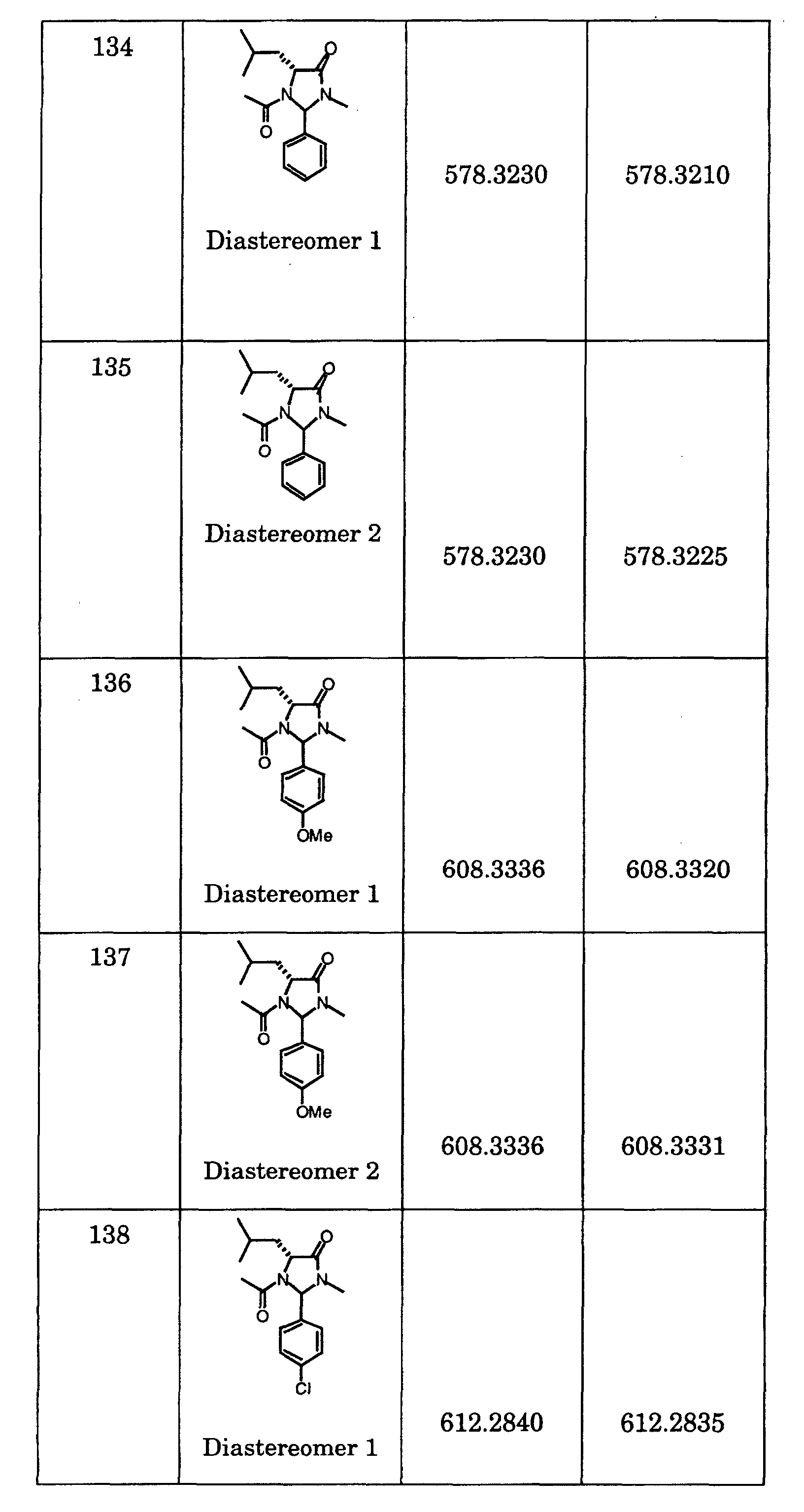

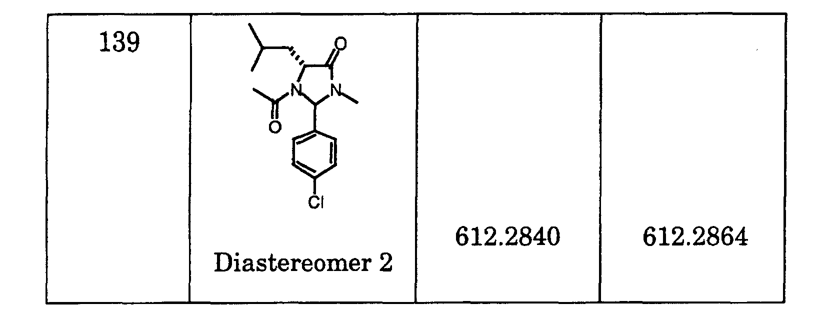

### Example 140. Synthesis of Wang resin linked N-[[1-(4-methoxyphenyl)cyclohexyl] carbonyl)-4-nitro-L-phenylalanine

A mixture of Fmoc-4-nitro-L-phenylalanine on Wang resin (581 mg, 0.36 mmol) in 10 mL of 25% piperidine in NMP (N-methylpyrrolidinone) was shaken for 30 minutes at room temperature. Solvent was filtered through a sintered funnel. With the resin still on the funnel, another 27 mL of 25% piperidine in NMP was added and the suspension was allowed to stand at room temperature for 30 minutes. After removal of the solvent by filtration, the resin was then washed with dichloromethane, DMF, isopropyl alcohol, dichloromethane sequentially and was dried under vacuum.

The above resin was placed into a 25 mL round bottom flask containing 4 mL of NMP. To it was added, 1-(4-methoxyphenyl)cyclohexanecarboxylic acid (337 mg, 1.44 mmol), diisopropylethylamine (DIEA, 343 µL, 1.98 mmol), and BOP reagent (637 mg, 1.44 mmol). The reeaction mixture was shaken overnight. A small aliquot was removed and analyzed by the Kaiser test which was negative for amine. The resin was collected by filtration and was washed with dichloromethane, DMF, isopropyl alcohol, dichloromethane and was dried under reduced pressure to give 580 mg (0.59 mmol/g) of Wang resin linked N-[[1-(4-methoxyphenyl)cyclohexylcarbonyl]-4-nitro-L-phenylalanine.

### Example 145. 2,6-Dimethyl-4-trifluoromethyl-3-pyridinecarboxylic acid.

A solution of 2,6-dimethyl-4-trifluoromethyl-3-pyridinecarboxylic acid ethyl ester in 40 mL of THF and 10 mL of 1 N sodium hydroxide solution was heated to reflux for 48 h. TLC of the mixture (3:7 methanol:dichloromethane) indicated that starting material was consumed. The mixture was acidified with acetic acid (5 mL) and evaporated to dryness. The residue was triturated with THF and the solution was concentrated to give 0.7 g of material containing some THF and acetic acid as indicated by NMR. This material was combined with the product of a similar experiment and was chromatographed on 90 g of silica gel, eluting with (3:7) methanol:dichloromethane to give 1.05 g of a solid. This material was diluted with toluene (6 mL) and evaporated several times to remove most of the acetic acid to afford after drying under high vacuum, 0.9 g of a white foam. LR-ES- MS (C9H6F3NO2): 218 (M-H).

### Example 146. Preparation of 4-[[(2,6-dichlorophenyl)carbonyl]amino]-N-[[1-(2-azidoethyl)cyclopentyl]carbonyl]-L-phenylalanine

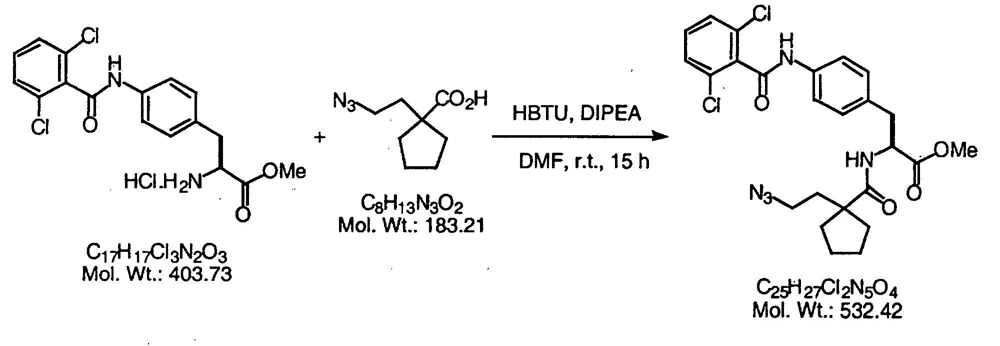

Using the general procedure described in example 46, 4-[[(2,6-dichlorophenyl)carbonyl]amino)-N-[[1-(2-azidoethyl)cyclopentyl]carbonyl)-L-phenylalanine methyl ester was prepared in 78% overall yield. HR MS: (C25H27Cl2N5O4): Obs mass, 532.1519. Calcd mass, 532.1518, (M+H).

### Example 147. Preparation of 4-[[(2,6-dichlorophenyl)carbonyl]amino]-N-[[1-(2-aminoethyl)cyclopentyl)carbonyl]-L-phenylalanine methyl ester

To a suspension of 4-[[(2,6-dichlorophenyl)carbonyl]amino]-N-[[1-(2-azidoethyl) cyclopentyl]-carbonyl]-L-phenylalanine methyl ester (15.47 mmol, 8.24 g) in THF (70 mL) was added a solution of 1.0 M trimethylphosphine in toluene (25 mmol, 25 mL) at 0 °C. The mixture was stirred 7 h at room termperature, at which time TLC analysis indicated the absence of starting material. Then, 3 equiv. of water (45 mmol, 0.82 mL) were added and the mixture was stirred for 15 h at room temperature. The solvent was removed under vacuum and the residue was azeotrophed two times with toluene to give a pasty material which was dissolved in THF:dichloromethane (-250 mL) and dried over anhydrous sodium sulfate. The solution was filtered through a plug of celite and the celite was washed with THF (100 mL). The combined filtrates were evaporated under vacuum to obtain 5 g (64%) of methyl 4-[[(2,6-dichlorophenyl)carbonyl]amino]-N-[[1-(2-aminoethyl)cyclopentyl]carbonyl]-L-phenylalanine ester as a pink solid. HR MS: (C25H29Cl2N3O4) Obs mass, 506.1625. Calcd mass, 506.1613, (M+H).

### Example 148. Preparation of 4-[[(2,6-dichlorophenyl)carbonyl]amino]-N-[[1-[2-[(1-oxoethyl)amino]ethyl] cyclopentyl]carbonyl]-L-phenylalanine methyl ester.

To a solution of methyl 4-[[(2,6-dichlorophenyl)carbonyl]amino]-N-[[1-(2-aminoethyl)cyclo pentyl]carbonyl]-L-phenylalanine ester (9.6 mmol, 5 g) in pyridine (70 mL) was added 2 equiv. of acetic anhydride (20 mmol, 2.04 g) at room temperature. The colored solution was stirred for 15 h at room temperature and then diluted with 200 mL of ethyl acetate. The ethyl acetate layer was washed successively with 1N hydrochloric acid (2 x 100 mL), brine solution (100 mL) and dried over anhydrous magnesium sulfate. Filtration of the drying agent and concentration of the solvent afforded a fluffy solid which was only 85% pure by HPLC. This solid was triturated with ethyl acetate (40 mL) and then hexane (20 mL) was added. The solid was collected by filtration and dried at air to afford 3.38 g (63%) of 4-[[(2,6-dichlorophenyl)carbonyl]amino]-N-[[1-[2-[(1-oxoethyl)amino)ethyl]cyclopentyl]carbonyl]-L-phenylalanine methyl ester as a yellow solid, mp 190-194 °C. HR MS (C27H31Cl2N3O5): Obs mass, 548.1696. Calcd mass, 548.1719 (M+H).

### Example 149. Preparation of 4-[[(2,6-dichlorophenyl)carbonyl]amino]-L-phenylalanine hydrochloride salt.

To a solution of methyl 4-[[(2,6-dichlorophenyl)carbonyl]amino]-L-phenylalanine hydrochloride salt (30 mmol, 12.1 g) in ethanol (300 mL) was added 1 N sodium hydroxide (90 mL). The mixture was heated to 40-45 °C for 1 h at which point TLC analysis of the reaction mixture indicated the absence of starting material and it was cooled to room temperature. The solvent was removed under vacuum and the residue was extracted with ether (2 x 100 mL) to remove neutral impurities. Then, the basic aqueous layer was acidified with 1 N hydrochloric acid to pH 2 to give a clear solution. The solution was lyopholized under high vacuum to afford 11.7 g (100%) of the title compound as a white amorphous solid. HR MS (C16H15Cl3N2O3): Obs. mass, 354.2014. Calcd. mass, 354.2053 (M+H).

### Example 152. Preparation of 4-[[(2,6-dichlorophenyl)carbonyl]amino]-L-phenylalanine on Wang resin

To the 4-[[(2,6-dichlorophenyl)carbonyl]amino]-N-[9H-fluoren-9-ylmethoxy)carbonyl]-L-phenylalanine on Wang resin prepared above (12.1 mmol, 17.4 g) was added 20% of piperidine in NMP at room temperature. The mixture was shaken for 1.5 h at room temperature and the resin was filtered and washed with DMF (2 x 20 mL). Then, the resin was suspended in 20% piperidine in NMP (100 mL) and the solvent was decanted. The resin was washed with dichloromethane (2 x 20 mL) and dried under high vacuum to obtain 13.5 g of 4-[[(2,6-dichlorophenyl)carbonyl]amino]-L-phenylalanine on Wang resin.

Example 153. N-[[1-(2-Azidoethyl)cyclopentyl]carbonyl]-4-[[(2,6-dichlorophenyl)carbonyl] amino]-L-phenylalanine on Wang resin was prepared from the product of example 152 using the general method described in example 64. The loading was 0.695 mmol/g of resin.

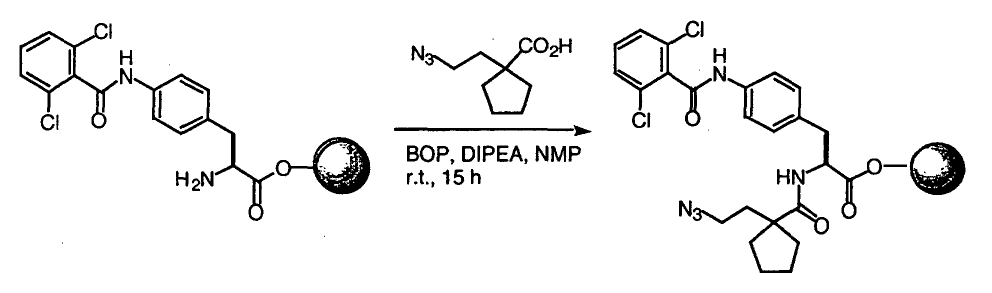

### Example 154. Preparation of N-[[1-(2-aminoethyl)cyclopentyl]carbonyl]-4-[[(2,6-dichlorophenyl)carbonyl]amino]-L-phenylalanine on Wang resin

To a suspension of N-[[1-(2-azidoethyl)cyclopentyl]carbonyl]-4-[[(2,6-dichlorophenyl)carbonyl]amino]-L-phenylalanine on Wang resin (9.33 mmol, 13.43 g, 0.695 mmol per g) in THF (60 mL) was added a solution of 1.0 M trimethylphosphine in THF (37.3 mmol, 37.3 mL) at 0 °C. The mixture was allowed to warm to room temperature and stir for 4 h at which time a Kaiser test was possitve for amine. The resin was filtered and was washed with DMF (4 x 20 mL), dichloromethane (4 x 10 mL), isopropanol (4 x 10 mL), DMF (2 x 20 mL) and dichloromethane (2 x 20 mL), respectively. After drying under high vacuum, 13.43 g of N-[[1-(2-aminoethyl)cyclopentyl]carbonyl]-4-[[(2,6-dichlorophenyl)carbonyl]amino]-L-phenylalanine on Wang resin was obtained.

### Example 155. Preparation of 4-[[(2,6-dichlorophenyl)carbonyl]amino]-N-[[1-[2-[[(1,1-dimethylethyl)carbonyl]amino]ethyl]cyclopentyl]carbonyl]-L-phenylalanine on Wang resin

To a mixture of N-[[1-(2-aminoethyl)cyclopentyl]carbonyl]-4-[[(2,6-dichlorophenylcarbonyl] amino]-L-phenylalanine on Wang resin (0.069 mmol, 100 mg, 0.695 mmol per gram loading) in pyridine (2 mL) was added excess of 2,2,2-trimethylacetyl chloride (0.28 mmol, 33 mg) at 0 °C. The mixture was allowed to warm to room temperature and shaken for 3 h at which time a Kaiser test was negative for amine. The resin was filtered and washed with dichloromethane (2 x 10 mL) and was dried under high vacuum to afford 4-[[(2,6-dichlorophenyl)carbonyl]aminoj-N-[[1-[2-[[N-(1,1-dimethylethyl)carbonyl]amino]ethyl]cyclopentyl]carbonyl]-L-phenylalanine on Wang resin.

Example 156. 4-[[(2,6-dichlorophenyl)carbonyl]amino]-N-[[1-[2-[[N-(1,1-dimethylethyl) carbonyl] amino] ethyl] cyclopentyl] carbonyl]-L-phenylalanine was prepared by cleavage of the product of example 155 from the resin with TFA using the general procedure described in example 64 to give 25 mg (62%) of a white solid. HR MS (C29H35Cl2N3O5): Obs mass, 576.2019. Calcd mass, 576.2032 (M+H).

### Example 157. Preparation of N-[[1-(2-methoxyethyl)cyclopentyl]carbonyl]-N-methyl-4-nitro-L-phenylalanine methyl ester.

To a suspension of N-[[1-(2-methoxyethyl)cyclopentyl]carbonyl]-4-nitro-L-phenylalanine methyl ester (2.73 mmol, 1.03 g) and silver oxide (10.9 mmol, 2.53 g) in DMF (25 mL) was added methyl iodide (160 mmol, 10 mL) at room temperature. The suspesion was stirred for 2 days at room temperature at which time TLC analysis of the mixture indicated the presence of starting material. An additional 10 mL (160 mmol) of methyl iodide and 2 g (8.6 mmol) of silver oxide were added, respectively. The suspension was stirred for 24 h and the solid was filtered through a pad of celite and was washed with ethyl acetate (30 mL) and methanol (30 mL). The filtrate was concentrated and the residue was extracted with ethyl acetate (3 x 30 mL).The organic layer was washed with water (20 mL) and brine solution (20 mL) and was dried over anhydrous magnesium sulfate. Filtration of the drying agent and removal of the solvent gave a crude compound which was purified by silica gel column chromatography to obtain 530 mg (50%) as a light brown oil. HR MS (C20H28N2O6): Obs. mass, 392.1940. Calcd. mass, 392.1947 (M+).

Example 158 to 167 using the general procedure described in examples 155 and 156, the analogues shown below were prepared from N-[[1-(2-aminoethyl)cyclopentyl]carbonyl] -4- [[(2,6-dichlorophenyl)carbonyl] amino]-L-phenylalanine on Wang resin.

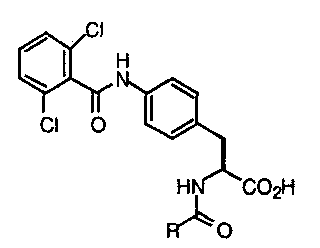

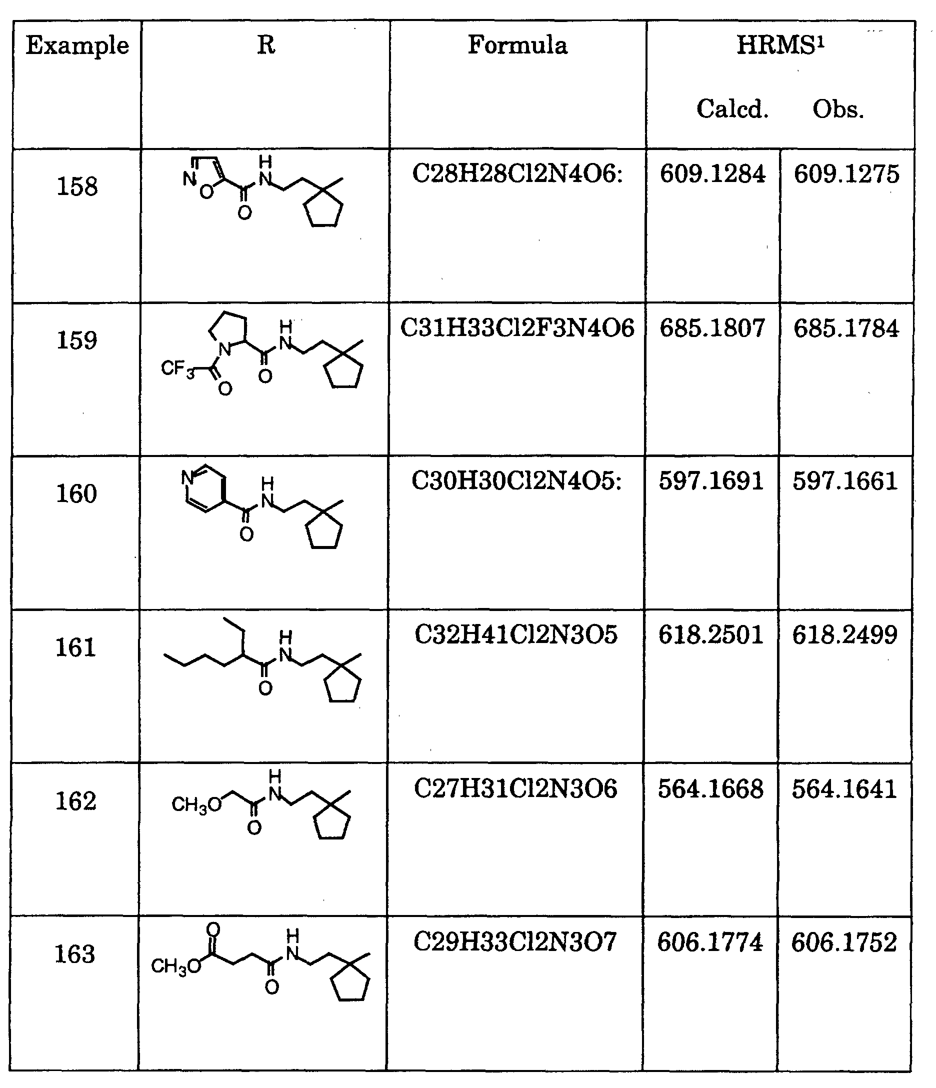

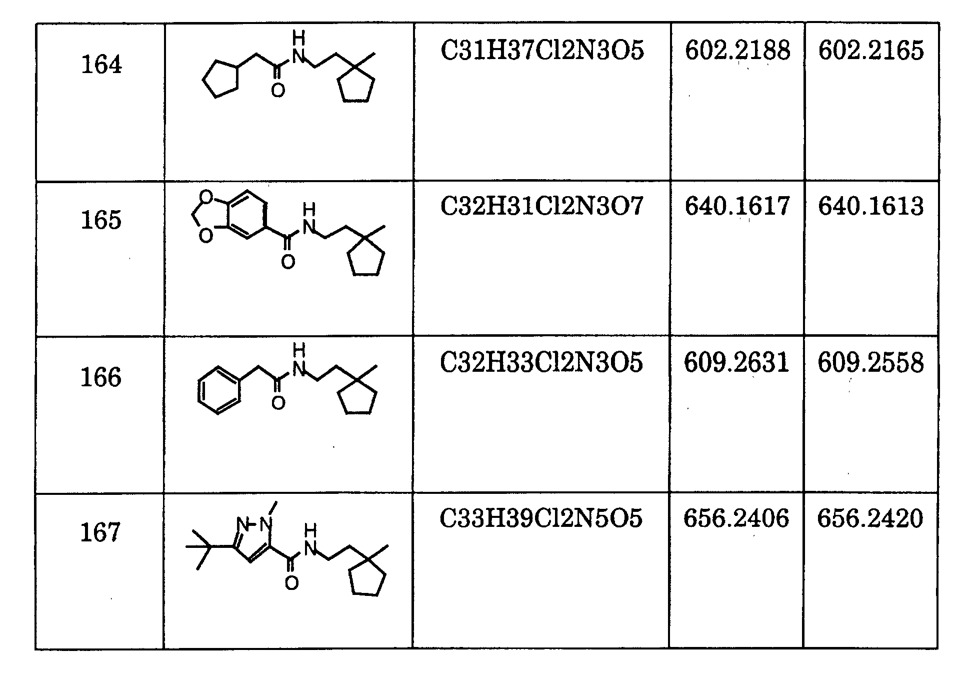

1. M+H ion unless otherwise indicated

### Example 168. Preparation of 1-(4-bromobutyl)cyclopentane carboxylic acid methyl ester.

To a solution of diisopropylamine (150 mmol, 21 mL) in THF (100 mL) was added dropwise a solution of n-butyl lithium (145 mmol, 58 mL, 2.5M) in hexanes at -10 °C while maintaining the temperature below 0 °C. After addition, the solution was stirred for 30 min at 0 °C. To this, a solution of methyl cyclopentane carboxylate (100 mmol, 13.1 g) in THF (20 mL) was added dropwise at -70 °C maintaining the internal temperature between -60 to - 70 °C. After addition, the reaction mixture was stirred for 1 h at -50 to -60 °C. Then, a solution of 1,4-dibromobutane (100 mmol, 21.59 g) in THF (20 mL) was added dropwise and the light brown suspension was stirred for 1 h at -60 to -70 °C. Then, it was allowed to warm to room temperature and stirred overnight. The reaction mixture was poured into a saturated solution of ammonium chloride (200 mL) and the organic compound was extracted into ether (2 x 100 mL). The combined extracts were washed with a saturated brine solution (150 mL) and dried over anhydrous magnesium sulfate. After filtration of the drying agent, the solution was concentrated under vacuum and the resulting residue was distilled at 120-133 °C/2.5 mm Hg to obtain 1-(4-bromobutyl)cyclopentane carboxylic acid methyl ester as a colorless oil (12.8 g, 48%). HR MS (C11H19BrO2): Obs mass, 262.0565. Calcd mass, 262.0568 (M+).

### Example 169. Preparation of 1-(4-cyanobutyl)cyclopentane carboxylic acid methyl ester

A solution of 1-(4-bromobutyl)cyclopentane carboxlic acid methyl ester was treated with a solution of 0.5 molar lithium cyanide in DMF (5 mmol, 10 mL) and stirred overnight at room temperature followed by heating at 75 °C for two hrs. The reaction mixture was evaporated to dryness, the residue was dissolved in ethyl acetate (30mL) and washed with sat'd sodium bicarbonate (2 x 10 mL), and brine (5 mL) and dried over magnesium sulfate to give a yellow oil (860 mg). Silica gel chromtography eluting with 1:3 ethyl acetate:hexane yielded a pale oil (83%, 693 mg). LR MS (C12H19NO2): Obs mass, 227 (M+NH4), 248 (M+K).

### Example 170. Preparation of 1-(3-bromopropyl)cyclopentane carboxylic acid methyl ester

To a solution of 1.5 molar LDA in cyclohexane (60 mmol, 40 mL) cooled to -70 °C was added a solution of cyclopentane carboxylic acid methyl ester (42 mmol, 5.6 g) in THF (15 mL) over 30 min. The mixture was stirred for 1 hr at -70 °C. Then a solution of 1,3-dibromopropane (60 mmol, 11.9 g) in THF (30 mL) which had been pre-cooled to -70 °C was added all at once and the reaction mixture was stirred at -70 °C for one hr then at room temperature overnight. The reaction mixture was poured into brine (200 mL), the layers were separated, the aqueous layer was extracted with ether (3 x 30 mL) and the combined organic layers were washed with brine (20 mL), dried (magnesium sulfate) and concentrated to give a yellow oil (15 g). Distillation through a short path apparatus with a 3" vigreux column gave a yellow oil, 7.96g (76%), bp 76-80°C at 0.3mm.

Example 171. 1-(3-Cyanopropyl)cyclopentane carboxylic acid methyl ester was prepared from 1-(3-bromopropyl)cyclopentane carboxylic acid methyl ester using the general method described in example 169 to give a 79% yield of a pale yellow oil. LR MS (C11H17NO2): Calcd mass, 196. Obs mass, 196 (M+H).

### Example 172. Preparation of 1-[4-(methylthio)butyl]cyclopentane carboxylic acid methyl ester.

(29514-112).

To a solution of 1-(4-bromobutyl)cyclopentane carboxylic acid methyl ester (38 mmol, 10 g) in DMF (100 mL) was added sodium thiomethoxide (72.6 mmol, 5.09 g). After addition of sodium thiomethoxide, the reaction was exothermic and the mixture became a light brown cloudy solution. The mixture was stirred for 15 h at room temperature and was poured into water (200 mL). The organic compound was extracted with ether (2 x 150 mL). The combined extracts were washed with brine (150 mL) and dried over anhydrous magnesium sulfate. After filtration of the drying agent, the solution was concentrated under vacuum and the resulting residue was purified by silica gel column chromatography to afford 4.43 g (51%) of a colorless oil. LR MS (C12H22O2S): 230 (M+).

Example 173. 1-(3-Methylthiopropyl)cyclopentane carboxylic acid methyl ester was prepared from 1-(3-bromopropyl)cyclopentane carboxylic acid methyl ester using the general procedure described in example 172 to give a 54% yield of a pale yellow oil.

### Example 174. Preparation of 1-[(4-(methylsulfonyl)butyl]cyclopentane carboxylic acid methyl ester

To a solution of 1-[4-(methylthio)butyl]cyclopentane carboxylic acid methyl ester (19.2 mmol, 4.43 g) in AcOH (20 mL) was added 30% hydrogen peroxide (10 mL). The reaction mixture was heated to 70 °C and stirred for 15 h at which time the TLC of the mixture indicated the absence of starting material. The reaction mixture was cooled to room temperature and was concentrated under vacuum. The residue was poured into saturated sodium bicarbonate solution and was extracted with ether (3 x 100 mL). The combined extracts were washed with a saturated solution of sodium chloride (200 mL) and dried over anhydrous magnesium sulfate. After filtration of the drying agent, the solvent was removed under vacuum and the resulting residue was purified by silica gel column chromatography to afford 4.94 g (98%) as a colorless oil. LR MS (C12H22O4S): 263 (M+H).

### Example 175. Preparation of 1-[4-(methylsulfonyl)butyl]cyclopentane carboxylic acid

To a solution of 1-[4-(methylsulfonyl)butyl]cyclopentane carboxylic acid methyl ester (18.8 mmol, 4.94 g) in a mixture of THF (38 mL) and methanol (38 mL) was added 1 N sodium hydroxide (38 mL). The mixture was heated to 50-55 °C for 15 h at which point TLC analysis of the reaction mixture indicated the absence of starting material and the mixture was allowed to cool to room temperature. The solvent was removed under vacuum and the residue was diluted with water (100 mL) and extracted with ether (2 x 50 mL) to remove any neutral impurities. Then, the basic aqueous layer was acidified with 1 N hydrochloric acid and the product was extracted with ethyl acetate (2 x 75 mL). The combined extracts were washed with brine solution and dried over anhydrous sodium sulfate. After filtration of the drying agent, the solution was concentrated under vacuum and the residue was dried under high vacuum to afford 4.31 g (92%) of the title compound as a low melting white solid. LR MS (C11H20O4S): 249 (M+H).

### Example 176. Preparation of 1-(2-bromoethyl)cyclopentane carboxylic acid methyl ester

To a solution of diisopropylamine (150 mmol, 21 mL) in THF (100 mL) was added dropwise a solution of n-butyl lithium (145 mmol, 58 mL 2.5M) in hexanes at -10 °C while maintaining the temperature below 0 °C. After addition, the solution was stirred for 30 min at 0 °C. To this, a solution of cyclopentane carboxylic acid methyl ester (100 mmol, 13.1 g) in THF (20 mL) was added dropwise at -70 °C maintaining the internal temperature between -60 to -70 °C. After addition, the reaction mixture was stirred for 1 h at -50 to -60 °C. Then, a solution of 1,2-dibromoethane (90 mmol, 16.91 g) in THF (20 mL) was added dropwise and the light brown suspension was stirred for 1 h at -60 to -70 °C. Then, it was allowed to warm to room temperature and was stirred overnight. The reaction mixture was poured into a saturated solution of ammonium chloride (200 mL) and the organic compound was extracted into ether (2 x 100 mL). The combined extracts were washed with a saturated solution of sodium chloride (150 mL) and dried over anhydrous magnesium sulfate. After filtration of the drying agent, the solution was concentrated under vacuum and the resulting residue was distilled at 95-105 °C/2.5 mm Hg to obtain 11.5 g (49%) of a colorless oil.

### Example 177. Preparation of 1-[2-(4-morpholino)ethyl]cyclopentane carboxylic acid methyl ester.

To a solution of 1-(2-bromoethyl)cyclopentane carboxylic acid methyl ester (2 mmol, 0.47 g) in DMF (10 mL) was added sodium iodide (0.3 mmol, 45 mg) and morpholine (10 mmol, 0.87 g). The reaction mixture was stirred for 3 days at room temperature at which time the TLC of the mixture indicated the absence of starting material. The mixture was diluted with ethyl acetate (100 mL) and washed successively with water (2 x 50 mL) and a saturated solution of sodium chloride (100 mL) and was dried over anhydrous magnesium sulfate. After filtration of the drying agent, the solution was concentrated under vacuum to afford 0.44 g (92%) of a colorless oil. HR MS (C13H23NO3): Obs mass, 241.1675. Calcd mass, 241.1678 (M+).

### Example 178. Preparation of 1-[2-(4-morpholino)ethyl] cyclopentane carboxylic acid.

To a solution of 1-[2-(4-morpholino)ethyl]cyclopentane carboxylic acid methyl ester (1.75 mmol, 0.42 g) in a mixture of THF (5 mL) and methanol (5 mL) was added 1 N sodium hydroxide (3.5 mL). The mixture was heated to 50-55 °C for 40 h at which point TLC analysis of the reaction mixture indicated the absence of starting material and the mixture was allowed to cool to room temperature. The solvent was removed under vacuum and the residue was diluted with water (100 mL) and extracted with ether (2 x 50 mL) to remove any neutral impurities. Then, the basic aqueous layer was neutralized with 1 N hydrochloric acid and extracted with ethyl acetate (2 x 75 mL). The aqueous layer was neutralized with saturated sodium carbonate solution and extracted with ethyl acetate (3 x 50 mL). TLC of the aqueous layer indicated the presence of some more product. Thus, all ethyl acetate extracts were combined with aqueous layer and concentrated. The solid residue was triturated with methanol. The undissolved solids were filtered and the filtrate was concentrated under vacuum. The resulting solid was dissolved again in methanol and concentrated HCl was added to form a salt. Then, the methanol was removed to obtain the HCl salt of 1-[2-(morpholino)ethyl]cyclopentane carboxylic acid (1.09 g, may contain some NaCl) as a white solid. LR MS (C12H21NO3): 228 (M+H).

### Example 179. Preparation of methyl 1-(3,3 difluoro-2-propylene)cyclopentane carboxylate

A solution of 0.89 M LDA (0.24 mmol, 27 mL) was prepared from diisipropylamine (75 mmol, 7.58 g) in THF (50 mL) and 2.5 M n-butyl lithium in hexane (72 mmol, 29 mL). The solution was cooled to -70 °C and cycylopentane carboxylic acid methyl ester (15 mmol, 1.92 g) in THF (10 mL) was added dropwise over 20 min while maintaining the temperature at -70 °C. The mixture was stirred an additional 1 h at -70 °C and a solution of trifluoropropyl bromide (15 mmol, 2.65 g) in THF (10 mL) was added over 15 min.. The reaction mixture was stirred 1 h at -70 °C and allowed to warm to room temperature overnight. The reaction mixture was poured into brine (150 mL) and the organic layer was separated. The aqueous layer was extracted with ether (30 mL), the combined organic layers were washed with brine (10 mL), dried over anhydrous magnesium sulfate and evaporated to dryness to give a yellow oil ((4.1 g) which gave after chromotography on silica gel (120 g, 10% ethyl acetate in hexane) pale yellow oil (56%, 1.72 g).

### Example 180. Preparation of 5-Iodo-2-pentanone ethylene ketal.

A solution of 5-chloro-2-pentanone (40 mmol, 6.59 g) in acetone (40 mL) was treated with NaI (60 mmol, 9 g) and refluxed overnight. After cooling to room temperature, the solids were filtered off and the supernatant was concentrated to a dark gummy solid. A 1:1 mixture of ether and pet. ether (20 mL) was added, the mixture was stirred for 30 min., filtered and evaporated to dryness to give 7.2 g of a red oil which upon distillation (34-36° at 0.3mm) gave 5 g of the ketone. To a solution of this ketone (23.6 mmol, 5 g) in toluene (40 mL) in a 100 mL flask fitted with a Dean-Stark trap was added ethylene glycol ( 27 mmol, 1.67 g ) and 100 mg of *para*-toluenesulfonic acid and the reaction mixture was reluxed 6 h. After cooling, the toluene solution was washed with 1N NaOH (20 mL), water (5x 20mL), and brine (5 mL) was dried over potassium carbonate, filtered and evaporated to dryness to yield, upon distillation, 4.8 g (47%) of 5-Iodo-2-pentanone ethylene ketal as a colorless oil bp 44-48 °C at 0.3 mm.

### Example 181. Preparation of 1-(4-(ethylenedioxy)pentyl)cyclopentane carboxylic acid methyl ester

A solution of lithium diisopropylamide•tetrahydrofuran 1.5M solution in cyclohexane (15 mmol, 10 mL) was cooled to -70 °C, and a solution of cyclopentane carboxylic acid methyl ester (10 mmol, 1.28 g) in THF (10 mL) was added dropwise over 15 min, maintaining the internal temperature at -60 to -70 °C. The yellow solution was stirred 1 h at -70 °C and a solution of 5-iodo-2-pentanone ethylene ketal (10 mmol, 1.28 g) in THF (10 mL) was added over 15 min, while maintaining temperature of -70 °C . After srirring for 1 h at -70°C, the reaction mixture was allowed to warm to room temperature overnight. The reaction mixture was poured into brine (150 mL) and the organic layer was separated. The aqueous layer was extracted with ether (20 mL), the combined organic layers were washed with brine (10 mL), dried over anhydrous magnesium sulfate and evaporated to dryness to give 2.5 g (97%) of a pale yellow oil.

### Example 182. Preparation of 4-Iodo-2-butanone ethylene ketal.

A solution of 4 bromo-2-butanone ethylene ketal (16.4 mmol,3.2 g) in acetone (30 mL) was treated with sodium iodide (24 mmol, 3.7 g) and sodium carbonate (50 mmol, 5.1 g) and was reluxed overnight. The resulting mixture was filtered, washed with acetone (10 mL) and evaporated to dryness to a white solid. The residue was triturated with a mixture of 1:1 ether: pet ether (20 mL), stirred 30 min., filtered and evaporated to give 3.74 g ( 94%) of a pale yellow oil. LR-ES (C13H22O4): 243 (M+H).

Example 183. 1-[3-(Ethylenedioxy)butyl]cyclocarboxylic acid methyl ester was prepared using the general method described in example 181 to give a 25% yield of a colorless oil. LR-ES MS (C13H22O4): 243 (M+H).

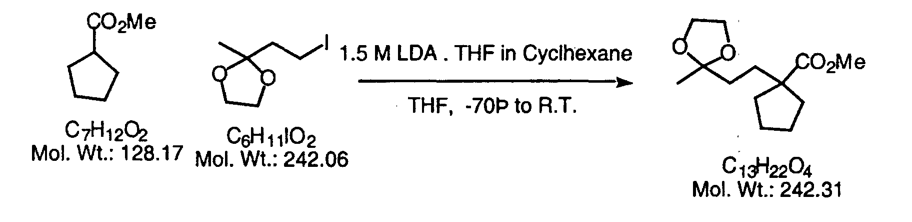

### Example 184. Preparation of 4-[[(2,6-dichlorophenyl)carbonyl]amino]-N-[[1-[3-(methylsulfonyl)propyl]cyclopentyl]carbonyl]-L-phenylalanine methyl ester

A solution of 4-[[(2,6-dichlorophenyl)carbonyl]amino]-N-[[1-[3-[(methylthio)propylcyclopentyl] carbonyl]-L-phenylalanine methyl ester (110 mg, 0.2 mmol) in dichloromethane (10 mL) was cooled in ice bath and treated with meta-chloroperbenzoic acid ( 0.7 mmol, 150 mg). After stirring at room temperature for 3 h, the reaction mixture was diluted with dichloromethane (30 mL) and washed with sat'd sodium bicarbonate (10 mL), and brine (5 mL) and dried over magnesium sulfate. Evaporation to a yellow oil and silica gel chromtography eluting with 5% methanol in dichloromethane yielded a white pasty solid (96%, 113 mg). LR-ES MS (C27H32N2O6Cl2S): 583 (M+H).

### Example 185. Preparation of 4-[[(2,6-dichlorophenyl)carbonyl]amino]-N-[[1-[3-[(methy sulfinyl)propyl]cyclopentyl]carbonyl]-L-phenylalanine methyl ester

A solution of 4-[[(2,6-dichlorophenyl)carbonyl]amino]-N-[[1-[3-(methylthio)propyl]cyclopentyl] carbonyl]-L-phenylalanine methyl ester (110 mg, 0.2 mmol) in a mixture of ethyl acetate (8 mL) and THF (3 mL) was treated with a solution of oxone (0.05 mmol, 31 mg) in water (2 mL) and the two phase system was stirred vigorously for 2 h at room temperature followed by further addition of oxone (0.05 mmol, 31 mg) and continued stirring overnight. After separation of the layers, the aqueous phase was extracted with ethyl acetate (5 mL), and the combined organic layers were washed with brine (3 mL), dried over magnesium sulfate and evaporated to dryness to give a white solid. Chromotography on silica gel eluting with methanol (7.5%) in dichloromethane gave a white solid (68%, 78 mg). LR-ES MS (C27H32N2O5Cl2S): 567 (M+H).

### Example 187. Preparation of 4-[[(2,6-dichlorophenyl)carbonyl]amino]-N-[[1-[2-[[(methylamino)carbonyl]amino]ethyl]cyclopentyl]carbonyl]-L-phenylalanine.

To a solution of 4-[[(2,6-dichlorophenyl)carbonyl]amino]-N-[[1-(2-aminoethyl)cyclopentyl] carbonyl]-L-phenylalanine methyl ester (40 mg, 0.080 mmol) in dichloromethane (1 mL) was added methyl isocyanate (5 mg, 0.095 mmol). The resultant mixture was stirred for 18 h. The reaction mixture was *concentrated in vacuo* and the crude urea was used in the next step without purification.

To a solution of the crude methyl ester (50 mg, 0.080 mmol) in MeOH (1 mL) was added a solution of LiOH (8 mg, 0.19 mmol) in water (0.5 mL). The mixture was stirred for 2 h and then it was acidified (pH -1-2) with 0.5M HCl. The reaction mixture was poured into a round bottom flask and concentrated in *vacuo.* Purification by reversed-phase HPLC, using a 15-95% acetonitrile-water gradient over 25 min., provided 30 mg (68%). HR MS (C26H30Cl2N4O5): Calcd mass, 549.1671. Obs mass, 549.1677 (M+H).

Examples 188 to 191. Using the general procedure described in example 187, and starting with 4-[[(2,6-dichlorophenyl)carbonyl]amino]-N-[[1-(2-aminoethyl)cyclopentyl]carbonyl]-L-phenylalanine methyl ester, the analogues listed below were prepared:

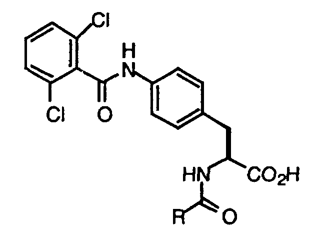

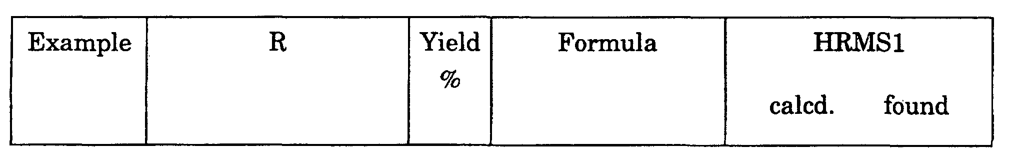

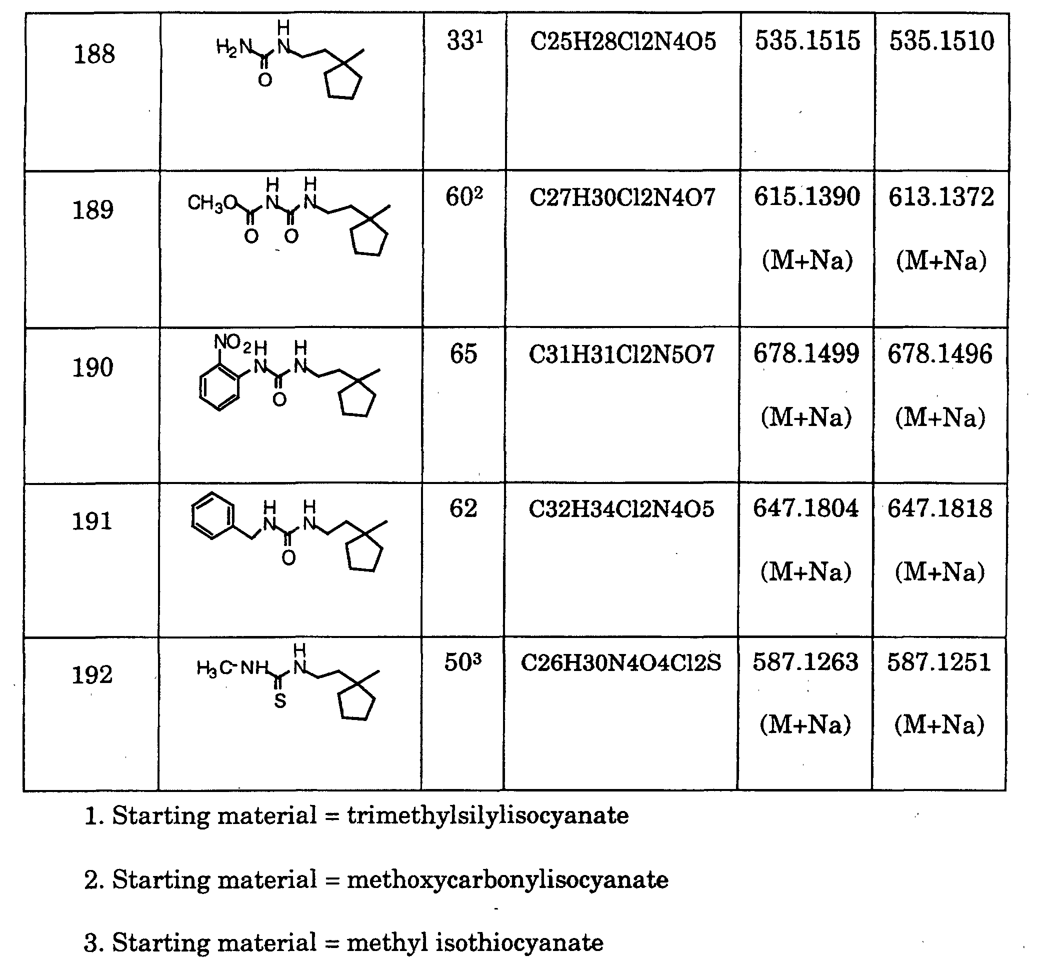

### Example 193. 4-[[(2,6-dichlorophenyl)carbonyl]amino]-N-[[2-[[(methoxy)carbonyl]amino]ethyl]cyclopentyl]carbonyl]-L-phenylalanine

To a solution of 4-[[(2,6-dichlorophenyl)carbonyl]amino]-N-[[1-(2-aminoethyl)cyclopentyl] carbonyl]-L-phenylalanine methyl ester (40 mg, 0.080 mmol) in dichloromethane (1 mL) was added diisopropylethylamine (20 mg, 0.16 mmol) and methyl chloroformate (7 mg, 0.080 mmol). The resultant mixture was stirred for 18 h. The reaction mixture was concentrated *in vacuo* and the crude carbamate was used in the next step without purification.

To a solution of the crude methyl ester (54 mg, 0.080 mmol) in MeOH (1 mL) was added a solution of LiOH (8 mg, 0.19 mmol) in water (0.5 mL). The mixture was stirred for 2 h and then it was acidified (pH ∼1-2) with 0.5M HCl. The reaction mixture was poured into a round bottom flask and concentrated *in vacuo.*

Purification by reversed-phase HPLC, using a 15-95% acetonitrile-water gradient over 25 min., provided 25 mg (57%). HR MS (C26H29N3O6Cl2): Calcd mass, 550.1511. Obs mass, 550.1524 (M+H).

### Example 194. Preparation of 4-[[(2,6-dichlorophenyl)carbonyl]amino]-N-[[1-[2-[(methylsulfonyl)amino] ethyl] cyclopentyl)carbonyl]-L-phenylalanine.

To a solution of 4-[[(2,6-dichlorophenyl)carbonyl]amino]-N-[[1-(2-aminoethyl)cyclopentyl] carbonyl]-L-phenylalanine methyl ester (40 mg, 0.080 mmol) in dichloromethane (1 mL) was added diisopropylethylamine (12 mg, 0.095 mmol) and methanesulfonyl chloride (9 mg, 0.080 mmol). The resultant mixture was stirred for 18 h. The reaction mixture was *concentrated in vacuo* and the crude sulfonamide was used in the next step without purification.

To a solution of the crude methyl ester (55 mg, 0.080 mmol) in MeOH (1 mL) was added a solution of LiOH (8 mg, 0.19 mmol) in water (0.5 mL). The mixture was stirred for 2 h and then it was acidified (pH -1-2) with 0.5M HCl. The reaction mixture was poured into a round bottom flask and concentrated *in vacuo.* Purification by reversed-phase HPLC, using a 15-95% acetonitrile-water gradient over 25 min., provided 28 mg (61%). HR MS (C25H29N3O6Cl2S): Calcd mass, 592.1052. Obs mass, 592.1068 (M+Na).

### Example 195. Preparation of 1-[2-[[(1,1-dimethylethoxy)carbonyl]amino]ethyl]cyclopentyl carboxylic acid methyl ester.

To a solution of 1-(2-azidoethyl)cyclopentane carboxylic acid methyl ester (5.00 g, 25.4 mmol) in ethyl acetate (100 mL) was added di-tert-butyl-dicarbonate (55.4 g, 254 mmol) and 10% Pd on carbon (1.5 g). The reaction mixture was shaken for 3 h under hydrogen gas (50 psi) on a Parr shaker apparatus. The reaction mixture was filtered through a pad of celite and the pad was washed with ethyl acetate (2 x 150 mL). The combined organic phase was transferred to a round bottom flask and concentrated *in vacuo.* Purification by flash column chromatography, using hexane-ethyl acetate (9:1), afforded 5.30 g (76%) as a light yellow oil. HR MS (C14H25NO4): Calcd: 272.1862. Obs mass, 272.1856 (M+H).

### Example 196. Preparation of 1-[2-[[(1,1-dimethylethoxy)carbonyl]amino]ethyl]cyclopentane carboxylic acid.

To a solution of the 1-[2-[[(1,1-dimethylethoxy)carbonyl]amino]ethyl]cyclopentane carboxylic acid methyl ester (10.6 g, 39.0 mmol) in THF/MeOH (3:1, 40 mL) was added a solution of LiOH ( 4.20 g, 98.0 mmol) in water (10 mL). The mixture was stirred for 2.5 h at 50 °C and then cooled to room temperature. The reaction mixture was poured into a round bottom flask and *concentrated in vacuo.* The mixture was diluted with H₂O (60 mL) and acidified with 1M HCl. The aqueous phase was extracted with ethyl acetate (3 x 200 mL) and the combined organic layer was dried over MgSO₄, filtered and concentrated *in vacuo.* Purification by flash column chromatography, using hexane-ethyl acetate (3:1), afforded 5.80 g (58%) as a white powder. HR MS (C13H23NO4): Calcd mass, 258.1705. Obs mass, 258.1700 [(M+H).

### Example 197. Preparation of 1-[2-[[(1,1-dimethylethoxy)carbonyl](methyl)amino]ethyl] cyclo-pentane carboxylic acid methyl ester.

To a slurry of NaH (1.20 g, 47.5 mmol) in DMF (20 Obs mass, mL) at 0 °C was added dropwise a solution of 1-[2-[[(1,1-dimethylethoxy)carbonyl]amino]ethyl]cyclopentane carboxylic acid (5.8 g, 22.6 mmol) in THF (25 mL). The resultant mixture was stirred for 1 h at 0 °C and methyl iodide (3.3 Obs mass, mL, 52.3 mmol) was added dropwise. The reaction mixture was stirred for 1 h at 0 °C and 5h at room temperature. The reaction was quenched with sat. ammonium chloride (20 Obs mass, mL) solution and transferred to a separatory funnel. The aqueous phase was extracted with ethyl acetate (3x100 mL) and the combined organic layer was dried over MgSO₄, filtered and concentrated *in vacuo.* Purification of the residue by flash column chromatography, using hexane-ethyl acetate (9:1), afforded 5.40 g (84%). HR MS (C15H27NO4): Calcd: 286.2018. Obs mass, 286.2021 (M+H).

### Example 198. Preparation of 1-[2-[[(1,1-dimethylethoxy)carbonyl](methyl)amino]ethyl] cyclopentane carboxylic acid.

To a solution of 1-[2-[[(1,1-dimethylethoxy)carbonyl](methyl)amino]ethyl]cyclopentane carboxylic acid methyl ester (4.00 g, 14.0 mmol) in THF/MeOH (2:1, 24 mL) was added a solution of LiOH (1.50 g, 35.0 mmol) in water (8 mL). The mixture was stirred overnight at 50 °C and then cooled to room temperature. The reaction mixture was poured into a round bottom flask and concentrated *in vacuo.* The mixture was diluted with H₂O (50 mL) and acidified with 1M HCl. The aqueous phase was extracted with ethyl acetate (3x150 mL) and the combined organic layer was dried over MgSO₄, filtered and concentrated *in vacuo.* Purification by flash column chromatography, using hexane-ethyl acetate (5:1), afforded (3.60 g, 95%) of a white powder. HR MS (C14H25NO4): Calcd mass, 272.1862. Obs mass, 272.1872 (M+H).

Example 199. Preparation of 4-[[(2,6-dichlorophenyl)carbonyl]amino]-N-[[1-[2-(methylamino) ethyl]cyclopentyl]carbonyl]-L-phenylalanine methyl ester was prepared by coupling of 4-[[(2,6-dichlorophenyl)carbonyl]amino]-L-phenylalanine methyl ester as prepared in example 45 with the product from example 198 using the general procedure described in example 146. The protecting Boc group was removed from the product by treatment with 4 N HCl in dioxane as described in example 45 and the product was used as is in subsequent steps.

### Example 200. Preparation of 4-[[(2,6-dichlorophenyl)carbonyl]amino]-N-[[1-[2-[(acetyl)(methyl) amino]ethyl]cyclopentyl]carbonyl]-L-phenylalanine.

Acetylation of the product of example 199 using the general procedure described in example 148 followed by ester hydrolysis using the general procedure described in example 47 gave the title compound in 75% yield. HR MS (C27H31Cl2N3O5): Calcd mass, 548.1719. Obs mass, 548.1716 (M+H).

### Example 201. Preparation of 4-[[(2,6-dichlorophenyl)carbonyl]amino]-N-[[1-[2-[[ (methylamino)carbonyl)(methyl)amino]ethyl]cyclopentyl]carbonyl]-L-phenylalanine.

Reaction of the product of example 199 with methyl isocyanate using the general procedure described in example 187 followed by ester hydrolysis using the general procedure described in example 47 gave the title compound in 69% yield. HR MS (C27H32Cl2N4O5): Calcd mass, 563.1828. Obs mass, 563.1816 (M+H).

### Example 202. Preparation of 4-[[(2,6-dichlorophenyl)carbonyl]amino]-N-[[1-[2-[(methoxycarbonyl)-(methyl)amino]ethyl]cyclopentyl]carbonyl]-L-phenylalanine.

Reaction of the product of example 199 with methyl chloroformate using the general procedure described in example 193 followed by ester hydrolysis using the general procedure described in example 47 gave the title compound in 70% yield. HR MS (C₂₇H₃₁Cl₂N₃O₆): Calcd mass, 586.1488. Obs mass, 586.1465 (M+Na).

### Example 203. 4-[[(2,6-dichlorophenyl)carbonyl]amino]-N-[[1-[2-[( trifluoroacetyl)amino] ethyl] cyclopentyl] carbonyl]-L-phenylalanine.

To a solution of 4-[[(2,6-dichlorophenyl)carbonyl]amino]-N-[[1-(2-aminoethyl)cyclopentyl] carbonyl]-L-phenylalanine (0.041 mmol, 20 mg) in DMF (2 mL) was added 2 equiv. of trifluoroacetic anhydride (0.082 mmol, 17.2 mg) at room temperature. The mixture was stirred for 15 h at room temperature at which time HPLC analysis of the reaction mixture indicated the absence of staring material. Then, without any work-up, it was purified by reverse phase HPLC to afford 5.7 mg (21%) of a white solid. HR MS: (C26H26Cl2N3O5): Obs. mass, 588.1280. Calcd. mass, 588.1269 (M+H).

### Example 204. Preparation of 4-[[(2,6-dichlorophenyl)carbonyl]amino]-N-[[1-[2-(dimethylamino)ethyl]cyclopentyl]carbonyl]-L-phenylalanine methyl ester.

To a mixture of N-[[1-(2-aminoethyl)cyclopentyl]carbonyl]-4-[[(2,6-dichlorophenyl)carbonyl]amino]-L-phenylalanine methyl ester (0.339 mmol, 0.17 g), zinc chloride (1.36 mmol, 0.185 g) and paraformaldehyde (1.36 mmol, 40.7 mg) was added dichloromethane (2 mL) at room temperature and the mixture was stirred for 1.5 h. Then, sodium borohydride (1.36 mmol, 51.3 mg) was added and the resulting mixture was stirred for 15 h at room temperature. The mixture was poured into NH₄OH (10 mL) and extracted with dichloromethane (2 x 20 mL). The combined organic layer was washed with brine solution (20 mL) and dried over anhydrous magnesium sulfate. Filtration of the drying agent and removal of the solvent gave a crude product which was purified by HPLC to afford 18 mg (10%) of a light yellow solid. HR MS (C27H33Cl2N3O4): Obs. mass, 534.1927. Calcd. mass, 534.1926 (M+H).

### Example 205. Preparation of 4-[[(2,6-dichlorophenyl)carbonyl]amino]-N-[[1-[2-[[(4-methoxyphenyl)carbonyl]amino]ethyl]cyclopentyl]carbonyl]-L-phenylalanine methyl ester.

To a solution of 4-[[(2,6-dichlorophenyl)carbonyl]amino]-N-[[1-(2-aminoethyl)cyclopentyl] carbonyl]-L-phenylalanine methyl ester (0.2 mmol, 101 mg) and 4-methoxybenzoyl chloride (0.25 mmol, 52.1 mg) in dichloromethane (1 mL) was added DIPEA (0.3 mmol, 38.7 mg) at room temperature. The reaction mixture was stirred for 15 h at room temperature and then diluted with 20 mL of dichloromethane. The dichloromethane layer was washed successively with water (20 mL) and brine solution (20 mL) and was dried over anhydrous magnesium sulfate. Filtration of the drying agent and concentration of the solvent afforded a crude product which was purified by revesrsed phase HPLC to obtain 0.1 g (78%) of a yellow syrup. HR MS (C33H35Cl2N3O6): Obs. mass, 662.1778. Calcd. mass, 662.1801 (M+Na).

Example 206. 4-[[(2,6-dichlorophenyl)carbonyl]amino]-N-[[1-[2-[[(3-trifluoromethylphenyl) carbonyl]amino]ethyl]cyclopentyl]carbonyl]-L-phenylalanine methyl ester was prepared from 4-[[(2,6-dichlorophenyl)carbonyl]amino]-N-[[1-(2-aminoethyl)cyclopentyl]carbonyl]-L-phenylalanine methyl ester and 3-trifluoromethylbenzoyl chloride using the general method described in example 205 to give a 99% yield of a white solid. HR MS C33H32Cl2N3O5): Obs. mass, 700.1596. Calcd. mass, 700.1569 (M+Na).

Example 207. 1-[4-(Azido)butyl]cyclopentane carboxylic acid methyl ester was prepared from 1-[4-(bromobutyl)]cyclopentane carboxylic acid methyl ester and sodium azide using the general procedure described in example 4 to give a syrup in 87% overall yield. HR MS (C11H19N3O2): Obs mass, 225.1523. Calcd mass, 225.1536 (M+).

Example 208. 1-[4-(Azido)butyl]cyclopentane carboxylic acid was prepared by hydrolysis of the ester prepared in example 207 using the general procedure described in example 15 to give a brown syrup in quantitative yield. HR MS (C10H17N3O2): Obs mass, 211.1285. Calcd mass, 211.1267 (M+).

Example 209. 1-(3-Azidopropyl)cyclopentane carboxylic acid was prepared from 1-(3-bromopropyl)cyclopentane carboxylic acid methyl ester using the general procedure described in example 4. The acid was isolated as an oil. LR ES MS (C9H15N3O2): 196.1 (M-H).

Example 210. 4-[[(2,6-dichlorophenyl)carbonyl]amino]-N-[[1-(4-azidobutyl)cyclopentyl] carbonyl]-L-phenylalanine methyl ester was prepared from 1-[4-(azido)butyl]cyclopentane carboxylic acid and of 4-[[(2,6-dichlorophenyl)carbonyl]amino]-L-phenylalanine methyl ester hydrochloride salt using the procedure described in example 46 to give a 99% yield of a white solid, mp 195-199 °C. HR MS (C27H31Cl2N5O4): Obs mass, 560.1833. Calcd mass, 560.1831 (M+H)

Example 211. 4-[[(2,6-dichlorophenyl)carbonyl]amino]-N-[[1-(4-aminobutyl)cyclopentyl] carbonyl]-L-phenylalanine methyl ester was prepared from 4- [[(2,6-dichlorophenyl)carbonyl]amino]-N-[[1-(4-azidobutyl)cyclopentyl]carbonyl]-L-phenylalanine methyl ester using the general procedure described in example 147 to give a 30% yield of a white solid. HR MS: (C27H33Cl2N3O4): Obs mass, 534.1352. Calcd mass, 534.1368 (M+H).

Example 212. 4-[[(2,6-dichlorophenyl)carbonyl]amino]-N-[[1-[4-[( acetyl)amino]butyl]cyclo pentyl]carbonyl]-L-phenylalanine methyl ester was prepared from 4-[[(2,6-dichlorophenyl)carbonyl]amino]-N-[[1-(4-aminobutyl)cyclopentyl]carbonyl]-L-phenylalanine methyl ester using the procedure described in example 148 in 80% overall yield as a white solid. HR MS (C29H35Cl2N3O5): Obs mass, 576.1690. Calcd mass, 576.1714 (M+H).

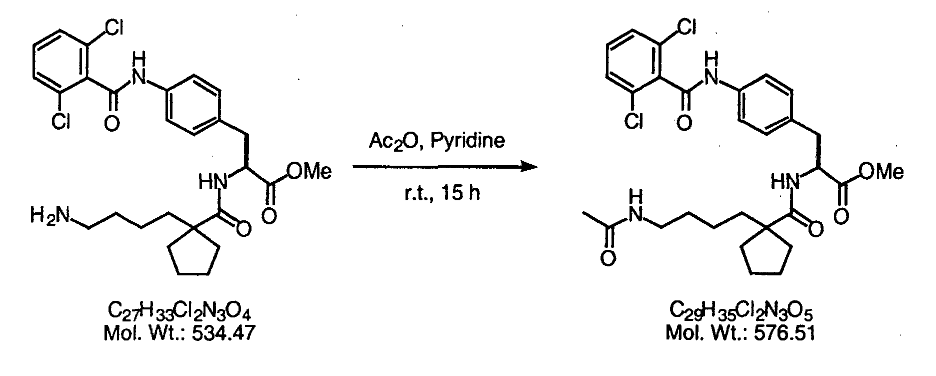

### Example 213. Preparation of 1-[(5-tetrazolyl)methyl]cyclopentane methyl ester.

To a solution of methyl 1-(1-cyanomethyl)cyclopentane carboxylate (5.5 mmol, 0.9 g) in toluene (15 mL) were added trimethylsilyl azide (11 mmol, 1.26 g) and dibutyltin oxide (0.55 mmol, 137 mg) at room temperature. The mixture was heated to 110 °C and stirred for 15 h. Then, the reaction mixture was cooled to room temperature and toluene was removed under vacuum. The brown residue was diluted with ethyl acetate (100 mL) and washed with saturated sodium bicarbonate solution (2 x 50 mL) and the starting material and some impurities remained in ethyl acetate. The aqueous sodium bicarbonate layer was neutralized with 3N HCl and extracted with ethyl acetate (2 x 50 mL). The combined extracts were washed with brine solution (50 mL) and dried over anhydrous magnesium sulfate. After filtration of the drying agent, the solution was concentrated under vacuum and the residue was dried under high vacuum to afford 0.31 g (27%) of a low melting white solid. HR MS (C9H14N4O2): Obs mass, 210.0218. Calcd mass, 210.0252 (M+).

### Example 214. Preparation of 1-[(1-tetrazolyl)methyl]cyclopentane carboxylic acid

Using the procedure described in example 15, 1-[(5-tetrazolyl)methyl]cyclopentane carboxylic acid was prepared from the corresponding ester in 67% overall yield as a white solid: mp 192-196 °C. HR MS (C8H12N4O2): Obs mass, 196.0329. Calcd mass, 196.0318 (M+).

### Example 215. Preparation of 4-[[(2,6-dichlorophenyl)carbonyl]amino]-N-[[1-(butyl)cyclopentyl]carbonyl]-L-phenylalanine methyl ester.

A mixture of 4-[[(2,6-dichlorophenyl)carbonyl]amino]-N-[[1-(3-butenyl)cyclopentyl]carbonyl]-L-phenylalanine methyl ester (0.19 mmol, 100 mg) and 10% palladium on carbon (200 mg) in EtOH (2 mL) was stirred under a hydrogen atmosphere at room temperature for 15 h. Then, the charcoal was filtered and washed with EtOH (10 mL). The filtrate was removed under vacuum to obtain 37.5 mg (37%) of as a white solid: mp 193-196 °C. HR MS: Obs. mass, 519.1818. Calcd. mass, 519.1817 (M+H).

Example 216. 4-[[(2,6-dichlorophenyl)carbonyl]amino]-N-[[1-[2-[[2-[[(1,1-dimethylethoxy) carbonyl]amino]-1-oxoethyl]amino]ethyl]cyclopentyl]carbonyl)-L-phenylalanine was prepared by coupling of the product from example 147 with Boc-glycine using the general HBTU protocol described in example 46, followed by treatment with NaOH to effect ester hydrolysis as described in example 47 to give a 75% yield. HR MS (C31H38Cl2N4O7): Calcd mass, 671.2016. Obs mass, 671.2002 (M+Na).

Example 217. 4-[[(2,6-dichlorophenyl)carbonyl]amino]-N-[[1-[2-[(2-amino-1-oxoethyl)amino]ethyl]yclopentyl]carbonyl]-L-phenylalanine was prepared by treatment of the product of example 216 with 4 N HCl in dioxane. HR MS (C26H30Cl2N4O5): Calcd mass, 571.1419. Obs mass, 571.1491 (M+Na).

Example 218. 1-(Cyanomethyl)cyclopentane carboxylic acid methyl ester was prepared from cyclopentane carboxylic acid methyl ester and chloroacetonitrile using the procedure described in example 7 to give a 38% yield. HR MS (C9H13NO2): Obs mass,
167.0173. Calcd mass, 167.0146 (M+).

Examples 219-229. Using the procedure described in example 15, the following cyclopentane carboxylic acids were prepared:

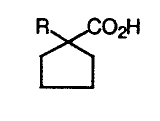

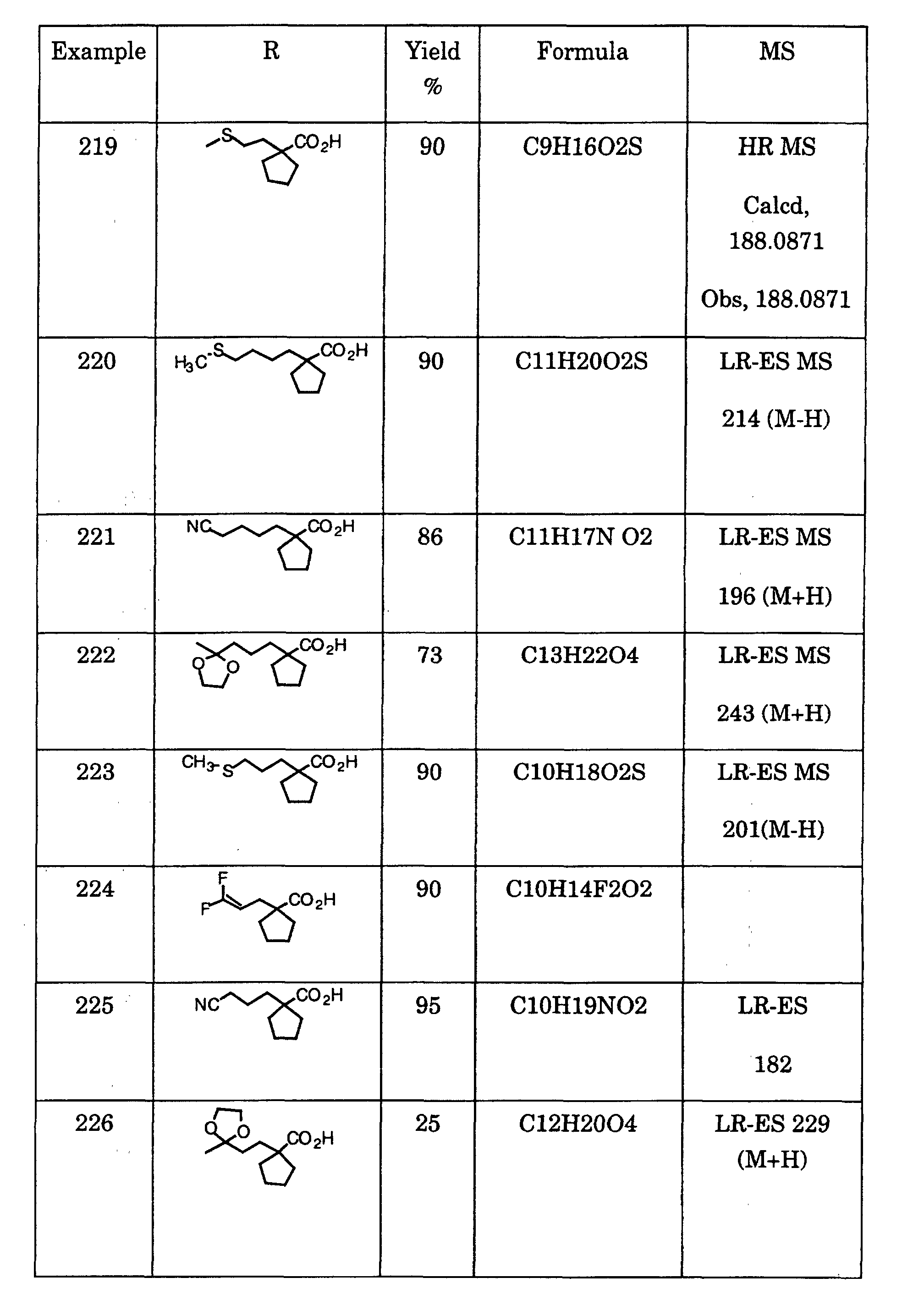

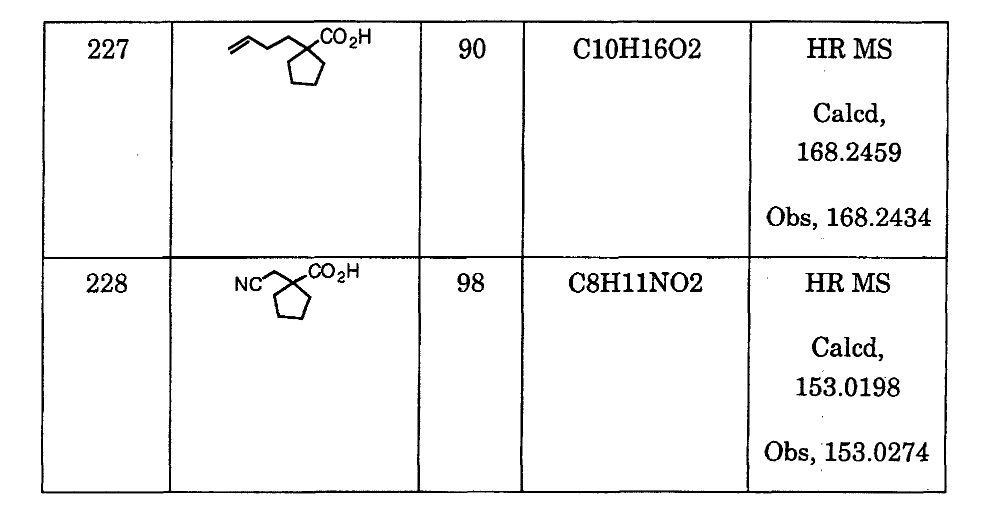

### Example 229. Preparation of methyl 1-(4-chlorobutyl)cyclopentane carboxylate.

1-(4-Chlorobutyl)cyclopentane carboxylic acid methyl ester was prepared from cyclopentane carboxylic acid methyl ester and 4-chloro-1-bromobutane using the procedure described in example 7 to give a 64%. yield. HR MS (C11H19ClO2): Obs. mass, 218.1072. Calcd. mass, 218.1074, (M+).

### Example 230. Preparation of 1-(3-butenyl)cyclopentane carboxylic acid methyl ester.

To a solution of diisopropylamine (225 mmol, 31.6 mL) in THF (150 mL) was added dropwise a solution of n-butyl lithium (217.5 mmol, 87 mL, 2.5M) in hexanes at -10 °C while maintaining the temperature below 0 °C. After addition, the solution was stirred for 30 min at 0 °C. To this, a solution of methyl cyclopentane carboxylate (150 mmol, 19.23 g) in THF (30 mL) was added dropwise at -70 °C maintaining the internal temperature between -60 to - 70 °C. After addition, the reaction mixture was stirred for 1 h at -50 to -60 °C. Then, a solution of 4-bromo-1-butene (142.2 mmol, 19.2 g) in THF (30 mL) was added dropwise and the light brown suspension was stirred for 1 h at -60 to -70 °C. Then, it was allowed to warm to room temperature and stirred overnight. The reaction mixture was poured into a saturated solution of ammonium chloride (250 mL) and the mixture was extracted with ether (2 x 150 mL). The combined extracts were washed with a saturated solution of sodium chloride (150 mL) and dried over anhydrous magnesium sulfate. After filtration of the drying agent, the solution was concentrated under vacuum and the residue was distilled at 63-67 °C/2.5 mm Hg to give 13.77 g (53%) of a colorless oil. HR MS (C11H16O2): Obs mass, 182.1311. Calcd mass, 182.1307 (M+).

### Example 231. Preparation of 1-[2-(methylthio)ethyl]cyclopentane carboxylic acid methyl ester.

To a solution of 1-(2-bromoethyl)cyclopentane carboxylic acid methyl ester (2.0 mmol, 472 mg) in DMF (5 mL) was added sodium thiomethoxide (2.65 mmol, 186 mg). The reaction mixture was stirred for 15 h at room temperature and was poured into water (30 mL). The organic compound was extracted into diethyl ether (2 x 20 mL). The combined extracts were washed with a saturated solution of sodium chloride (50 mL) and dried over anhydrous magnesium sulfate. After filtration of the drying agent, the solution was concentrated under vacuum and the resulting residue was purified by silica gel column chromatography to afford 334 mg (82%) of a colorless oil. HR MS C10H18O2S): Obs mass, 202.1024. Calcd mass, 202.1028 (M+).

### Example 232. Preparation of 1-[4-(methoxy)butyl]cyclopentane carboxylic acid.

To a solution of 1-(4-chlorobutyl)cyclopentane carboxylic acid methyl ester (30 mmol, 6.56 g) in a mixture of THF (60 mL) and methanol (60 mL) was added 1 N sodium hydroxide (60 mL). The mixture was heated to 40-45 °C for 15 h at which point TLC analysis of the reaction mixture indicated the absence of starting material and it was cooled to room temperature. The solvent was removed under vacuum and the residue was diluted with water (100 mL) and extracted with ether (2 x 100 mL) to remove any neutral impurities. Then, the basic aqueous layer was acidified with 1 N hydrochloric acid and the product was extracted with ethyl acetate (2 x 75 mL). The combined extracts were washed with brine solution and were dried over anhydrous sodium sulfate. After filtration of the drying agent, the solution was concentrated under vacuum and the residue was purified by silica gel column chromatography to afford 4.2 g (68%) of 1-(4-chlorobutyl)cyclopentane carboxylic acid as a liquid and 1.3 g (22%) of 1-[4-(methoxy)butyl]cyclopentane carboxylic acid as a viscous oil. HR MS (C11H20O3): Obs mass, 200.0175. Calcd mass, 200.0143 (M+).

Examples 233 - 248. Using the general coupling procedure described in example 46, the following analogues were prepared:

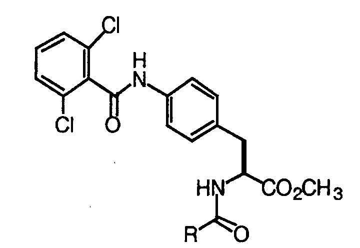

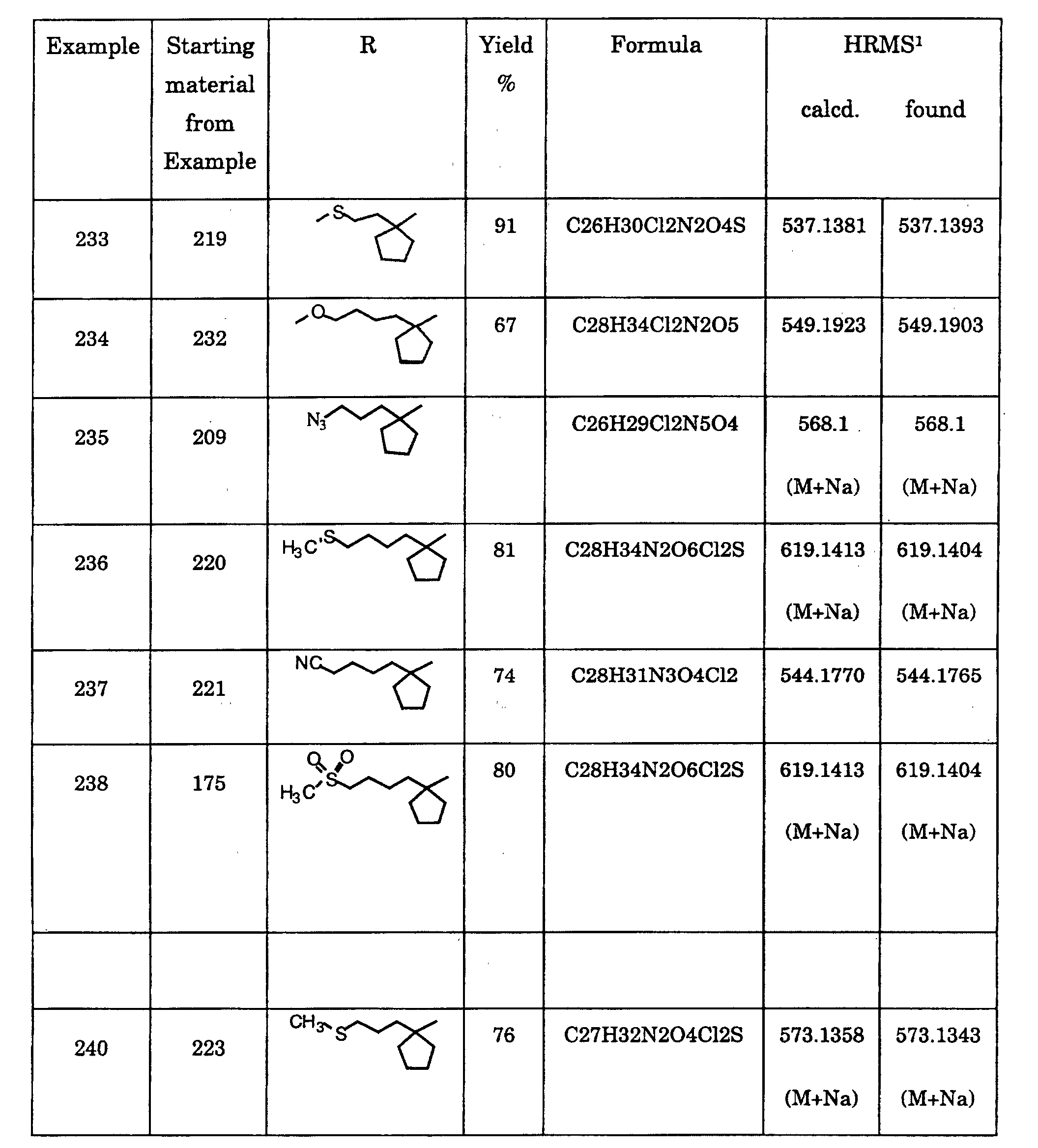

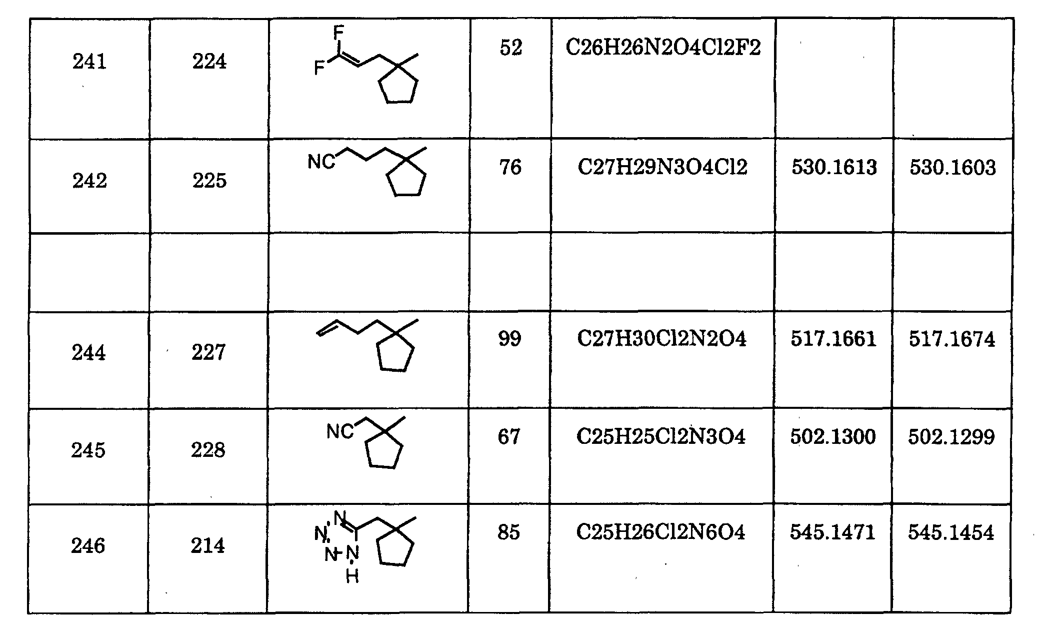

1. M+H ion unless otherwise indicated

Example 247. 4-[[(2,6-Dichlorophenyl)carbonyl]amino]-N-[[1-[(2-morpholinyl)ethyl] cyclopentyl)carbonyl)-L-phenylalanine methyl ester was prepared from 4-[[(2,6-Dichlorophenyl)carbonyl]amino]-L-phenylalanine methyl ester and 1-[(2-morpholinyl)ethyl)cyclopentane carboxylic acid using the general coupling procedure described in example 46 to provide a 62% yield. HRMS (C29H35Cl2N3O5): Obs. mass, 576.2531. Calcd. mass, 576.2582, (M+H).

### Example 248. Preparation of 4-[[(2,6-dichlorophenyl)carbonyl]amino]-N-[[1-(4-hydroxybutyl)cyclopentyl]carbonyl]-L-phenylalanine methyl ester.

To a suspension of 4-[[(2,6-dichlorophenyl)carbonyl]amino]-N-[[1-(3-butenyl)cyclopentyl] carbonyl]-L-phenylalanine methyl ester (3.13 mmol, 1.62 g) and anhydrous copper (I) chloride (5.0 mmol, 500 mg) in THF (30 mL) was added solid sodium borohydride (5.0 mmol, 200 mg) at -5 °C over 5 min. After the addition, the reaction mixture was allowed to warm to room temperature and the brown reaction mixture was stirred for 36 h at which time TLC analysis of the mixture indicated the absence of starting material. Then, the excess hydride was quenched by the addition of water (5 mL) and the reaction mixture was cooled to 0 °C. To this, a solution of sodium acetate (20 mL, 3.0N) was added dropwise maintaining the temperature below 10 °C this was followed by H₂O₂ (25 mL, 30%). After addition of hydrogen peroxide, the reaction mixture was allowed to warm to room temperature and was stirred for 3 h and followed by 1 h at 40-45 °C to complete the hydrolysis. Then, it was poured into a mixture of water (50 mL) and ethyl acetate (50 mL). The two layers were separated and the aqueous layer was extracted with ethyl acetate (2 x 30 mL). The comined extracts were washed with brine solution and dried over anhydrous magnesium sulfate. Filtration of the drying agent and removal of the solvent gave a crude product which was purified by silica gel column chromatography to afford 1.04 g (62%) of a white amorphous solid. HR MS (C27H32Cl2N2O5): Obs. mass, 535.1758. Calcd. mass, 535.1766, M+H).

Examples 249-274. The compounds shown below were prepared from the corresponding methyl esters according to the procedure given in example 47 .

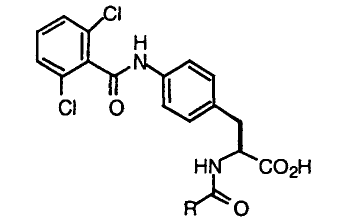

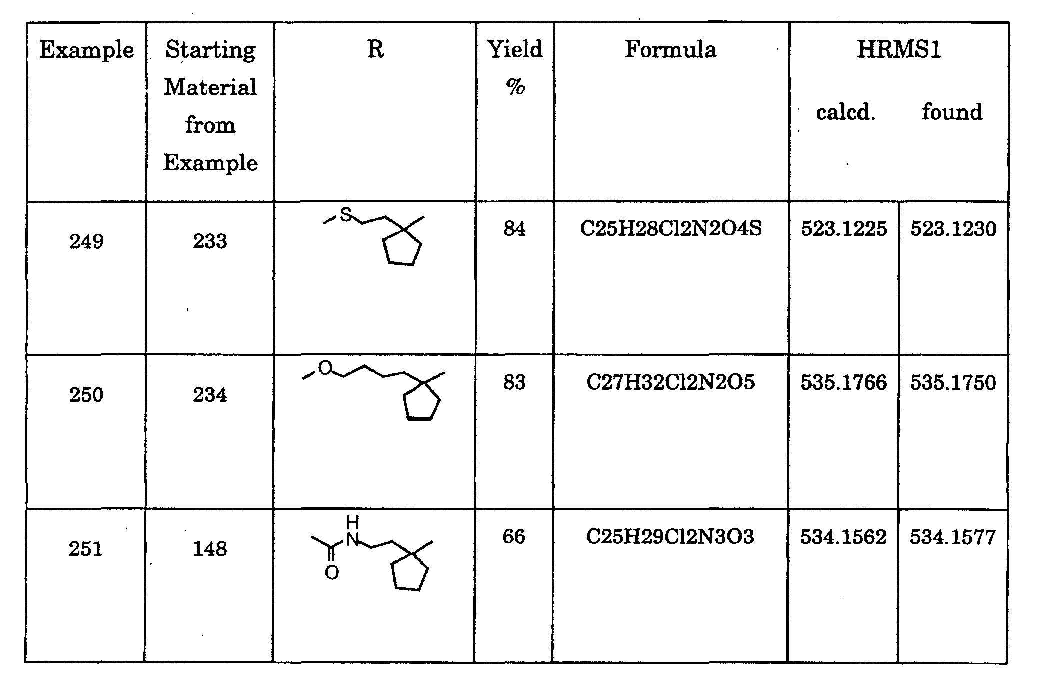

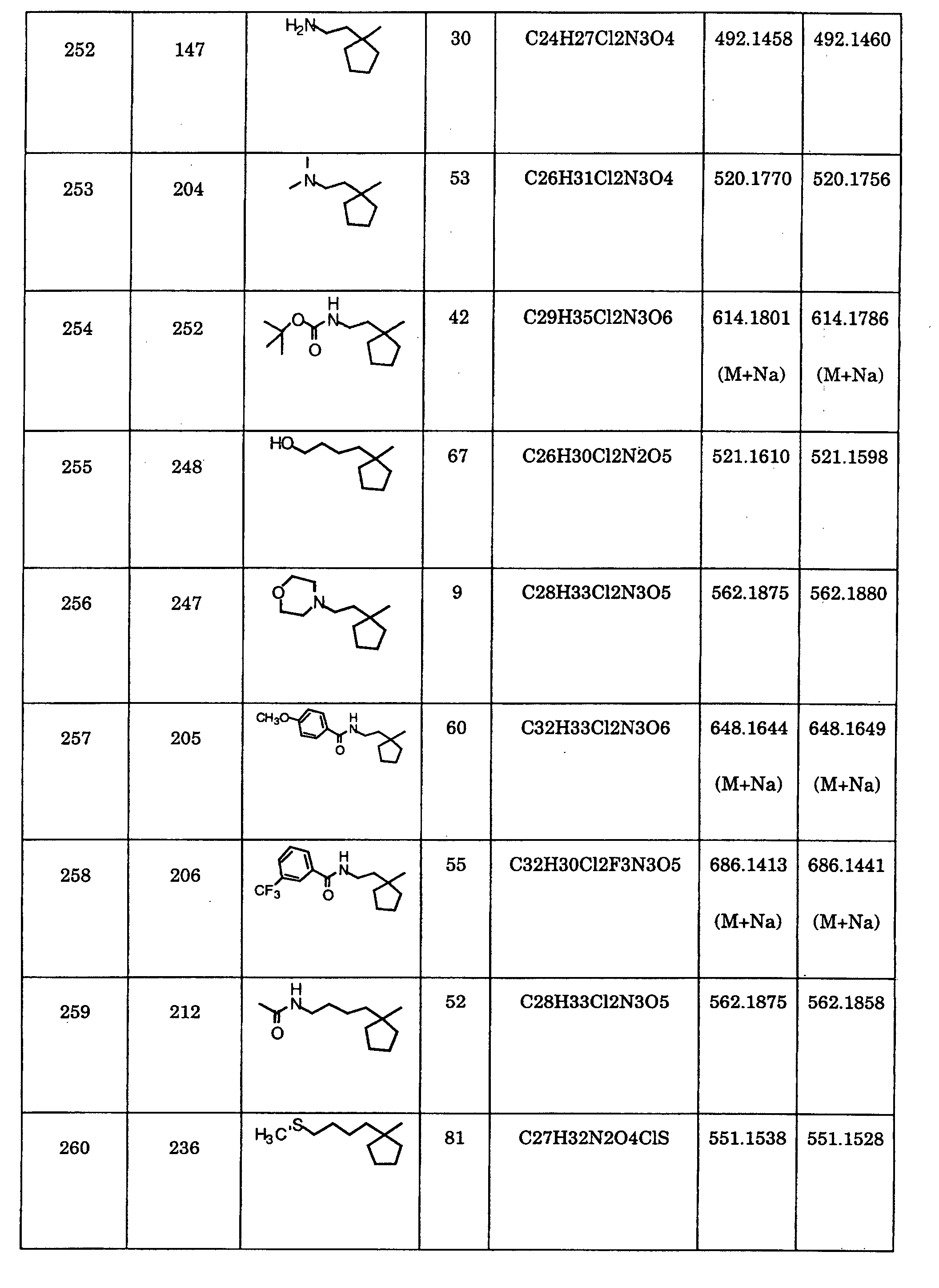

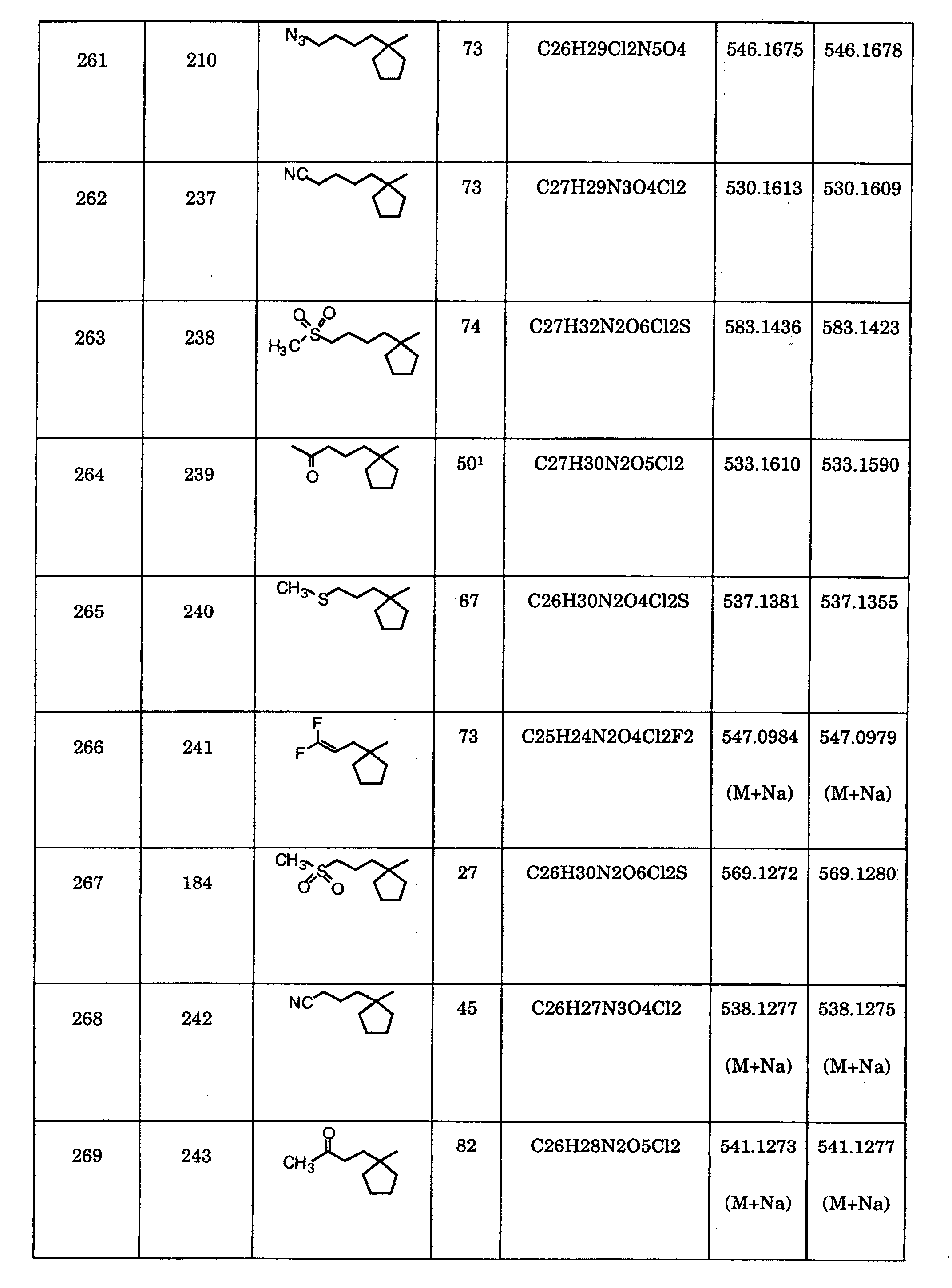

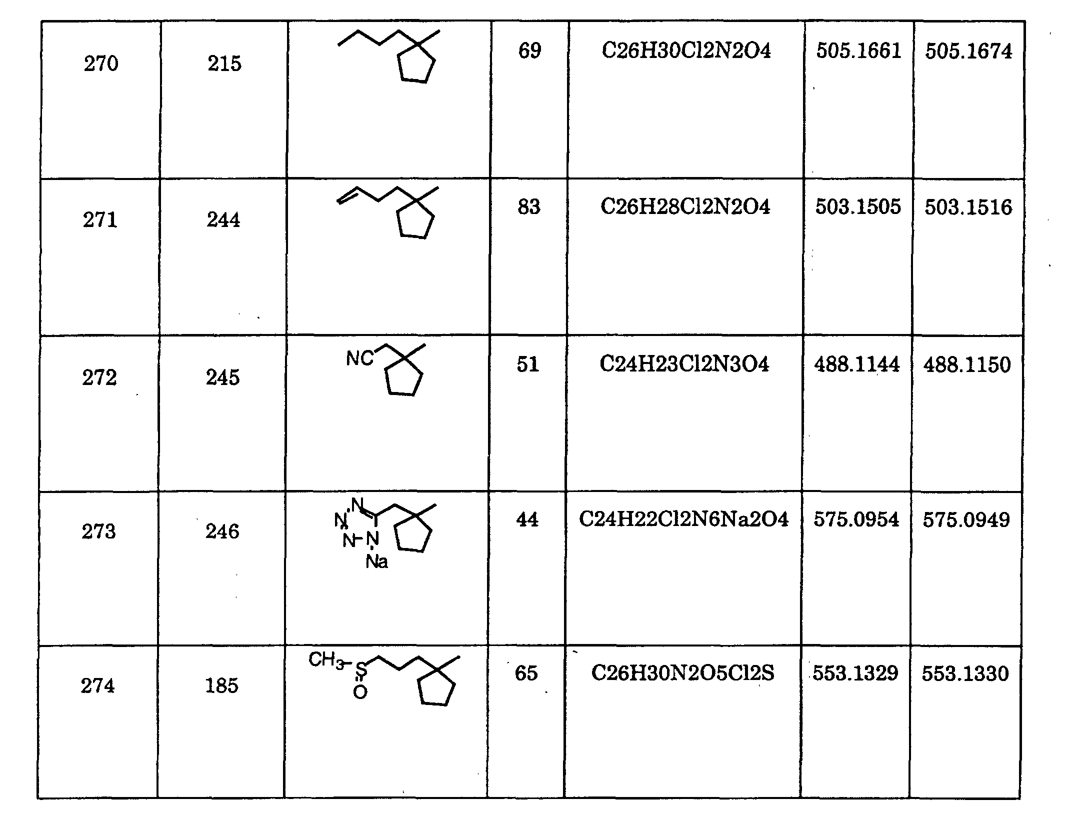

1. M+H ion unless otherwise indicated

Example 275. N-[[1-(3-(Acetylamino)propyl]cyclopentyl]carbonyl]-4-[[(2,6-dichlorophenyl) carbonyl]amino]-L-phenylalanine methyl ester was prepared in 50% yield from N-[[1-(3-(azidopropyl)cyclopenyl]carbonyl]-4-[[(2,6-dichlorophenyl)carbonyl]amino]-L-phenylalanine methyl ester using the general procedure described in examples 211 and 212.

Example 276. N-[[1-(3-(Acetylamino)propyl]cyclopenyl]carbonyl]-4-[[(2,6-dichlorophenyl) carbonyl]amino]-L-phenylalanine was prepared from N-[[1-(3-(Acetylamino)propyl]cyclopenyl] carbonyl]-4-[[(2,6-dichlorophenyl)carbonyl]amino]-L-phenylalanine methyl ester by hydrolysis using the general procedure described in example 47 to give a 70% yield. HR MS (C27H31Cl2N3O5): Obs mass, 570.1533. Calcd mass, 570.1539 (M+Na).

Example 277. General method for the preparation of morpholinoethyl esters from 4-(substituted)-N-acyl-L-phenylalanine derivatives.

To a solution of a 4-(substituted)-N-acyl-L-phenylalanine (0.5 mmol) and 2-morpholinoethanol (0.131 g, 1.0 mmol) in THF (5 mL) was added diisopropylcarbodimide (94.6 mg, 0.75 mmol) and 4-dimethylaminopyridine (30.5 mg, 0.25 mmol) at room temperature. The resulting mixture was stirred at room temperature until time TLC analysis of the reaction mixture indicated the absence of acid, typically 15 h. Then, the mixture was diluted with water (50 mL) and the THF was removed under vaccum and the residue was extracted with dichloromethane (3 x 25 mL). The combined extracts were washed with water (2 x 50 mL), brine solution (50 mL) and dried over MgSO₄. Filtration of the drying agent and concentration of the solvent gave a white residue which was purified by silica gel column chromatography eluting with dichloromethane-ethyl acetate mixtures to obtain the target product.

Examples 278-356. Procedure for the preparation of 4-[(4R)-3-acyl-5-oxo-2-substituted-4-substituted-1-imidazolidinyl]-N-[[1-[2-(acetylamino)ethyl]cyclopentyl]carbonyl]-L-phenylalanines

A 250 mL flask was charged with 4-nitro-N-Fmoc-L-phenylalanine (20.7 g, 47.8 mmol) and NMP (30 mL). The mixture was warmed up to accelerate dissolution. After it was cooled to room temperature, the mixture was treated with 2,6-dichlorobenzoyl chloride (20 g, 95.6 mmol) and pyridine (12 mL, 143.4 mmol). This mixture was shaken for 5 min and was added to a suspension of Wang resin (21.7 g, 1.1 mmol/g) in NMP (60 mL). The mixture was then shaken at room temperature overnight. After removal of solvent by filtration, the resin was washed with DMF (4 x 60 mL), MeOH (4 x 60 mL), DMF (4 x 60 mL) and finally dichloromethane (4 x 60 mL). The resin was then dried under vacuum at room temperature overnight to give 34.27 g of resin with loading of 0.668 mmol/g determined by the UV method (Barry Bunin, *The Combinatorial Index,* p. 219 (Academic Press, 1998)).

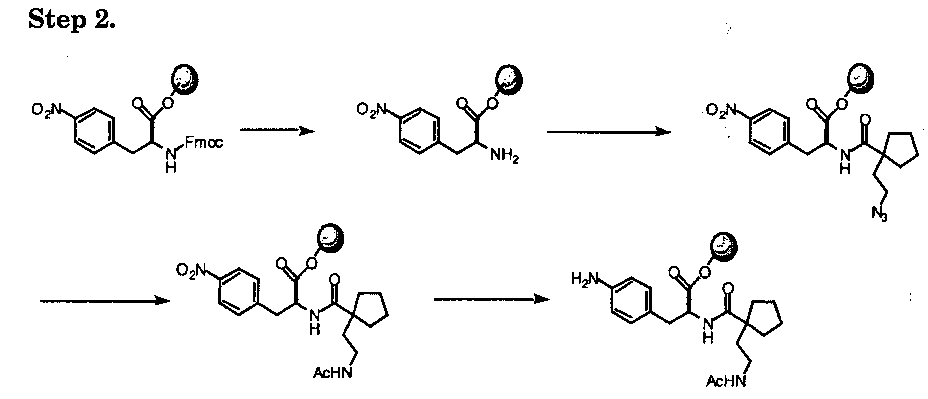

The above resin (25.5 g) was treated with 160 mL of 20% piperidine in NMP and shaken for 15 min and was filtered. This process was then repeated 2 times. The resin was then washed with DMF (4 x 100 mL), MeOH (4 x 100 mL), DMF (4 x 100 mL) and finally dichloromethane (4 x100 mL). The resin was then dried under vacuum at room temperature overnight to give 22 g of resin.

A portion of this free amine resin (13 g) was suspended in 80 mL of NMP and was treated with 1-1-(2-azidoethyl)cyclopentane carboxylic acid (4.8 g, 26.05 mmol) followed by DIEA (15mL) and BOP reagent (15.4 g, 34.2 mmol). The reaction was shaken at room temperature overnight. After filtration, the resin was washed with DMF (4 x 60 mL), MeOH (4 x 60 mL), DMF (4 x 60 mL) and finally dichloromethane (4 x 60 mL). The resin was then dried under vacuum at room temperature overnight to give azide resin. This resin was reduced by treatement with trimethylphosphine (1.0 M in THF, 30 mL) in 20 mL of THF at room temperature for 4 hr and then was treated with water (3 mL) for 30 min. After filtration, the resin was washed with DMF (4 x 60 mL), MeOH (4 x 60 mL), DMF (4 x 60 mL) and finally dichloromethane (4 x 60 mL). The resin was then dried under vacuum at room temperature overnight to give free aminoethyl resin (13.19 g).

A portion of the above resin (6.3 g) was suspended in dichloromethane (40 mL) and was treated with acetic anhydride (2 mL, 21 mmol) and DIEA (3.6 mL, 21 mmol). The above mixtrue was shaken at room temperature overnight. After filtration, the resin was washed with DMF (4 x 40 mL), MeOH (4 x 40 mL), DMF (4 x 40 mL) and finally dichloromethane (4 x 40 mL). The resin was then dried under vacuum at room temperature overnight to give acetamide resin (6.27 g). A small sample of resin was collected and treated with 50% TFA in dichloromethane to give the cleavage product, N-[[1-[2-(acetylamino)ethyl]cyclopentyl]carbonyl)-4-nitro-L-phenylalanine. LS MS (M-H, m/z: 391). The remainder of the resin was then treated with SnCl2 (2M in DMF, 40 mL) at room temperature overnight. After filtration, the resin was washed with DMF (4 x 40 mL), MeOH (4 x 40 mL), DMF (4 x 40 mL) and finally dichloromethane (4 x 40 mL). It was then dried under vacuum at room temperature overnight to give 4-amino-N-[[1-[2-(acetylamino)ethyl]cyclopentyl)carbonyl)-L-phenylalanine on resin (6.1 g).

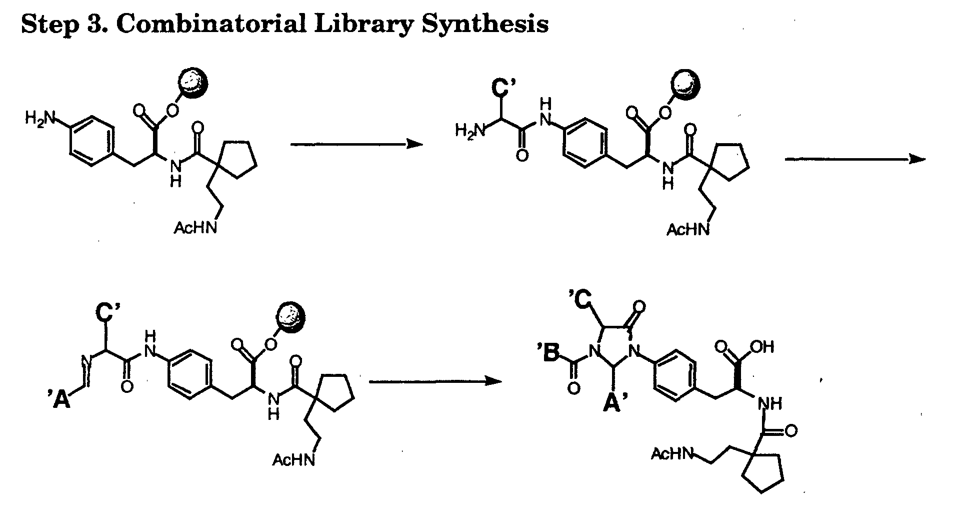

The above free 4-amino-N-[[1-[2-(acetylamino)ethyl]cyclopentyl]carbonyl]-L-phenylalanine on resin was split into 5 reaction vessels. To each vessel was added, in parallel, 8 mL of DMF followed by a Fmoc-D-amino acid (selected from: Fmoc-D-phenylalanine (C1), Fmoc-D-4-chlorophenylalanine (C2), Fmoc-D-3-pyridinylalanine (C3), Fmoc-D-4-methoxyphenylalanine (C4) and Fmoc-D-alanine (C5), 2.25 mmol), HBTU (1.4 g, 3.75 mmol) and DIEA (0.75 mL). The above mixture was shaken at room temperature overnight. The resin from each vessel was filtered, washed with DMF (4 x 20 mL), MeOH (4 x 20 mL), DMF (4 x 20 mL) and finally dichloromethane (4 x 20 mL). These resins were then individually dried under vacuum at room temperature overnight to give 5 individual Fmoc-D-amino acid containing resins. Each of the five individual batches of resin obtained above was treated in parallel with 10 mL of 20% piperidine in NMP under shaking for 15 min and was filtered. This process was then repeated two times to give the following five derivatives after individual drying: N-[[1-[2-(acetylamino)ethyl]cyclopentyl]carbonyl)-4-[[(2R)-2-amino-1-oxo-3-phenylpropyl]amino]-L-phenylalanine on Wang resin, N-[[1-[2-(acetylamino)ethyl]cyclopentyl]carbonyl]-4-[[(2R)-2-amino-3-(4-chloropheny)-1-oxopropyl]amino]-L-phenylalanine on Wang resin, N-[[1-[2-(acetylamino)ethyl]cyclopentyl]carbonyl]-4-[[(2R)-2-amino-1-oxo-3-(3-pyridinyl)propyl]amino] -L-phenylalanine on Wang resin, N-[[1-[2-(acetylamino)ethyl]cyclopentyl]carbonyl]-4-[[(2R)-2-amino-3-(4-methoxyphenyl)-1-oxopropyl]amino]-L-phenylalanine on Wang resin, and N-[[1-[2-(acetylamino)ethyl]cyclopentyl]carbonyl]-4-[((2R)-2-amino-1-oxopropyl)amino]-L-phenylalanine on Wang resin.

A library of imidazolidin-4-ones was prepared as discrete compounds using the IRORI AccuTag - 100 Combinatorial Chemistry System (IRORI AccuTag - 100 Combinatorial Chemistry System The technique for labeling a reaction vessel and use of the reaction vessels referred to herein as Microkans is described in the User's Guide, 1996, IRORI, 11025 North Torrey Pines Road, La Jolla, CA 92037). IRORI is registered trademark of IRORI. IRORI, AccuTag, Microkan and Synthesis Manager are trademarks of IRORI.

Each of the five resin derivatives was split into 16 Microkans containing radio frequency tags to give a total of 80 Microkan reaction vessels. Using the Synthesis Manager to read the radio frequency tags in each, these were then sorted into 4 groups of 20 so that each group had 4 Microkans containing each of the five resin bound D-aminoacids prepared above. Each group of 20 microkans was individually placed in a reaction vessel containing a mixture of dry solvent (THF/methyl orthoformate = 1/1, 80 mL) and one of four aldehydes (benzaldehyde (A1); 4-pyridylaldehyde (A2), 4-chlorobenzaldehyde (A3) or phenylpropionaldehyde (A4) (20 x 1.33 mmol ). The above mixtures were shaken at room temperature for 3 days to form imine intermediates. After removal of the solvent by decantation, the groups of Microkans were individually washed with dry THF (2 x 20 mL).

The resulting 80 Microkans were then sorted into 4 groups of 20 with each group incorporating one example of each of the D-amino acids (C1 to C5) combined with each of the four aldehydes (A1 to A4) according to the table shown below:

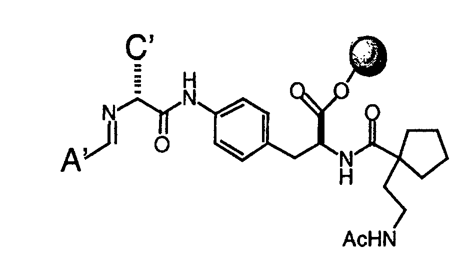

| | | | | |
|---|---|---|---|---|
| C'-1/A'-1 | C'-2/A'-1 | C'-3/A'-1 | C'-4/A'-1 | C'-5/A'-1 |
| C'-1/A'-2 | C'-2/A'-2 | C'-3/A'-2 | C'-4/A'-2 | C'-5/A'-2 |
| C'-1/A'-3 | C'-2/A'-3 | C'-3/A'-3 | C'-4/A'-3 | C'-5/A'-3 |
| C'-1/A'-4 | C'-2/A'-4 | C'-3/A'-4 | C'-4/A'-4 | C'-5/A'-4 |
| A'-1 = phenyl C'-1 = benzyl | | | | |
| A'-2 = 4-pyridyl C'-2 = 4-chlorobenzyl | | | | |
| A'-3 = 4-chlorophenyl C'-3 = 3-pyridinylmethyl | | | | |
| A'-4 = 2-phenylethyl C'-4 = 4-methoxybenzyl | | | | |
| C'-5 = methyl | | | | |

Each group of 20 Microkans was individually placed in a reaction vessel containing dry solvent (THF/methyl orthoformate = 1/1, 10 mL). To the first reaction vessel was then added acetic anhydride (B1) (5.5 mmol) and the resulting mixture was shaken at 90 °C for 4 hr. The remaining three reaction vessels were individually treated with an anhydride: butryric anhydride (B2), succinic anhdride (B3) and phenoxyacetic anhyride (B4) and subjected to the same reaction conditions in parallel. After filtration, each of the four groups of Microkans was individually washed with DMF (4 x 40 mL), MeOH (4 x 40 mL), DMF (4 x 40 mL) and finally dichloromethane (4 x 40 mL). The Microkans were then sorted into separate vials using the Synthesis Manager to identify each by means of the individual radio freqency tags. Each vial was treated with cleavaging reagent 50% TFA/dichloromethane (2.5 mL). The vials were shaken for 2 hr at room temperature and the resulting mixtures were filtered. The filtrate from each reaction was concentrated to dryness to give the crude product which was then treated with ether under shaking at room temperature for 20 min. The suspension was allowed to stand at room temperature for 15-30 min and the ether was removed. The ether wash was repeated and residues were dissolved in MeCN/H2O (2/1). A portion of this solution was set aside for analysis and the balance was freeze-dried to give crude products. The analytical samples were analyzed by LCMS using a Micromass platform II with a 5 min linear gradent (5% MeCN in water to 95% MeCN in water contain 0.01% of TFA) to determine purity while recording the MS spectra to verify the identity of the major peak. The subset of this library derived from 4-pyridylaldehide (A-2) was removed from the collection because of low purity scores. The remaining 60 members were purified by reversed-phase HPLC to give a library of imidazolidinones with purity greater than 90%. During the separation, some of the diastereomers could be separated to give the diastereomers at the 2-position of the imidazolidine ring designated diastereomers 1 and 2 (based upon the order of elution) while others were assayed as mixtures of diastereomers. The molecular weights of these purified products were comfirmed by ESMS (M-H, M, or M+H) as shown in table below.

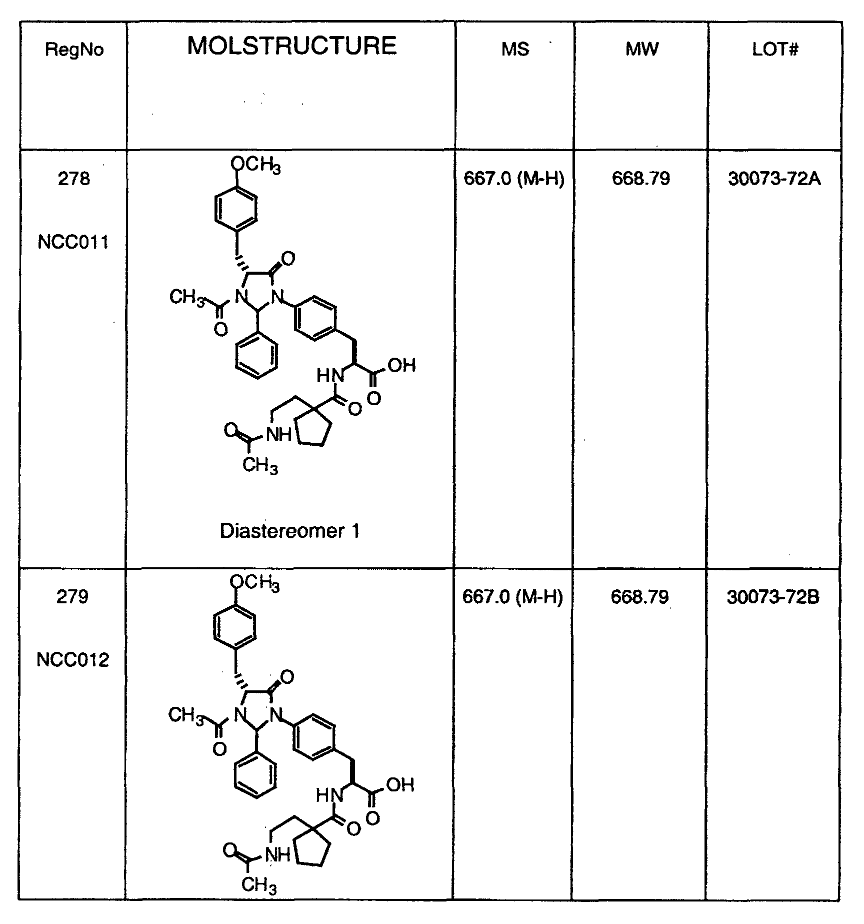

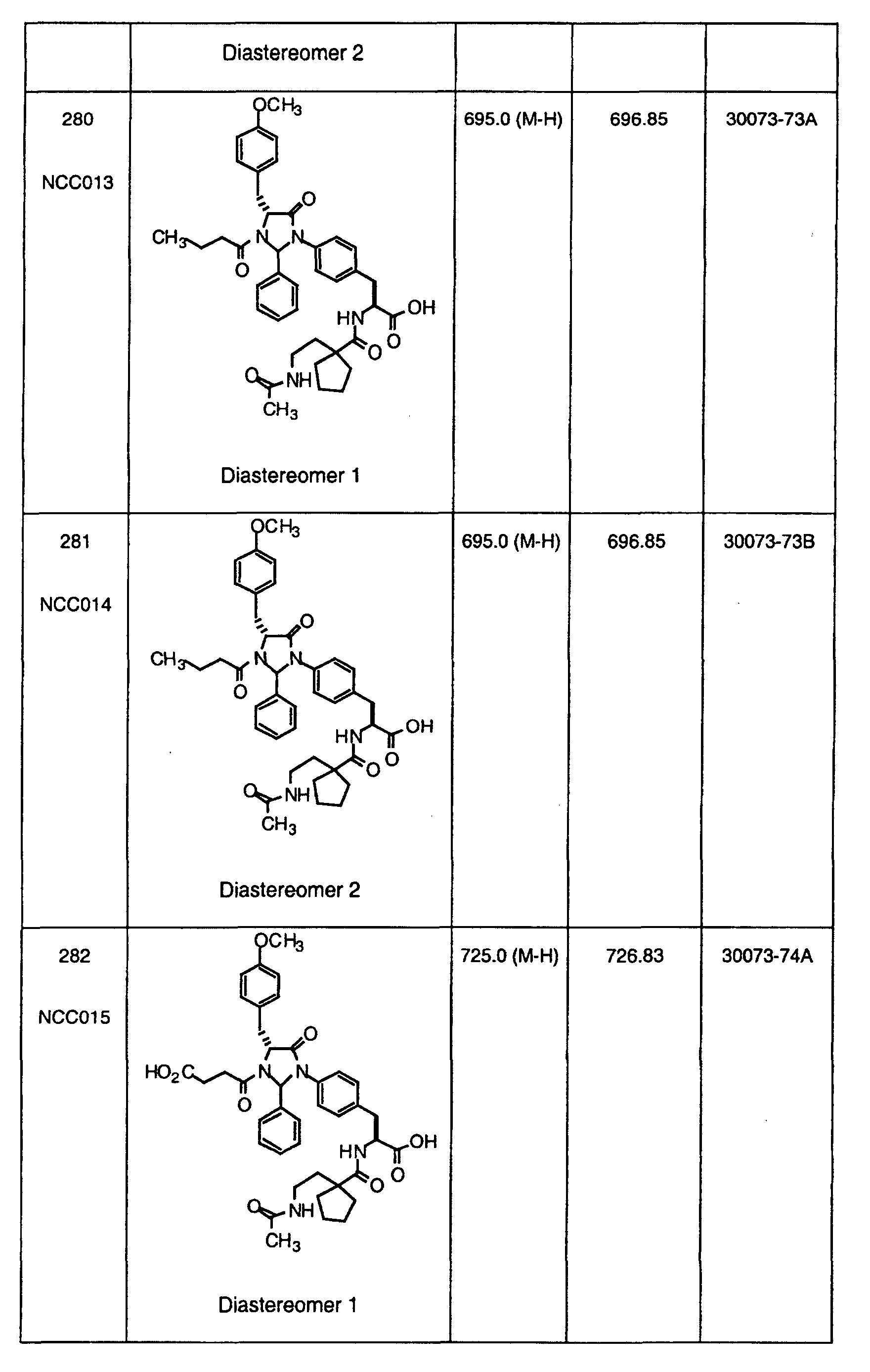

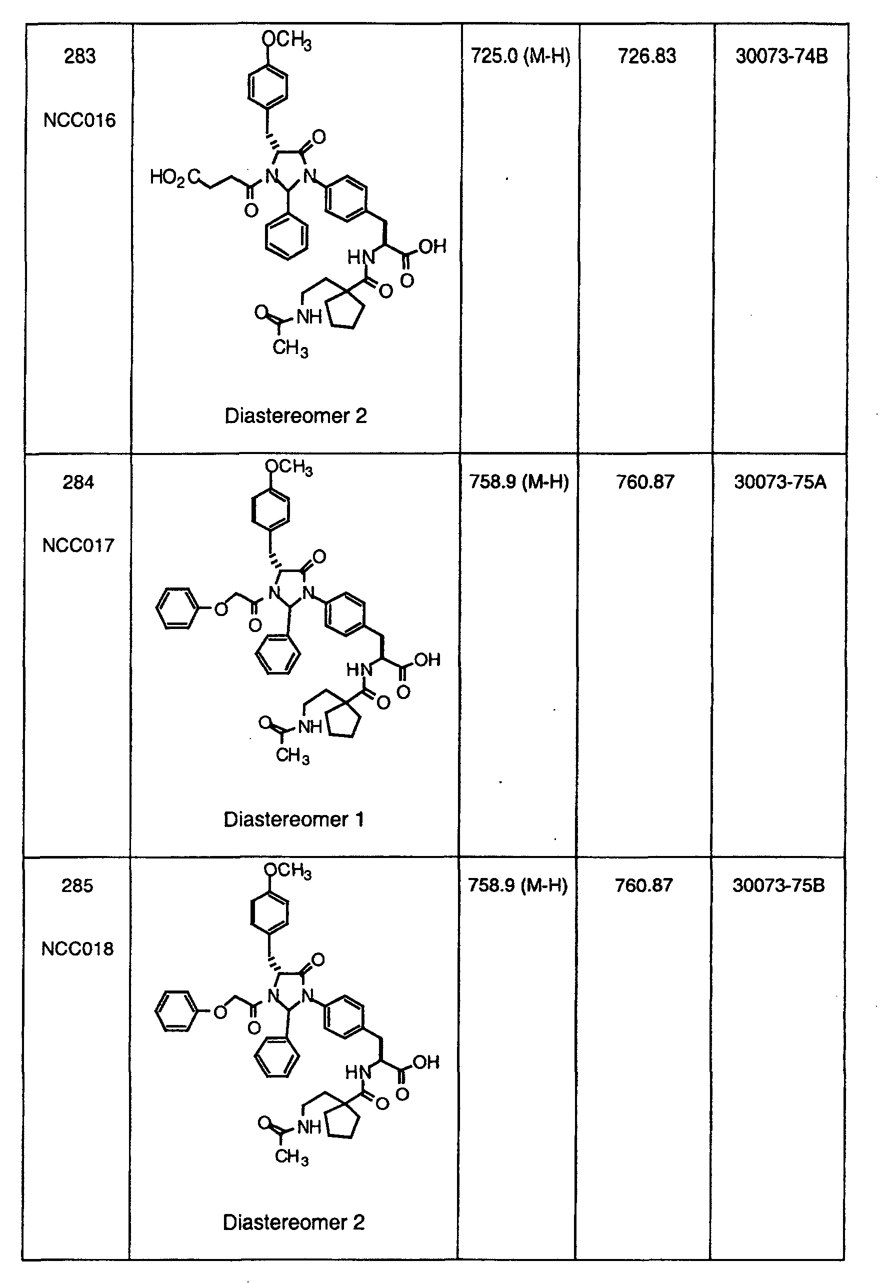

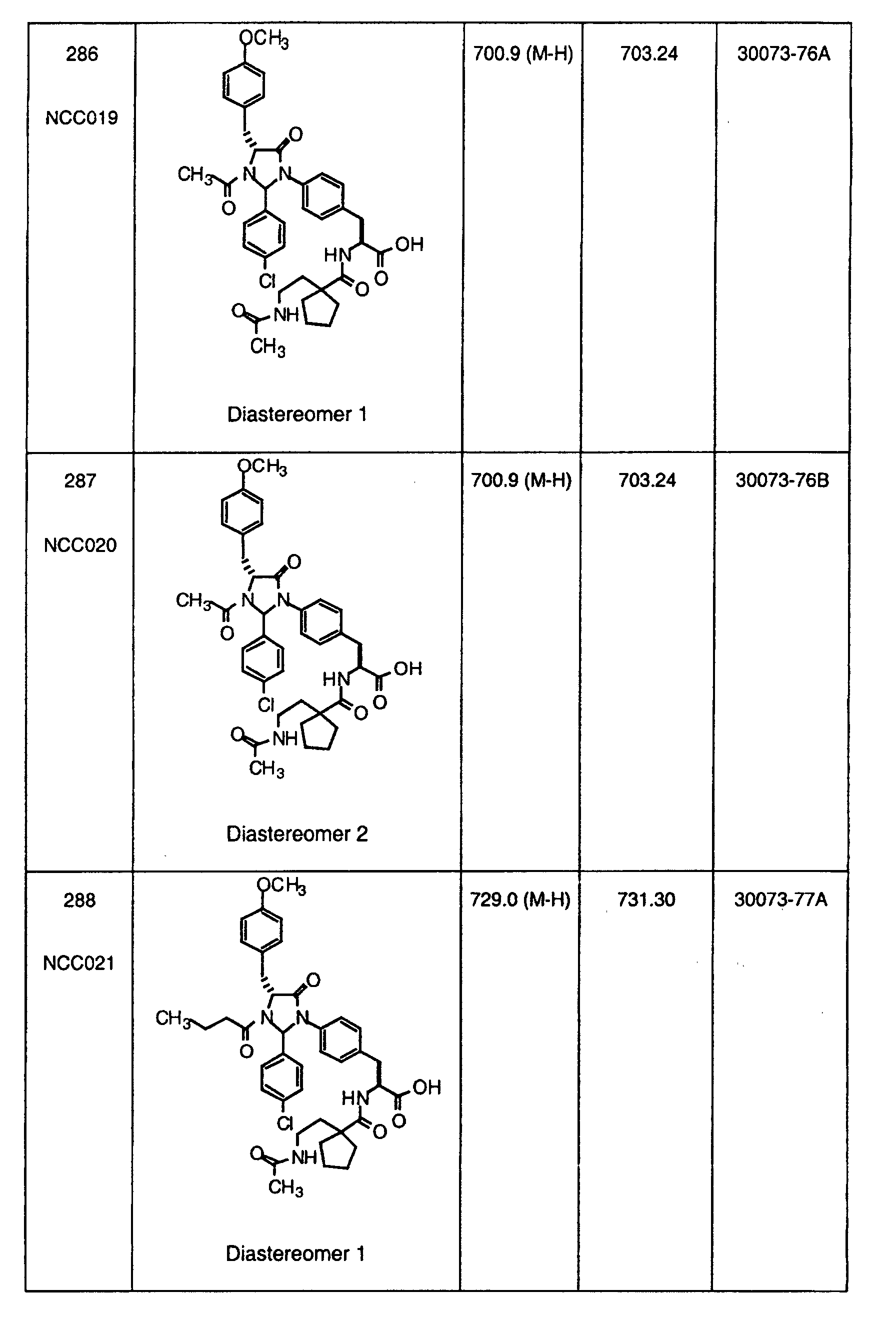

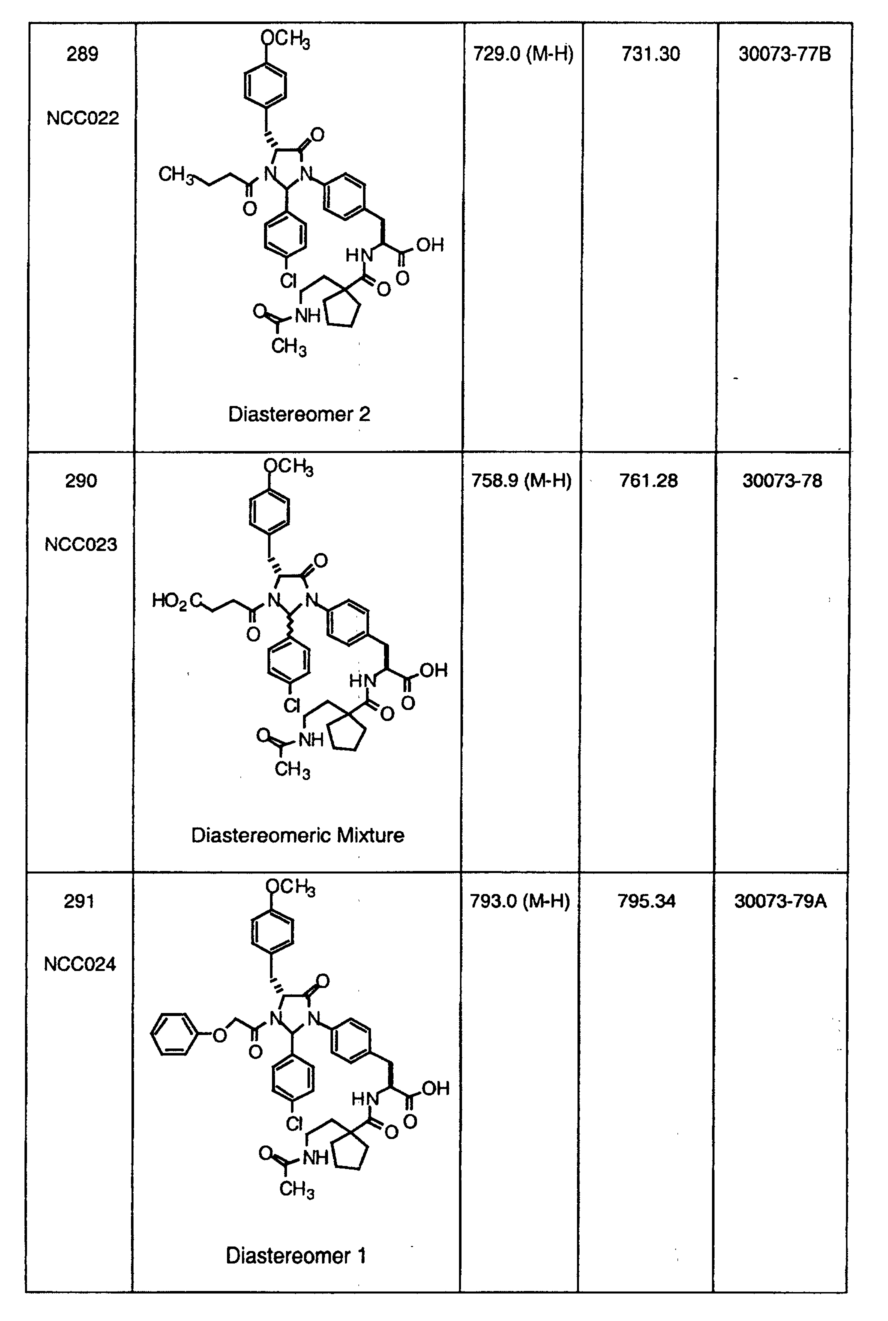

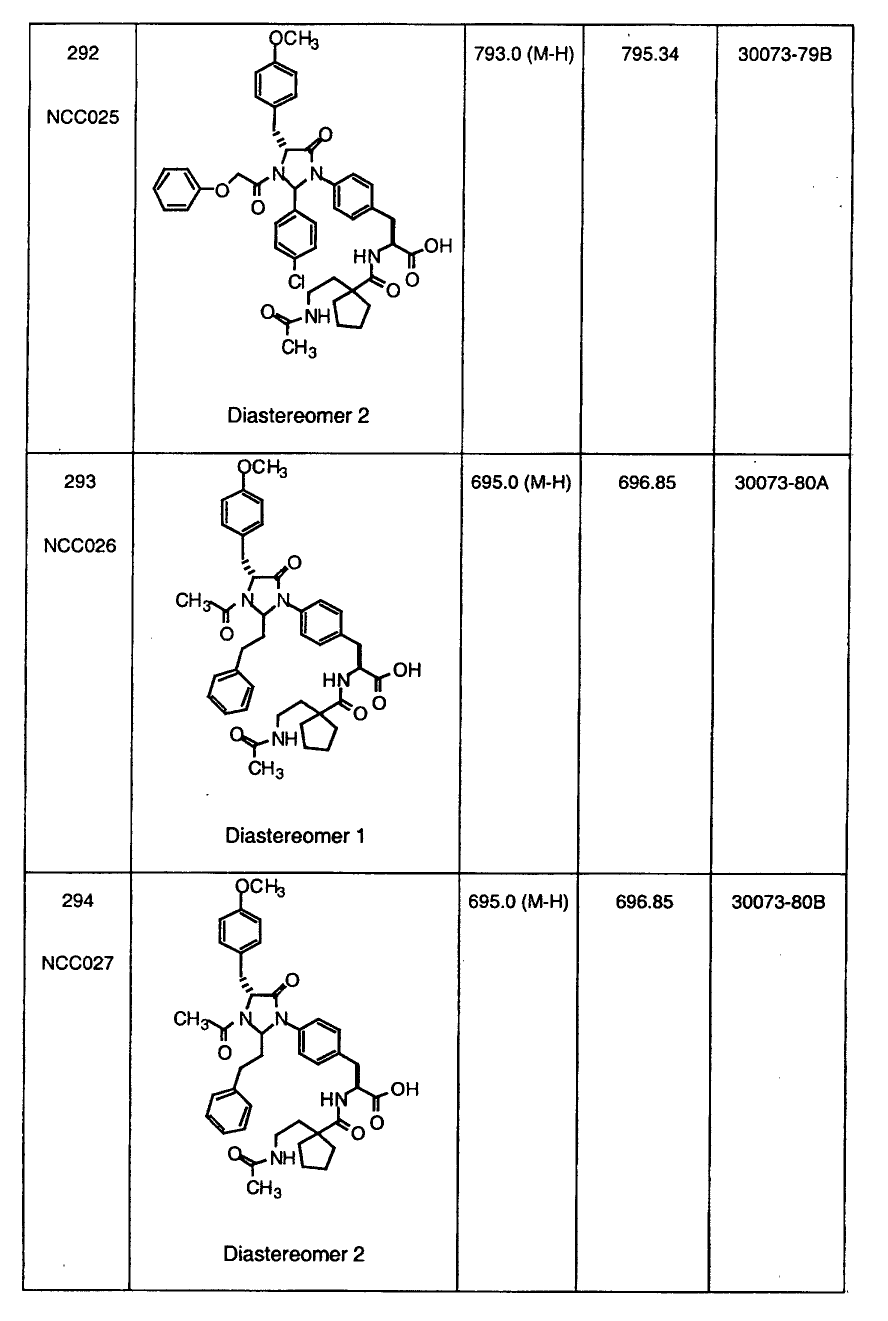

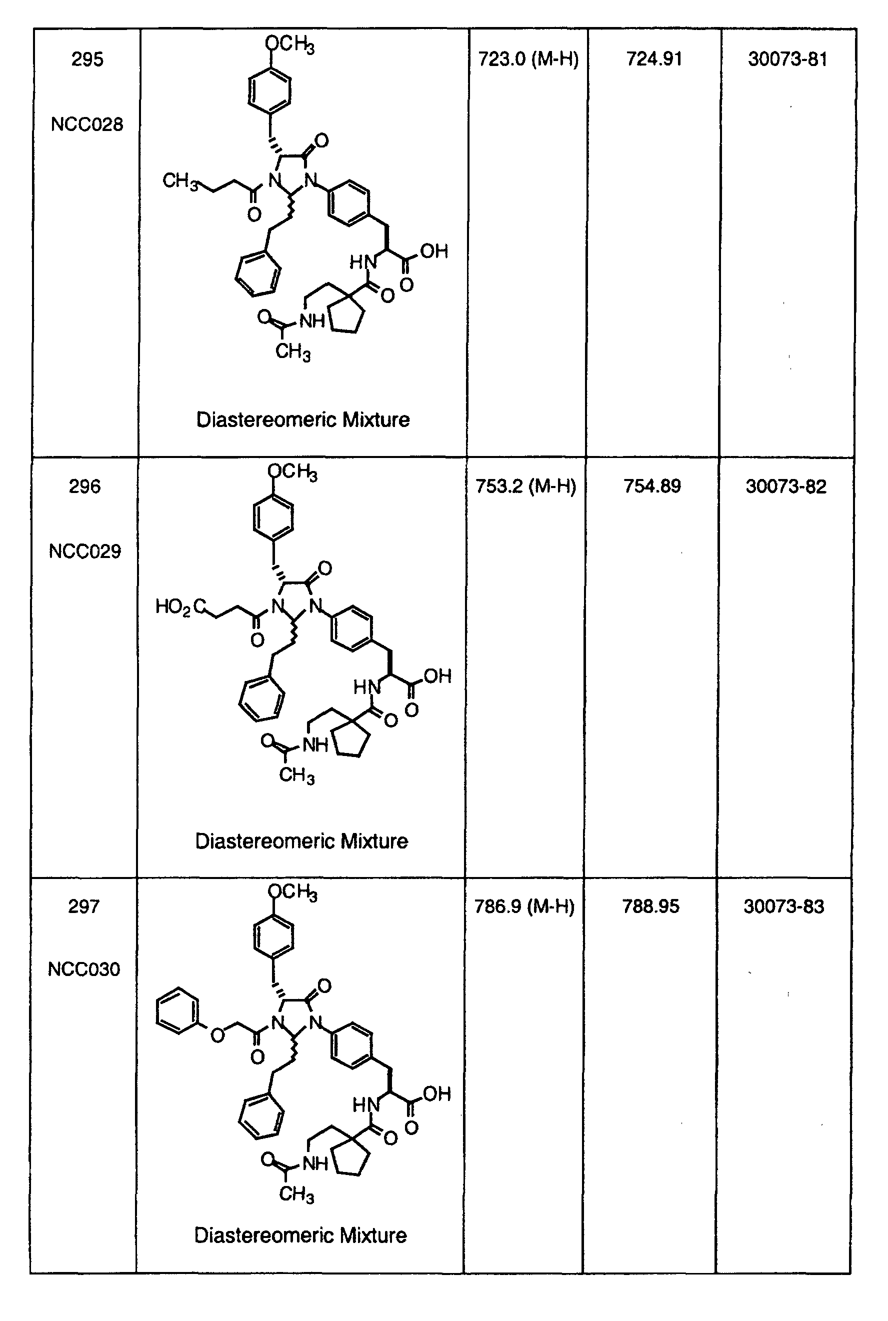

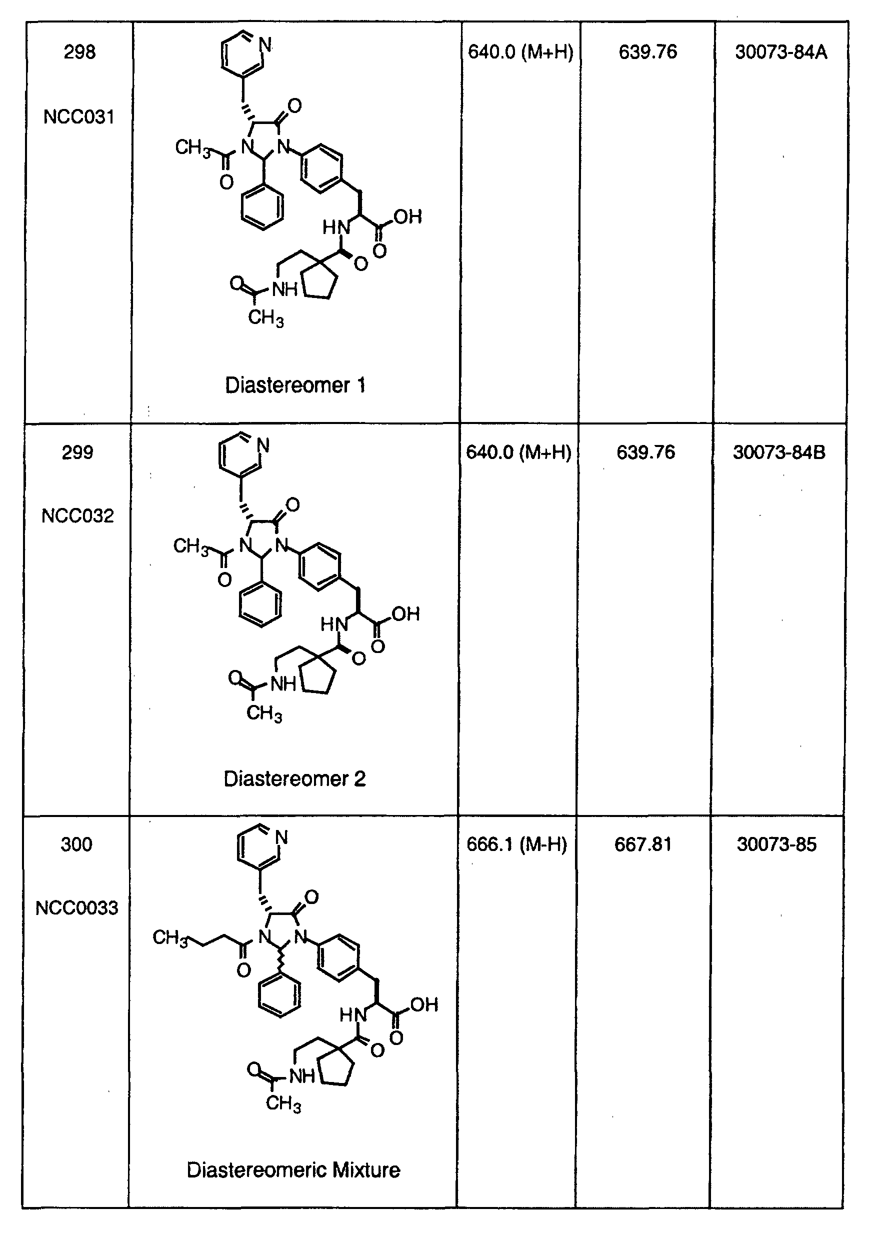

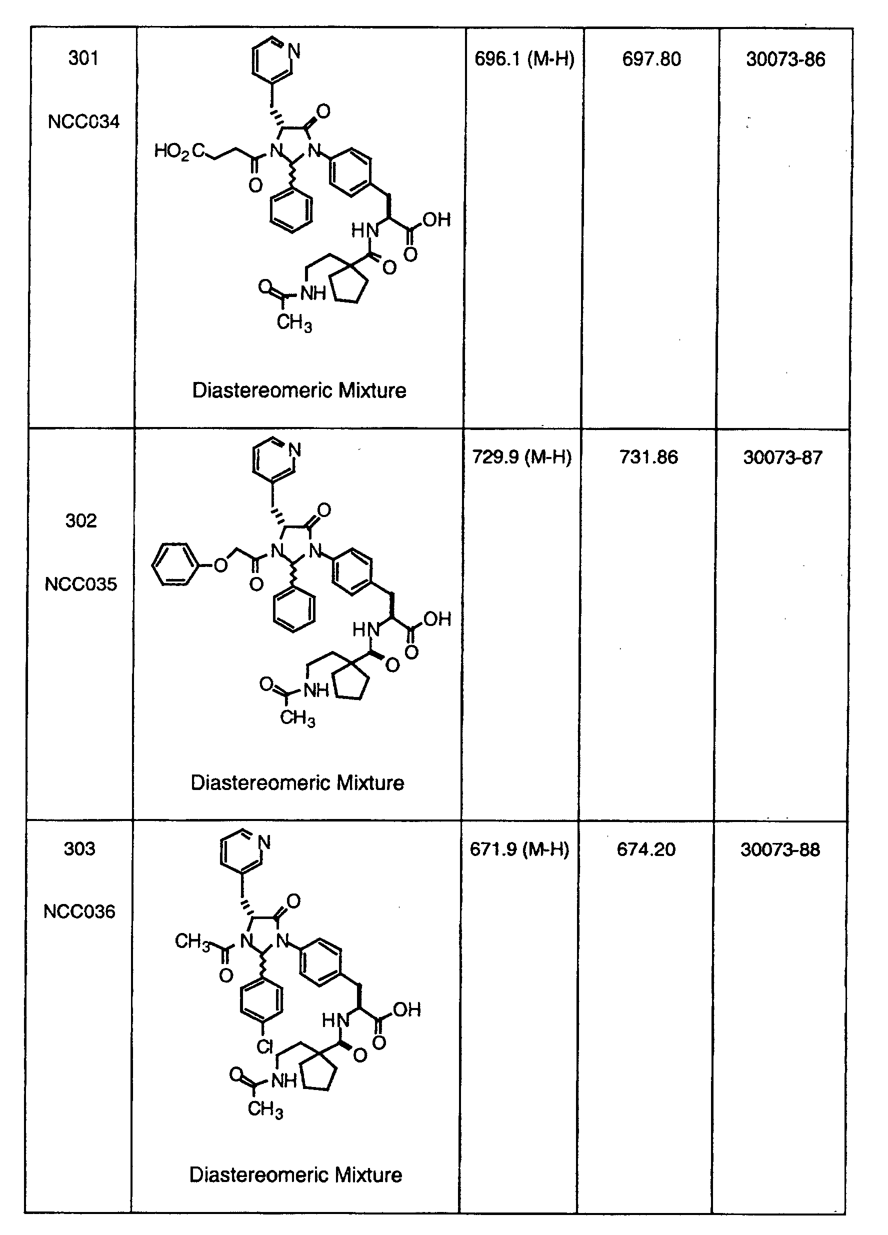

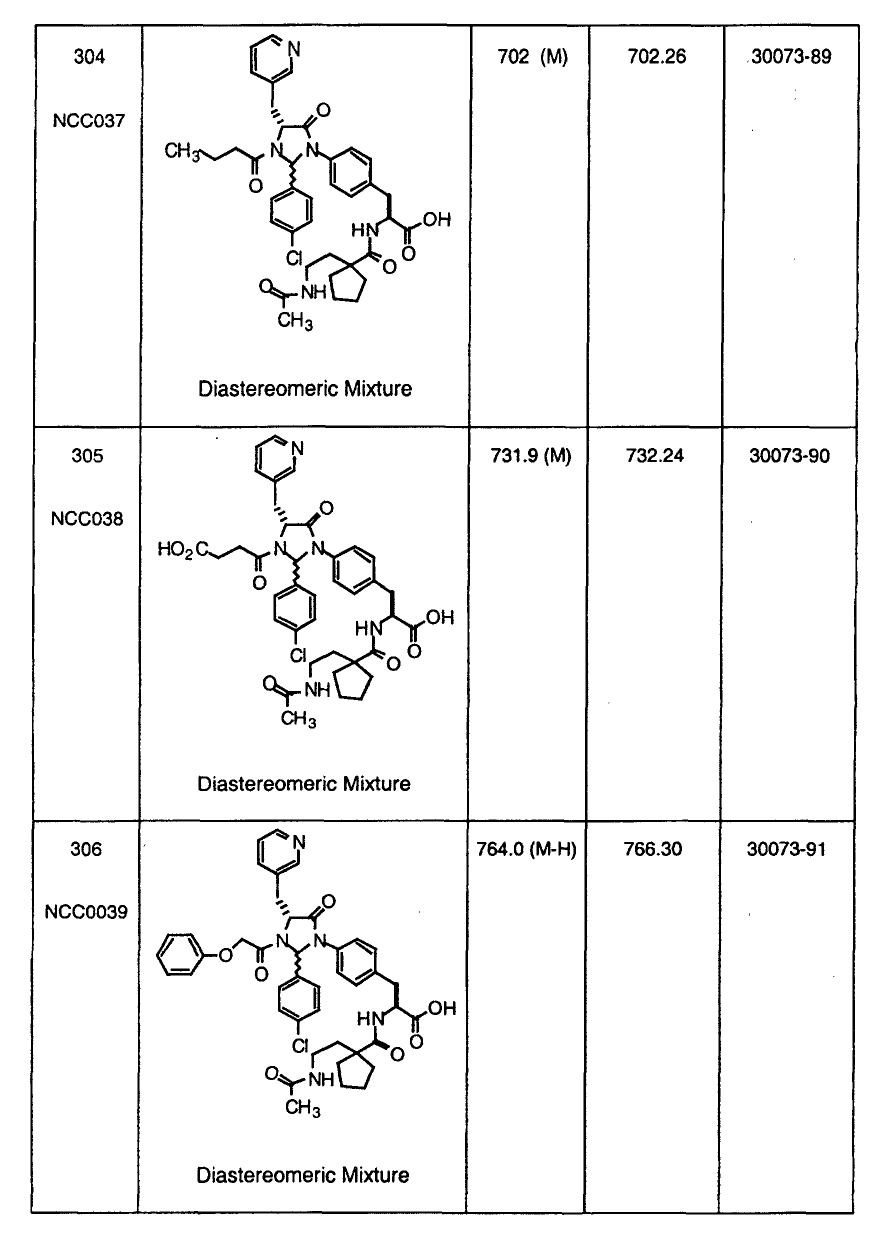

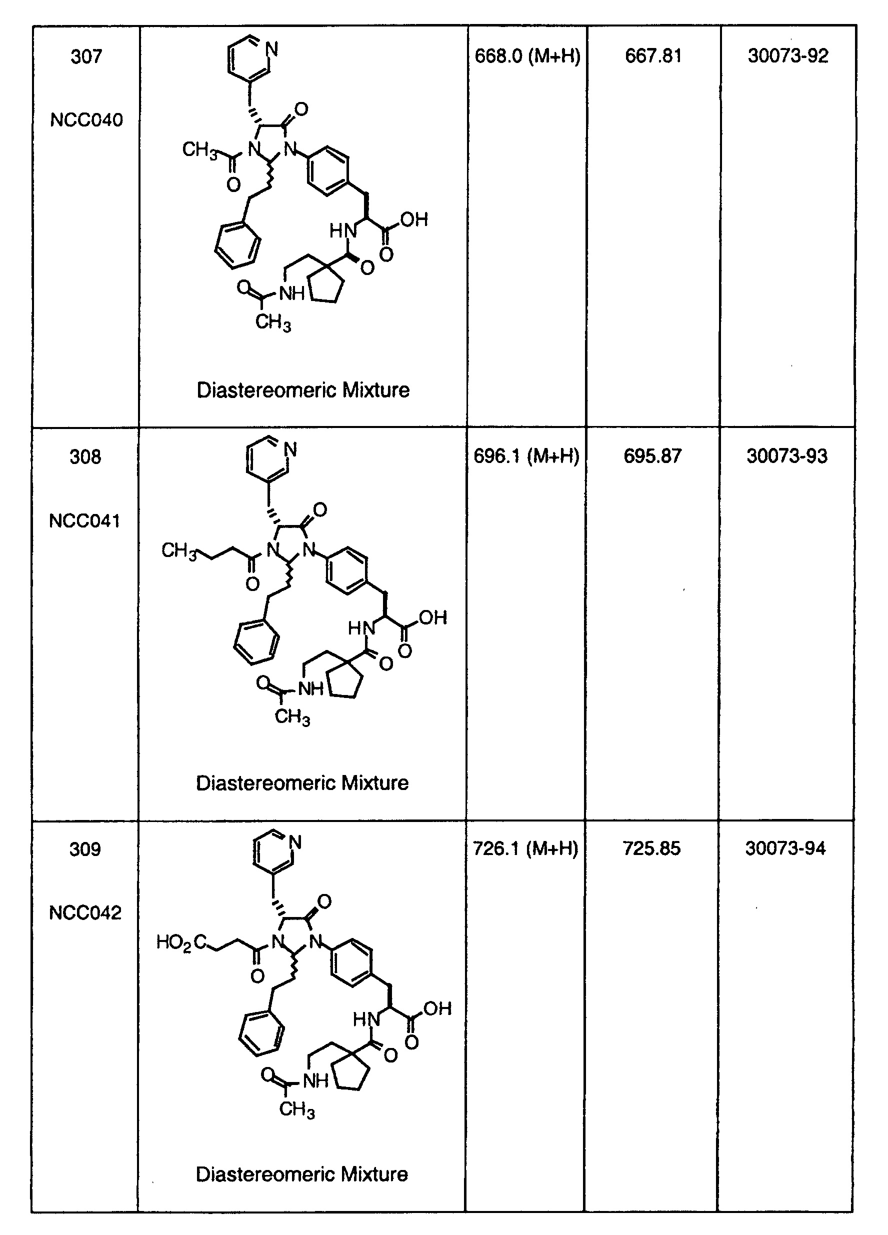

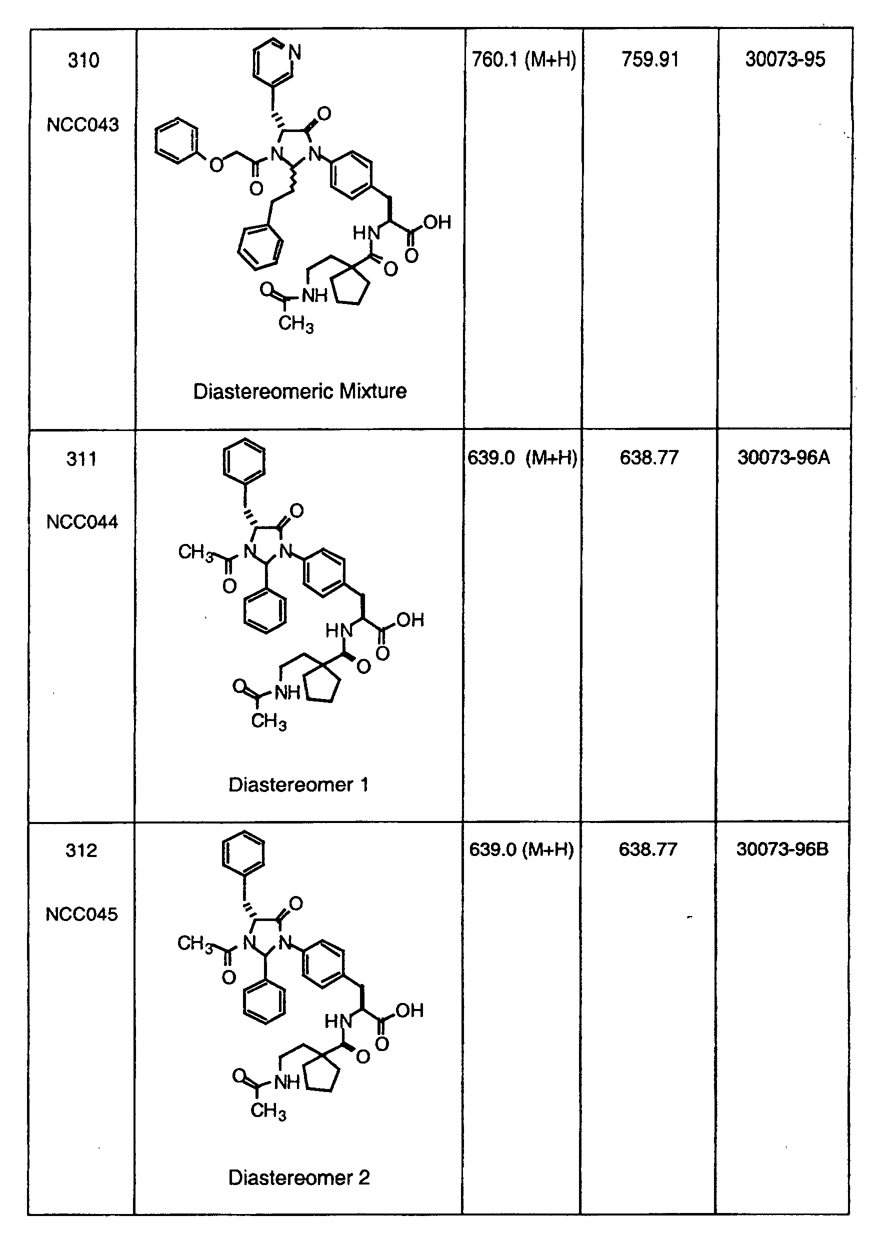

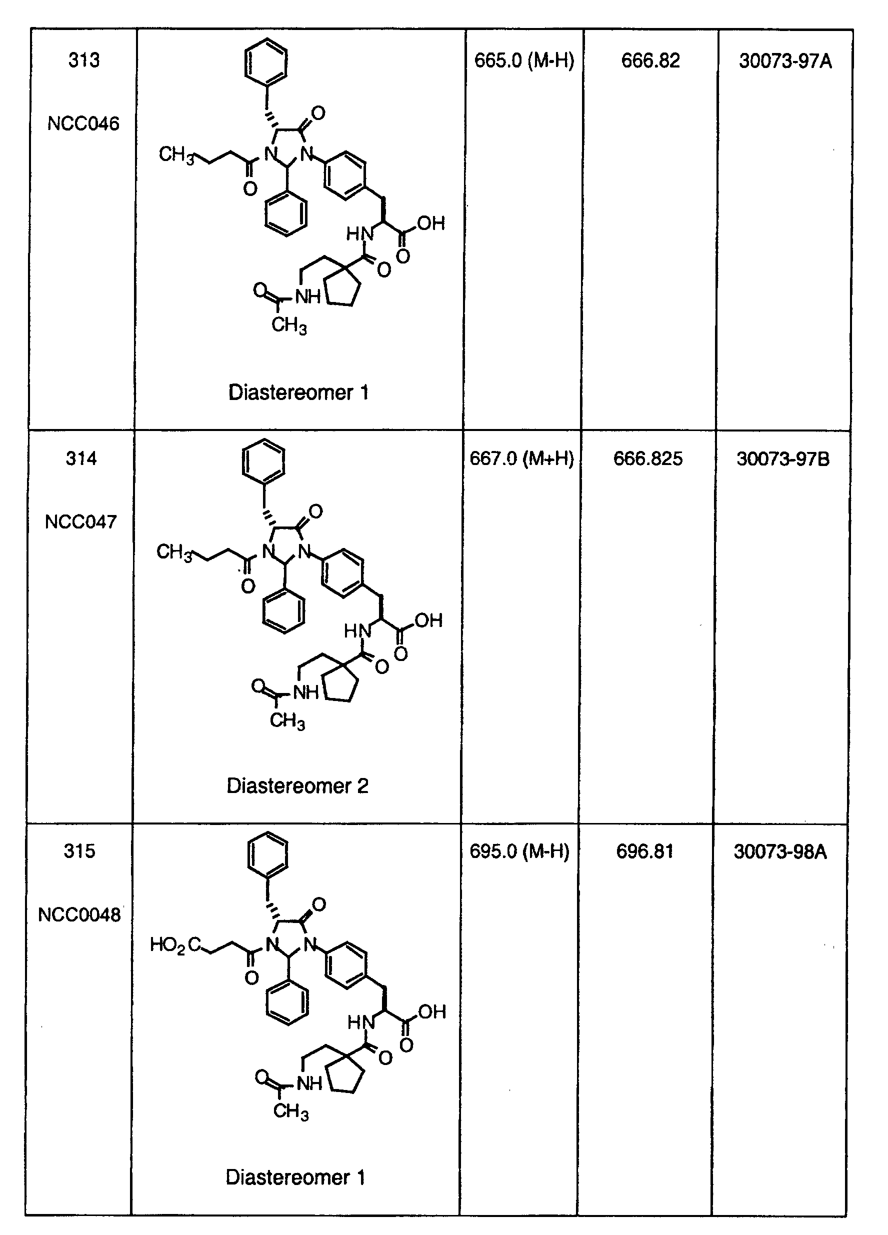

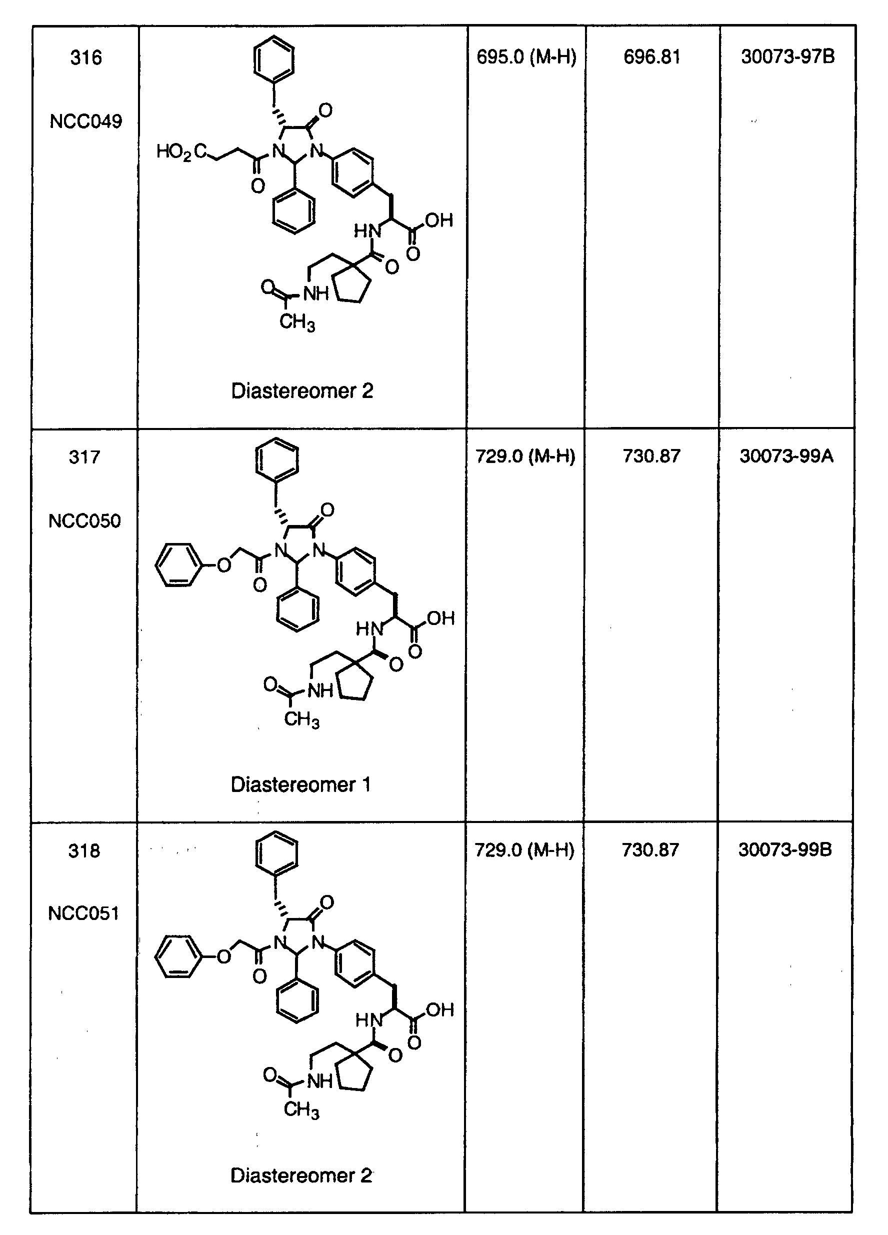

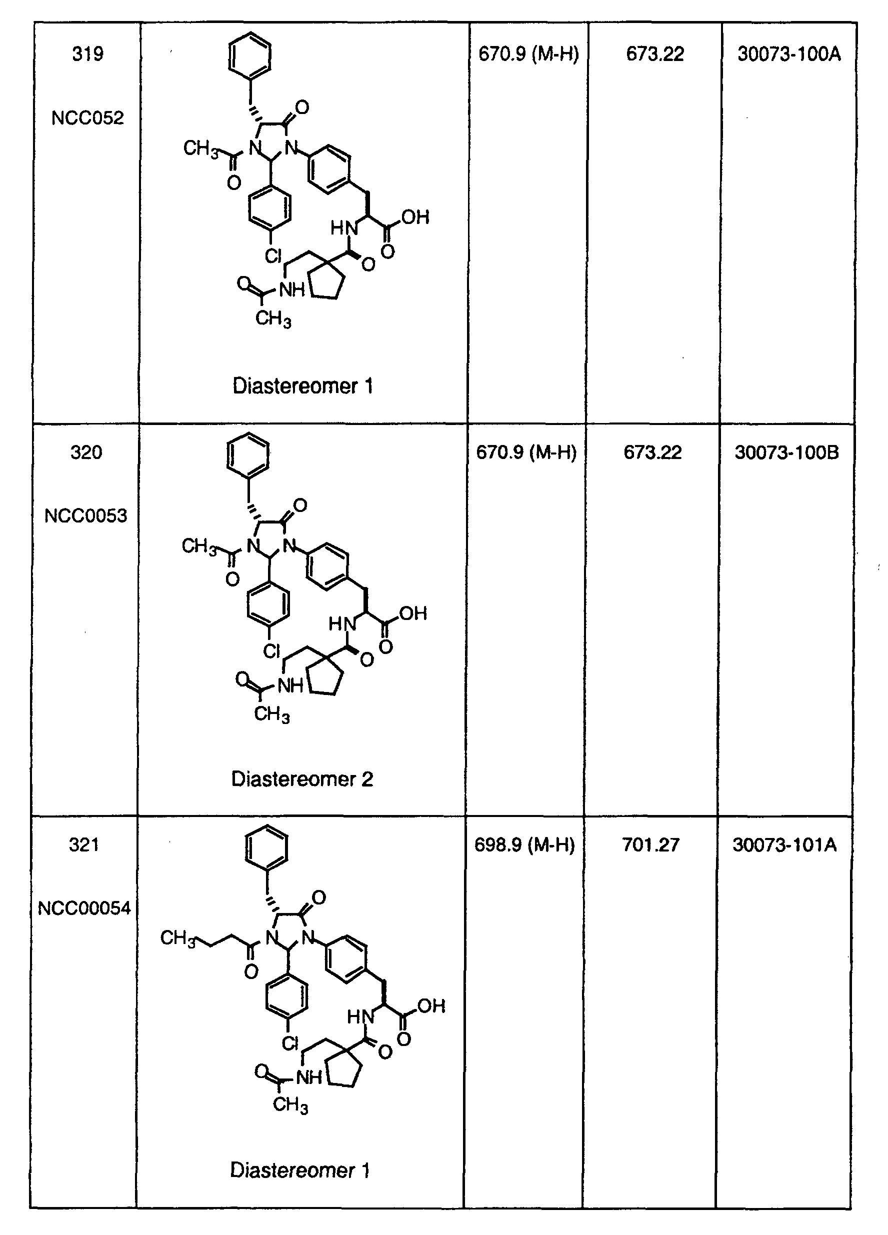

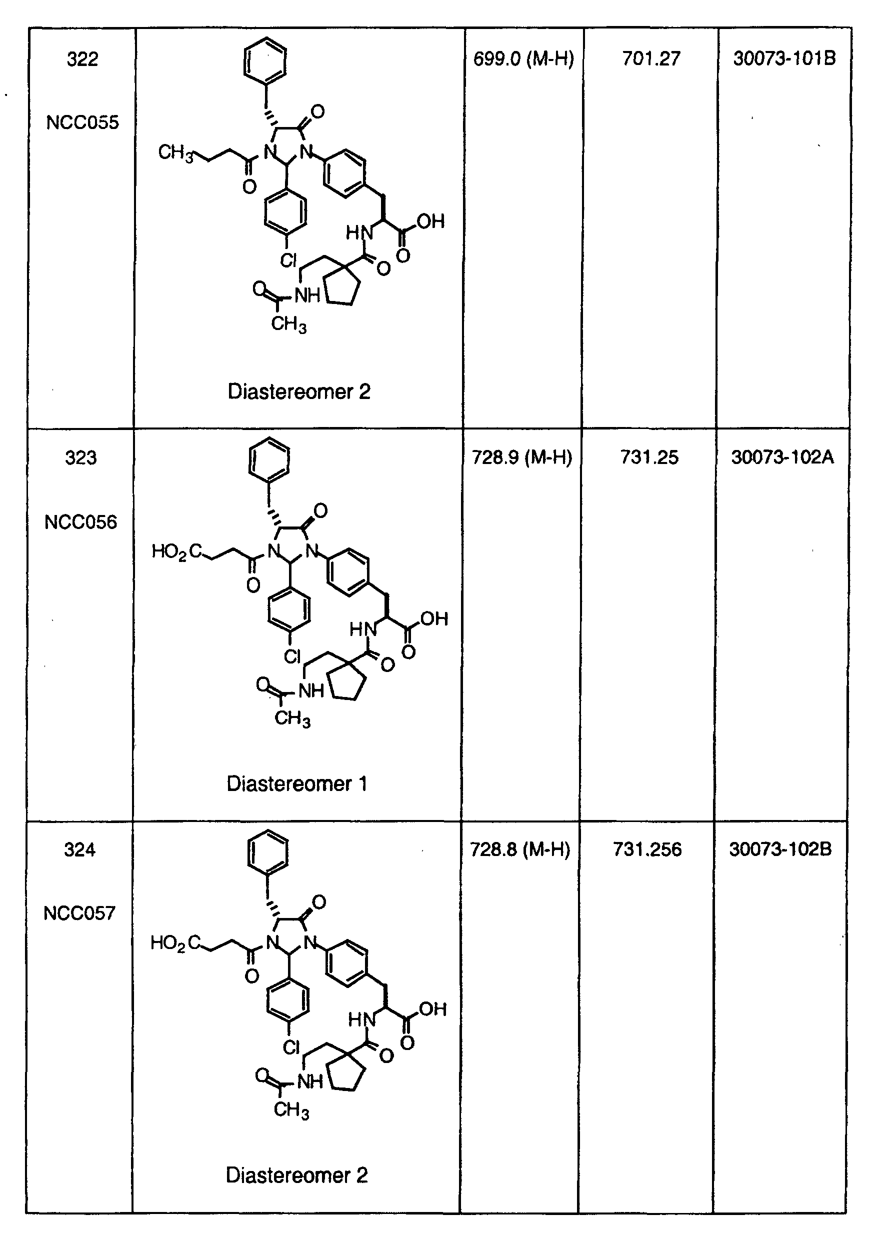

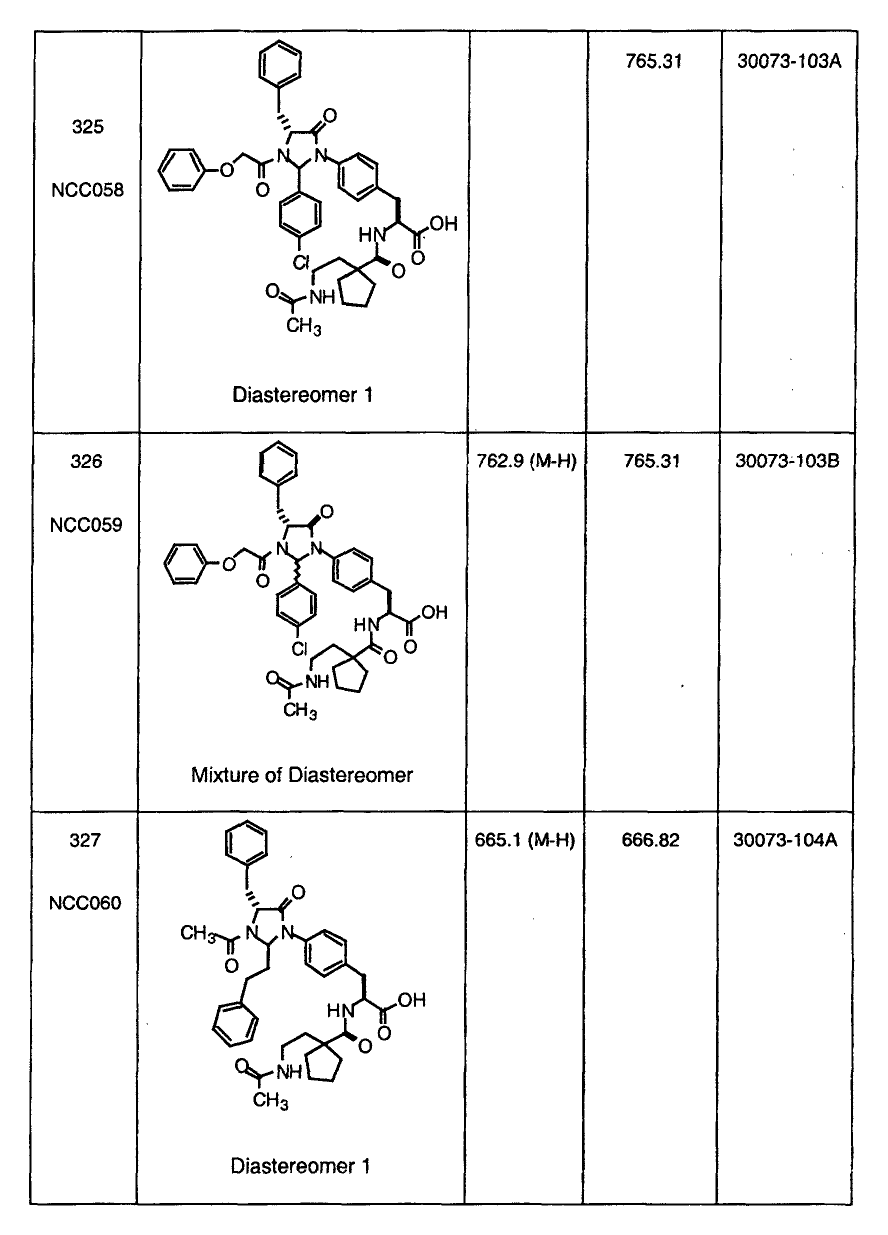

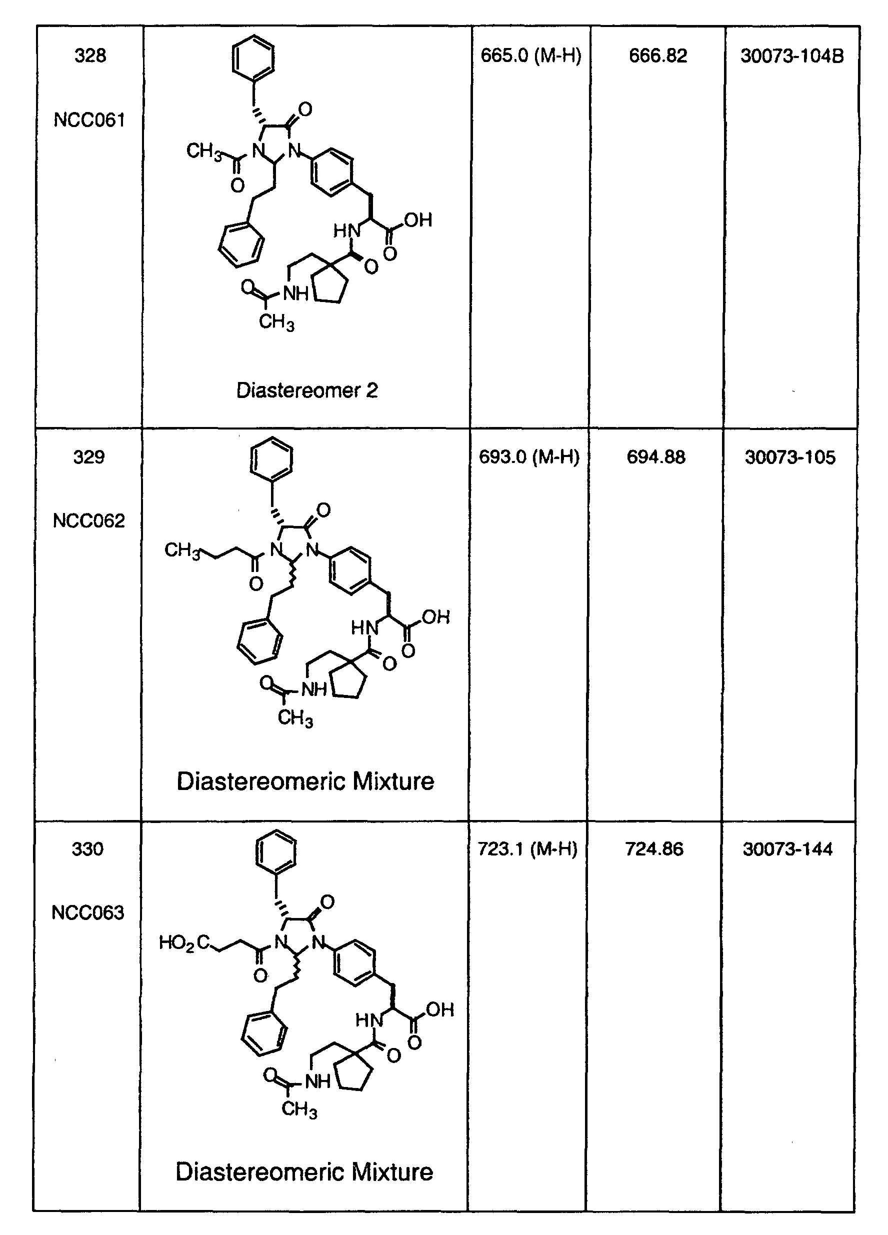

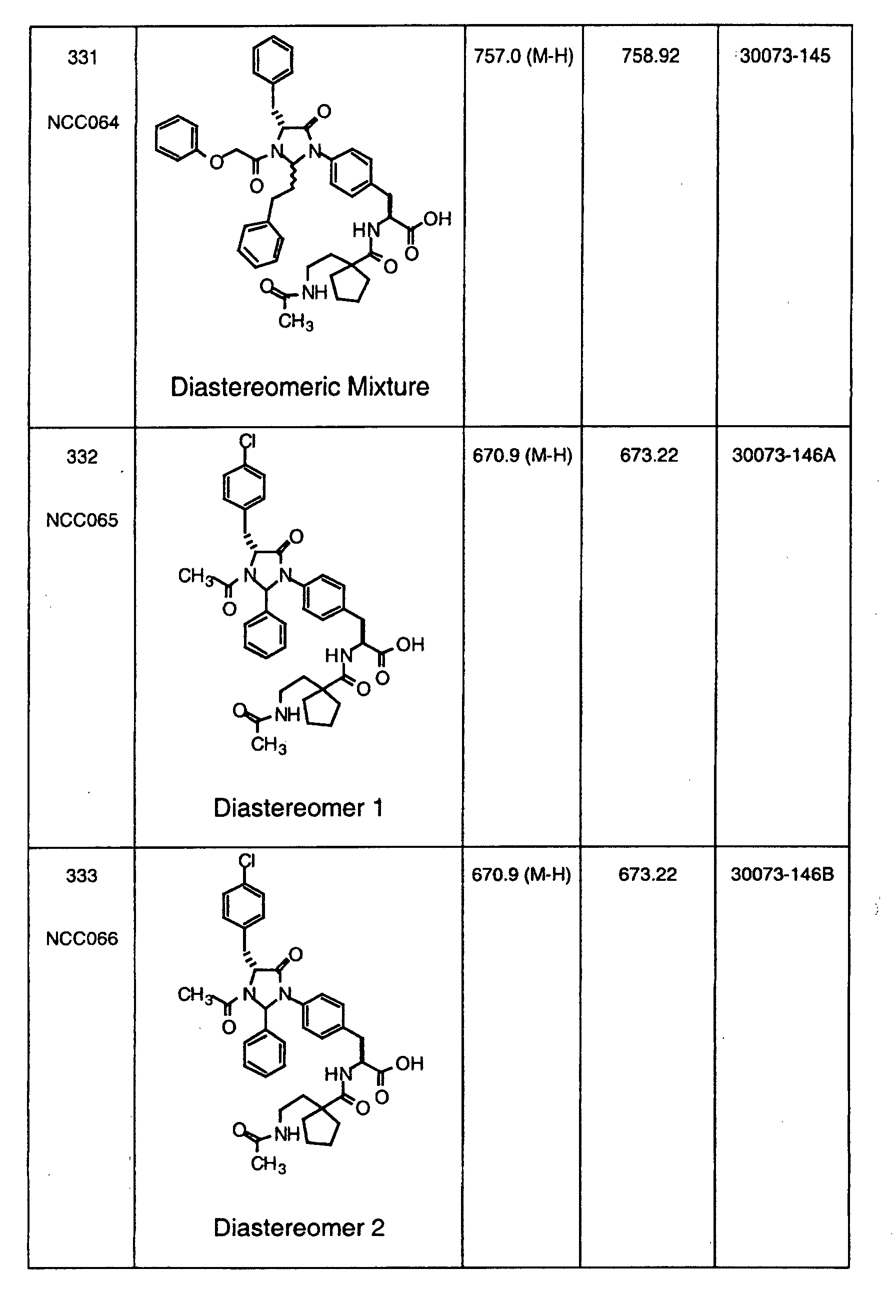

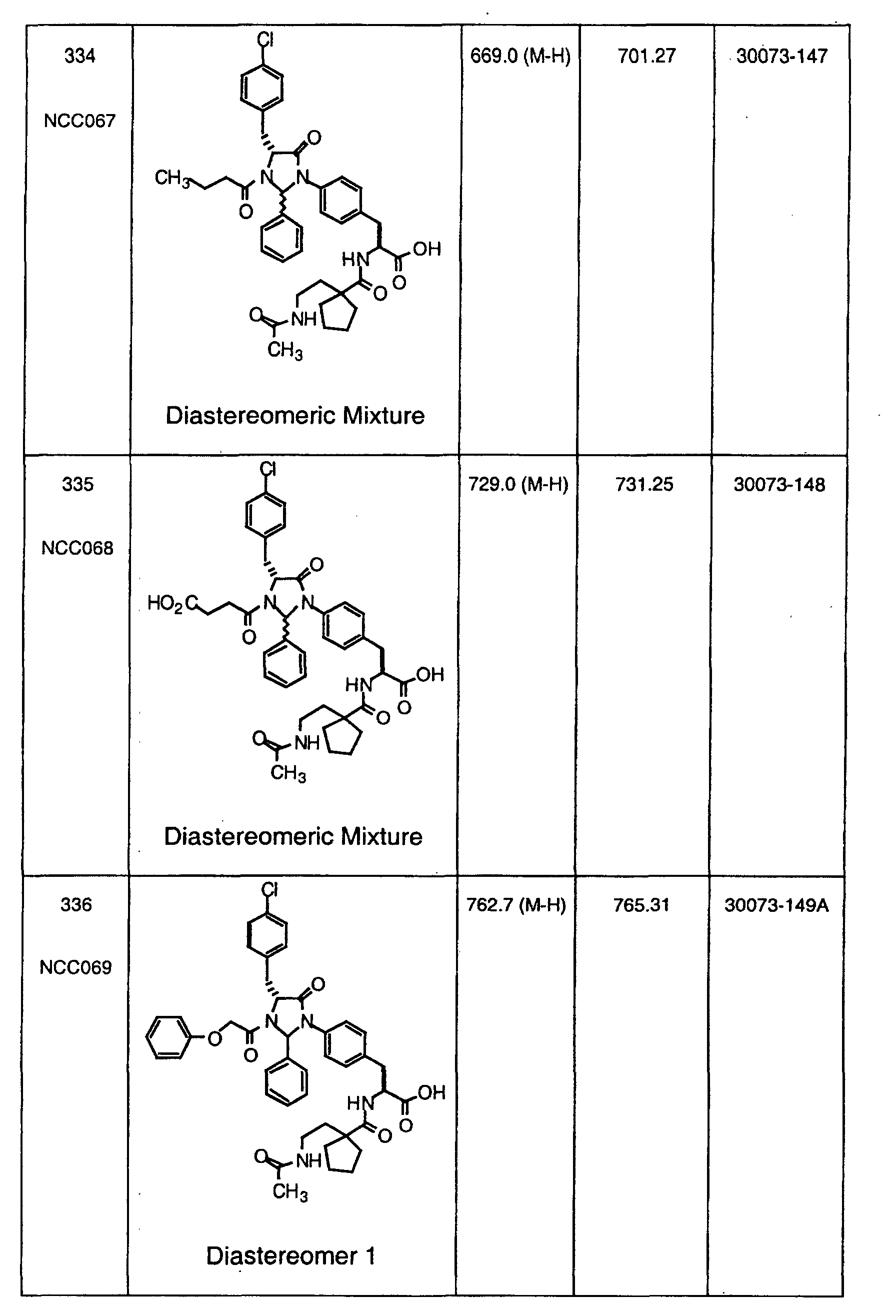

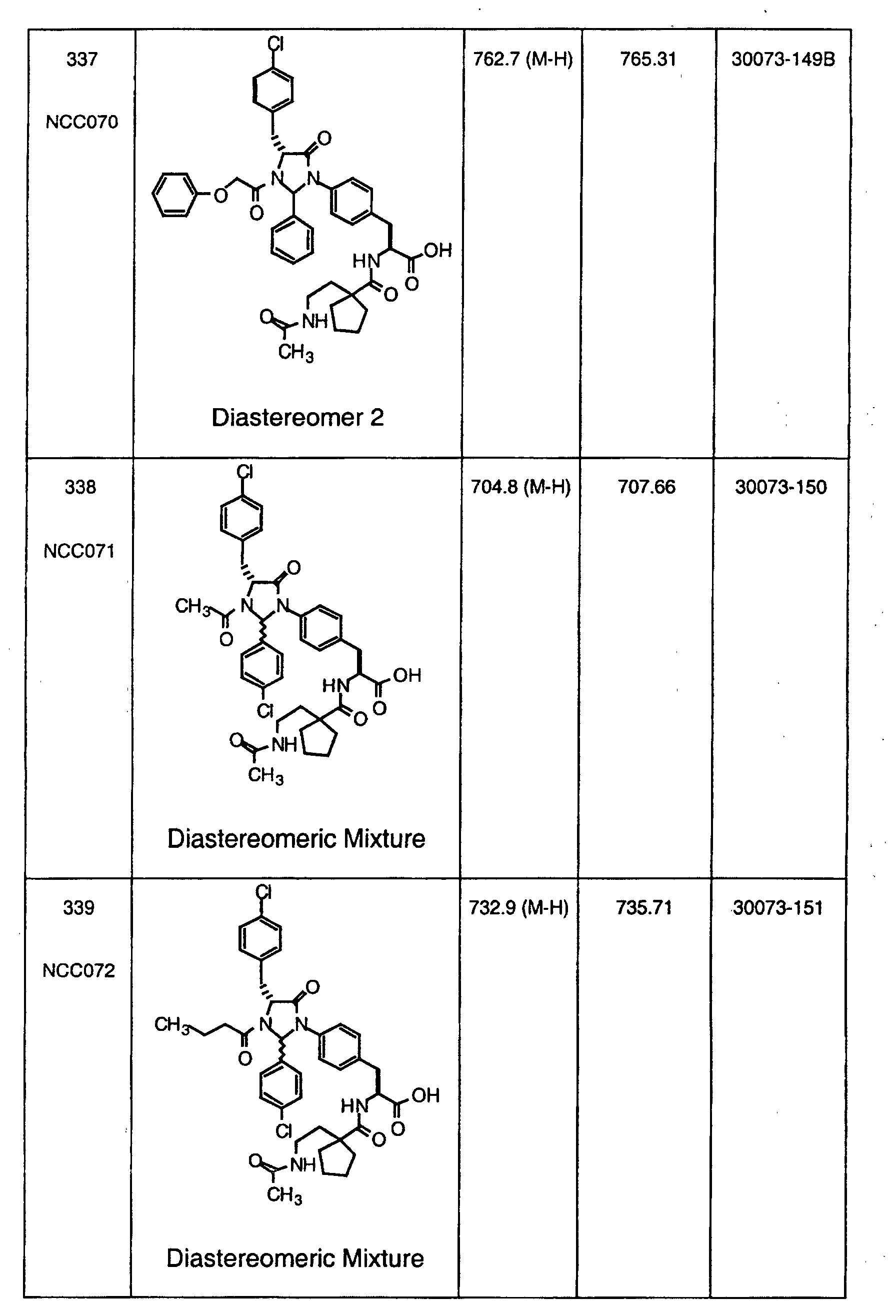

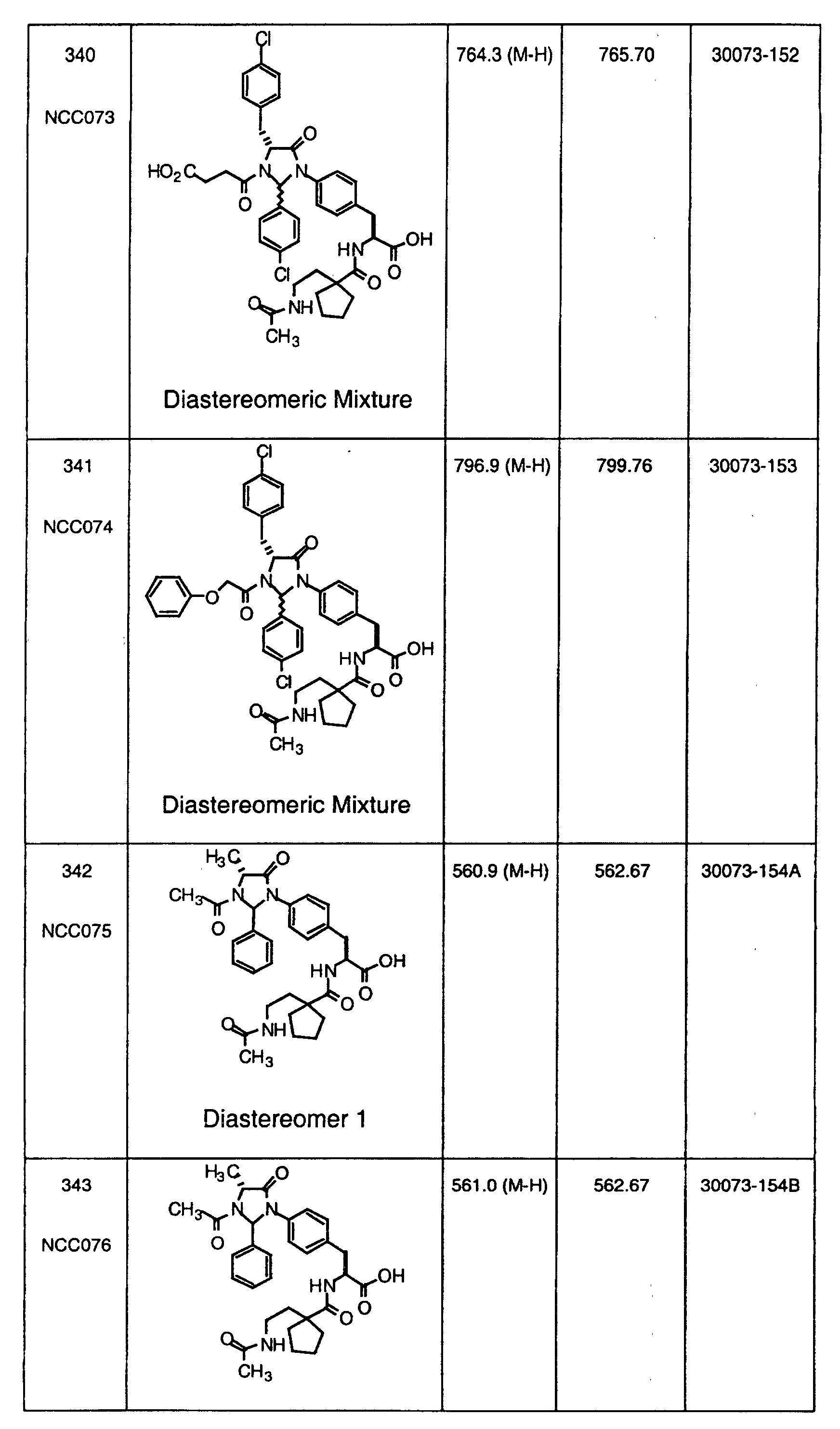

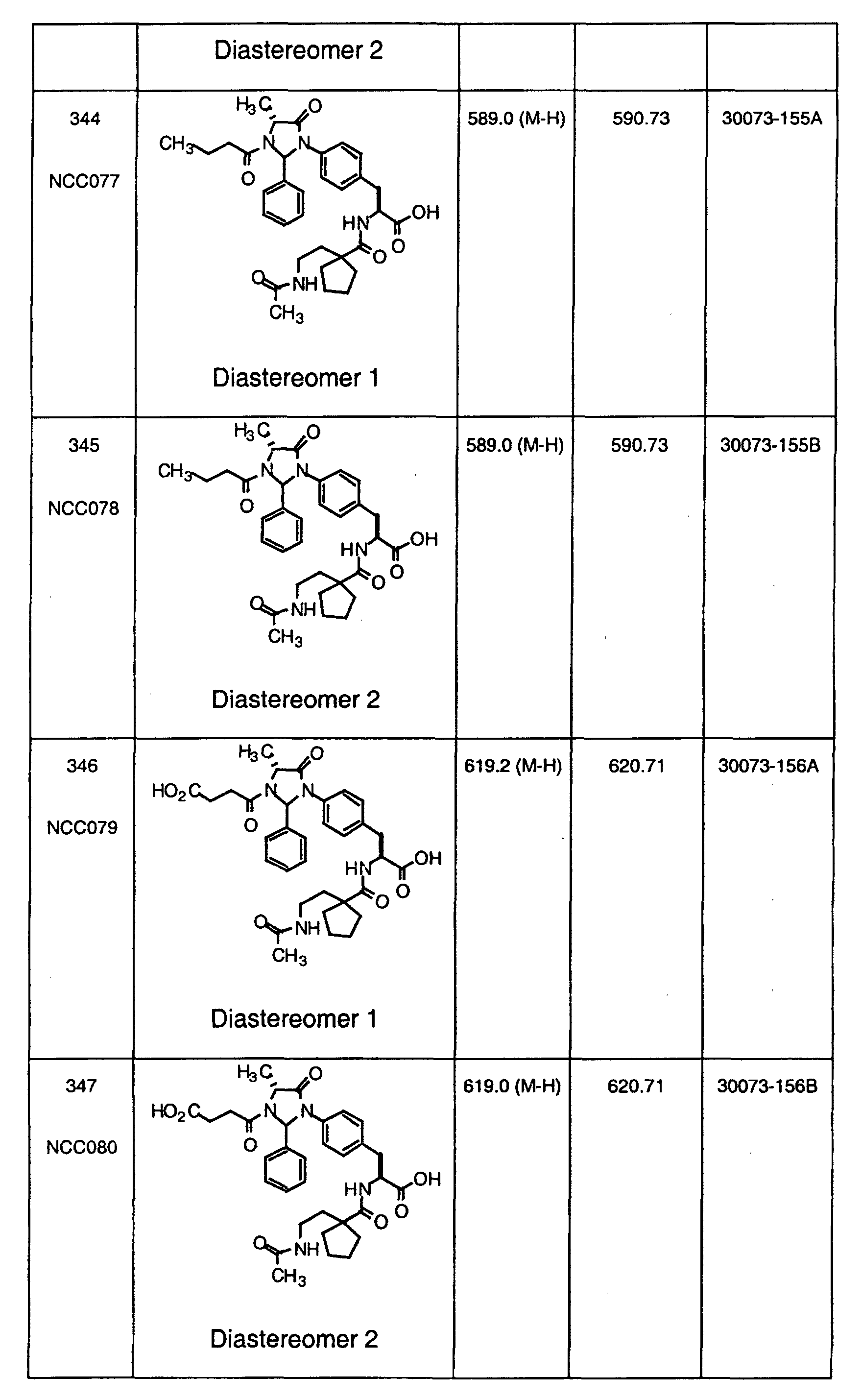

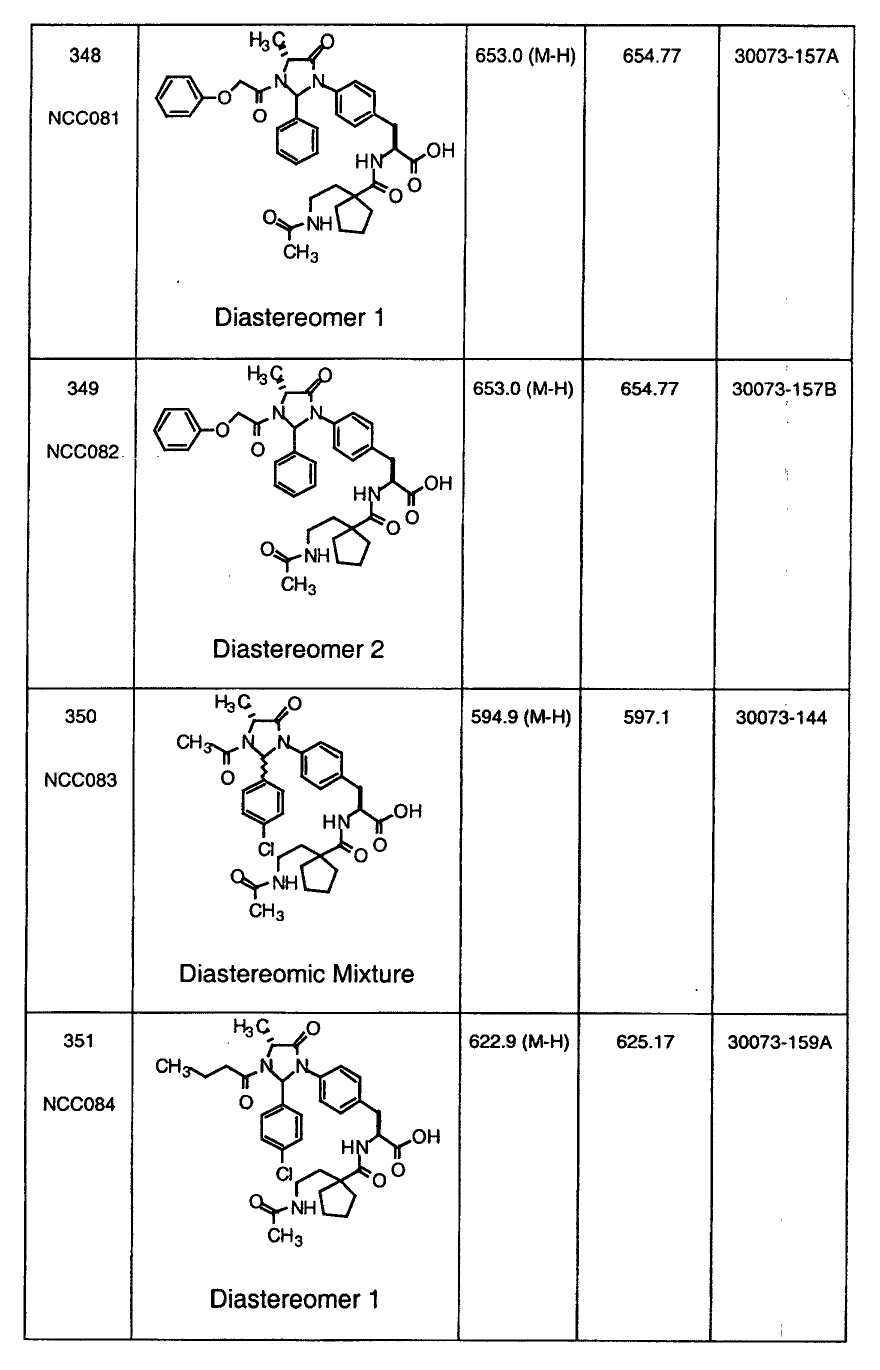

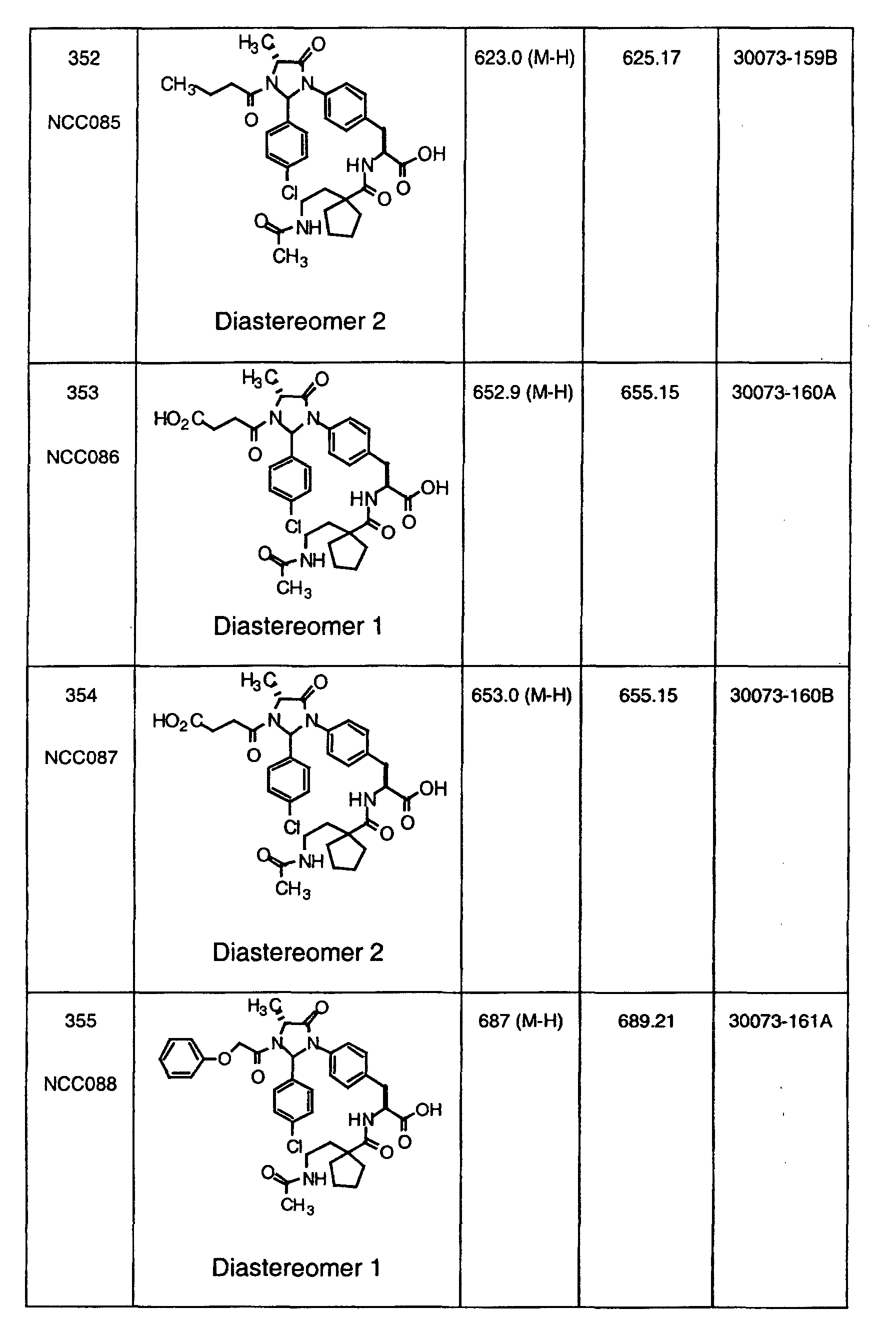

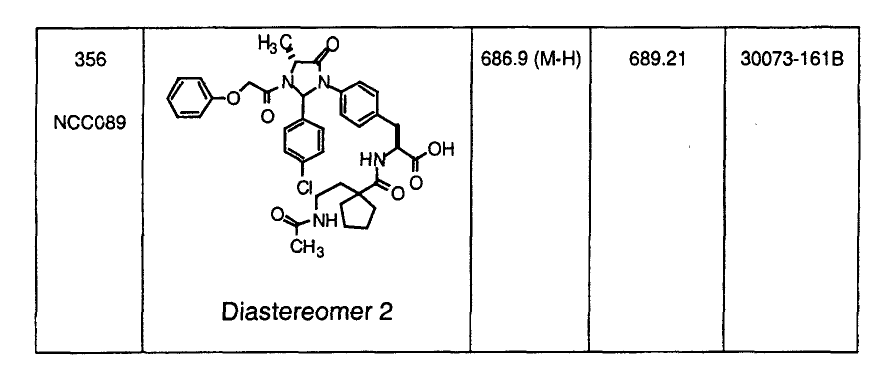

### Example 357. Preparation of 4-[[(2,6-dichlorophenyl)carbonyl]amino]-N-[[1-[(2-(methylsulfinyl)ethyl]cyclopentyl]carbonyl]-L-phenylalanine.

To a suspension of 4-[[(2,6-dichlorophenyl)carbonyl]amino]-N-[[1-[(2-(methylthio)ethyl]cyclopentyl]carbonyl]-L-phenylalanine (0.095 mmol, 50 mg) in ethyl acetate (4 mL) was added THF (1.5 mL) to afford a clear solution. Then, water (3 mL) and oxone (0.048 mmol, 30 mg) was added at room temperature. The mixture was stirred for 15 h at which time TLC analysis of the mixture indicated the absence of starting material. The solid was collected by filtration and washed with water. This material was purified by reverse phase HPLC to afford 26.3 mg (51%) of a white solid: mp 255-258 °C. HR MS (C25H28Cl2N2O5S): Obs mass, 539.1187. Calcd mass, 539.1174 (M+H).

### Example 358. Preparation of 4-[[(2,6-dichlorophenyl)carbonyl]amino]-N-[[1-[(2-[(methylsulfonyl)ethyl]cyclopentyl]carbonyl]-L-phenylalanine.

To a suspension of 4-[[(2,6-dichlorophenyl)carbonyl]amino]-N-[[1-[(2-(methylthio)ethyl]cyclopentyl]carbonyl]-L-phenylalanine (0.095 mmol, 50 mg) in ethyl acetate (4 mL) was added THF (1 mL) to afford a clear solution. Then, water (2 mL) and oxone (0.019 mmol, 12 mg) was added at room temperature. The mixture was stirred for 15 h and the precipitated sulfoxide was collected by filtration and washed with water. Then, the solid was redissolved in acetic acid (2 mL) and treated with 30% hydrogen peroxide (0.7 mL). The mixture was stirred for 15 h at room temperature at which time the TLC analysis indicated the absence of sulfoxide. This mixture was directly purified by reverse phase HPLC to afford 14 mg (66%) of a white solid, mp 184-187 °C. HR MS (C25H28Cl2O6S): Obs mass, 577.0928. Calcd mass, 577.0944 (M+Na).

### Example 359. Preparation of 4-[(2-chloro-5-bromophenylcarbonyl)aminol-N-[(1,1-dimethylethyoxy)carbonyl]-L-phenylalanine methyl ester..

To a mixture of 4-amino-N-[(1,1-dimethylethoxy)carbonyl)-L-phenylalanine methyl ester (20 mmol, 5.88 g), 2-chloro-5-bromobenzoic acid (22 mmol, 5.18 g) and HBTU (22 mmol, 8.34 g) in DMF (70 mL) was added diisopropylethylamine (50 mmol, 8.7 mL) at room temperature. The suspension was stirred for 48 h at which time TLC analysis of the mixture indicated the absence of starting material. Then, the mixture was diluted with water (100 mL) and the solids were collected by filtration and washed with water (150 mL). After air drying, the crude product was purified by silica gel column chromatography to obtain 1.02 g (10%) of a white solid: mp 158-161 °C. HR MS (C22H24BrClN2O5): Ob, 533.0442. Calcd mass, 533.0455 (M+Na).

### Example 360. Preparation of 4-[(2-chloro-5-cyanophenylcarbonyl)amino]-N-[(1,1-dimethylethoxy)carbonyl]-L-phenylalanine methyl ester.

To a mixture of 4-[(2-chloro-5-bromophenylcarbonyl)aminol-N-[(1,1-dimethylethoxy)carbonyl]-L-phenylalanine methyl ester (2 mmol, 1.02 g), zinc cyanide (1.3 mmol, 152 mg) and Pd(PPh₃)₄ (0.2 mmol, 231 mg) was added distilled and deoxygenated DMF (8 mL) at room temperature. The suspension was heated to 80-85 °C and stirred for 15 h at which time TLC analysis of the mixture indicated the absence of starting material. Then, the reaction mixture was cooled to room temperature and diluted with ethyl acetate (70 mL) and washed with 20% aqueous ammonium hydroxide (50 mL), brine solution (50 mL) and was dried over anhydrous magnesium sulfate. Filtration of the drying agent and concentration of the solvent gave a crude product which was purified by silica gel column chromatography to obtain 555 mg (61%) of a white solid, mp 185-187 °C. HR MS (C23H24ClN3O5): Obs mass, 480.1301. Calcd mass, 480.1302 (M+Na).

### Example 361. Preparation of 4-[(2-chloro-5-cyanophenylcarbonyl)amino]-L-phenylalanine methyl ester TFA salt.

To a solution of 4-[(2-chloro-5-cyanophenylcarbonyl)amino]-N-[(1,1-dimethylethoxy)carbonyl]-L-phenylalanine methyl ester (1.2 mmol, 0.55 g) in dichloromethane (12 mL) was added trifluoroacetic acid (3 mL) at room temperature. The reaction mixture was stirred for 15 h at room temperature at which time TLC analysis of the mixture indicated the absence of starting material. Then, the solvent was removed under vacuum and the residue was azeotroped with toluene (2 x 10 mL) and dried under high vacuum to afford 0.43 g (100%) of a yellow solid. HR MS (C18H16ClN303): Obs mass, 358.0963. Calcd mass, 358.0959 (M+H).

### Example 362. Preparation of 4-[(2-chloro-5-cyanophenylcarbonyl)amino]-N-[[1-(2-methoxyethyl)cyclopentyl]carbonyl]-L-phenylalanine methyl ester.

To a solution of 4-[(2-chloro-5-cyanophenylcarbonyl)amino]-L-phenylalanine methyl ester TFA salt (0.55 mmol, 0.35 g), HBTU (0.65 mmol, 0.24 g) and 1-(2-methoxyethyl)cyclopentane carboxylic acid (0.65 mmol, 0.11 g) in DMF (3 mL) was added diisopropylethylamine (1.65 mmol, 0.29 mL) at room temperature. The clear solution was stirred for 15 h at room temperature and diluted with 50 mL of ethyl acetate. Then, the ethyl acetate layer was washed successively with 0.5N hydrochloric acid (2 x 20 mL), saturated sodium bicarbonate solution (2 x 20 mL) and brine solution and was dried over anhydrous magnesium sulfate. Filtration of the drying agent and concentration of the solvent gave a crude product which was purified by silica gel column chromatography to afford 0.25 g (87%) of a white solid: mp 172-175 °C. HR MS (C27H30ClN3O5): Obs mass, 512.1949. Calcd mass, 512.1953 (M+H).

### Example 363. Preparation of 4-[(2-chloro-5-cyanophenylcarbonyl)amino]-N-[[1-(2-methoxyethyl)cyclopentyl]carbonyl]-L-phenylalanine.

To a mixture of 4-[(2-chloro-5-cyanophenylcarbonyl)amino]-N-[[1-(2-methoxyethyl) cyclopentyl]carbonyl]-L-phenylalanine methyl ester (0.1 mmol, 51 mg) and lithium iodide (1.0 mmol, 133 mg) was added pyridine (2 mL) at room temperature. The solution was refluxed for 15 h at which time TLC analysis of the mixutre indicated the absence of starting material. Then, the mixture was cooled to room temperature and diluted with water (15 mL) and the bulk of the pyridine was removed under reduced pressure. Then it was extracted with ether (2 x 15 mL) to remove any neutral impurities. The aqueous layer was acidified with 1N HCl and the precipitated white solid was collected by filtration and washed with 20 mL of water and 20 mL of hexane. After air-drying, the crude product was crystallized from acetonitrile to afford 20 mg (40%) of a white solid: mp 169-172 °C. HR MS (C26H26ClN3O5): Obs mass, 498.1802. Calcd mass, 498.1795, M+H).

### Example 364. Preparation of 4-[[(2,4-dimethylpyridin-3-yl)carbonyl]amino]-N-[[1-[4-(methylsulfonyl)butyl]cyclopentyl]carbonyl]-L-phenylalanine.

a. To a solution of 2,4-dimethylpyridinecarboxylic acid (0.6 mmol, 102 mg) in dichloromethane (3 mL) was added a drop of DMF and oxalyl chloride (0.78 mmol, 99 mg) at 0 °C (ice bath). The solution was stirred at this temperature for 30 min, warmed to room temperature and stirred for an additional 1h. Then, the solvent and excess oxalyl chloride was removed under vacuum and the residue was dried under high vacuum. To this 4-amino-N-[[1-[4-(methylsulfonyl)butyl]cyclopentyl]carbonyl]-L-phenylalanine methyl ester (0.5 mmol, 212 mg) was added and the mixture was dissolved in dichloromethane (5 mL). To this clear solution was added DIPEA (2.0 mmol, 0.258 g) at room temperature. The mixture was stirred for 15 h at which time TLC analysis of the mixture indicated the absence of starting material. Then, the mixture was diluted with dichloromethane (20 mL) and water (100 mL). The two layers were separated and the organic layer was washed with saturated sodium bicarbonate solution (20 mL), brine solution (30 mL) and dried over anhydrous magnesium sulfate. Filtration of the drying agent and removal of the solvent gave a crude product which was purified by silica gel column chromatography to afford 0.232 g (80%) of a white solid. HR MS (C29H39N3O6S): Obs. mass,558.2629. Calcd. mass, 558.2638, M+H).
b. Preparation of 4-[[(2,4-dimethyl-3-pyridinyl)carbonyl]amino]-N-[[1-[4-(methylsulfonyl) butyl]cyclopentyl]carbonyl]-L-phenylalanine.

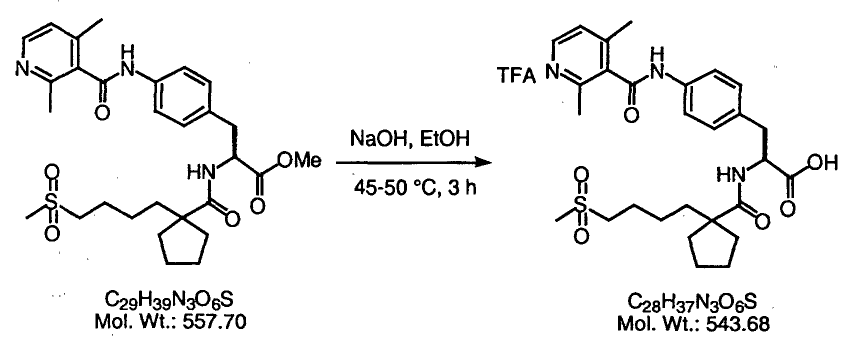

Using the procedure described in example 47, 4-[[(2,4-dimethyl-3-pyridyl)carbonyl] amino]-N-[[1-[4-(methylsulfonyl)butyl]cyclopentyl]carbonyl]-L-phenylalanine methyl ester prepared above was hydrolyzed in 88% yield to give a white solid. HR MS (C28H37N3O6S): Obs. mass, 544.2471. Calcd. mass, 544.2481 (M+H).

### Example 365. 4-[(4R)-3-acetyl-5-oxo-2-phenyl-4-(phenylmethyl)-1-imidazolidinyl]-N-[[1-[4-(methylsulfonyl)butyl]cyclopentyl]carbonyl]-L-phenylalanine.

a. Synthesis of N-[(1,1-dimethylethoxy)carbonyl]-4-[[(2R)-2-amino-1-oxo-3-phenylpropyl]amino]-L-phenylalanine methyl ester
   To the solution of 4-amino-N-[(1,1-dimethylethoxy)carbonyl]-L-phenylalanine methyl ester (5.09 g, 17 mmol) in DMF (60 mL) was added Fmoc-D-Phenylalanine (8.70 g, 22.5 mmol), DIPEA (12 mL, 69 mmol) and HBTU (8.50 g, 22.5 mmol). The mixture was then stirred at room temperature for 4 h. The reaction mixture was diluted with water (150 mL) and the light yellow solid which precipitated was collected by filtration. This solid was then redissolved in 60 mL of acetone and the solution was treated with 100 mL of water. The solid was collected by filtration and was washed with 1N HCl, H₂O. After drying at 60 °C under vaccum overnight, a light yellow solid was obtained (13.2 g). A portion of this solid (2.51 g, 3.78 mmol) was dissolved in 15 mL of DMF and to the solution was added 1.5 mL of piperidine. The above solution was stirred at room temperature for 45 min. After removal of the solvent, the residue was recrystillized from ethyl acetate-hexane to give N-[(1,1-dimethylethoxy)carbonyl]-4-[[(2R)-2-amino-1-oxo-3-phenylpropyl]amino]-L-phenylalanine methyl ester (1.36 g, 3.0 mmol ) in 81.5 % yield. LR MS 442 (M+H).
b. Synthesis of 4- (3-acetyl-5-oxo-2-phenyl-4-phenylmethyl-1-imidazolidinyl)-N-[(1,1,-dimethylethoxy)carbonyl]-L-phenylalanine methyl ester
   A solution of above amine (1.48, 3.35 mmol) and benzaldehyde (376 µl, 3.7 mmol) in dichloromethane (10 mL) and methyl orthoformate (10 mL) was stirred at room temperature for 3 days. The reaction flask was then warmed to 90° C and acetic anhydride (neat, 1.8 mL) was added. The resulting mixture was stirred at 110° C for 4 hr. The solvent was then evaporated and crude product was purified by silica gel chromatography (ethyl acetate:hexane = 1:1) to give 4-(3-acetyl-5-oxo-2-phenyl-4-phenylmethyl-1-imidazolidinyl)-N-[(1,1,-dimethylethoxy) carbonyl]-L-phenylalanine methyl ester diatereomer 1 (417 mg) and diastereomer 2 (1.25 g) These compounds are diastereomeric at the 2-position of the imidazolidinone ring. Both diastereomers gave LR MS (C33H37N3O6): 572 (M+H).
c. 4-(3-acetyl-5-oxo-2-phenyl-4-phenylmethyl-1-imidazolidinyl)-N-[(1,1-dimethylethoxy) carbonyl]-L-phenylalanine methyl ester (Diastereomer 1) (415 mg, 0.7 mmol) was treated with 10 mL of 4N HCl in dioxane at room temperature for 2 hr. After removal of solvent, the residue was dried overnight under vacuum. The residue (241 mg, 0.471 mmol) was dissolved in DMF (4 mL) and was treated with 1-(4-methylsulfonyl)butyl)cyclopentane carboxylic acid (153 mg, 0.617 mmol), HBTU (234 mg, 0.617 mmol) and DIEA (246 µL, 1.42 mmol) at room temperature for 4 hr. The mixture was diluted with 30 mL of ethyl acetate, the mixture was washed with 1N HCl, water and brine ( 8 mL each), After it was dried over MgSO4, the solvent was removed and the residue was filtered through silica gel eluting with ethyl acetate:hexane = 4:1 to give 4-(3-acetyl-5-oxo-2-phenyl-4-phenylmethyl-1-imidazolidinyl)-N-[[1-[4-(methyl-sulfonyl)butyl]cyclopentyl]carbonyl]-L-phenylalanine methyl ester diastereomer 1 (147 mg, 0.2 mmol) in 44% yield. LR MS: 702 (M+H).
d. 4-(3-acetyl-5-oxo-2-phenyl-4-phenylmethyl-1-imidazolidinyl)-N-[[1-[4-(methylsulfonyl) butyl]cyclopentyl]carbonyl]-L-phenylalanine methyl ester diastereomer 1 (90 mg, 0.128 mmol) in EtOH (3 mL) was treated with NaOH (1N, 0.3 mL) at room temperature for 30 min. The resulting solution was acidified with 1 drop of HOAc and was purified by HPLC (C-18, linear gradent from 5% acetonitrile to 95% in water over 30 min) to give 84 mg (95%, 0.122 mmol) of 4-(3-acetyl-5-oxo-2-phenyl-4-phenylmethyl-1-imidazolidinyl)-N-[[1-[4-(methylsulfonyl)butyl] cyclopentyl]carbonyl]-L-phenylalanine diastereomer 1. LR MS: 688 (M+H).

Example 366. 4-[(4R)-3-Acetyl-5-oxo-2-phenyl-4-(3-pyridinylmethyl)-1-imidazolidinyl]-N-[(1-phenylcyclopentyl)carbonyl]-L-phenylalanine was prepared from Fmoc-D-3-pyridinylalanine, benzaldehyde and 4-amino-N-[(1,1-dimethylethoxy)carbonyl]-L-phenylalanine methyl ester using the general procedure described in example 365.
MS: 631 (M+H)

### Example 367. Preparation of 4-(5-bromo-1,3-dioxo-2H-isoindol-2-yl)-N-[(1,1-dimethylethoxy)carbonyl]-L-phenylalanine methyl ester.

To a suspension of 4-amino-N-[(1,1-dimethylethoxy)carbonyl]-L-phenylalanine methyl ester (6.78 mmol, 1.99 g) in dichloromethane (90 mL) was added a solution of 4-bromophthalic anhydride (6.78 mmol, 1.54 g) in dichloromethane (30 mL) and 1,1'-carbonyldiimidazole (6.78 mmol, 1.1 g) at room temperature. The resulting solution was stirred for 15 h at which time TLC analysis of the mixture indicated the absence of starting material. The mixture was diluted with water (100 mL) and layers were separated. The aqueous layer was extracted with dichloromethane (2 x 100 mL) and the combined extracts were washed with brine solution and dried over anhydrous magnesium sulfate.

Filtration of the drying agent and concentration of the solvent under vacuum gave a crude product which was purified by silica gel column chromatography to obtaine 2.4 g (70%) of a white solid: mp 168-170 °C. HR MS C23H23BrN2O6): Obs mass, 525.0656. Calcd mass, 525.0637 (M+Na).

### Example 368. Preparation of 4-(5-cyano-1,3-dioxo-2H-isoindol-2-yl)-N-[(1,1-dimethylethoxy) carbonyl]-L-phenylalanine methyl ester.

To a mixture of 4-(5-bromo)1,3-dioxo-2H-isoindol-2-yl)-N-[(1,1-dimethylethoxy)carbonyl]-L-phenylalanine methyl ester (0.5 mmol, 0.25 g), zinc cyanide (0.3 mmol, 35 mg) and Pd(PPh₃)₄ (0.05 mmol, 57.7 mg) was added distilled and deoxygenated DMF (2 mL) at room temperature. The suspension was heated to 80-85 °C and was stirred for 15 h under argon an atmosphere. At this time TLC analysis of the mixture indicated the absence of starting material. The reaction mixture was cooled to room temperature and was diluted with ethyl acetate (50 mL) and was washed with 20% aqueous ammonium hydroxide (50 mL) and brine solution (50 mL) and was dried over anhydrous magnesium sulfate. Filtration of the drying agent and concentration afforded a crude product which was purified by silica gel column chromatography to afford 170 mg (75%) of a white solid. HR MS (C24H23N3O6): Obs mass, 472.1472. Calcd mass, 472.1485 (M+Na).

### Exmaple 369. Preparation of 4-(5-cyano-1,3-dioxo-2H-isoindol-2-yl)-L-phenylalanine methyl ester TFA salt.

To a solution of 4-(5-cyano-1,3-dioxo-2H-isoindol-2-yl)-N-[(1,1,-dimethylethoxy)carbonyl]-L-phenylalanine methyl ester (1.33 mmol, 0.6 g) in dichloromethane (12 mL) was added trifluoroacetic acid (3 mL) at room temperature. The reaction mixture was stirred for 15 h at room temperature at which time TLC analysis of the mixture indicated the absence of starting material. The solvent was removed under vacuum and the residue was azeotrophed with toluene (2 x 10 mL) and dried under high vacuum to afford 0.46 g (100%) of a yellow solid. HR MS (C19H15N3O4): Obs mass, 350.0156. Calcd mass, 350.0183 (M+H).

Example 372. 4-[[(2,4-Dimethyl-3-pyridinyl)carbonyl)amino)-N-[[1-[4-(methylsulfonyl) butyl]cyclopentyl]carbonyl]-L-phenylalanine ethyl ester was prepared using the general method described in example 123 starting with 4-[[(2,4-dimethylpyridin-3-yl)carbonyl]amino]-N-[[1-[4-(methylsulfonyl)butyl]cyclopentyl]carbonyl]-L-phenylalanine.

Example 373. 4-[[(2,6-Dichlorophenyl)carbonyl]amino]-N-[[1-(2-methoxyethyl)cyclopentyl]carbonyl]-L-phenylalanine ethyl ester was prepared using the general method described in example 123 starting with 4-[[(2,6-dichlorophenyl)carbonyl]amino]-N-[[1-(2-methoxyethyl)cyclopentyl]carbonyl]-L-phenylalanine.

Example 374. 4-[[(2,6-Dichlorophenyl)carbonyl]amino]-N-[[-1-(4-(methylsulfonyl)butyl] cyclopentyl]carbonyl]-L-phenylalanine ethyl ester was prepared using the general method described in example 123 starting with 4-[[(2,6-dichlorophenyl)carbonyl]amino]-N-[[-1-[4-(methylsulfonyl)butyl]cyclopentyl]carbonyl]-L-phenylalanine.

Example 375. 4-[(4R)-3-acetyl-4-(phenylmethyl)-5-oxo-2-phenyl-1-imidazolidinyl]-N-[[1-[4-(methylsulfonyl)butyl]cyclopentyl]carbonyl)-L-phenylalanine ethyl ester was prepared using the method described in example 123 from 4-[(4R)-3-acetyl-4-(phenylmethyl)-5-oxo-2-phenyl-1-imidazolidinyl]-N-[[1-[4-(methylsulfonyl)butyl]cyclopentyl]carbonyl]-L-phenylalanine.

Example 377. 4-[[(2,6-Dichlorophenyl)carbonyl]amino]-N-[[1-(2-methoxyethyl)cyclopentyl] carbonyl]-L-phenylalanine 2-(4-morpholino)ethyl ester was prepared using the general method described in example 277 starting with 4-[[(2,6-dichlorophenyl)carbonyl]amino]-N-[[1-(2-methoxyethyl)cyclopentyl] carbonyl]-L-phenylalanine.

Example 378. 4-[(2,4-Dimethyl-3-pyridinyl)carbonyl]amino]-N-1-[(4-methylsulfonyl)butyl] cyclopentyl]carbonyl-L-phenylalanine 2-(4-morpholino)ethyl ester was prepared from 4-[(2,4-dimethyl-3-pyridinyl)carbonyl]amino]-N-1-[(4-methylsulfonyl)butyl]cyclopentyl]carbonyl-L-phenylalanine using the procedure described in example 277.

Example 379. 4-[[(2,6-dichlorophenyl)carbonyl]amino]-N-[[1-[4-(methylsulfonyl)butyl] cyclopentyl]carbonyl]-L-phenylalanine 2-(4-morpholino)ethyl ester was prepared from 4-[[(2,6-dichlorophenyl)carbonyl]amino]-N-[[1-[4-(methylsulfonyl)butyl]cyclopentyl]carbonyl]-L-phenylalanine using the procedure described in example 277.

Example 380. 4-[(4R)-3-acetyl-4-(phenylmethyl)-5-oxo-2-phenyl-1-imidazolidinyl)-N-[[1-[4-(methylsulfonyl)butyl]cyclopentyl]carbonyl]-L-phenylalanine 2-(4-morpholino)ethyl ester was prepared from 4-[(4R)-3-acetyl-4-(phenylmethyl)-5-oxo-2-phenyl-1-imidazolidinyl]-N-[[1-[4-(methylsulfonyl)butyl]cyclopentyl]carbonyl]-L-phenylalanine using the procedure described in example 277.

Example 380a. 4-[[(2,6-Dichlorophenyl)carbonyl]amino]-N-[[1-(2-methoxyethyl)cyclopentyl] carbonyl]-L-phenylalanine 2-(N,N-diethylamino)ethyl ester was prepared using the general method described in example 277 starting with 4-[[(2,6-dichlorophenyl)carbonyl]amino]-N-[[1-(2-methoxyethyl)cyclopentyl]carbonyl]-L-phenylalanine.

Example 381. 4-[(2,6-Dimethyl-4-trifluoromethyl-3-pyridinyl)carbonyl]amino]-N-[[1-[4-(methylsulfonyl)butyl]cyclopentyl]carbonyl]-L-phenylalanine was prepared from 4-amino-N-[[1-[4-(methylsulfonyl)butyl]cyclopentyl]carbonyl]-L-phenylalanine methyl ester and 2,6-dimethyl-4-trifluoromethyl-3-pyridine carboxylic acid using the general procedure described in example 364.

### Example 382. Preparation of 4-trifluoromethyl-5-pyrimidine carboxylic acid.

A solution of 2-chloro-4-trifluoromethyl-5-pyrimidine carboxylic acid benzyl ester in cyclohexene (3 mL, 30 mmole) and ethanol (9 mL) was treated with 10 % palladium on carbon and the resulting mixture was heated to reflux for 1 h. The mixture was cooled to room temperature and filtered through a pad of Celite and concentrated to give a quantitative yield of a gummy, off-white solid. LR ES MS (C6H3F3N2O2): 191 (M-H).

Examples 383-387. The 4-[[(heteroaryl)carbonyl]amino]-N-[[1-[4-(methylsulfonyl)butyl] cyclopentyl]carbonyl]-L-phenylalanine derivatives shown in the table below were prepared from 4-amino-N-[[1-[4-(methylsulfonyl)butyl]cyclopentyl]carbonyl]-L-phenylalanine methyl ester and the appropriate heteroaromatic carboxylic acids using the general procedure described in example 34

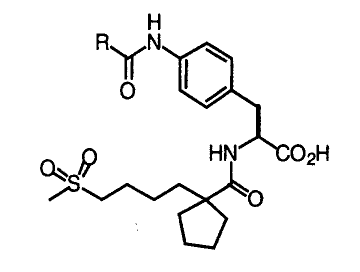

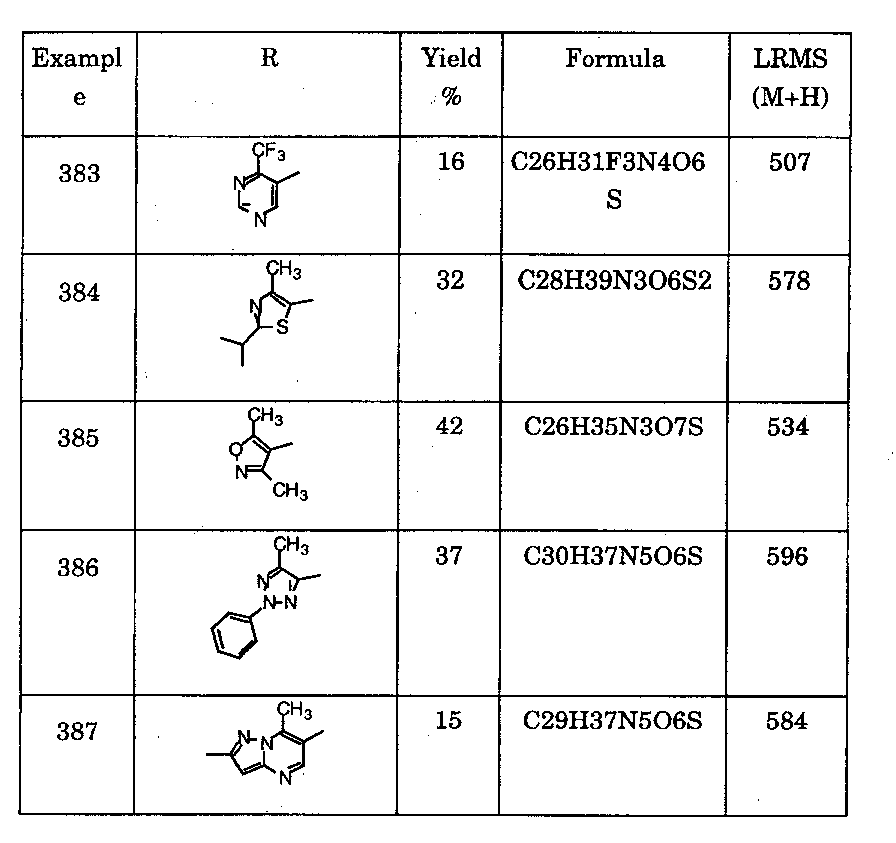

### Example 388. Preparation of 4-amino-N-methyl-N-[[1-(2-methoxyethyl)cyclopentyl]carbonyl]-L-phenylalanine methyl ester.

To a mixture of N-[[1-(2-methoxyethyl)cyclopentyl]carbonyl]-N-methyl-4-nitro-L-phenylalanine methyl ester (1.35 mmol, 530 mg), zinc dust (-325 mesh, 13.5 mmol, 0.88 g, 10 equiv.) and ammonium chloride (20.2 mmol, 1.08 g, 15 equiv.) was added methanol (10 mL) and water (5 mL) at room temperature. After addition of water, the reaction was exothermic. The suspension was stirred for 2 h at room temperature at which time TLC analysis of the mixture indicated the absence of starting material, the reaction mixture was filtered through a pad of celite and the filter cake was washed with methanol (50 mL) and water (40 mL). The mixture was concentrated and extracted with ethyl acetate (3 x 30 mL). The combined extracts were washed with brine solution (30 mL) and dried over anhydrous magnesium sulfate. Filtration of the drying agent and concentration of the solvent afforded 490 mg (100%) of a yellow oil. HR MS (C20H30N2O4): Obs. mass,362.2202. Calcd. mass, 362.2206 (M+).

Example 389. 4-[[(2,6-Dichlorophenyl)carbonyl]amino]-N-methyl-N-[[1-(2-methoxyethyl) cyclopentyl]carbonyl]-L-phenylalanine methyl ester was prepared from of 4-amino-N-methyl-N-[[1-(2-methoxyethyl)cyclopentyl]carbonyl]-L-phenylalanine methyl ester and 1-(2-methoxyethyl)cyclopentane carboxylic acid using the general procedure described in example 44 to afford a 64% yield of a white solid. HR MS (C27H32Cl2N2O5): Obs. mass, 535.1742. Calcd. mass, 535.1766 (M+H).

Example 390. 4-[[(2,6-dichlorophenyl)carbonyl]amino]-N-methyl-N-[[1-(2-methoxyethyl) cyclopentyl]carbonyl]-L-phenylalanine was prepared from 4-[[(2,6-dichlorophenyl) carbonyl] amino]-N-[[1-(2-methoxyethyl)cyclopentyl)carbonyl]-N-methyl-L-phenylalanine methyl ester using the general procedure described in example 47 to give a 43% yield of a white solid. HR MS (C₂₆H₃₀Cl₂N₂O₅): Obs. mass, 519. 1453. Calcd mass, 519. 1454 (M-H).

### Example 391. Preparation of 1-(2-methoxyethyl)cyclopentane carboxylate methyl ester.

To a solution of diisopropylamine (21 mL, 150 mmol) in THF (100 mL) was added dropwise a solution of n-butyl lithium (58 mL, 145 mmol) in hexanes at - 10 °C while maintaining the temperature below 0 °C. After addition, the solution was stirred for 30 min at 0 °C. To this, a solution of methyl cyclopentane carboxylate (12.8 g, 100 mmol) in THF (20 mL) was added dropwise at -70 °C maintaining the internal temperature between -60 to -70 °C. After addition, the reaction mixture was stirred for 30 min at -50 to -60 °C. Then, a solution of 2-methoxy ethyl bromide (12.5 g, 90 mmol) in THF (20 mL) was added dropwise and the light brown suspension was stirred for 30 min at - 60 to -70 °C. Then, it was allowed to warm to room temperature and stirred overnight. The reaction mixture was poured into a saturated solution of ammonium chloride (250 mL) and was extracted with ether (2 x 100 mL). The combined extracts were washed with a saturated solution of sodium chloride (100 mL) and dried over anhydrous magnesium sulfate. After filtration of the drying agent, the solution was concentrated under vacuum to afford 16.55 g of crude product as a black liquid. Distillation at 70-75 °C/1.5 mm Hg afforded 7.98 g of a colorless oil and a further 2.76 g as a light yellow oil for a total yield of 10.74 g (64%). HR MS (C10H18O3): Obs. mass, 186.1257. Calcd. mass, 186.1256 (M+).

### Example 392. Preparation of 1-(2-methoxyethyl)cyclopentane carboxylic acid.

To a solution of 1-(2-methoxyethyl)cyclopentane carboxylate methyl ester (7.987 g, 42.9 mmol) in a mixture of THF (170 mL) and methanol (170 mL) was added 1 N sodium hydroxide (170 mL). The mixture was heated to 40 °C for 2 h at which point TLC (ether:hexane 1:1, iodine chamber) analysis indicated the absence of starting material and the mixture was cooled to room temperature. The solvent was removed under vacuum and the residue was diluted with water (100 mL) and was extracted with ether (2 x 200 mL) to remove any neutral impurities. The basic aqueous layer was acidified with 1 N hydrochloric acid and the product was extracted with ethyl acetate (2 x 100 mL). The combined extracts were washed with brine solution and dried over sodium sulfate. After filtration of the drying agent, the solution was concentrated under vacuum and the residue was dried under high vacuum to afford 6.183g (82%) of a light brown oil. HR MS (C9H16O3 Obs. mass, 172.0154. Calcd. mass, 172.0126 (M+).

### Example 393. Preparation of N-[(1,1-dimethylethoxy)carbonyl]-4-nitro-L-phenylalanine methyl ester.

To suspesion of of 4-nitro-N-[(1,1-dimethylethoxy)carbonyl]-L-phenylalanine (226.2 mmol, 70.2 g) and sodium carbonate (1.13 mol, 95 g) in DMF (500 mL) was added methyl iodide (1.13 mol, 70.4 mL) at room temperature. The suspesion was stirred for 15 h at room temperature at this time TLC analysis of the mixture indicated the absence of starting acid and the excess methyl iodide and some DMF were removed under high vacuum. The residue was poured into water (2 L) and stirred at room temperature as a precipitate formed slowly over 72 h. The precipitated solids were collected by filtration and washed with water (2 L). After air and vacuum drying, 72 g (98%) of a light yellow solid, mp 95-96 °C were obtained. ¹H-NMR, (DMSO-d₆) (400 MHz) δ 8.16 (d, 2H, J = 20 Hz), 7.53 (d, 2H, J = 20 Hz), 7.39 (d, 1H, J = 22 Hz), 4.26-4.28 (m, 1H), 3.6 (s, 3H), 2.96-3.19 (m, 2H), 1.25 (s, 9H). ¹³C NMR, CDCl₃ (100 Mhz) δ 172.04, 155.29, 146.27, 145.96, 130.48, 123.18, 78.36, 54.44, 51.9, 36.1, 27.99. HR MS: Obs. mass, 325.1404. Calcd. mass, 325.1400 (M+H).

### Example 394. Preparation of of 4-amino-N-[(1,1-dimethylethoxy)carbonyl]-L-phenylalanine methyl ester.

To a mixture of of N-[(1,1-dimethylethoxy)carbonyl]-4-nitro-L-phenylalanine methyl ester (222 mmol, 72 g), zinc dust (-325 mesh, 2.2 mol, 145.2 g, 10 equiv) and ammonium chloride (3.3 mol, 178.1 g, 15 equiv) was added methanol (1 L) and water (500 mL) at room temperature. After addition of water, an exothermic reaction ensued and the internal temperature rose to 45 to 50 °C. The suspension was stirred for 30 min to 1 h at room temperature, at which time TLC analysis of the mixture indicated the absence of starting material, and the reaction mixture was filtered through a pad of celite and the filtered cake was washed with methanol (1 L) and water (500 mL). Concentration to remove most of the methanol afforded white solid which was collected by filtration and washed with water. After air drying, 65.5 g of a white solid, mp 86-89 °C was obtained. ¹H-NMR (DMSO-d₆) (400 MHz) δ 6.9 (d, 2H, J = 20 Hz), 6.62 (d, 2H, J = 20 Hz), 7.39 (d, 1H, J = 22 Hz), 4.26-4.28 (m, 1H), 3.68 (s, 3H), 2.96-3.19 (m, 2H), 1.25 (s, 9H). HR MS: Obs. mass, 294.1614. Calcd. mass, 294.1621 (M+).

### Example 395. Preparation 4-[[(2,6-dichlorophenyl)carbonyl)amino]-N-[(1,1-dimethylethoxy)carbonyl]-L-phenylalanine methyl ester.

To a solution of 4-amino-N-[(1,1-dimethylethoxy)carbonyl]-L-phenylalanine methyl ester (127.6 mmol, 37.57 g) and 2,6-dichlorobenzoyl chloride (140.6 mmol, 29.45 g) in dichloromethane (350 mL) was added diisopropylethylamine (192 mmol, 33.4 mL) at room temperature. The brown solution was stirred for 15 h at room temperature to afford a white suspension. At this time, TLC analysis of the mixture indicated the absence of starting material. The solids were collected by filtration and were washed with dichloromethane (150 mL) and air dried to obtain 52.75 g (88.4%) of a white solid: mp 192-194 °C. ¹H NMR (DMSO-d₆) (400 MHz) δ 10.68 (s, 1H), 7.47-7.6 (m, 5H), 7.2-7.29 (m, 3H), 4.12-4.17 (m, 1H), 3.62 (s, 3H), 2.79-2.99 (m, 2H), 1.33 (s, 9H). ¹³C NMR, CDCl₃ (100 Mhz) d 172.49, 161.82, 155.37, 136.99, 136.36, 131.28, 131.16, 129.48, 128.19, 119.31, 78.27, 55.3, 51.76, 35.9, 27.77. HR MS: Obs. mass, 466.1069. Calcd. mass, 466.1062).

### Example 396. Preparation of 4-[[(2,6-dichlorophenyl)carbonyl]amino]-L-phenylalanine methyl ester hydrochloride salt.

Solid 4-[[(2,6-dichlorophenyl)carbonyl]amino]-N-[(1,1-dimethylethoxy)carbonyl]-L-phenyl alanine methyl ester (92.97 mmol, 43.45 g) in dioxane (90 mL) was treated with 166 mL of 4.0 N hydrochloric acid in dioxane at room temperature. After 5 minutes, the solids went into solution and the mixture was stirred for 2 h. The reaction mixture was concentrated a yellow syrup and 250 mL of ethyl ether was added. A gum was formed which was dissolved in THF (100 mL) and methanol (100 mL). The solvent was removed under vacuum to obtain 43.7 (100%) of a white solid: mp 180-195 °C. ¹H NMR (DMSO-d₆) (400 MHz) δ 10.81 (s, 1H), 7.76 (d, 2H, J = 22 Hz), 7.58 (d, 2H, J = 18 Hz), 7.51 (t, 1H, J = 15 Hz), 7.24 (d, 2H, J = 22 Hz),4.23-4.26 (m, 1H), 3.56 (s, 3H), 3.14-3.17 (m, 2H). ¹³C NMR, CDCl₃ (100 Mhz) d 169.03, 161.72, 137.56, 136.11, 131.19, 130.95, 129.93, 129.79, 128.06, 119.46, 53.17, 52.6, 35.13. HR MS (C17H16Cl2N2O3•HCl): Obs. mass, 367.0611. Calcd. mass, 367.0616 (M+H).

### Example 397. Preparation of 4-[[(2,6-dichlorophenyl)carbonyl]amino]-N-[[1-(2-methoxyethyl)cyclopentyl]carbonyl]-L-phenylalanine methyl ester.

To a solution of 4-[[(2,6-dichlorophenyl)carbonyl]amino]-L-phenylalanine methyl ester hydrochloride salt (43.03 mmol, 11.75 g) and 1-(2-methoxyethyl)cyclopentane carboxylic acid (43.5 mmol, 7.5 g) in DMF (130 mL) was added HBTU (43.5 mmol, 16.5 g) and diisopropylethylamine (108.8 mmol, 19.02 mL) at room temperature. The clear solution was stirred 23 h at room temperature and was diluted with 200 mL of ethyl acetate. The ethyl acetate layer was washed successively with 0.5 N hydrochloric acid (2 x 100 mL), saturated sodium bicarbonate solution (2 x 100 mL), brine solution and was dried over anhydrous magnesium sulfate. Filtration of the drying agent and concentration of the solvent gave 18.86 g (84%) of a white solid, mp 85-87 °C. ¹H NMR (DMSO-d₆) (400 MHz) δ 10.65 (s, 1H), 7.88 (d, 1H, J = 19 Hz), 7.47-7.59 (m, 5H), 7.21 (d, 2H, J = 19 Hz), 4.47-4.53 (m, 1H), 3.64 (s, 3H), 2.88-3.1 (m, 7H), 1.76-1.98 (m, 4H), 1.23-1.47 (m, 6H). HR MS C26H30Cl2N2O5): Obs. mass, 521.1586. Calcd. mass, 521.1610 (M+H).

### Example 398. Preparation of 4-[[(2,6-dichlorophenyl)carbonyl]amino]-N-[[1-(2-methoxyethyl)cyclopentyl]carbonyl]-L-phenylalanine.

To a suspension of 4-[[(2,6-dichlorophenyl)carbonyl]amino]-N-[[1-(2-methoxyethyl) cyclopentyl]carbonyl]-L-phenylalanine methyl ester (20.17 mmol, 10.52 g) in ethanol (80 mL) and tetrahydrofuran (10 mL) was added aqueous 1.0 N sodium hydroxide (80 mL) at room temperature. The mixture was heated to 50 °C and the resulting clear solution was stirred overnight. Then, the ethanol solution was concentrated and diluted with 50 mL of water and was extracted with 200 mL of ether to remove neutral impurities. The aqueous layer was acidified with 1N HCl and the precipitated white solid was collected by filtration and washed with 200 mL of water and 200 mL of hexane. After air-drying, 8.4 g (82%) of as a white solid: mp 136-140 °C was obtained. ¹H NMR, DMSO-d₆ (400 MHz) δ 10.64 (s, 1H), 7.73 (d, 1H, J = 22 Hz), 7.46-7.59 (m, 5H), 7.22 (d, 2H, J = 22 Hz), 4.43-4.48 (m, 1H), 2.87-3.11 (m, 7H), 1.76-2.01 (m, 4H), 1.23-1.47 (m, 6H). HR MS (C25H28Cl2N2O5): Obs. mass, 507.1464. Calcd. mass, 507.1454 (M+H).

### Example 399. Preparation of 3-(1-methyltetrazol-5-yl)benzyl chloride.

### a. Preparation of 3-chloromethylbenzoic acid.

A 2 L, three-necked, round bottom flask equipped with a mechanical stirrer, thermometer and condenser with an open end (no nitrogen inlet tube was connected to avoid pressure build-up in the apparatus) was charged with 109 g (800 mmol) of m-toluic acid and 320 mL of chlorobenzene. The mixture was heated to ca. 90 °C with a steam bath to give a homogeneous solution and 53.4 g (400 mmol, 0.5 equiv) of N-chlorosuccinimide (NCS) and 800 mg (3.3 mmol) of benzoyl peroxide (BPO) were added. The yellow solution was stirred at ca. 95 °C for 2.5 h. Then, 26.7 g (200 mmol, 0.25 equiv) of NCS and 400 mg (1.65 mmol) of BPO were added and the mixture was stirred at 95 °C for 2.5 h. Then, a further 26.7 g (200 mmol, 0.25 equiv) of NCS and 400 mg (1.65 mmol) of BPO were added and the mixture was stirred at 95 °C for 2.5 h. To the reaction mixture was added 480 mL of water and the resulting suspension was allowed to cool to room temperature with stirring overnight. To the yellow slurry was added 480 mL of hexane. The resulting suspension was stirred at room temperature for 30 min, then filtered through a coarse sintered glass filter. The collected solid was washed thoroughly with 2 x 130 mL of water and then with 2 x 130 mL of hexane, and dried by suction for 2.5 h. The solid was then suspended in 800 mL of water and the mixure was heated on a steam bath for 30 min. After standing at room temperature overnight, the white solid was collected by filtration and dried by suction for 1.5 h. Further drying at 55 °C under high vacuum overnight yielded 73.7 g (54.0%) of 3-chloromethylbenzoic acid; mp 134-136 °C.

### b. Preparation of 3-(chloromethyl)-N-methylbenzamide.

A 250 mL, round bottom flask equipped with a magnetic stirrer, reflux condenser and calcium chloride drying tube was charged with 34.1 g (200 mmol) of 3-chloromethylbenzoic, acid and 125 mL of toluene (dried over molecular sieves 4A). To this suspension was added 21.9 mL (300 mmol) of thoinyl chloride and the mixture was heated to 85-90 °C for 15 h. While heating, evolution of gas, presumably hydrogen chloride and sulfur dioxide, was observed. The reaction mixture was cooled to room temperature and excess thionyl chloride and toluene were removed under vacuum. The resulting oily residue was azeotroped with 100 mL of toluene, then dried under high vacuum for 1 h to give the crude acid chloride.

A 1 L, three necked, round bottom flask equipped with a magnetic stirrer, addition funnel, thermometer and argon bubbler was charged with the crude acid chloride obtained above, and 400 mL of dichloromethane (dried over molecular sieves 4A). After the solution was cooled to -5 to 0 °C (using an ice-sodium chloride bath), 14.9 g (220 mmol) of methylamine hydrochloride was added in one portion. To this mixture was added 69.6 mL (400 mmol) of diisopropylethylamine (DIPEA) dropwise over 15-20 min, while maintaining the temperature of the reaction mixture below 2 °C. After completion of the addition, the mixture was stirred for 45 min at 0 to 5 °C, then allowed to warm to room temperature. After stirring for 15 min at room temperature, TLC analysis indicated complete reaction. The reaction mixture was diluted with 250 mL of water, and stirred for 5 minutes. The two layers were separated and the aqueous phase was extracted with 2 x 100 mL of dichloromethane. The combined organic layers were washed successively with 300 mLof water, and 300 mL of saturated sodium chloride solution. After drying over anhydrous magnesium sulfate, the solution was concentrated by rotary evaporation under house vacuum. The residue was further dried in vacuo to give a light yellow solid. This solid was dissolved in 110 mL of toluene at -60-70 °C. The resulting solution was allowed to cool to room temperature and seeded with crystals of product, then stored in a refrigerator overnight. The resulting precipitate was collected by filtration and washed with 30 mL of hexane. After drying under high vacuum, 29.4 g (80.0% yield) of 3-(chloromethyl)-N-methylbenzamide was obtained as a light yellow solid; mp 59-61 °C.

### c. Preparation of 3-(1-methyltetrazol-5-yl)benzyl chloride.

A 250 mL round bottom flask equipped with a magnetic stirrer, reflux condenser and calcium chloride drying tube was charged with 27.2 g (148 mmol) of 3-(chloromethyl)-N-methylbenzamide, 100 mL of toluene (dried over molecular sieves 4A). To this solution was added 16.2 mL (222 mmol) of thionyl chloride and the mixture was heated to 85-90 °C for 15 h (Note 6). While heating, evolution of gas, presumably hydrogen chloride and sulfur dioxide, was observed. After cooling to room temperature, excess thionyl chloride and toluene were removed under vacuum. The resulting residue was azeotroped with 100 mL of toluene, then dried under high vacuum for 1 h to give the crude imidoyl chloride. A 500 mL, three necked, round bottom flask equipped with a magnetic stirrer, thermometer and argon bubbler was charged with 11.6 g (178 mmol) of sodium azide and 140 mL of acetonitrile (freshly opened bottle). To this suspension was added 23.7 mL (187 mmol) of chlorotrimethylsilane and the mixture was stirred for 1.5 h at room temperature. After cooling to 0 °C, a solution of the crude imidoyl chloride, prepared above, in 40 mL of acetonitrile was added. This heterogeneous mixture was stirred for 1-2 h at 0 °C, then allowed to warm to room temperature and stirred for 15 h. TLC analysis indicated complete reaction. The reaction mixture was quenched by the addition of 150 mL of water, then diluted with 150 mL of ethyl acetate. The two layers were separated and the aqueous phase was extracted with 2 x 100 mL of ethyl acetate. The combined organic layers were washed successively with 200 mL of water, and 200 mL of saturated sodium chloride solution. After drying over anhydrous magnesium sulfate, the solution was concentrated. The residue was further dried in vacuo to give a light yellow solid (29.7 g). This solid was dissolved in 220 mL of 5.5:4.5 hexane:ethyl acetate at ~60-70 °C. The resultant solution was allowed to cool to room temperature and was seeded with crystals of product, then stored in a refrigerator overnight. The resulting precipitate was collected by filtration and washed with 50 mL of hexane. After drying by suction, 24.5 g (79.5% yield) of 3-(1-methyltetrazol-5-yl)benzyl chloride was obtained as a white amorphous solid; mp 63-65 °C.

Example 400. 1-[[3-(1-Methyltetrazol-5-yl)phenyl]methyl]cyclobutane carboxylic acid methyl ester was prepared from 3-(1-methyltetrazol-5-yl)benzyl chloride using the general method described in example 7 to give a 77 % yield of a viscous oil. HR MS: obs. mass, 287.1514. Calcd. masss, 287.1508 (M+H).

Example 401. 1-[[3-(1-Methyltetrazol-5-yl)phenyl]methyl]cyclobutane carboxylic acid was prepared from 1-[[3-(1-methyltetrazol-5-yl)phenyl]methyl]cyclobutane carboxylic acid methyl ester using the general procedure described in example 15 to give an 83% yield of a viscous oil. HR MS: obs. mass, 273.1226. Calcd. mass, 273.1238 (M+H).

Example 402. 4-Amino-N-[(1-[4-(methylsulfonyl)butyl]cyclopentyl]carbonyl]-L-phenylalanine methyl ester was prepared from 4-nitro-L-phenylalanine methyl ester and 4-(methylsulfonyl)butyl]cyclopentane carboxylic acid using the general procedure described in example 26. HR MS (C21H32N2O5S): Obs mass, 425.2121. Calcd mass, 425.2110 (M+H).

Example 403. 1-(4-Bromobutyl)cyclobutane carboxylic acid methyl ester was prepared from 1,4-dibromobutane and cyclobutane carboxylic acid methyl ester using the general procedure described in example 168.

Example 404. 1-[4-(Methylthio)butyl]cyclobutane carboxylic acid methyl ester was prepared from 1-(4-bromobutyl)cyclobutane carboxylic acid methyl ester and sodium methylmercaptan using the procedure described in example 172.

Example 405. 1-[4-(methylsulfonyl)butyl]cyclobutane carboxylic acid was prepared from 1-[4-(methylthio)butyl]cyclobutane carboxylic acid methyl ester using the general procedures described in examples 174 and 175.

Example 406. 4-[[(2,6-dichlorophenyl)carbonyl]amino]-N-[[1-[4-(methylsulfonyl)butyl] cyclobutane]carbonyl]-L-phenylalanine was prepared from 4-[[(2,6-dichlorophenyl)carbonyl] amino]-L-phenylalanine methyl ester and 1-[4-(methylsulfonyl) butyl]cyclobutane carboxylic acid using the procedure described in examples 46 and 47.

Example 407. 4-[(4R)-3-Acetyl-4-(phenylmethyl)-5-oxo-2-phenyl-1-imidazolidinyl]-N-[(1-phenylcyclopentyl)carbonyl]-L-phenylalanine was prepared from Fmoc-D-phenylalanine, benzaldehyde and 4-amino-N-[(1,1-dimethylethoxy)carbonyl]-L-phenylalanine methyl ester using the general procedure described in example 365

Example 408. 4-[3-Acetyl-5-oxo-2-[(3-pyridinyl)methyl]-4-phenylmethyl-1-imidazolidinyl]-N-[[1-[4-(methylsulfonyl)butyl]cyclopentyl]carbonyl]-L-phenylalanine methyl ester was prepared from 4-amino-N-[(1,1-dimethylethoxy)carbonyl]-L-phenylalanine methyl ester and Fmoc-D-pyridinylalanine using the general method described in example 365.

### Example 409. VLA-4 / VCAM-1 Screening Assay

VLA-4 antagonist activity, defined as ability to compete for binding to immobilized VCAM-1, was quantitated using a solid-phase, dual antibody ELISA. VLA-4 (α4β1 integrin) bound to VCAM-1 was detected by a complex of anti-integrin β1 antibody: HRP-conjugated anti-mouse IgG: chromogenic substrate (K-Blue). Initially, this entailed coating 96 well plates (Nunc Maxisorp) with recombinant human VCAM-1 (0.4 µg in 100 µl PBS), sealing each plate and then allowing the plates to stand at 4°C for ^{~}18 hr. The VCAM-coated plates were subsequently blocked with 250 µl of 1% BSA/0.02% NaN₃ to reduce non-specific binding. On the day of assay, all plates were washed twice with VCAM Assay Buffer (200 µl/well of 50 mM Tris-HCl, 100 mM NaCl, 1 mM MnCl₂, 0.05% Tween 20; pH 7.4). Test compounds were dissolved in 100% DMSO and then diluted 1:20 in VCAM Assay Buffer supplemented with 1 mg/mL BSA (i.e., final DMSO = 5%). A series of 1:4 dilutions were performed to achieve a concentration range of 0.005 nM - 1.563 µM for each test compound. 100 µl per well of each dilution was added to the VCAM-coated plates, followed by 10 µl of Ramos cell-derived VLA-4. These plates were sequentially mixed on a platform shaker for 1 min, incubated for 2 hr at 37°C, and then washed four times with 200 µl/well VCAM Assay Buffer. 100 µl of mouse anti-human integrin β1 antibody was added to each well (0.6 µg/mL in VCAM Assay Buffer + 1 mg/mL BSA) and allowed to incubate for 1 hr at 37°C. At the conclusion of this incubation period, all plates were washed four times with VCAM Assay Buffer (200 µl/well). A corresponding second antibody, HRP-conjugated goat anti-mouse IgG (100 µl per well @ 1:800 dilution in VCAM Assay Buffer + 1 mg/mL BSA), was then added to each well, followed by a 1 hr incubation at room temperature and concluded by three washes (200µl/well) with VCAM Assay Buffer. Color development was initiated by addition of 100 µl K-Blue per well (15 min incubation, room temp) and terminated by addition of 100 µl Red Stop Buffer per well. All plates were then read in a UV/Vis spectrophotometer at 650 nM. Results were calculated as % inhibition of total binding (i.e., VLA-4 + VCAM-1 in the absence of test compound). The results are provided in the following table:

**Table 2**

| Example | conc. nM | % Inh. @ conc. | IC₅₀ nM |
|---|---|---|---|
| 27 | | | 2.7 |
| 28 | | | 22.6 |
| 29 | | | 3.6 |
| 30 | | | 6.1 |
| 31 | | | 9.8 |
| 35 | | | 5.0 |
| 36 | | | 4.6 |
| 37 | | | 5.0 |
| 40 | | | 2.1 |
| 41 | | | 1.7 |
| 42 | | | 0.61 |
| 43 | | | 0.63 |
| 47 | | | 2.5 |
| 48 | | | 0.44 |
| 49 | | | 4.7 |
| 50 | | | 1.3 |
| 51 | | | 1.6 |
| 52 | | | 1.1 |
| 53 | | | 0.5 |
| 54 | | | 0.82 |
| 55 | | | 0.47 |
| 56 | | | 0.94 |
| 57 | | | 0.42 |
| 58 | | | 0.58 |
| 59 | | | 0.26 |
| 60 | | | 1.05 |
| 64 | | | 3.0 |
| 65 | 100 | 92" | 2.4 |
| | 10 | 72 | |
| 66 | 100 | 85" | |
| | 10 | 47 | |
| 67 | 100 | 81 | |
| | 10 | 41 | |
| 68 | 100 | 86 | 8.2 |
| | 10 | 49 | |
| 69 | 100 | 87 | 5.8 |
| | 10 | 49 | |
| 70 | 100 | 86 | 7.0 |
| | 10 | 49 | |
| 71 | 100 | 86 | 2.5 |
| | 10 | 51 | |
| 72 | 100 | 80 | |
| | 10 | 39 | |
| 73 | 100 | 90 | |
| | 10 | 64 | |
| 74 | 100 | 86 | 13.6 |
| | 10 | 50 | |
| 75 | 100 | 92 | 3.8 |
| | 10 | 68 | |
| 76 | 100 | 93 | 4.6 |
| | 10 | 73 | |
| 77 | 100 | 93 | 5.6 |
| | 10 | 67 | |
| 78 | 100 | 93 | 9.0 |
| | 10 | 63 | |
| 79 | 100 | 95 | 1.2 |
| | 10 | 77 | |
| 80 | 100 | 89 | 6.4 |
| | 10 | 53 | |
| 81 | 100 | 93 | 1.4 |
| | 10 | 73 | |
| 82 | | | 7.3 |
| 83 | | | 1.1 |
| 84 | | | 4.4 |
| 85 | | | 0.43 |
| 86 | | | 6.8 |
| 87 | | | 1.5 |
| 88 | | | 3.9 |
| 89 | | | 81 |
| 90 | | | 1.04 |
| 91 | | | 40 |
| 92 | | | 3.0 |
| 93 | | | 1.9 |
| 94 | | | 0.96 |
| 95 | | | 0.69 |
| 96 | | | 17.4 |
| 97 | | | 0.50 |
| 98 | | | 11.7 |
| 99 | | | 2.1 |
| 100 | | | 0.26 |
| 101 | | | 18.3 |
| 102 | | | 0.95 |
| 103 | | | 0.70 |
| 104 | | | 10.4 |
| 105 | | | 0.83 |
| 106 | | | 13.5 |
| 109 | | | 62 |
| 110 | | | 8.2 |
| 111* | | | 9.4 |
| 113 | | | 43 |
| 114 | | | 155 |
| 115 | | | 22.7 |
| 116 | | | 0.88 |
| 118 | | | 0.35 |
| 119 | | | 5.0 |
| 120 | | | 2.9 |
| 121 | | | 66 |
| 122 | | | 2.1 |
| 128 | | | 5.0 |
| 129 | | | 153 |
| 130 | | | 6.6 |
| 131 | | | 63 |
| 132 | | | 2.8 |
| 133 | | | 60 |
| 134 | | | 0.19 |
| 135 | | | 17 |
| 136 | | | 0.73 |
| 137 | | | 19 |
| 138 | | | 0.42 |
| 139 | | | 38 |
| 141 | | | 3.9 |
| 142 | | | 1.7 |
| 143 | | | 0.85 |
| 144 | | | 0.75 |
| 156 | | | 1.15 |
| 158 | | | 5 |
| 159 | | | 6.4 |
| 160 | | | 5.8 |
| 161 | | | 9.4 |
| 162 | | | 1.2 |
| 163 | | | 2.4 |
| 164 | | | 6.2 |
| 165 | | | 11 |
| 166 | | | 2.5 |
| 167 | | | 9.2 |
| 187 | | | 0.26 |
| 192 | | | 0.14 |
| 193 | | | 0.55 |
| 194 | | | 0.33 |
| 200 | | | 0.81 |
| 201 | | | 1.2 |
| 202 | | | 1.0 |
| 203 | | | 0.64 |
| 216 | | | 2.9 |
| 217 | | | 2.0 |
| 249 | | | 0.29 |
| 250 | | | 0.32 |
| 251 | | | 0.37 |
| 252 | | | 5.1 |
| 253 | | | 3.4 |
| 254 | | | 2.0 |
| 255 | | | 0.5 |
| 256 | | | 2.4 |
| 257 | | | 40 |
| 258 | | | 14 |
| 259 | | | 3.6 |
| 270 | | | 1.1 |
| 271 | | | 0.55 |
| 272 | | | 1.0 |
| 273 | | | 1.5 |
| 363 | | | 0.64 |
| 371 | | | 1.65 |

| | | | |
|---|---|---|---|
| * 4-[(RS)-2,3,5,6,7,7a-hexahydro-1,3-dioxo-1H-pyrrolo[3,4-c]pyridin-2-yl]-N-[[1-[(4-methoxyphenyl)methyl]cyclopentyl]carbonyl]-L-phenylalanine | | | |

### Example 410. Ramos (VLA-4) / VCAM-1 Cell-Based Screening Assay Protocol

### Materials:

Soluble recombinant human VCAM-1 (mixture of 5- and 7-Ig domain) was purified from CHO cell culture media by immunoaffinity chromatography and maintained in a solution containing 0.1 M Tris-glycine (pH 7.5), 0.1 M NaCl, 5 mM EDTA, 1 mM PMSF, 0.02% 0.02% NaN₃ and 10 µg/mL leupeptin. Calcein-AM was purchased from Molecular Probes Inc.

### Methods:

VLA-4 (α4β1 integrin) antagonist activity, defined as ability to compete with cell-surface VLA-4 for binding to immobilized VCAM-1, was quantitated using a Ramos-VCAM-1 cell adhesion assay. Ramos cells bearing cell-surface VLA-4, were labeled with a fluorescent dye (Calcein-AM) and allowed to bind VCAM-1 in the presence or absence of test compounds. A reduction in fluorescence intensity associated with adherent cells (% inhibition) reflected competitive inhibition of VLA-4 mediated cell adhesion by the test compound.

Initially, this entailed coating 96 well plates (Nunc Maxisorp) with recombinant human VCAM-1 (100 ng in 100 µl PBS), sealing each plate and allowing the plates to stand at 4°C for ^{~}18 hr. The VCAM-coated plates were subsequently washed twice with 0.05% Tween-20 in PBS, and then blocked for 1hr (room temperature) with 200 µl of Blocking Buffer (1% BSA/0.02% thimerosal) to reduce non-specific binding. Following the incubation with Blocking Buffer, plates were inverted, blotted and the remaining buffer aspirated. Each plate was then washed with 300 µl PBS, inverted and the remaining PBS aspirated.

Test compounds were dissolved in 100% DMSO and then diluted 1:25 in VCAM Cell Adhesion Assay Buffer (4 mM CaCl₂, 4 mM MgCl₂ in 50 mM TRIS-HCl, pH 7.5) (final DMSO = 4%). A series of eight 1:4 dilutions were performed for each compound (general concentration range of 1 nM - 12,500 nM). 100 µl/well of each dilution was added to the VCAM-coated plates, followed by 100 µl of Ramos cells (200,000 cells/well in 1% BSA/PBS). Plates containing test compounds and Ramos cells were allowed to incubate for 45 min at room temperature, after which 165 µl/well PBS was added. Plates were inverted to remove non-adherent cells, blotted and 300 µl/well PBS added. Plates were again inverted, blotted and the remaining buffer gently aspirated. 100 µl Lysis Buffer (0.1% SDS in 50 mM TRIS-HCl, pH 8.5) was added to each well and agitated for 2 min on a rotary shaking platform. The plates were then read for fluorescence intensity on a Cytofluor 2300 (Millipore) fluorecence measurement system (excitation = 485 nm, emission = 530 nm). The results are shown in the following table:

**Table 3**

| Example | Conc. (nM) | % Inh @ Conc. | IC₅₀ in Ramos Cell Based Assay (nM) |
|---|---|---|---|
| 43 | | | 36.5 |
| 53 | | | 37.5 |
| 55 | | | 18.5 |
| 95 | | | 34.5 |
| 100 | | | 54.5 |
| 56 | | | 31.5 |
| 57 | | | 27 |
| 58 | | | 47 |
| 59 | | | 13.5 |
| 156 | | | 28 |
| 158 | | | 124 |
| 159 | | | 313 |
| 160 | | | 328 |
| 162 | | | 23 |
| 163 | | | 85 |
| 187 | | | 5.7 |
| 188 | | | 36 |
| 189 | | | 130 |
| 190 | | | 650 |
| 191 | | | 322 |
| 192 | | | 22 |
| 193 | | | 40 |
| 194 | | | 27 |
| 200 | | | 19 |
| 201 | | | 19 |
| 202 | | | 31 |
| 203 | | | 47 |
| 216 | | | 356 |
| 217 | | | 211 |
| 249 | | | 62 |
| 250 | | | 28 |
| 251 | | | 9 |
| 252 | | | 228 |
| 253 | | | 89 |
| 254 | | | 360 |
| 255 | | | 27 |
| 256 | | | 151 |
| 257 | | | 635 |
| 258 | | | 1,034 |
| 259 | | | 129 |
| 260 | | | 130 |
| 276 | | | 22 |
| 260 | | | 94 |
| 261 | | | 184 |
| 262 | | | 39 |
| 263 | | | 5 |
| 270 | | | 150 |
| 271 | | | 67 |
| 272 | | | 96 |
| 273 | | | 104 |
| 278 | | | 2 |
| 280 | | | 4.2 |
| 282 | | | 1.8 |
| 283 | 10 | 32 | |
| 284 | | | 2.9 |
| 286 | | | 24 |
| 288 | 10 | 92 | |
| 291 | 10 | 48 | |
| 293 | | | 40 |
| 299 | | | 1.7 |
| 300 | 10 | 32 | |
| 303 | 10 | 70 | |
| 304 | 10 | 35 | |
| 305 | 10 | 37 | |
| 311 | | | 3 |
| 313 | 10 | 68 | |
| 315 | 10 | 71 | |
| 316 | 10 | 22 | |
| 317 | 10 | 17 | |
| 319 | 10 | 68 | |
| 321 | 10 | 59 | |
| 323 | 10 | 76 | |
| 324 | 10 | 20 | |
| 322 | | | 5 |
| 342 | 10 | 24 | |
| 344 | | | 19 |
| 346 | 10 | 23 | |
| 349 | 10 | 58 | |
| 350 | | | 13 |
| 351 | | | 13 |
| 357 | | | 25 |
| 358 | | | 75 |
| 363 | | | 11 |
| | | | |

### Example 411. Oral Dosage Form

| Item | Ingredients | mg/tablet | | | |
|---|---|---|---|---|---|
| 1 | Compound of invention | 25 | 100 | 250 | 500 |
| 2 | Anhydrous lactose | 83 | 35 | 19 | 38 |
| 3 | Croscarmellose sodium | 6 | 8 | 16 | 32 |
| 4 | Povidone K30 | 5 | 6 | 12 | 24 |
| 5 | Magnesium stearate | 1 | 1 | 3 | 6 |
| | | | | | |
| | Total weight (mg) | 120 | 150 | 300 | 600 |
| Manufacturing procedure: | | | | | |
| 1. Mix items 1,2 ,3 in a suitable mixer for 15 minutes. | | | | | |
| 2. Granulate the powder mix from step 1 with 20% PVP K30 solution. | | | | | |
| 3. Dry the granulation in step 2 at 50° C. | | | | | |
| 4 Pass the granulation from step 3 through a suitable milling equipment. | | | | | |
| 5. Add the item 5 to the milled granulation from Step 4 and mix for 3 minutes. | | | | | |
| 6. Compress the granulation from Step 5 on a suitable press. | | | | | |

### Example 412. Aerosol Administration Formulation

| **Ingredients** | **Qty/mL** |
|---|---|
| Compound of invention | 3-150 mg* |
| Sodium chloride | 8.0 mg |
| Phophate buffer (20 mM)pH 7.0* q.s. | 1.0 mL |

| | |
|---|---|
| * Depending upon activity of the compound | |

pH can be adjusted with Sodium hydroxide solution (1 N) or HCl solution (10%w/v)

### Procedure:

1. Dissolve the drug substance in the buffer.
2. Filter the solution through a 0.22 micron filter.

The particle size distribution after nebulizing the above solution (as measured using Malvern Mastersizer X) is in the range of 1-6 microns.

## Claims

1. A compound of the formula: 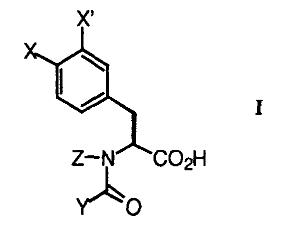 wherein:
X' is H, halogen, or C₁₋₆ alkyl, and X is a group of the formula: 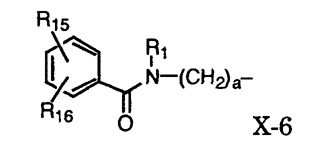
wherein:
R₁ is hydrogen or C₁₋₆ alkyl,
R₁₅ is hydrogen, halogen, nitro, C₁₋₆ alkyl sulfonyl, cyano, C₁₋₆ alkyl, C₁₋₆ alkoxy, C₁₋₆ alkoxycarbonyl, carboxy, C₁₋₆ alkyl aminosulfonyl, perfluoro C₁₋₆ alkyl, C₁₋₆ alkylthio, hydroxy C₁₋₆ alkyl, alkoxy C₁₋₆ alkyl, alkylthio C₁₋₆ alkyl, alkylsulfinyl C₁₋₆ alkyl, alkylsulfonyl C₁₋₆ alkyl, C₁₋₆ alkylsulfinyl, C₁₋₆ alkylcarbonyl, aryloxy, aroyl, aryl or a group of the formula R₁₇-C≡C-,
R₁₆ is H, halogen, nitro, cyano, C₁₋₆ alkyl, OH, perfluoro C₁₋₆ alkyl, or C₁₋₆ alkylthio, and
R₁₇ is H, aryl, heteroaryl, or C₁₋₆ alkyl which is unsubstituted or substituted by OH, aryl, or heteroaryl, and
a is 0 or 1;
or X is a group of the formula: 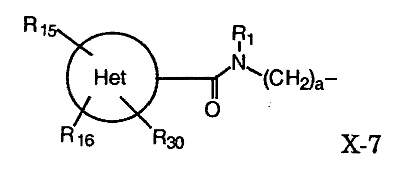 wherein 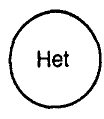 is a 5- or 6-membered heteroaromatic ring containing 1, 2 or 3 heteroatoms selected from N,O, and S;
or 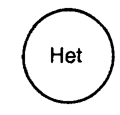 is a 9- or 10-membered bicyclic heteroaromatic ring containing 1, 2, 3
or 4 heteroatoms selected from O, S, and N,
a, R₁, R₁₅ and R₁₆ are as defined in X-6, and
R₃₀ is hydrogen or C₁₋₆ alkyl, or is absent;
or X is a group of the formula: 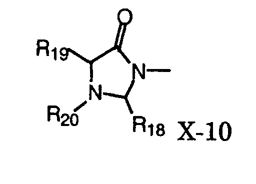 wherein:
R₁₈ is hydrogen, substituted or unsubstituted C₁₋₆ alkyl, aryl, heteroaryl, arylalkyl or heteroaryl alkyl,
R₁₉ is unsubstituted or substituted C₁₋₆ alkyl, aryl, heteroaryl, arylalkyl or heteroarylalkyl, and
R₂₀ is unsubstituted or substituted C₁₋₆ alkyl, C₁₋₆ alkylcarbonyl optionally substituted by carboxyl, aroyl or aryloxy;
Y is a group of the formula: 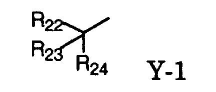
wherein:
R₂₂ and R₂₃ are independently aryl, heteroaryl or C₁₋₆ alkyl which is unsubstituted or substituted by one or more chloro, bromo, nitro, hydroxy, C₁₋₆ alkoxy, aryl, C₁₋₆ alkylcarbonyl, aroyl or cyano,
R₂₄ is aryl, cyano, alkylsulfonyl or C₁₋₆ alkyl or alkenyl up to C₆ unsubstituted or substituted by an aryl or heteroaryl ring, and when R₂₂ is aryl and R₂₃ is aryl or C₁₋₆ alkyl, R₂₄ is H, and the total number of carbon atoms in R₂₂, R₂₃ and R₂₄ is from 6 to 14; or
Y is a 3-7 membered ring of the formula: 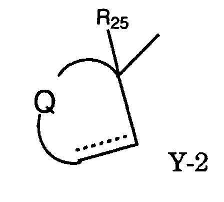
wherein:
R₂₅ is C₁₋₆ alkyl, unsubstituted or fluorine substituted alkenyl up to C₆, or a group of formula R₂₆-(CH₂)ₑ-,
R₂₆ is aryl, heteroaryl, azido, cyano, hydroxy, C₁₋₆ alkoxy, C₁₋₆ alkoxycarbonyl, C₁₋₆ alkylcarbonyl, C₁₋₆ alkylthio, C₁₋₆ alkyl sulfonyl, C₁₋₆ alkyl sulfinyl, perfluoro C₁₋₆ alkylcarbonyl, nitro, or R₂₆ is a group of formula -NR₂₈R₂₉, wherein:
R₂₈ is H or C₁₋₆ alkyl,
R₂₉ is hydrogen, C₁₋₆ alkyl, C₁₋₆ alkoxycarbonyl, optionally substituted aminocarbonyl, optionally substituted C₁₋₆ alkylcarbonyl, aroyl, heteroaroyl, heterocycloalkylcarbonyl, C₁₋₆ alkyl sulfonyl, C₁₋₆ alkylaminothiocarbonyl, or
R₂₈ and R₂₉ taken together with the nitrogen atom to which they are attached form a 4, 5 or 6-membered saturated heterocyclic ring containing one or two heteroatoms with the second heteroatom being O, S, or N-R₂₇;
Q is -(CH₂)_{f} O-, -(CH₂)_{f} S-, -(CH₂)_{f}-, or when f=0, a bond,
R₂₇ is H, C₁₋₆ alkyl, aryl, C₁₋₆ alkylcarbonyl, aroyl or C₁₋₆ alkoxycarbonyl, the carbon atoms in said ring are unsubstituted or substituted by C₁₋₆ alkyl or halogen,
e is an integer from 0 to 4,
f is an integer from 0 to 3,
and the dotted line means a bond which may be absent or present; and
Z is hydrogen or C₁₋₆ alkyl;
and the pharmaceutically acceptable salts and esters thereof
the term "substituted C₁₋₆ alkyl" , if not defined otherwise, means a C₁₋₆ alkyl group substituted by one or more groups selected independently from cycloalkyl, nitro, aryloxy, aryl, hydroxy, halogen, cyano, C₁₋₆ alkoxy, C₁₋₆ alkylcarbonyl, C₁₋₆ alkylthio, C₁₋₆ alkyl sulfinyl, C₁₋₆ alkyl sulfonyl and substituted amino;
the term "aryl" means a mono- or bicylic aromatic group which is unsubstituted or substituted by C₁₋₆ alkyl, C₁₋₆ alkoxy, hydroxy C₁₋₆ alkyl, hydroxy, hydroxyalkoxy, halogen, C₁₋₆ alkylthio, C₁₋₆ alkylsulfinyl, C₁₋₆ alkylsulfonyl, cyano, nitro, perfluoro-alkyl, alkanoyl, aroyl, aryl alkynyl, heteroaryl, lower alkynyl and C₁₋₆ alkylcarbonylamino;
the term "aryl C₁₋₆ alkyl" means a C₁₋₆ alkyl group in which one or more hydrogen atoms is/are replaced by an aryl or heteroaryl group;
the term "heteroaryl" means an unsubstituted or substituted 5- or 6-membered monocyclic hetereoaromatic ring or a 9- or 10-membered bicyclic hetereoaromatic ring containing 1,2,3 or 4 hetereoatoms which are independently N, S or O and which may be substituted with substitutents as defined above for "aryl";
the term "optionally substituted C₁₋₆ alkylcarbonyl" means C₁₋₆ alkyl groups bonded via a carbonyl group which alkyl group may be substituted by one or more groups selected from hydroxycarbonyl, aroyl or aryloxy, C₁₋₆ alkoxy, flouro, phenyl, cycloalkyl, C₁₋₆ alkoxy carbonyl, amino or C₁₋₆ alkoxycarbonyl amino;
the term "aroyl" means an mono- or bicyclic aryl or heteroaryl group as defined before bonded via a carbonyl group;
the term "cycloalkyl" means an unsubstituted or substituted 3- to 7-membered carbocyclic ring which may be substituted by hydroxy, halogen, cyano, C₁₋₆ alkoxy, C₁₋₆ alkylcarbonyl, C₁₋₆ alkyl, aroyl, C₁₋₆ alkylthio, C₁₋₆ alkyl sulfinyl, C₁₋₆ alkyl sulfonyl, aryl, heteroaryl and substituted amino
the term "heterocycloalkyl" means an unsubstituted or substituted 5- to 6-membered carbacyclic ring in which one or two of the carbon atoms has been replaced by heteroatoms independently selected from O, S and N.

2. A compound according to claim 1, wherein X' is hydrogen.

3. A compound according to claim 1 or 2, wherein Z is hydrogen.

4. A compound according to anyone of claims 1-3, wherein in Y-1, R₂₂ and R₂₃ are C₁₋₆ alkyl or phenyl, and R₂₄ is C₁₋₆ alkyl except when R₂₂ is aryl and R₂₃ is aryl or C₁₋₆ alkyl, then R₂₄ is hydrogen.

5. A compound according to anyone of claims 1-4, wherein Y-1 is selected from the group consisting of:

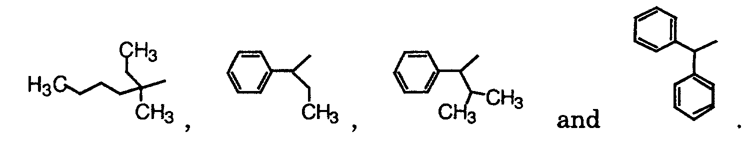

6. A compound according to any one of claims 1-3, wherein in Y-2 Q is-(CH₂)_{f}- or, when f=0, a bond.

7. A compound according to claim 6, wherein f is 1, 2 or 3.

8. A compound according to any one of claims 1-3, wherein in Y-2 R₂₆ is aryl, heteroaryl, azido, cyano, hydroxy, C₁₋₆ alkoxy, C₁₋₆ alkoxycarbonyl, C₁₋₆ alkylcabonyl, C₁₋₆ alkylthio, C₁₋₆ alkyl sulfonyl, C₁₋₆ alkyl sulfinyl, nitro, or R₂₆ is a group of formula -NR₂₈R₂₉ wherein R₂₈ is H or C₁₋₆ alkyl, R₂₉ is hydrogen, C₁₋₆ alkyl, C₁₋₆ alkoxycarbonyl, optionally substituted aminocarbonyl, optionally substituted C₁₋₆ alkylcarbonyl, aroyl, C₁₋₆ alkyl sulfonyl or R₂₈ and R₂₉ taken together with the nitrogen to which they are attached form a 4, 5 or 6-membered saturated heterocyclic ring which can contain one oxygen atom.

9. A compound according to any one of claims 1-3, wherein R₂₈ is H.

10. A compound according to claim 8 or claim 9, wherein R₂₆ is aryl unsubstituted or mono-substituted by halogen, C₁₋₆ alkoxy, C₁₋₆ alkyl, cyano, or tetrazolyl which is unsubstituted or substituted by methyl or R₂₆ is phenyl disubstituted by C₁₋₆ alkoxy.

11. A compound according to claim 10, wherein R₂₆ is phenyl unsubstituted or mono-substituted by chloro, methoxy or methyl.

12. A compound according to claim 8, wherein R₂₆ is CH₃S(O)₂-, CH₃S-, CH₃SO-, CH₃O-, CH₃CO-, NC-, N₃-, or HO-.

13. A compound according to claim 8, wherein the alkyl group in the C₁₋₆ alkylcarbonyl group of R₂₉ is unsubstituted or substituted by C₁₋₆ alkoxy, flouro, phenyl, cycloalkyl, C₁₋₆ alkoxy carbonyl, amino or C₁₋₆ alkoxycarbonyl amino.

14. A compound according to claim 13, wherein the unsubstituted or substituted C₁₋₆ alkylcarbonyl group is CH₃CO-, (CH₃)₃CCO-, CH₃(CH₂)₃CHCH₃CO-, CH₃OCH₂CO-, CF₃CO-, C₆H₅CH₂CO-, CH₃OCO(CH₂)₂CO-, cyclopentylCH₂CO-, H₂NCH₂CO or (CH₃)₃COCONH(CH₂)₂CO-.

15. A compound according to claim 8, wherein in R₂₉ the aminocarbonyl group is unsubstituted or substituted by C₁₋₆ alkyl, C₁₋₆ alkoxy carbonyl, monocyclic aryl or benzyl.

16. A compound according to claim 15, wherein the unsubstituted or substituted aminocarbonyl group is H₂NCO-, CH₃NHCO-, CH₃OCONHCO-, C₆H₄NO₂NHCO- or C₆H₅CH₂NHCO-.

17. A compound according to claim 1, wherein R₂₉ is (CH₃)₃COCO-, methylaminothiocarbonyl, 4-methoxyphenylcarbonyl, 3-triflourmethylphenylcarbonyl, 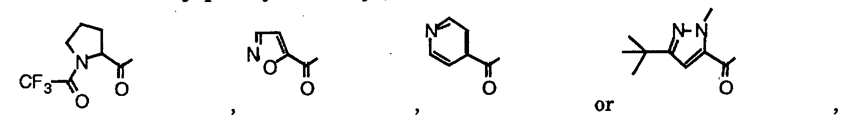 -NR₂₈R₂₉ is -NH₂ or -N(CH₃)₂ and R₂₈ and R₂₉ taken together is 4-morpholinyl.

18. A compound according to any one of claims 1-3, wherein Y-2 is selected from a group represented by one of the following formulas:

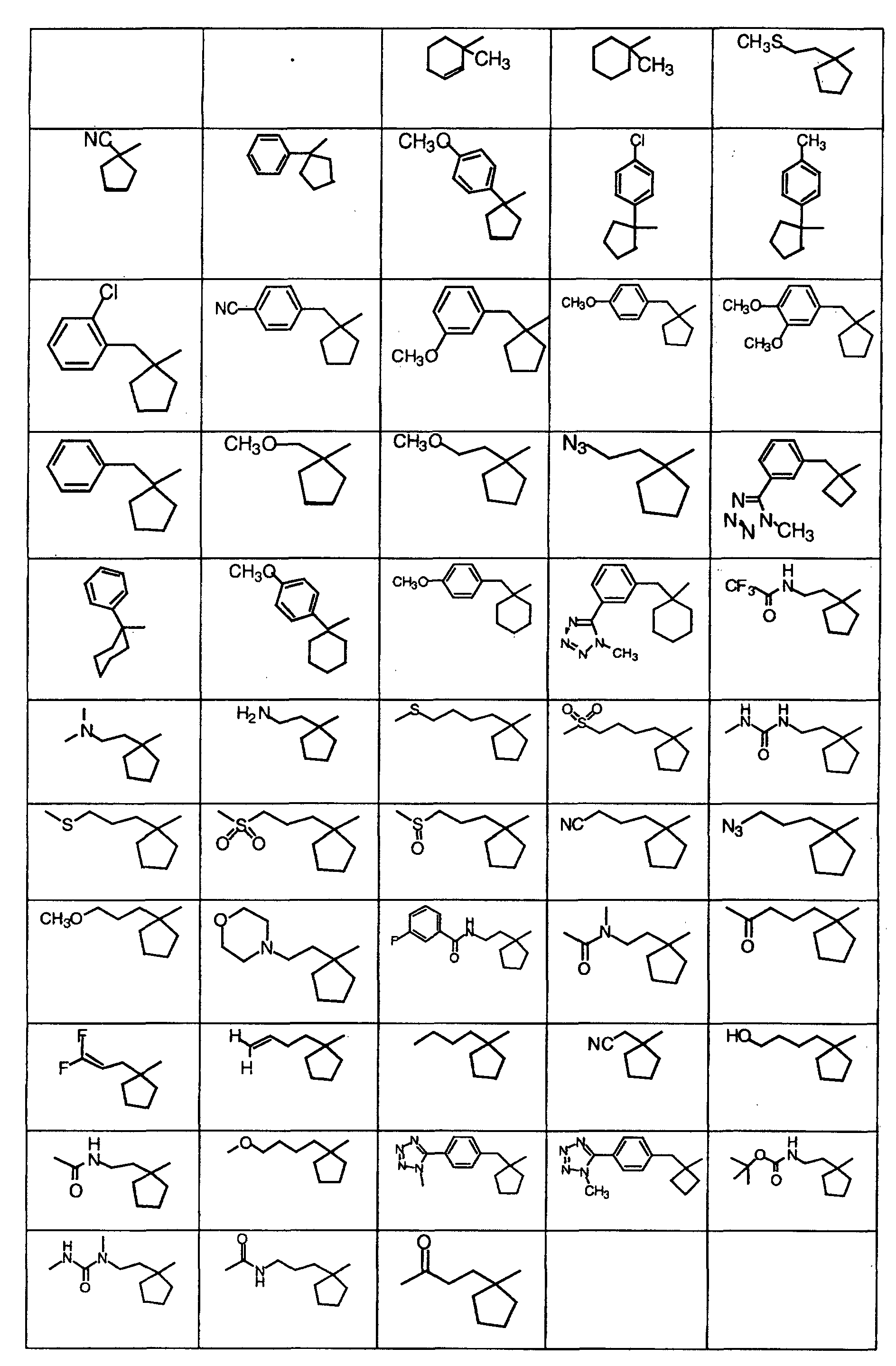

19. A compound according to any one of claims 1-3, wherein in X-6 R₁₅ and R₁₆ are independently H, halogen, nitro, perflouro C₁₋₆alkyl, C₁₋₆ alkyl, cyano or R₁₅ is phenoxy and R₁₆ is H.

20. A compound according to claim 19, wherein in X-6 the groups R₁₅ and R₁₆ are independently hydrogen, methyl, nitro, chloro, trifluoromethyl or cyano.

21. A compound according to any one of claims 1-3, wherein in X-6 R₁ is hydrogen.

22. A compound according to any one of claims 1-3, wherein a in X-6 is 0.

23. A compound according to any one of claims 1-3, wherein X-6 is selected from the group consisting of:

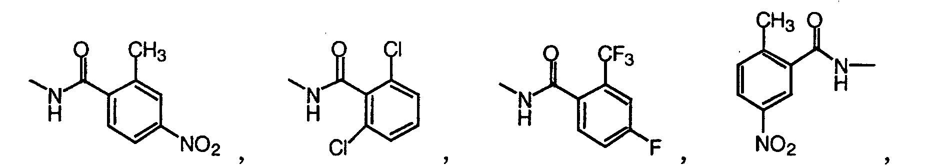

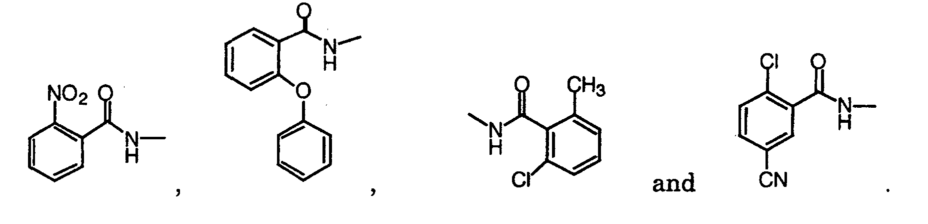

24. A compound according to any one of claims 1-3, wherein in X-7 Het is a 5-or 6-membered monocyclic heteroaromatic ring containing 1, 2 or 3 nitrogens, or a nitrogen and a sulfur, or a nitrogen and an oxygen.

25. A compound according to claim 24, wherein the heteroaromatic ring alone is

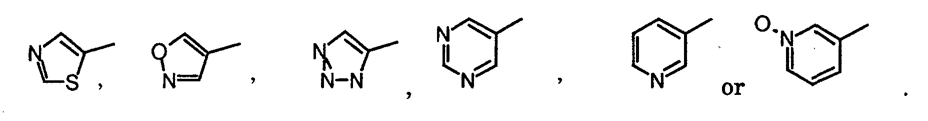

26. A compound according to any one of claims 1-3, wherein in X-7 Het is a bicyclic heteroaromatic ring containing from 1 to 3 nitrogens as the heteroatoms.

27. A compound according to claim 26, wherein the bicyclic heteroaromatic ring is 4-quinolinyl, 1-isoquinolinyl or

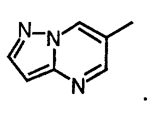

28. A compound according to any one of claims 1-3, wherein in X-7 R₁₅ is hydrogen, nitro, C₁₋₆ alkyl sulfonyl, cyano, C₁₋₆ alkyl, C₁₋₆ alkoxy, perfluoro C₁₋₆ alkyl, C₁₋₆ alkylthio, C₁₋₆ alkylcarbonyl, or aryl.

29. A compound according to claim 28, wherein R₁₅ is isopropyl, methyl or phenyl.

30. A compound according to any one of claims 1-3, wherein R₁₆ in X-7 is hydrogen, halogen, nitro, cyano, C₁₋₆ alkyl or perfluoro C₁₋₆ alkyl.

31. A compound according to claim 30, wherein R₁₆ is methyl or triflouromethyl.

32. A compound according to any one of claims 1-3, wherein R₃₀ in X-7 is hydrogen or C₁₋₆ alkyl.

33. A compound according to any one of claims 1-3, wherein X-7 is selected from the group consisting of 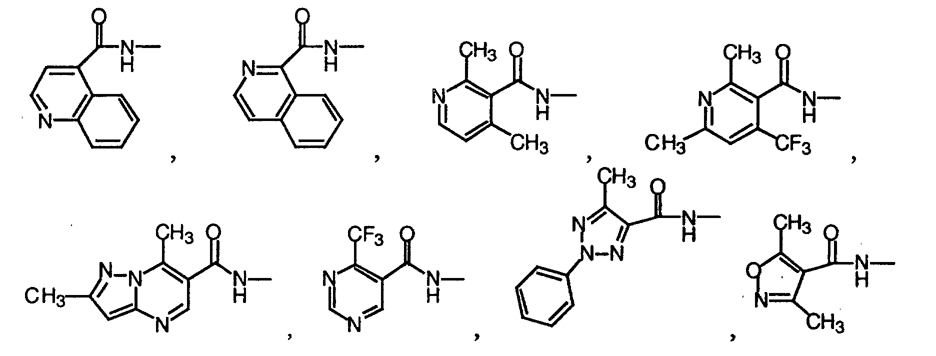 and

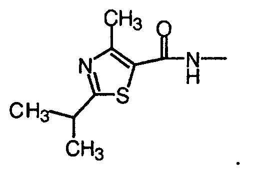

34. A compound according to any one of claims 1-3, wherein in X-10 R₁₈ is phenyl wherein the phenyl ring is unsubstituted or monosubstituted by halogen, or is phenyl C₁₋₆ alkyl.

35. A compound according to claim 34, wherein R₁₈ is phenyl, chlorophenyl or phenylethyl.

36. A compound according to any one of claims 1-3, wherein in X-10 R₁₉ is C₁₋₆ alkyl which is unsubstituted or substituted by pyridyl or phenyl wherein the phenyl ring is unsubstituted or monosubstituted by C₁₋₆ alkoxy or halogen.

37. A compound according to claim 36, wherein R₁₉ is methyl, isobutyl, benzyl, 4-chlorobenzyl, 4-methoxybenzyl or 2-pyridylmethyl.

38. A compound according to any one of claims 1-3, wherein in X-10 R₂₀ is optionally substituted C₁₋₆ alkylcarbonyl.

39. A compound according to claim 38, wherein R₂₀ is acetyl, butyryl, phenoxyacetyl, succinyl or glutaryl.

40. A compound according to any one of claims 1-3, wherein X-10 is selected from the group consisting of:

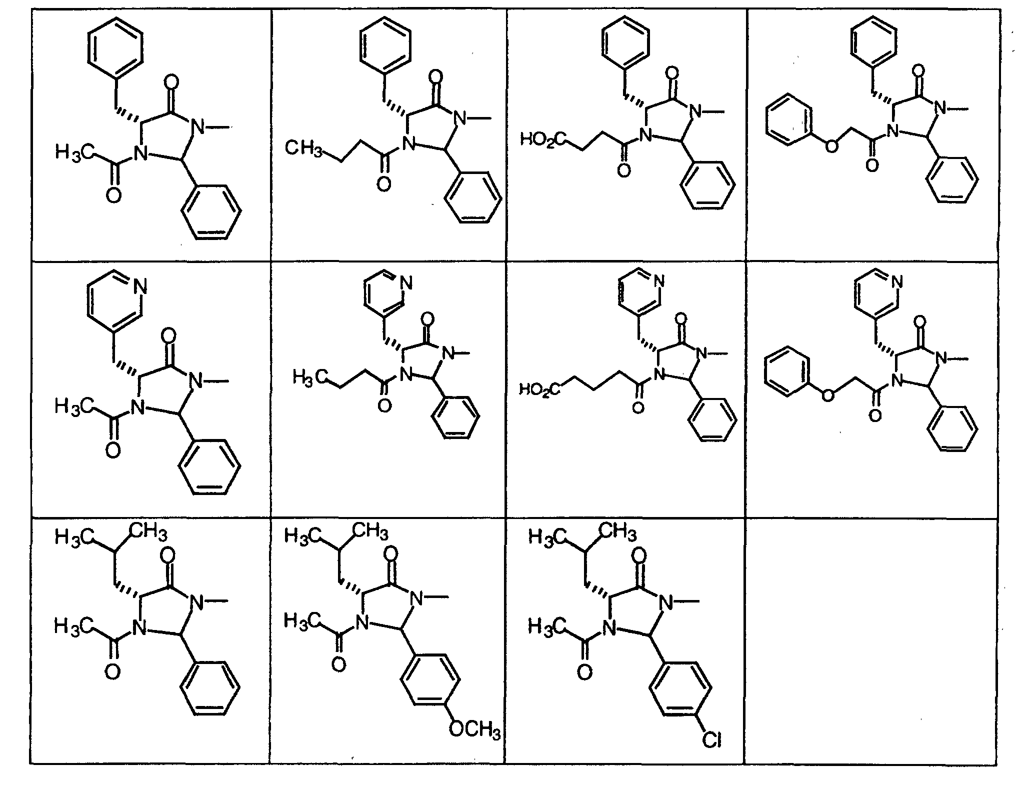

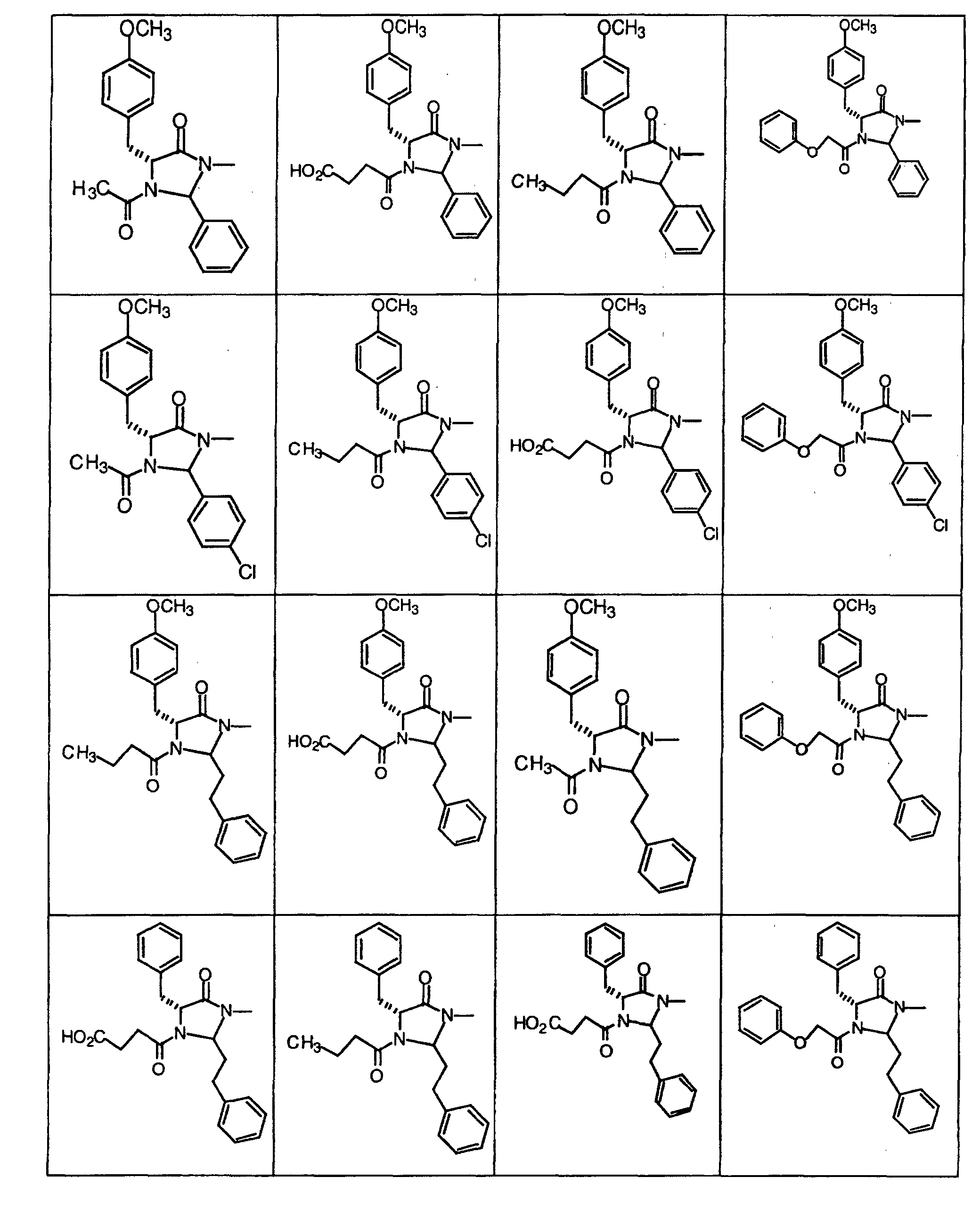

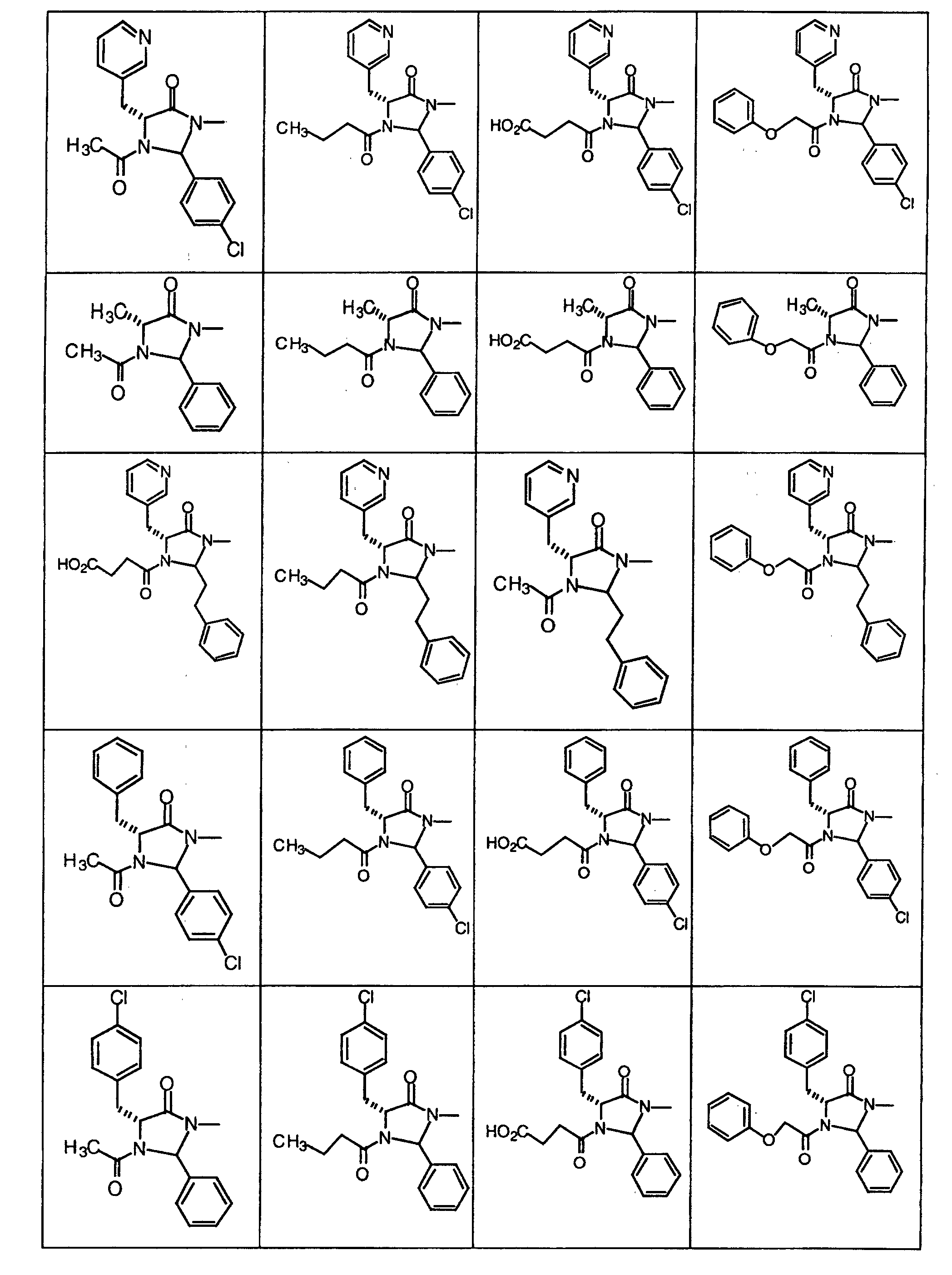

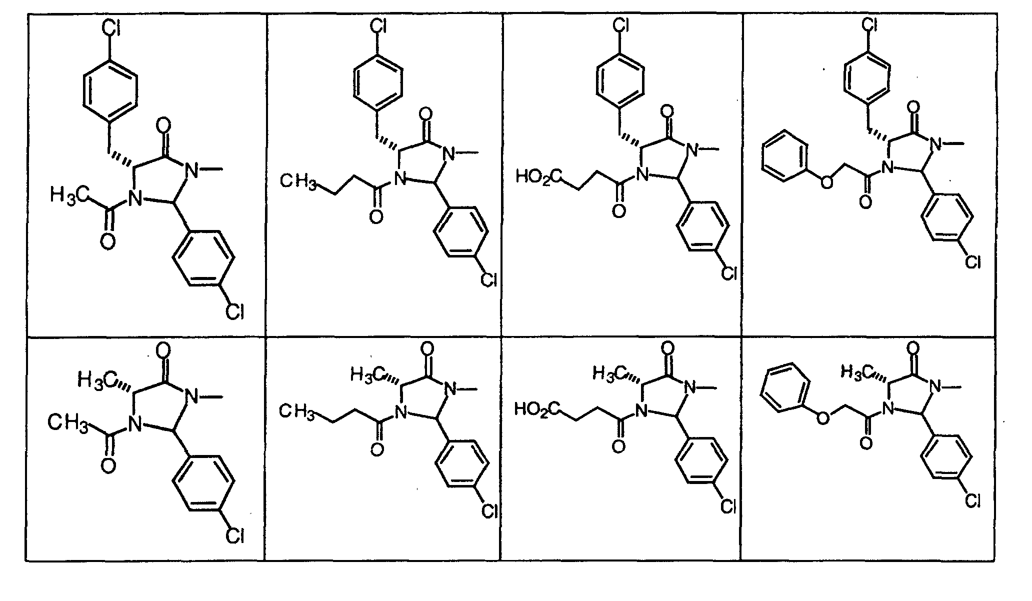

41. A compound according to claim 1 selected from the group consisting of:
4-[[(2,6-dichlorophenyl)carbonyl]amino]-N-[[1-[(4-methoxyphenyl) methyl]cyclopentyl]carbonyl]-L-phenylalanine,
N- [[1- [(4-methoxyphenyl)methyl]cyclopentyl]carbonyl]-4-[[(2-nitrophenyl) carbonyl] amino]-L-phenylalanine,
N-[[1-[(4-methoxyphenyl)methyl]cyclopentyl]carbonyl]-4-[((2-methyl-5-nitrophenyl)carbonyl]amino]-L-phenylalanine,
N-[[1-[(4-methoxyphenyl)methyl]cyclopentyl]carbonyl]-4-[(4-quinolinylcarbonyl)amino]-L-phenylalanine,
N-[[1-(phenylmethyl)cyclopentyl]carbonyl]-4-[(4-quinolinylcarbonyl)amino)-L-phenylalanine,
4-[[(2,6-dichlorophenyl)carbonyl]amino]-N-[[1-(phenylmethyl) cyclopentyl]carbonyl]-L-phenylalanine,
4-[[(2-nitrophenyl)carbonyl]amino]-N-[[1-(phenylmethyl)cyclopentyl] carbonyl]-L-phenylalanine,
4-[[(2-methyl-5-nitrophenyl)carbonyl]amino]-N-[[1-(phenylmethyl) cyclopentyl]carbonyl]-L-phenylalanine,
4-[[(2,6-dichlorophenyl)carbonyl]amino]-N-[[1-[2-[[N-(1,1-dimethylethyl) carbonyl]amino]ethyl]cyclopentyl]carbonyl]-L-phenylalanine,
4-[[(2,6-dichlorophenyl)carbonyl]amino]-N-[[1-[2-[(trifluoroacetyl) amino]ethyl]cyclopentyl]carbonyl]-L-phenylalanine,
4-[[(2,6-dichlorophenyl)carbonyl]amino]-N-[[1-[2-[(2-amino-1-oxoethyl)amino] ethyl]yclopentyl]carbonyl]-L-phenylalanine,
4-[[(2,6-dichlorophenyl)carbonyl]amino]-N-[[1-[2-[[2-[[(1,1-dimethylethoxy) carbonyl]aminol-1-oxoethyl]amino]ethyl]cyclopentyl]carbonyl]-L-phenylalanine,
4-[[(2,6-dichlorophenyl)carbonyl]amino]-N-[[2-[[(methoxy)carbonyl] amino]ethyl]cyclopentyl]carbonyl]-L-phenylalanine,
4-[[(2,6-dichlorophenyl)carbonyl]amino]-N-[[1-[2-[(methylsulfonyl) amino] ethyl] cyclopentyl] carbonyl]-L-phenylalanine,
4-[[(2,6-dichlorophenyl)carbonyl]amino]-N-[[1-[2-[(acetyl)(methyl) amino] ethyl]cyclopentyl]carbonyl]-L-phenylalanine,
4- [ (2,6-dichlorophenyl)carbonyl] amino]-N-[[1-[2-[(methylamino)carbonyl] (methyl)amino] ethyl] cyclopentyl]carbonyl]-L-phenylalanine,
4-[[(2,6-dichlorophenyl)carbonyl]amino]-N-[[1-[2-[(methoxycarbonyl)-(methyl)amino] ethyl] cyclopentyl]carbonyl]-L-phenylalanine,
4- [(2,6-dichlorophenyl)carbonyl]amino]-N-[[1-(2-methoxyethyl)cyclopentyl] carbonyl]-L-phenylalanine,
4- [[(2,6-dichlorophenyl)carbonyl] amino] -N- [[1- [(2- ((methylsulfonyl)ethyl] cyclopentyl]carbonyl]-L-phenylalanine,
4-[[(2,6-dichlorophenyl)carbonyl]amino]-N-[[1-[(2-(methylsulfinyl)ethyl] cyclopentyl]carbonyl]-L-phenylalanine,
4-[(2,6-Dimethyl-4-trifluoromethyl-3-pyridinyl)carbonyl]amino]-N-[[1-[4-(methylsulfonyl)butyl]cyclopentyl]carbonyl]-L-phenylalanine,
4- [ [(2,4-dimethylpyridin-3-yl)carbonyl]amino]-N-[[1-[4-(methylsulfonyl) butyl]cyclopentyl]carbonyl]-L-phenylalanine, or
4-[[(2,6-dichlorophenyl)carbonyl]amino]-N-[[1-(4-methoxyphenylmethyl) cyclohexyl]carbonyl]-L-phenylalanine.

42. A compound according to claim 1 having the formula selected from the group consisting of

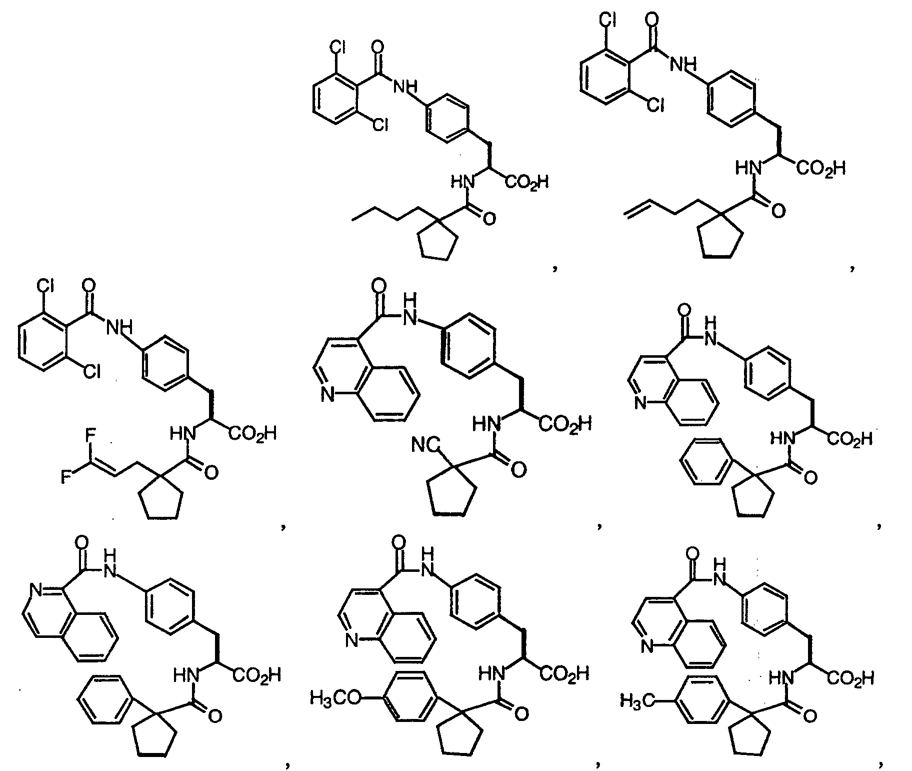

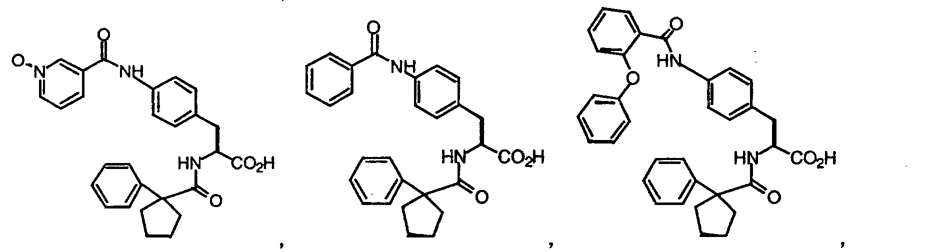

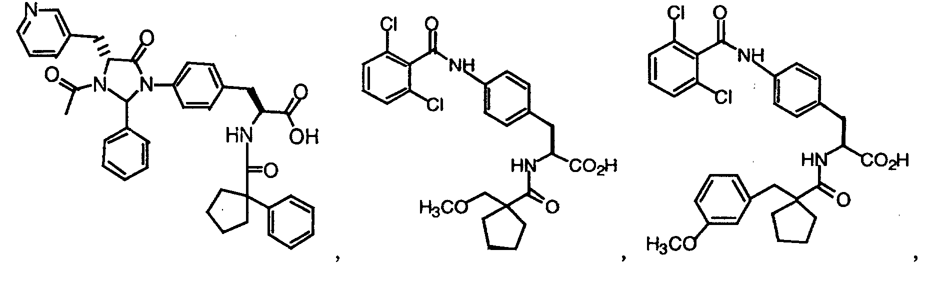

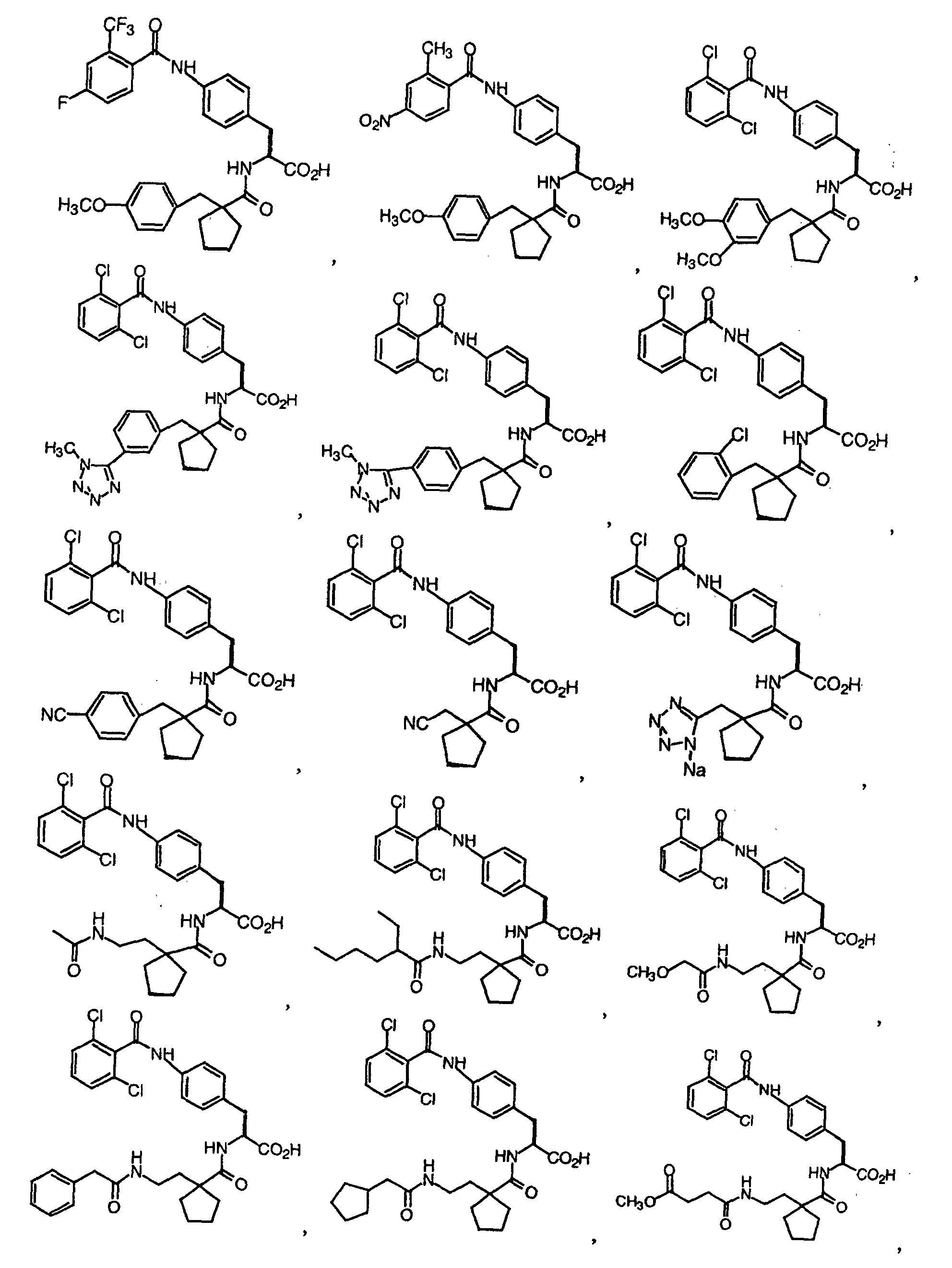

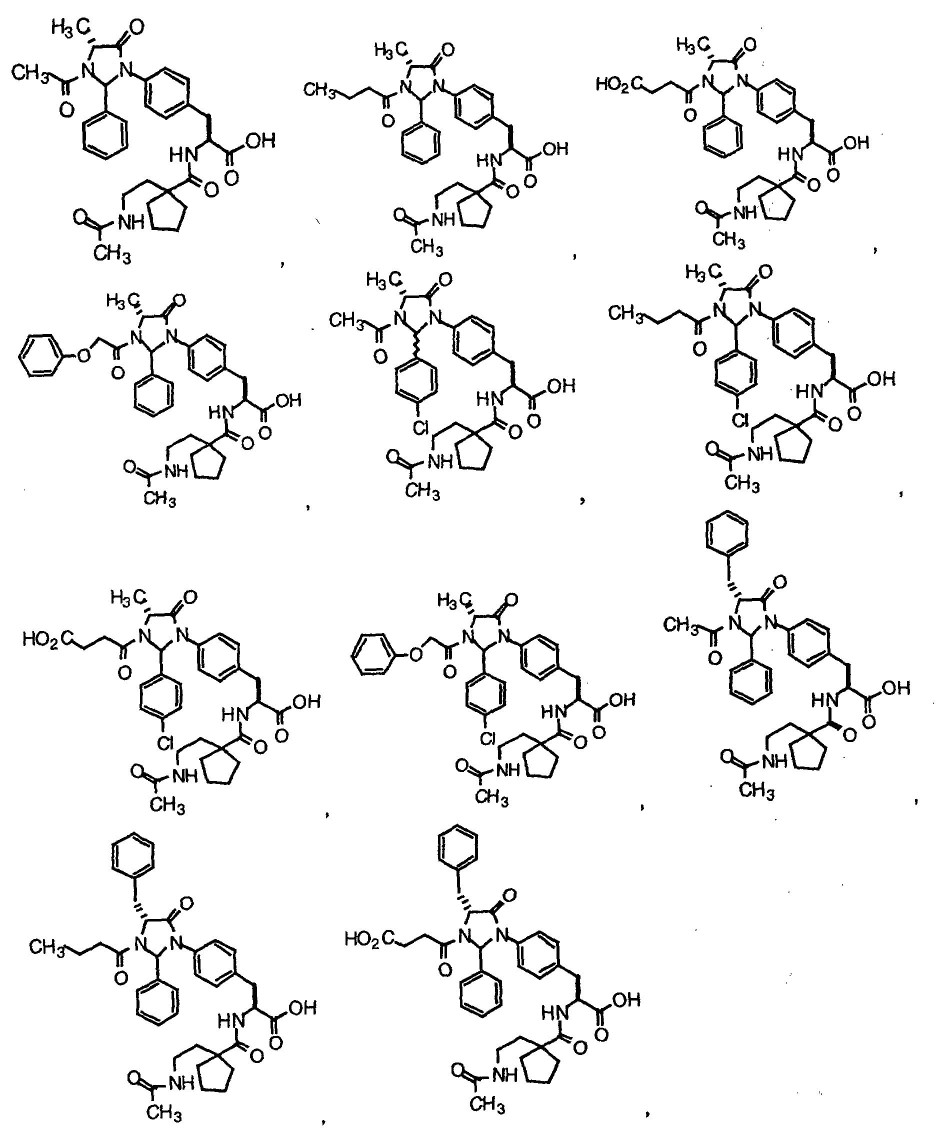

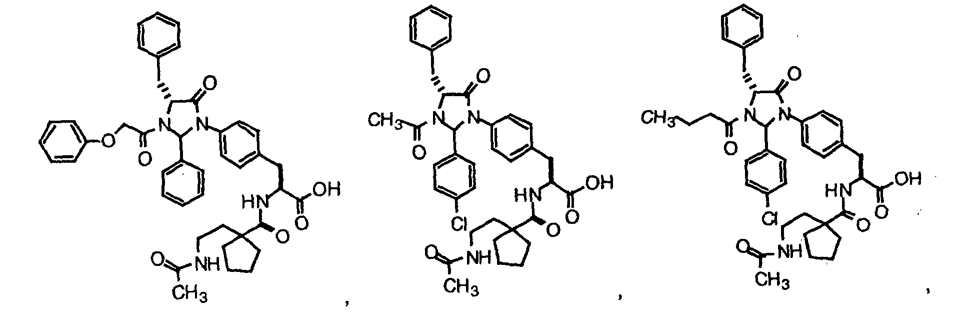

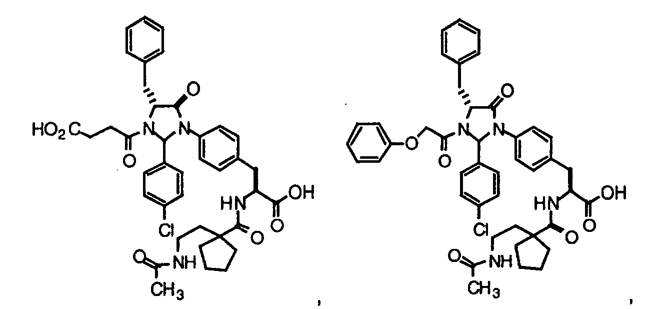

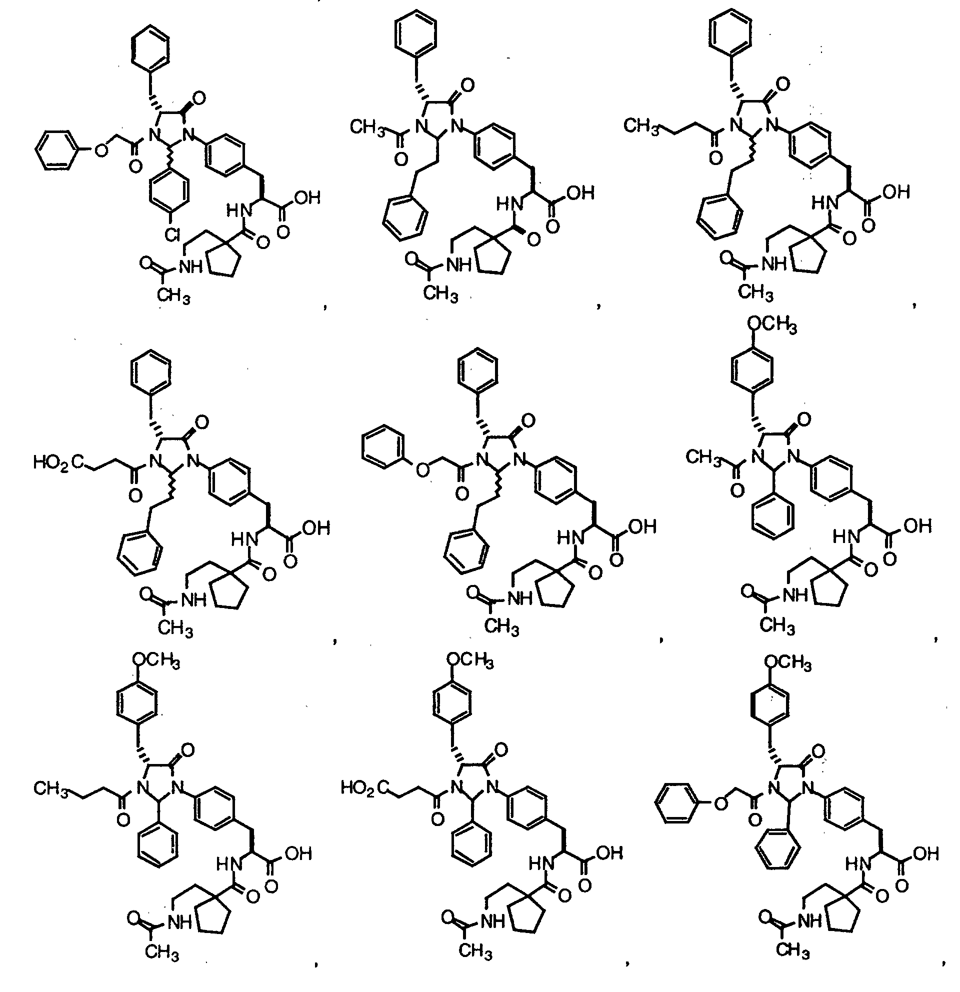

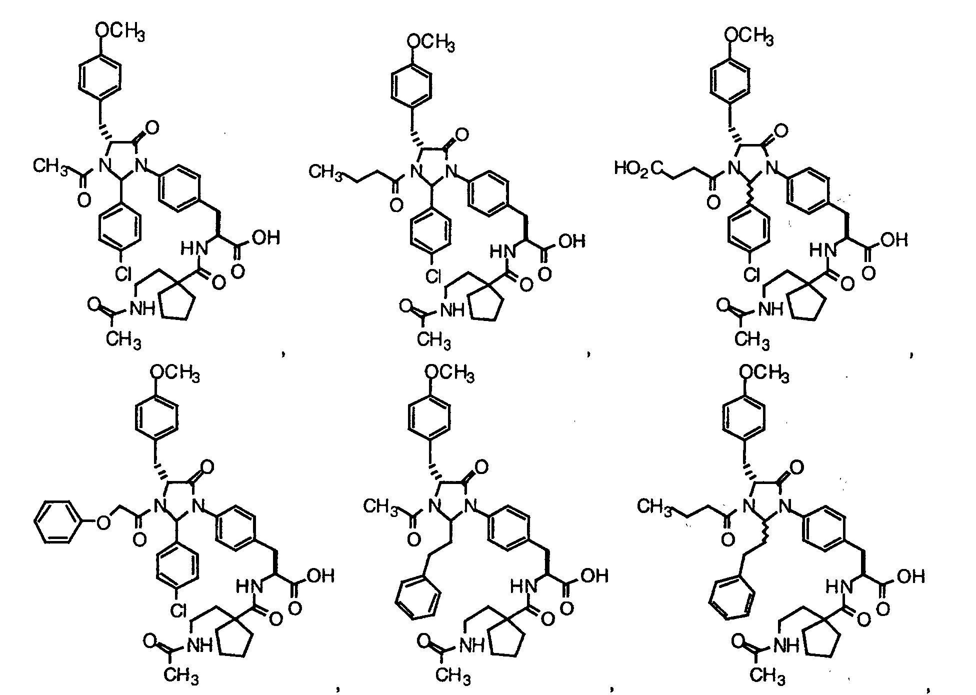

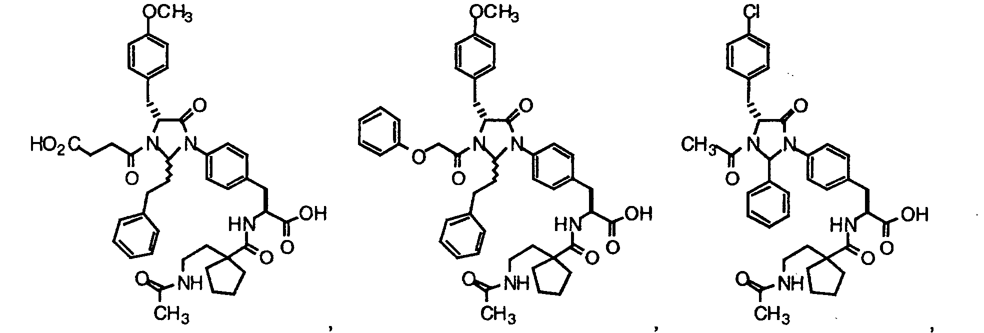

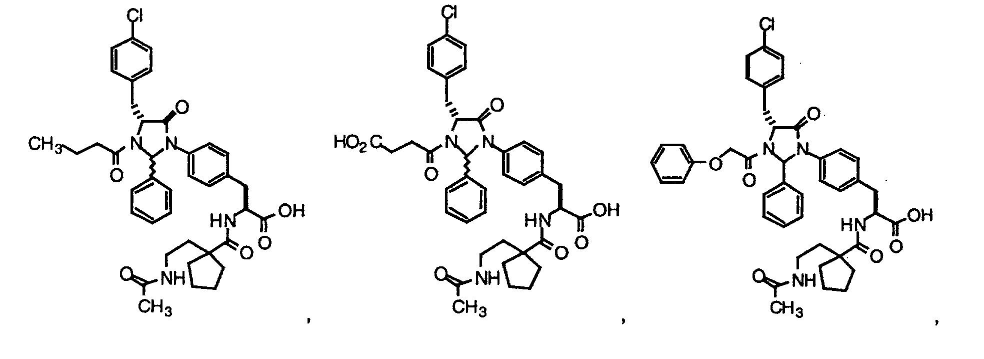

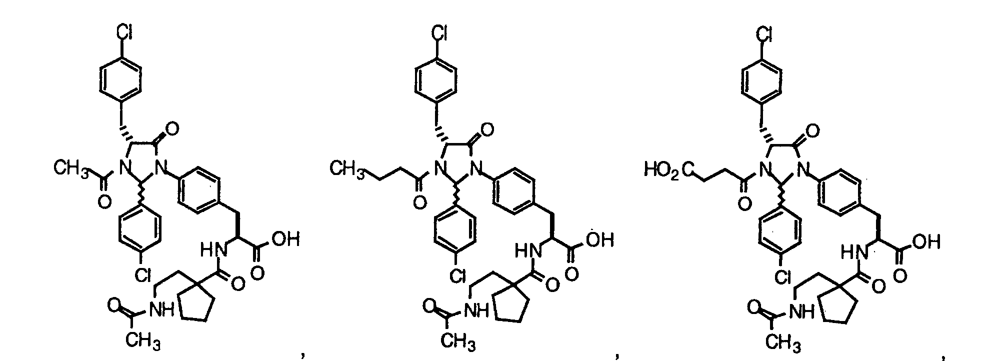

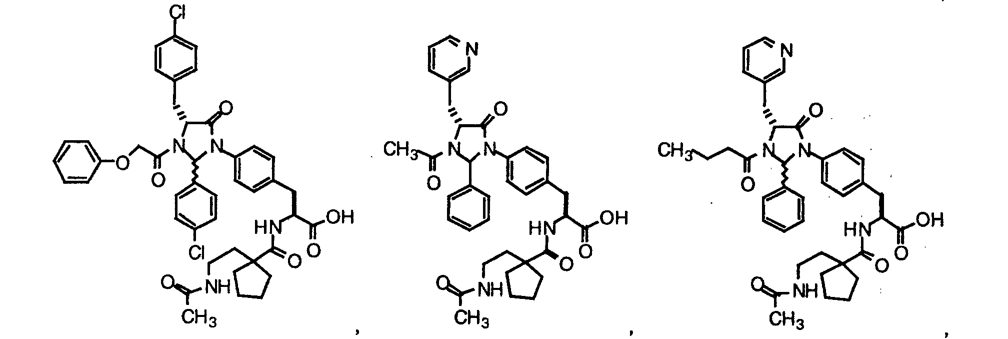

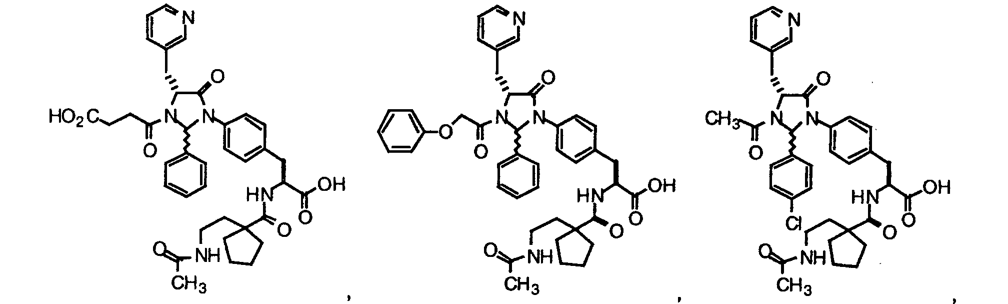

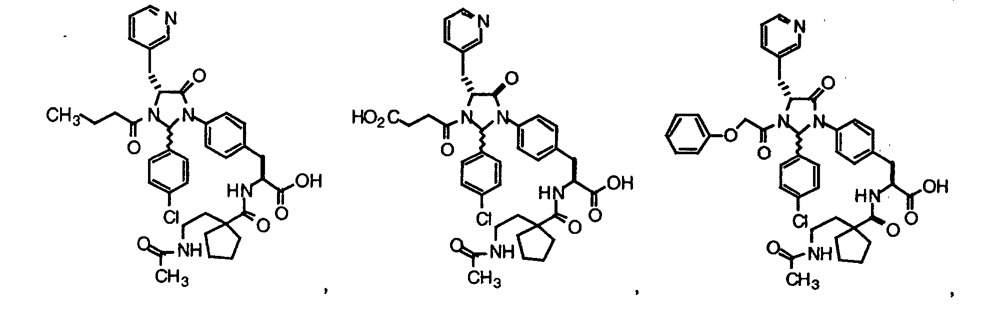

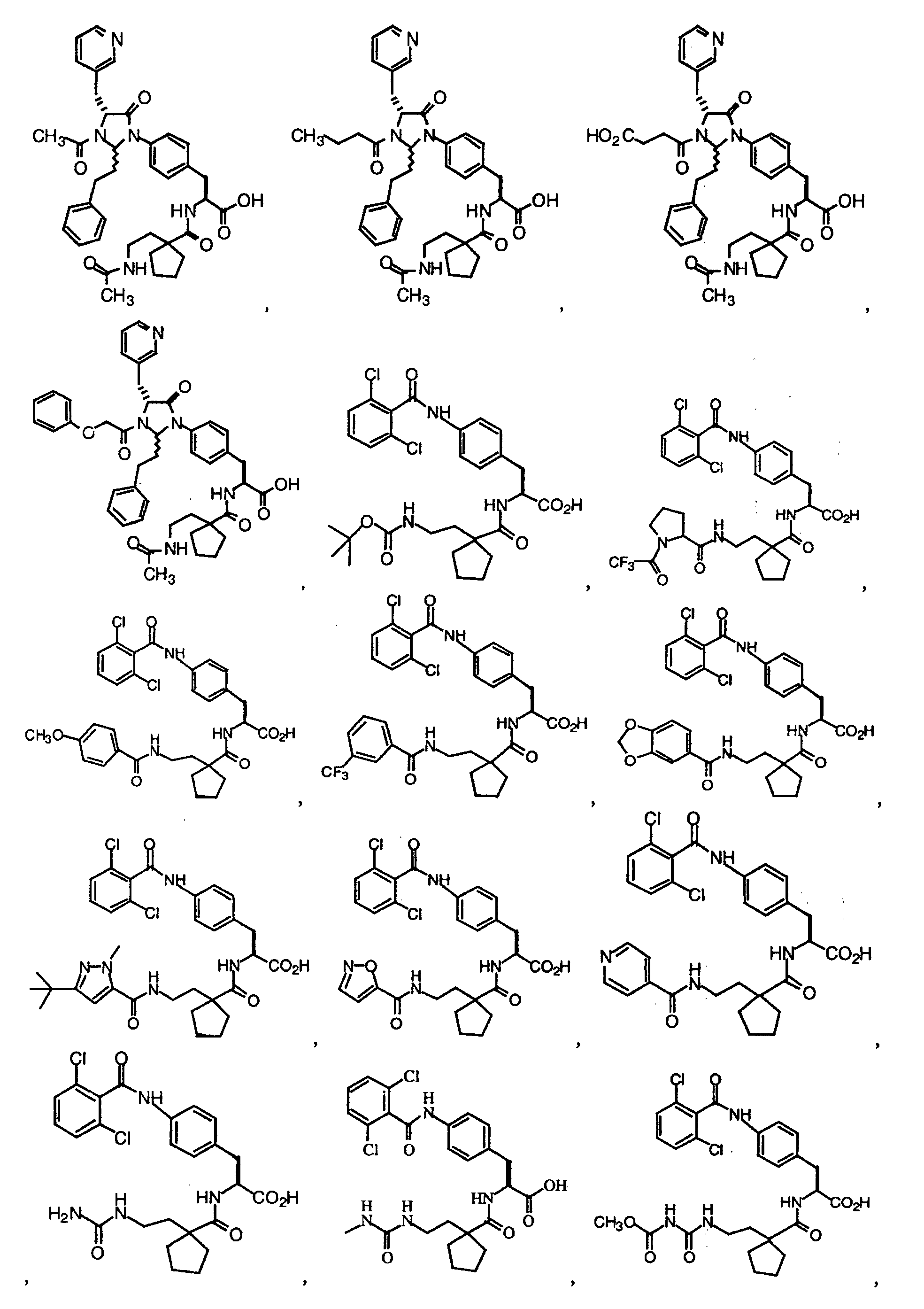

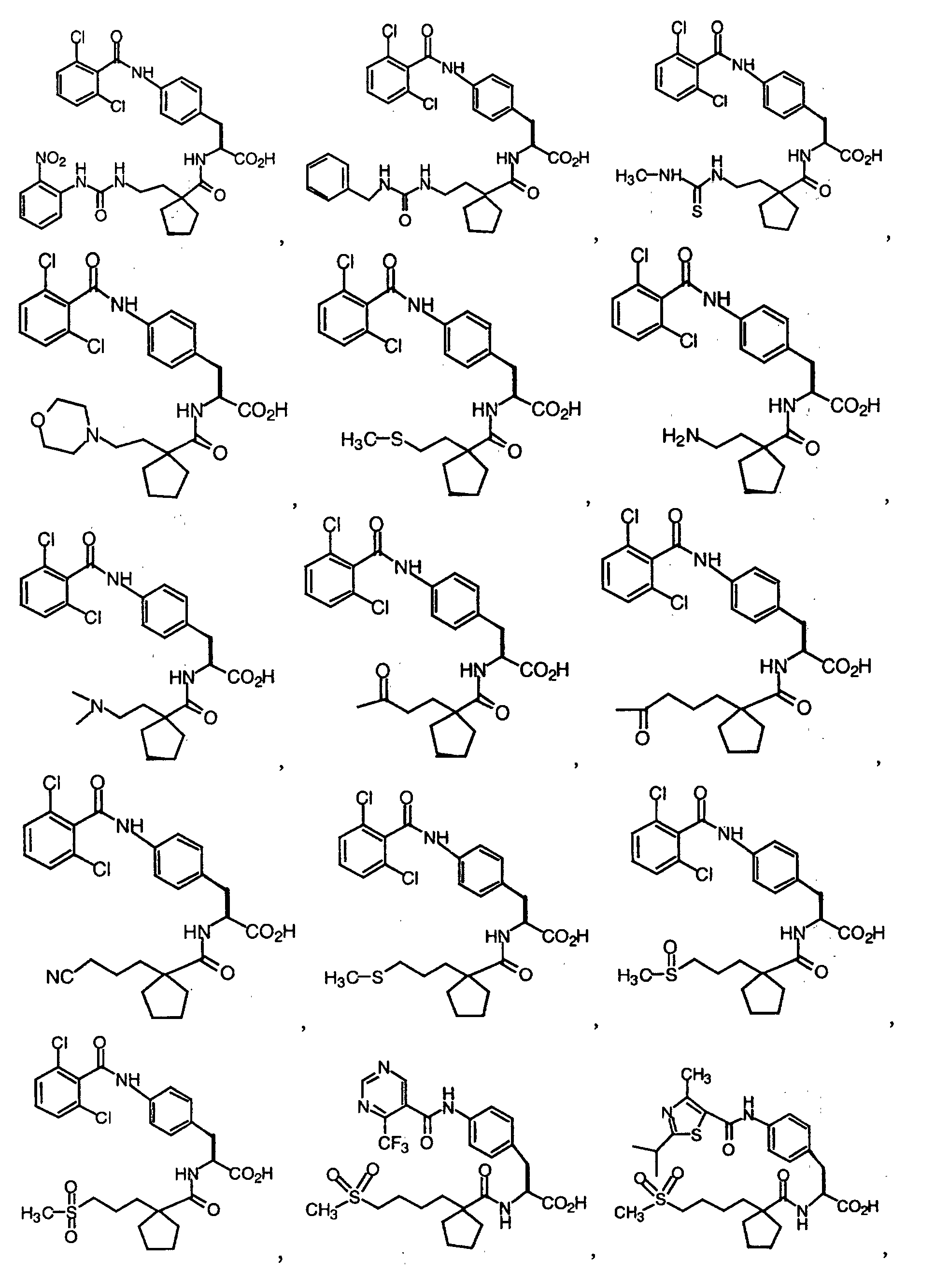

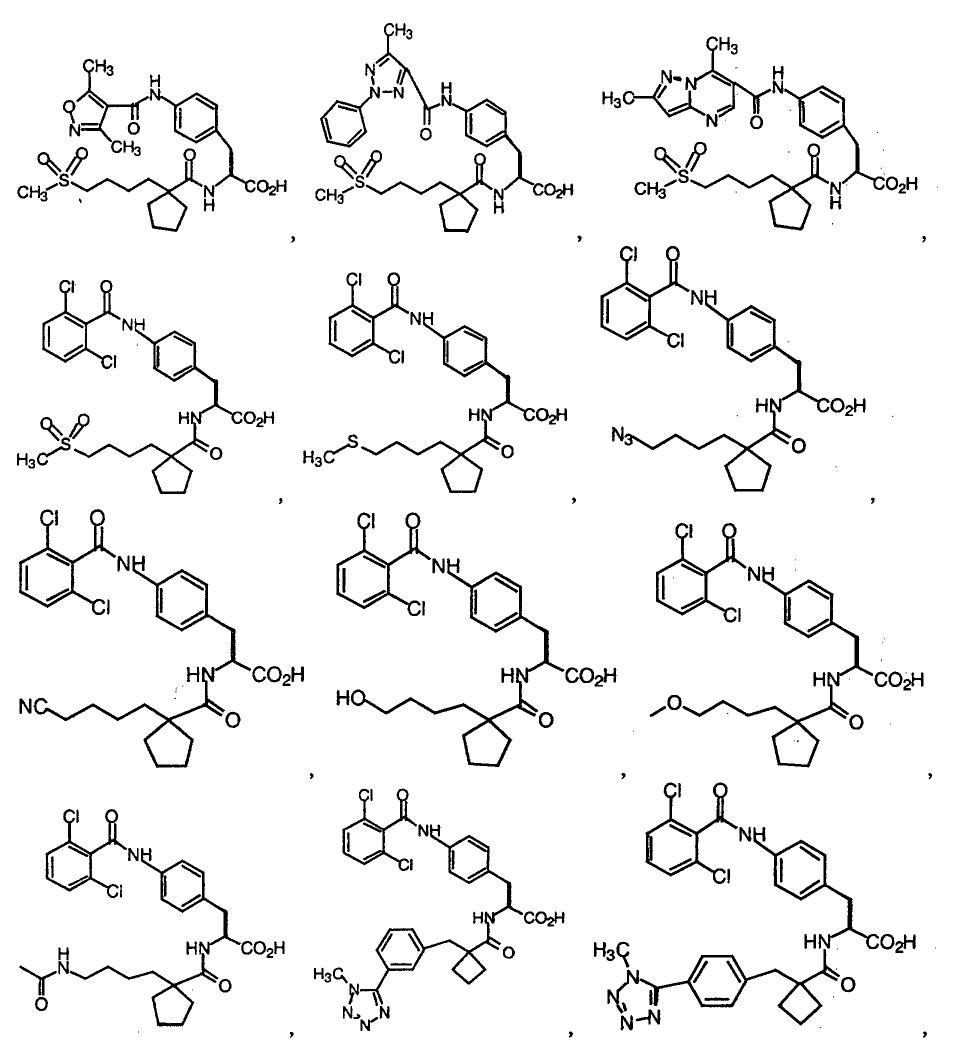

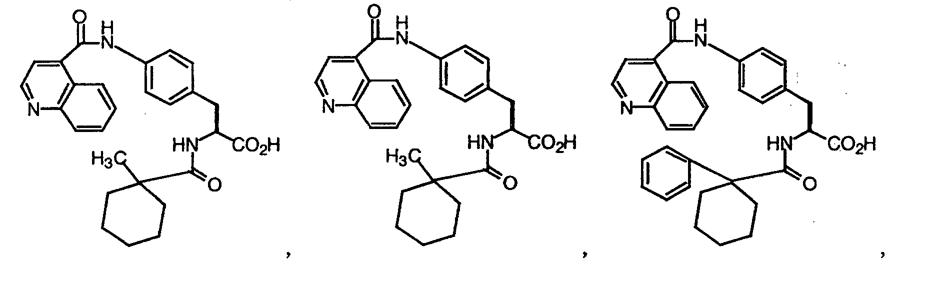

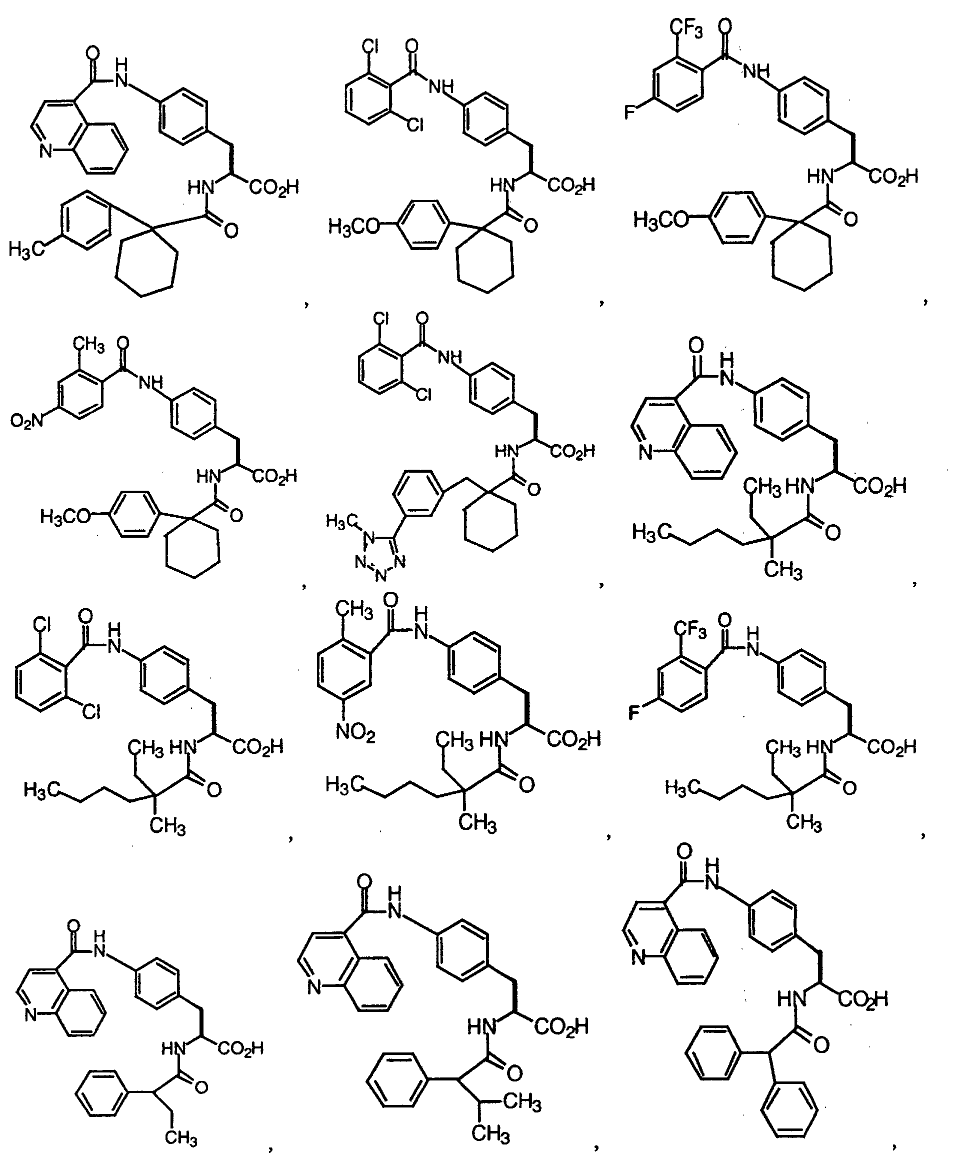

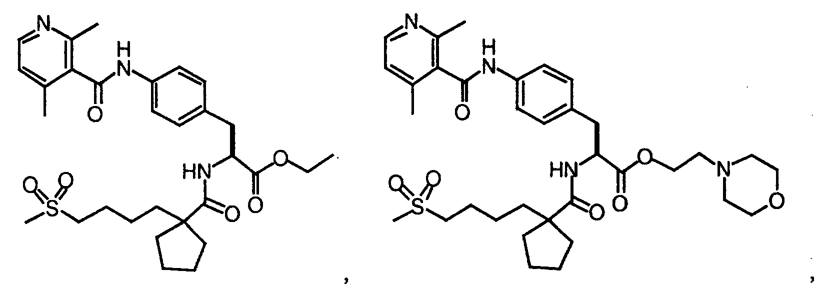

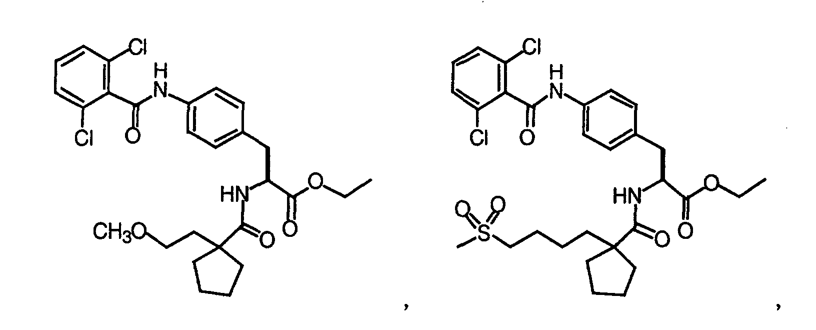

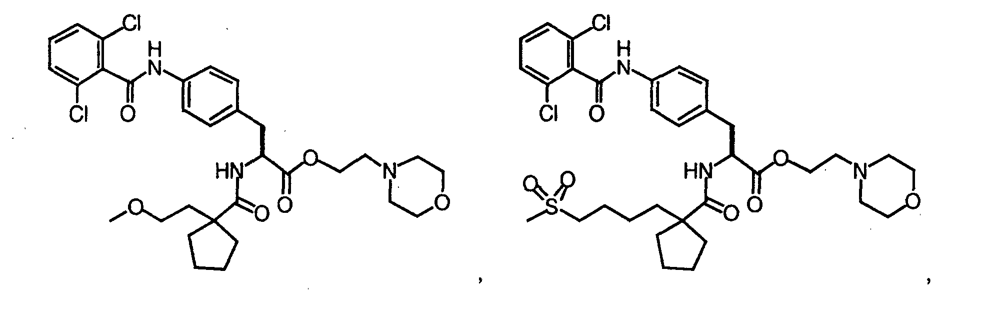

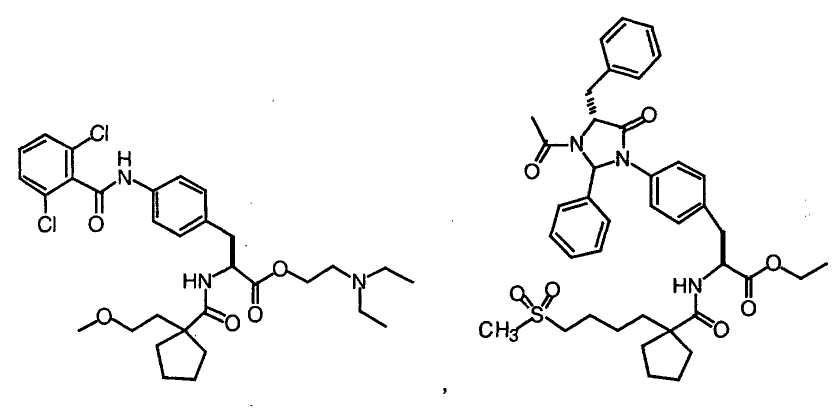

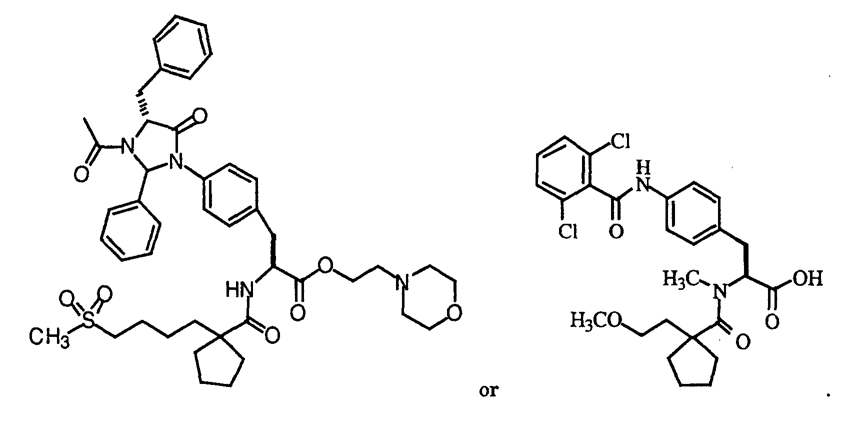

43. A process for the preparation of a compound of formula 1 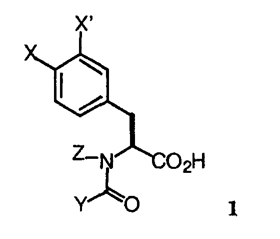 wherein X, X', Z and Y are as defined in claim 1,
**characterized in that** in a compound of formula 1b 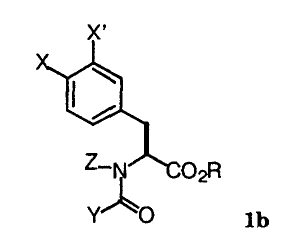 wherein X, X', Z and Y are as defined and R is a protecting group or a solid support,
the protecting group or the solid support is cleaved of and, if desired, converting a compound of formula 1 into a pharmaceutically acceptable salt.

44. Compounds according to any one of the claims 1-42 for use as a medicament.

45. Compounds according to any one of the claim 44 for use as a medicament in the treatment of rheumatoid arthritis, multiple sclerosis, inflammatory bowel disease or asthma.

46. A medicament containing a compound according to any one of claims 1-42 and a therapeutically inert carrier material.

47. A medicament according to claim 46 for the treatment of rheumatoid arthritis, multiple sclerosis, inflammatory bowel disease or asthma.

48. A process for the production of a medicament, especially for the treatment of rheumatoid arthritis, multiple sclerosis, inflammatory bowel disease or asthma, which process comprises bringing a compound according to any one of claims 1-42 into a galenical administration form together with a therapeutically inert carrier material and, if desired, one or more additional therapeutically active substances.

49. Use of a compound according to any one of claims 1-42 in the preparation of a medicament for the treatment of rheumatoid arthritis, multiple sclerosis, inflammatory bowel disease or asthma.

## Patentansprüche

1. Verbindung der Formel 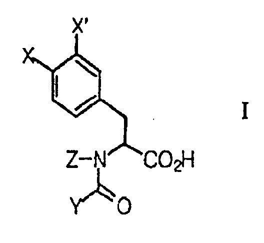 worin:
X' H, Halogen oder C₁₋₆-Alkyl darstellt und X eine Gruppe der Formel: 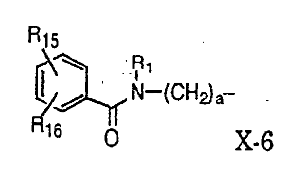
darstellt,
worin:
R₁ Wasserstoff oder C₁₋₆-Alkyl darstellt,
R₁₅ Wasserstoff, Halogen, Nitro, C₁₋₆-Alkylsulfonyl, Cyano, C₁₋₆-Alkyl, C₁₋₆-Alkoxy, C₁₋₆-Alkoxycarbonyl, Carboxy, C₁₋₆-Alkylaminosulfonyl, Perfluor-C₁₋₆-alkyl, C₁₋₆-Alkylthio, Hydroxy-C₁₋₆-alkyl, Alkoxy-C₁₋₆-alkyl, Alkylthio-C₁₋₆-alkyl, Alkylsulfinyl-C₁₋₆-alkyl, Alkylsulfonyl-C₁₋₆-alkyl, C₁₋₆-Alkylsulfinyl, C₁₋₆-Alkylcarbonyl, Aryloxy, Aroyl, Aryl oder eine Gruppe der Formel R₁₇-C≡C- darstellt,
R₁₆ H, Halogen, Nitro, Cyano, C₁₋₆-Alkyl, OH, Perfluor-C₁₋₆-alkyl oder C₁₋₆-Alkylthio darstellt und
R₁₇ H, Aryl, Heteroaryl oder C₁₋₆-Alkyl, das unsubstituiert oder mit OH, Aryl oder Heteroaryl substituiert ist, darstellt und
a 0 oder 1 ist;
oder X eine Gruppe der Formel : 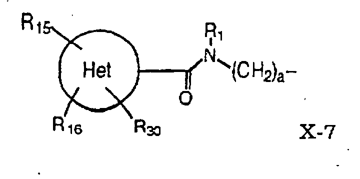 darstellt,
worin 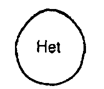 einen 5- oder 6-gliedrigen heteroaromatischen Ring, enthaltend 1, 2 oder 3 Heteroatome, ausgewählt aus N, O und S, darstellt;
oder 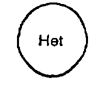 einen 9- oder 10-gliedrigen bicyclischen heteroaromatischen Ring, enthaltend 1, 2, 3 oder 4 Heteroatome, ausgewählt aus O, S und N, darstellt,
a, R₁, R₁₅ und R₁₆ wie in X-6 definiert sind und
R₃₀ Wasserstoff oder C₁₋₆-Alkyl darstellt oder nicht vorliegt;
oder X eine Gruppe der Formel: 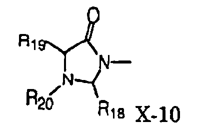 darstellt,
worin:
R₁₈ Wasserstoff, substituiertes oder unsubstituiertes C₁₋₆-Alkyl, Aryl, Heteroaryl, Arylalkyl oder Heteroarylalkyl darstellt,
R₁₉ unsubstituiertes oder substituiertes C₁₋₆-Alkyl, Aryl, Heteroaryl, Arylalkyl oder Heteroarylalkyl darstellt und
R₂₀ unsubstituiertes oder substituiertes C₁₋₆-Alkyl, C₁₋₆-Alkylcarbonyl, gegebenenfalls substituiert mit Carboxyl, Aroyl oder Aryloxy, darstellt;
Y eine Gruppe der Formel: 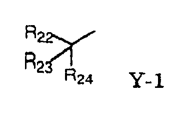 darstellt,
worin :
R₂₂ und R₂₃ unabhängig Aryl, Heteroaryl oder C₁₋₆-Alkyl, das unsubstituiert oder mit einem oder mehreren Chlor, Brom, Nitro, Hydroxy, C₁₋₆-Alkoxy, Aryl, C₁₋₆-Alkylcarbonyl, Aroyl oder Cyano substituiert ist, darstellen,
R₂₄ Aryl, Cyano, Alkylsulfonyl oder C₁₋₆-Alkyl oder Alkenyl, bis zu C₆ unsubstituiert oder substituiert mit einem Aryl- oder Heteroarylring, darstellt, und wenn R₂₂ Aryl darstellt und R₂₃ Aryl oder C₁₋₆-Alkyl darstellt, R₂₄ H darstellt und die Gesamtanzahl an Kohlenstoffatomen in R₂₂, R₂₃ und R₂₄ 6 bis 14 ist; oder
Y einen 3-7-gliedrigen Ring der Formel: 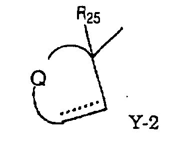 darstellt,
worin:
R₂₅ C₁₋₆-Alkyl, unsubstituiertes oder Fluor-substituiertes Alkenyl bis zu C₆, oder eine Gruppe der Formel R₂₆-(CH₂)ₑ- darstellt,
R₂₆ Aryl, Heteroaryl, Azido, Cyano, Hydroxy, C₁₋₆-Alkoxy, C₁₋₆-Alkoxycarbonyl, C₁₋₆-Alkylcarbonyl, C₁₋₆-Alkylthio, C₁₋₆-Alkylsulfonyl, C₁₋₆-Alkylsulfinyl, Perfluor-C₁₋₆-alkylcarbonyl, Nitro darstellt, oder R₂₆ eine Gruppe der Formel -NR₂₈R₂₉ darstellt, worin:
R₂₈ H oder C₁₋₆-Alkyl darstellt,
R₂₉ Wasserstoff, C₁₋₆-Alkyl, C₁₋₆-Alkoxycarbonyl, gegebenenfalls substituiertes Aminocarbonyl, gegebenenfalls substituiertes C₁₋₆-Alkylcarbonyl, Aroyl, Heteroaroyl, Heterocycloalkylcarbonyl, C₁₋₆-Alkylsulfonyl, C₁₋₆-Alkylaminothiocarbonyl darstellt, oder
R₂₈ und R₂₉, zusammengenommen mit dem Stickstoffatom, an das sie gebunden sind, einen 4-, 5- oder 6-gliedrigen, gesättigten heterocyclischen Ring, enthaltend ein oder zwei Heteroatome, wobei das zweite Heteroatom O, S oder N-R₂₇ darstellt, bilden;
Q -(CH₂)_{f}O-, -(CH₂)_{f}S-, (CH₂)_{f}- ist, oder, wenn f=0, eine Bindung darstellt,
R₂₇ H, C₁₋₆-Alkyl, Aryl, C₁₋₆-Alkylcarbonyl, Aroyl oder C₁₋₆-Alkoxycarbonyl darstellt, wobei die Kohlenstoffatome in dem Ring unsubstituiert oder mit C₁₋₆-Alkyl oder Halogen substituiert sind,
e eine ganze Zahl von 0 bis 4 ist,
f eine ganze Zahl von 0 bis 3 ist,
und die Punktlinie eine Bindung bedeutet, die nicht vorliegen oder vorliegen kann; und
Z Wasserstoff oder C₁₋₆-Alkyl darstellt;
und die pharmazeutisch verträglichen Salze und Ester davon,
wobei der Begriff "substituiertes C₁₋₆-Alkyl", wenn nicht anderweitig definiert, eine C₁₋₆-Alkylgruppe, substituiert mit einer oder mehreren Gruppen, unabhängig ausgewählt aus Cycloalkyl, Nitro, Aryloxy, Aryl, Hydroxy, Halogen, Cyano, C₁₋₆-Alkoxy, C₁₋₆-Alkylcarbonyl, C₁₋₆-Alkylthio, C₁₋₆-Alkylsulfinyl, C₁₋₆-Alkylsulfonyl und substituiertem Amino, bedeutet;
der Begriff "Aryl" eine mono- oder bicyclische aromatische Gruppe bedeutet, die unsubstituiert oder mit C₁₋₆-Alkyl, C₁₋₆-Alkoxy, Hydroxy-C₁₋₆-alkyl, Hydroxy, Hydroxyalkoxy, Halogen, C₁₋₆-Alkylthio, C₁₋₆-Alkylsulfinyl, C₁₋₆-Alkylsulfonyl, Cyano, Nitro, Perfluoralkyl, Alkanoyl, Aroyl, Arylalkinyl, Heteroaryl, Niederalkinyl und C₁₋₆-Alkylcarbonylamino substituiert ist;
der Begriff "Aryl-C₁₋₆-alkyl" eine C₁₋₆-Alkylgruppe bedeutet, worin ein oder mehrere Wasserstoffatome durch eine Aryl- oder Heteroarylgruppe ersetzt ist/sind;
der Begriff "Heteroaryl" einen unsubstituierten oder substituierten 5- oder 6-gliedrigen monocyclischen heteroaromatischen Ring oder einen 9- oder 10-gliedrigen bicyclischen heteroaromatischen Ring, enthaltend 1, 2, 3 oder 4 Heteroatome, die unabhängig N, S oder O sind, und die mit Substituenten, wie vorstehend für "Aryl" definiert, substituiert sein können, bedeutet;
der Begriff "gegebenenfalls substituiertes C₁₋₆-Alkylcarbonyl" C₁₋₆-Alkylgruppen, gebunden über eine Carbonylgruppe, wobei die Alkylgruppe mit einer oder mehreren Gruppen, ausgewählt aus Hydroxycarbonyl, Aroyl oder Aryloxy, C₁₋₆-Alkoxy, Fluor, Phenyl, Cycloalkyl, C₁₋₆-Alkoxycarbonyl, Amino oder C₁₋₆-Alkoxycarbonylamino, substituiert sein kann, bedeutet;
der Begriff "Aroyl" eine mono- oder bicyclische Aryloder Heteroarylgruppe, wie vorstehend definiert, die über eine Carbonylgruppe gebunden ist, bedeutet;
der Begriff "Cycloalkyl" einen unsubstituierten oder substituierten 3- bis 7-gliedrigen carbocyclischen Ring, der mit Hydroxy, Halogen, Cyano, C₁₋₆-Alkoxy, C₁₋₆-Alkylcarbonyl, C₁₋₆-Alkyl, Aroyl, C₁₋₆-Alkylthio, C₁₋₆-Alkylsulfinyl, C₁₋₆-Alkylsulfonyl, Aryl, Heteroaryl und substituiertem Amino substituiert sein kann, bedeutet;
der Begriff "Heterocycloalkyl" einen unsubstituierten oder substituierten 5- bis 6-gliedrigen carbocyclischen Ring, worin ein oder zwei der Kohlenstoffatome durch Heteroatome, unabhängig ausgewählt aus O, S und N, ersetzt wurden, bedeutet.

2. Verbindung nach Anspruch 1, worin X' Wasserstoff darstellt.

3. Verbindung nach Anspruch 1 oder 2, worin Z Wasserstoff darstellt.

4. Verbindung nach einem der Ansprüche 1-3, worin Y-1, R₂₂ und R₂₃ C₁₋₆-Alkyl oder Phenyl darstellen und R₂₄ C₁₋₆-Alkyl darstellt, mit der Ausnahme, dass wenn R₂₂ Aryl darstellt und R₂₃ Aryl oder C₁₋₆-Alkyl darstellt, dann R₂₄ Wasserstoff darstellt.

5. Verbindung nach einem der Ansprüche 1-4, worin Y-1 ausgewählt ist aus der Gruppe, bestehend aus:

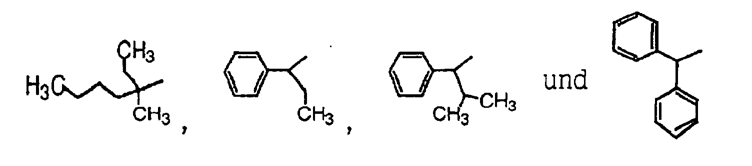

6. Verbindung nach einem der Ansprüche 1-3, worin in Y-2 Q -(CH₂)_{f}- oder, wenn f=0, eine Bindung darstellt.

7. Verbindung nach Anspruch 6, worin f 1, 2 oder 3 ist.

8. Verbindung nach einem der Ansprüche 1-3, worin in Y-2 R₂₆ Aryl, Heteroaryl, Azido, Cyano, Hydroxy, C₁₋₆-Alkoxy, C₁₋₆-Alkoxycarbonyl, C₁₋₆-Alkylcarbonyl, C₁₋₆-Alkylthio, C₁₋₆-Alkylsulfonyl, C₁₋₆-Alkylsulfinyl, Nitro darstellt, oder R₂₆ eine Gruppe der Formel -NR₂₈R₂₉ darstellt, worin R₂₈ H oder C₁₋₆-Alkyl darstellt, R₂₉ Wasserstoff, C₁₋₆-Alkyl, C₁₋₆-Alkoxycarbonyl, gegebenenfalls substituiertes Aminocarbonyl, gegebenenfalls substituiertes C₁₋₆-Alkylcarbonyl, Aroyl, C₁₋₆-Alkylsulfonyl darstellt, oder R₂₈ und R₂₉, zusammengenommen mit dem Stickstoffatom, an das sie gebunden sind, einen 4-, 5-oder 6-gliedrigen, gesättigten heterocyclischen Ring bilden, der ein Sauerstoffatom enthalten kann.

9. Verbindung nach einem der Ansprüche 1-3, worin R₂₈ H darstellt.

10. Verbindung nach Anspruch 8 oder Anspruch 9, worin R₂₆ Aryl, unsubstituiert oder monosubstituiert mit Halogen, C₁₋₆-Alkoxy, C₁₋₆-Alkyl, Cyano, oder Tetrazolyl, das unsubstituiert oder mit Methyl substituiert ist, darstellt oder R₂₆ Phenyl, disubstituiert mit C₁₋₆-Alkoxy, darstellt.

11. Verbindung nach Anspruch 10, worin R₂₆ Phenyl, unsubstituiert oder monosubstituiert mit Chlor, Methoxy oder Methyl, darstellt.

12. Verbindung nach Anspruch 8, worin R₂₆ CH₃S(O)₂-, CH₃S-, CH₃SO-, CH₃O-, CH₃CO-, NC-, N₃- oder HO- darstellt.

13. Verbindung nach Anspruch 8, worin die Alkylgruppe in der C₁₋₆-Alkylcarbonylgruppe von R₂₉ unsubstituiert oder mit C₁₋₆-Alkoxy, Fluor, Phenyl, Cycloalkyl, C₁₋₆-Alkoxycarbonyl, Amino oder C₁₋₆-Alkoxycarbonylamino substituiert ist.

14. Verbindung nach Anspruch 13, worin die unsubstituierte oder substituierte C₁₋₆-Alkylcarbonylgruppe CH₃CO-, (CH₃)₃CCO-, CH₃(CH₂)₃CHCH₃CO-, CH₃OCH₂CO-, CF₃CO-, C₆H₅CH₂CO-, CH₃OCO(CH₂)₂CO-, CyclopentylCH₂CO-, H₂NCH₂CO oder (CH₃)₃COCONH(CH₂)₂CO- darstellt.

15. Verbindung nach Anspruch 8, worin in R₂₉ die Aminocarbonylgruppe unsubstituiert oder mit C₁₋₆-Alkyl, C₁₋₆-Alkoxycarbonyl, monocyclischem Aryl oder Benzyl substituiert ist.

16. Verbindung nach Anspruch 15, worin die unsubstituierte oder substituierte Aminocarbonylgruppe H₂NCO-, CH₃NHCO-, CH₃OCONHCO-, C₆H₄NO₂NHCO- oder C₆H₅CH₂NHCO- darstellt.

17. Verbindung nach Anspruch 1, worin R₂₉ (CH₃)₃COCO-, Methylaminothiocarbonyl, 4-Methoxyphenylcarbonyl, 3-Trifluormethylphenylcarbonyl, 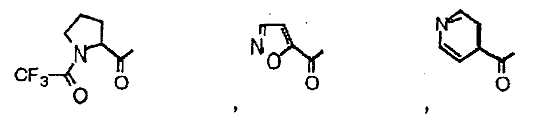 oder 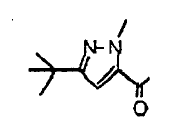 darstellt, -NR₂₈R₂₉ -NH₂ oder -N(CH₃)₂ darstellt und R₂₈ und R₂₉ zusammengenommen 4-Morpholinyl darstellen.

18. Verbindung nach einem der Ansprüche 1-3, worin Y-2 ausgewählt ist aus einer Gruppe, wiedergegeben durch die nachstehenden Formeln:

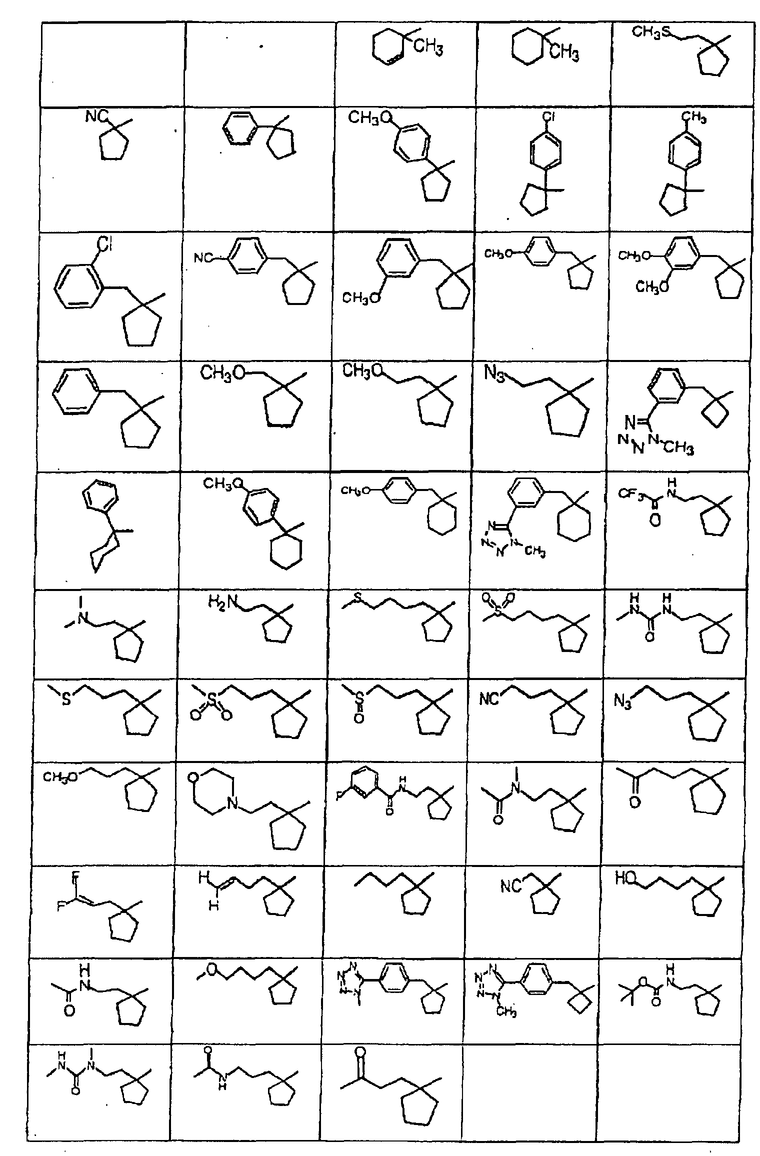

19. Verbindung nach einem der Ansprüche 1-3, worin in X-6 R₁₅ und R₁₆ unabhängig H, Halogen, Nitro, Perfluor-C₁₋₆alkyl, C₁₋₆-Alkyl, Cyano darstellen oder R₁₅ Phenoxy darstellt und R₁₆ H darstellt.

20. Verbindung nach Anspruch 19, worin in X-6 die Gruppen R₁₅ und R₁₆ unabhängig Wasserstoff, Methyl, Nitro, Chlor, Trifluormethyl oder Cyano darstellen.

21. Verbindung nach einem der Ansprüche 1-3, worin in X-6 R₁ Wasserstoff darstellt.

22. Verbindung nach einem der Ansprüche 1-3, worin a in X-6 0 ist.

23. Verbindung nach einem der Ansprüche 1-3, worin X-6 ausgewählt ist aus der Gruppe, bestehend aus:

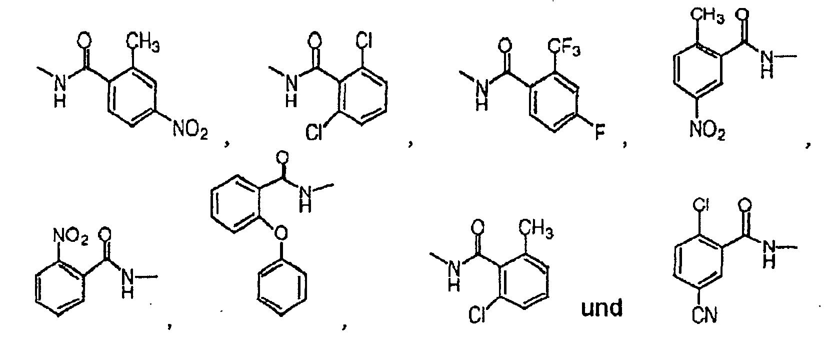

24. Verbindung nach einem der Ansprüche 1-3, worin in X-7 Het einen 5- oder 6-gliedrigen monocyclischen heteroaromatischen Ring, enthaltend 1, 2 oder 3 Stickstoffatome, oder ein Stickstoffatom und ein Schwefelatom, oder ein Stickstoffatom und ein Sauerstoffatom, darstellt.

25. Verbindung nach Anspruch 24, worin der heteroaromatische Ring allein 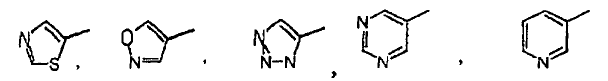 oder 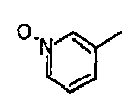 darstellt.

26. Verbindung nach einem der Ansprüche 1-3, worin in X-7 Het einen bicyclischen heteroaromatischen Ring, enthaltend 1 bis 3 Stickstoffatome als die Heteroatome, darstellt.

27. Verbindung nach Anspruch 26, worin der bicyclische heteroaromatische Ring 4-Chinolinyl, 1-Isochinolinyl oder 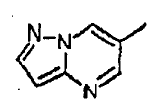 darstellt.

28. Verbindung nach einem der Ansprüche 1-3, worin in X-7 R₁₅ Wasserstoff, Nitro, C₁₋₆-Alkylsulfonyl, Cyano, C₁₋₆-Alkyl, C₁₋₆-Alkoxy, Perfluor-C₁₋₆-alkyl, C₁₋₆-Alkylthio, C₁₋₆-Alkylcarbonyl oder Aryl darstellt.

29. Verbindung nach Anspruch 28, worin R₁₅ Isopropyl, Methyl oder Phenyl darstellt.

30. Verbindung nach einem der Ansprüche 1-3, worin in X-7 R₁₆ Wasserstoff, Halogen, Nitro, Cyano, C₁₋₆-Alkyl oder Perfluor-C₁₋₆-alkyl darstellt.

31. Verbindung nach Anspruch 30, worin R₁₆ Methyl oder Trifluormethyl darstellt.

32. Verbindung nach einem der Ansprüche 1-3, worin in X-7 R₃₀ Wasserstoff oder C₁₋₆-Alkyl darstellt.

33. Verbindung nach einem der Ansprüche 1-3, worin X-7 ausgewählt ist aus der Gruppe, bestehend aus 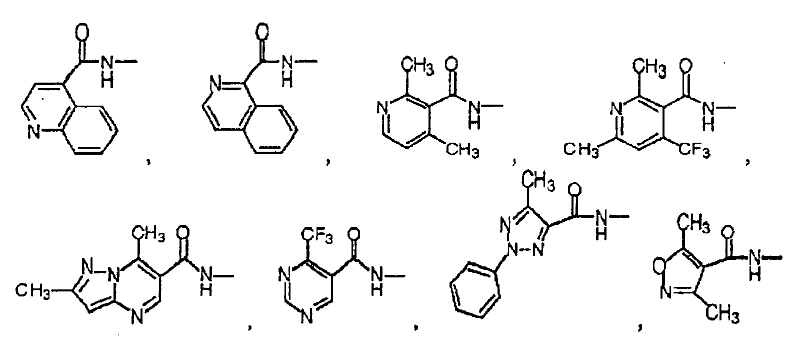 und

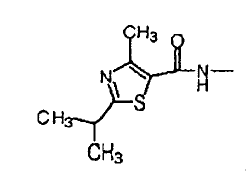

34. Verbindung nach einem der Ansprüche 1-3, worin in X-10 R₁₈ Phenyl darstellt, wobei der Phenylring unsubstituiert oder mit Halogen monosubstituiert ist oder Phenyl-C₁₋₆alkyl darstellt.

35. Verbindung nach Anspruch 34, worin R₁₈ Phenyl, Chlorphenyl oder Phenylethyl darstellt.

36. Verbindung nach einem der Ansprüche 1-3, worin in X-10 R₁₉ C₁₋₆-Alkyl darstellt, das unsubstituiert oder mit Pyridyl oder Phenyl substituiert ist, wobei der Phenylring unsubstituiert oder mit C₁₋₆-Alkoxy oder Halogen monosubstituiert ist.

37. Verbindung nach Anspruch 36, worin R₁₉ Methyl, Isobutyl, Benzyl, 4-Chlorbenzyl, 4-Methoxybenzyl oder 2-Pyridylmethyl darstellt.

38. Verbindung nach einem der Ansprüche 1-3, worin in X-10 R₂₀ gegebenenfalls substituiertes C₁₋₆-Alkylcarbonyl darstellt.

39. Verbindung nach Anspruch 38, worin R₂₀ Acetyl, Butyryl, Phenoxyacetyl, Succinyl oder Glutaryl darstellt.

40. Verbindung nach einem der Ansprüche 1-3, worin X-10 ausgewählt ist aus der Gruppe, bestehend aus:

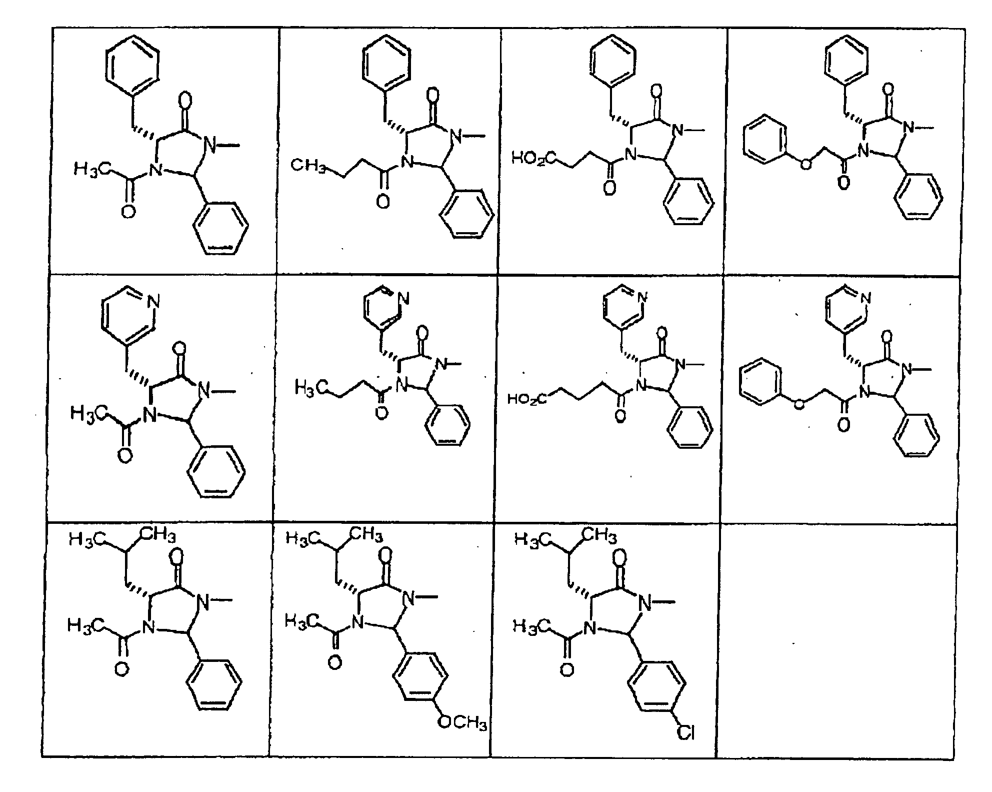

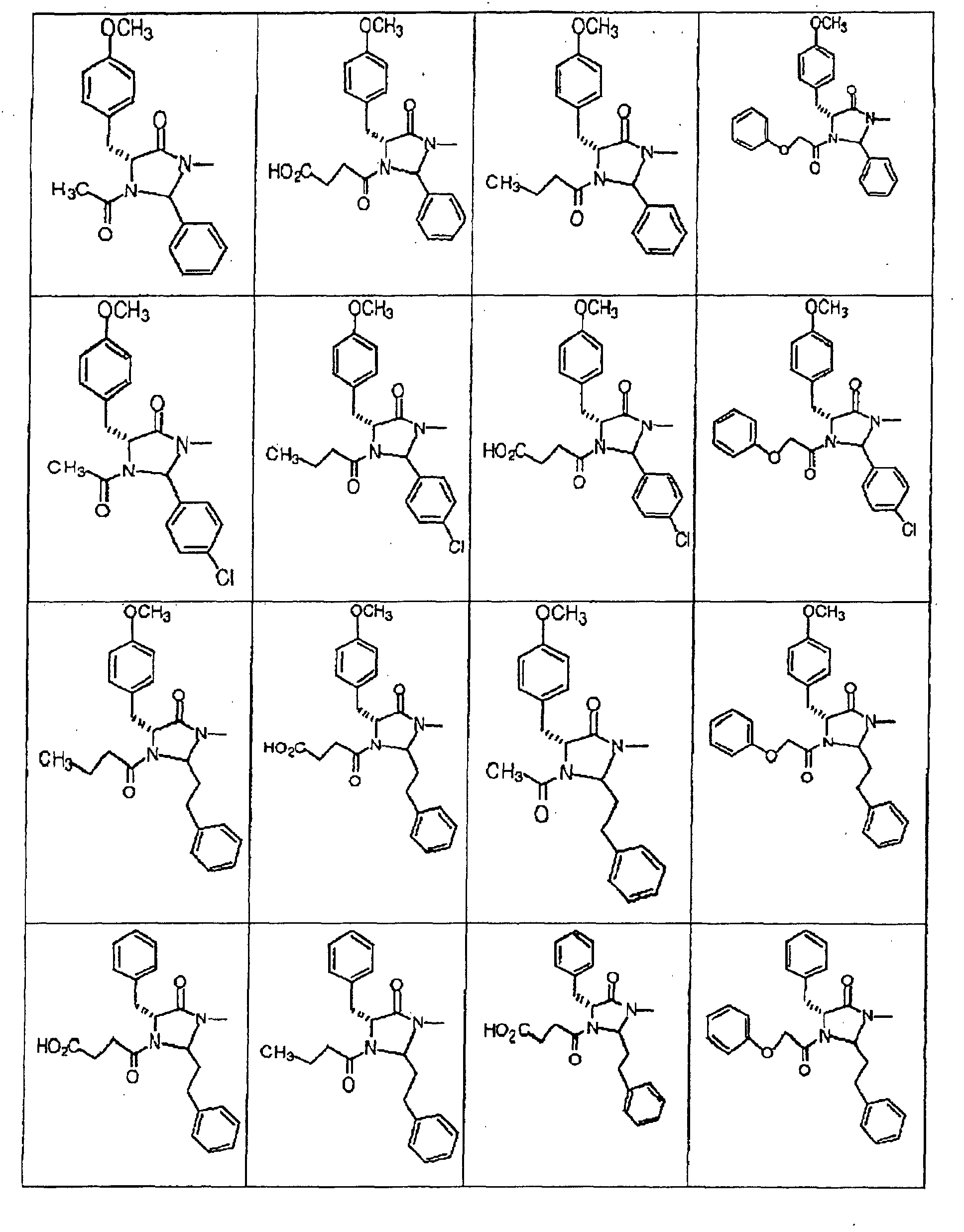

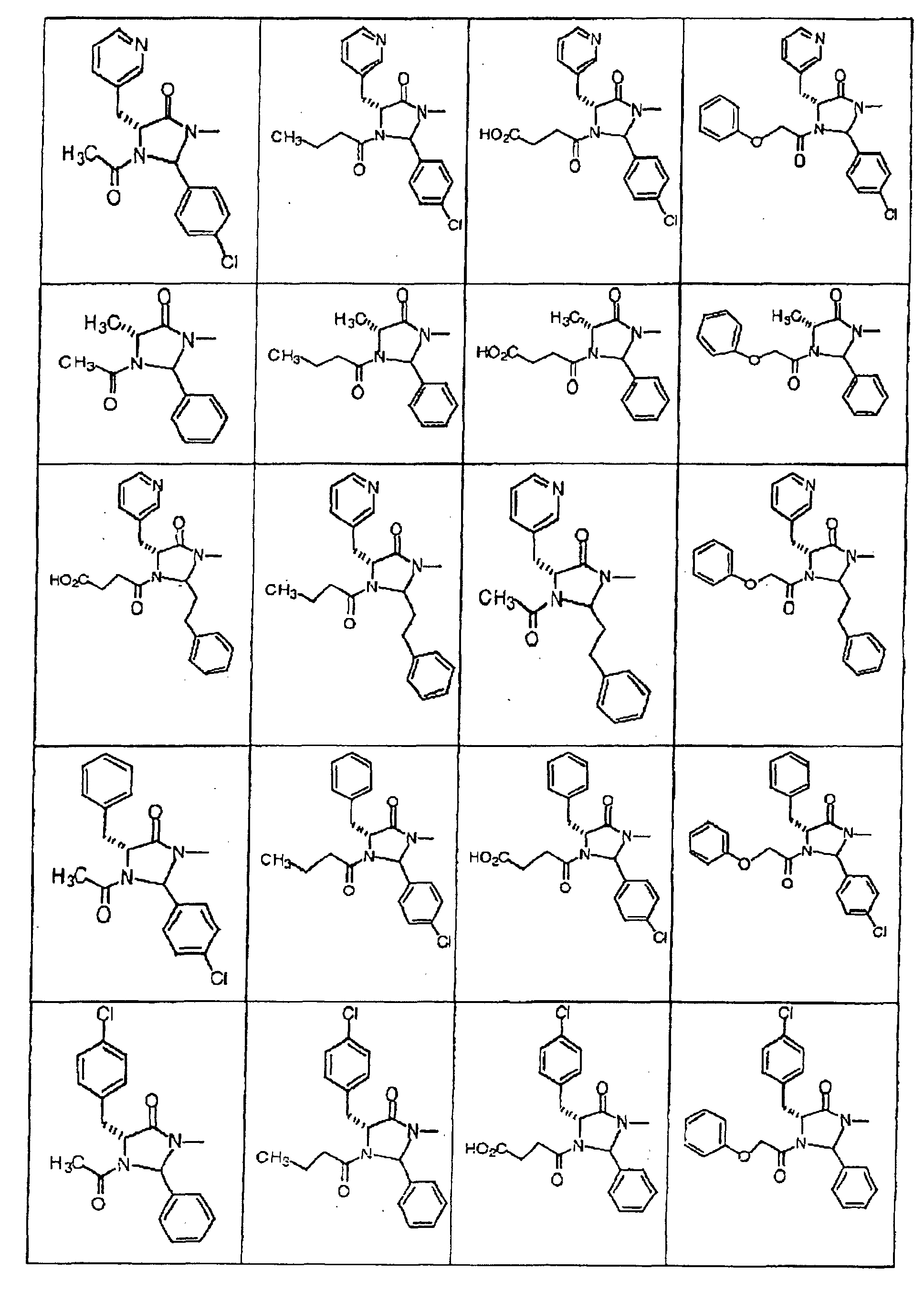

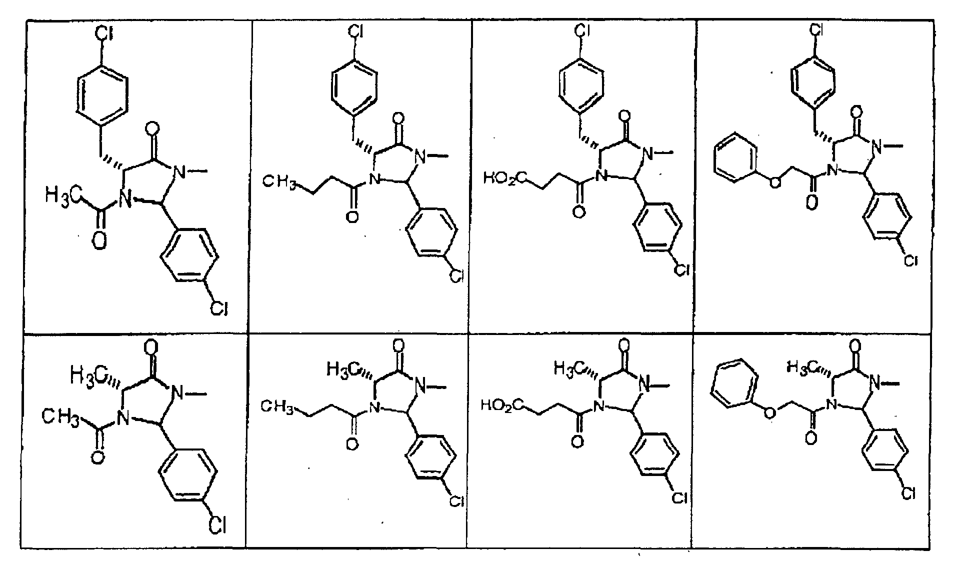

41. Verbindung nach Anspruch 1, ausgewählt aus der Gruppe, bestehend aus:
4-[[(2,6-Dichlorphenyl)carbonyl]amino]-N-[[1-[(4-methoxyphenyl)methyl]cyclopentyl]carbonyl]-L-phenylalanin,
N-[[1-[(4-Methoxyphenyl)methyl]cyclopentyl]carbonyl]-4-[[(2-nitrophenyl)carbonyl]amino]-L-phenylalanin,
N-[[1-[(4-Methoxyphenyl)methyl]cyclopentyl]carbonyl]-4-[[(2-methyl-5-nitrophenyl)carbonyl]amino]-L-phenylalanin,
N-[[1-[(4-Methoxyphenyl)methyl]cyclopentyl]carbonyl]-4-[(4-chinolinylcarbonyl)amino]-L-phenylalanin,
N-[[1-(Phenylmethyl)cyclopentyl]carbonyl]-4-[(4-chinolinylcarbonyl)amino]-L-phenylalanin,
4-[[(2,6-Dichlorphenyl)carbonyl]amino]-N-[[1-(phenylmethyl)cyclopentyl]carbonyl]-L-phenylalanin,
4-[[(2-Nitrophenyl)carbonyl]amino]-N-[[1-(phenylmethyl)cyclopentyl]carbonyl]-L-phenylalanin,
4-[[(2-Methyl-5-nitrophenyl)carbonyl]amino]-N-[[1-(phenylmethyl)cyclopentyl]carbonyl]-L-phenylalanin,
4-[[(2,6-Dichlorphenyl)carbonyl]amino]-N-[[1-[2-[[N-(1,1-dimethylethyl)carbonyl]amino]ethyl]cyclopentyl]carbonyl]-L-phenylalanin,
4-[[(2,6-Dichlorphenyl)carbonyl]amino]-N-[[1-[2-[(trifluoracetyl)amino]ethyl]cyclopentyl]carbonyl]-L-phenylalanin,
4-[[(2,6-Dichlorphenyl)carbonyl]amino]-N-[[1-[2-[(2-amino-1-oxoethyl)amino]ethyl]cyclopentyl]carbonyl]-L-phenylalanin,
4-[[(2,6-Dichlorphenyl)carbonyl]amino]-N-[[1-[2-[[2-[[(1,1-dimethylethoxy)carbonyl]amino]-1-oxoethyl]amino]-ethyl]cyclopentyl]carbonyl]-L-phenylalanin,
4-[[(2,6-Dichlorphenyl)carbonyl]amino]-N-[[2-[[(methoxy)carbonyl]amino]ethyl]cyclopentyl]carbonyl]-Lphenylalanin,
4-[[(2,6-Dichlorphenyl)carbonyl]amino]-N-[[1-[2-[(methylsulfonyl)amino]ethyl]cyclopentyl]carbonyl]-Lphenylalanin,
4-[[(2,6-Dichlorphenyl)carbony]amino]-N-[[1-[2-[(acetyl)(methyl)amino)ethyl]cyclopentyl]carbonyl]-L-phenylalanin,
4-[[(2,6-Dichlorphenyl)carbonyl]amino]-N-[[1-[2-[[(methylamino)carbonyl](methyl)amino]ethyl]cyclopentyl]carbonyl]-L-phenylalanin,
4-[[(2,6-Dichlorphenyl)carbonyl]amino]-N-[[1-[2-[(methoxycarbonyl)(methyl)amino]ethyl]cyclopentyl]carbonyl]-L-phenylalanin,
4-[[(2,6-Dichlorphenyl)carbonyl]amino]-N-[[1-(2-methoxyethyl)cyclopentyl]carbonyl]-L-phenylalanin,
4-[[(2,6-Dichlorphenyl)carbonyl]aminol-N-[[1-[(2-methylsulfonyl)ethyl]cyclopentyl)carbonyl]-L-phenylalanin,
4-[[(2,6-Dichlorphenyl)carbonyl]amino]-N-[[1-[(2-(methylsulfinyl)ethyl]cyclopentyl]carbonyl]-L-phenylalanin,
4-[[(2,6-Dimethyl-4-trifluormethyl-3-pyridinyl)carbonyl]amino]-N-[[1-[4-(methylsulfonyl)butyl]cyclopentyl]-carbonyl]-L-phenylalanin,
4-[[(2,4-Dimethylpyridin-3-yl)carbonyl]amino]-N-[[1-[4-(methylsulfonyl)butyl]cyclopentyl]carbonyl]-L-phenylalanin oder
4-[[(2,6-Dichlorphenyl)carbonyl]amino]-N-[[1-(4-methoxyphenylmethyl)cyclohexyl]carbonyl]-L-phenylalanin.

42. Verbindung nach Anspruch 1 mit der Formel, ausgewählt aus der Gruppe, bestehend aus

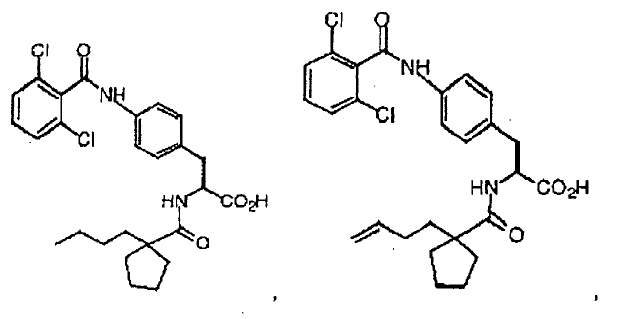

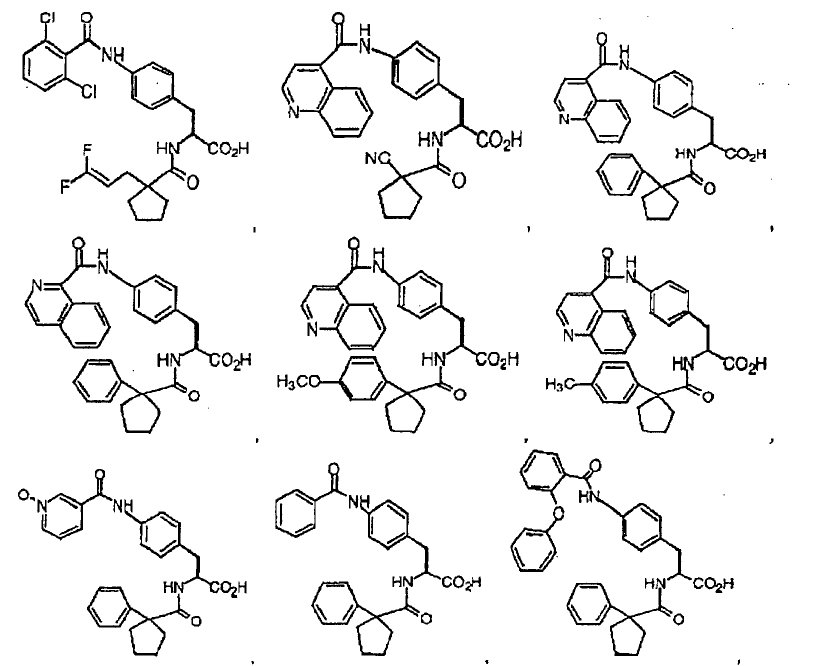

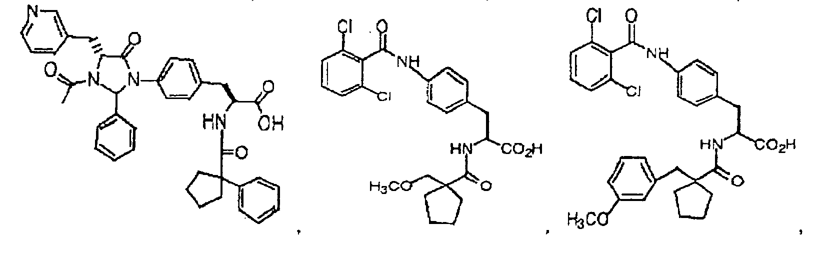

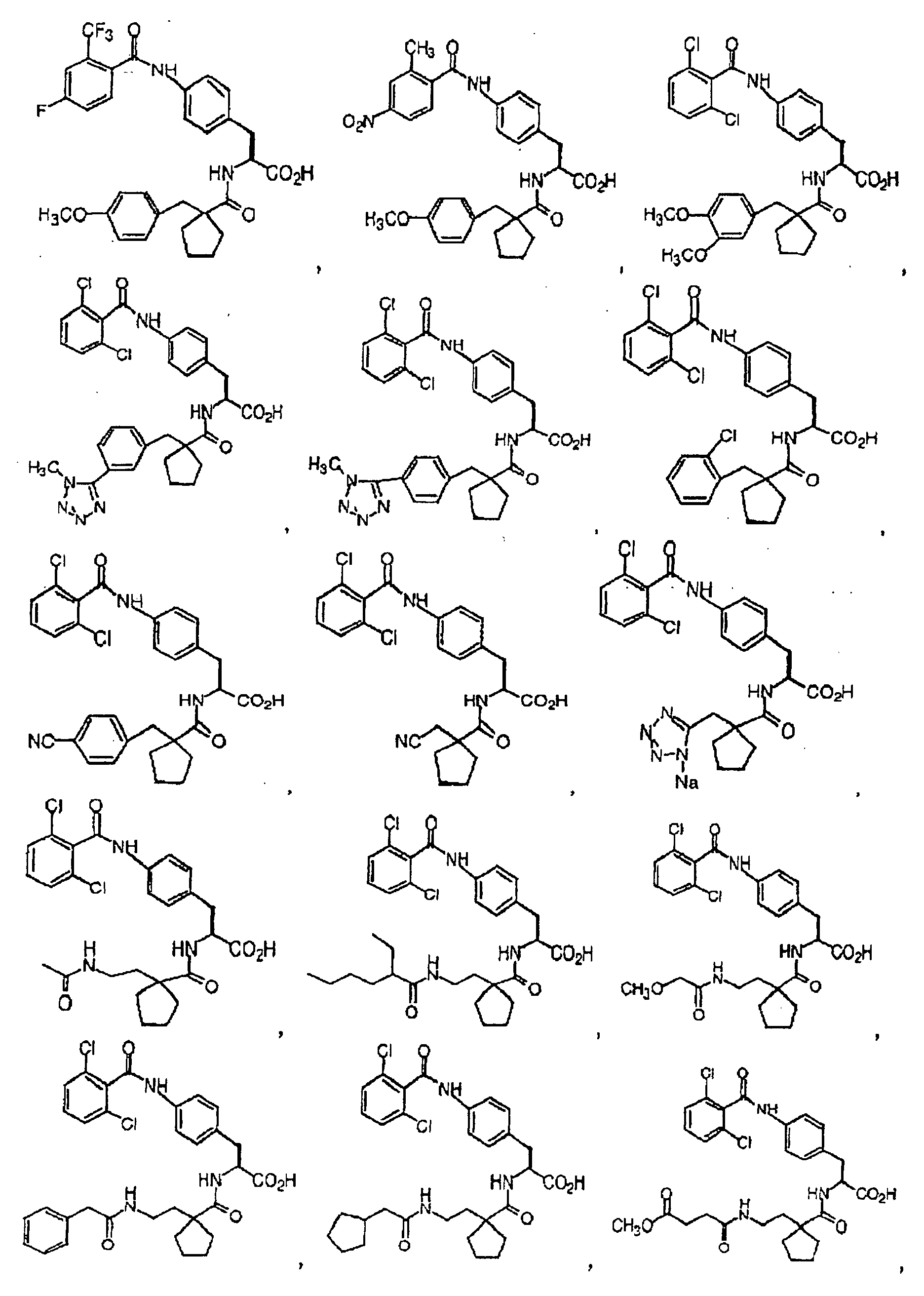

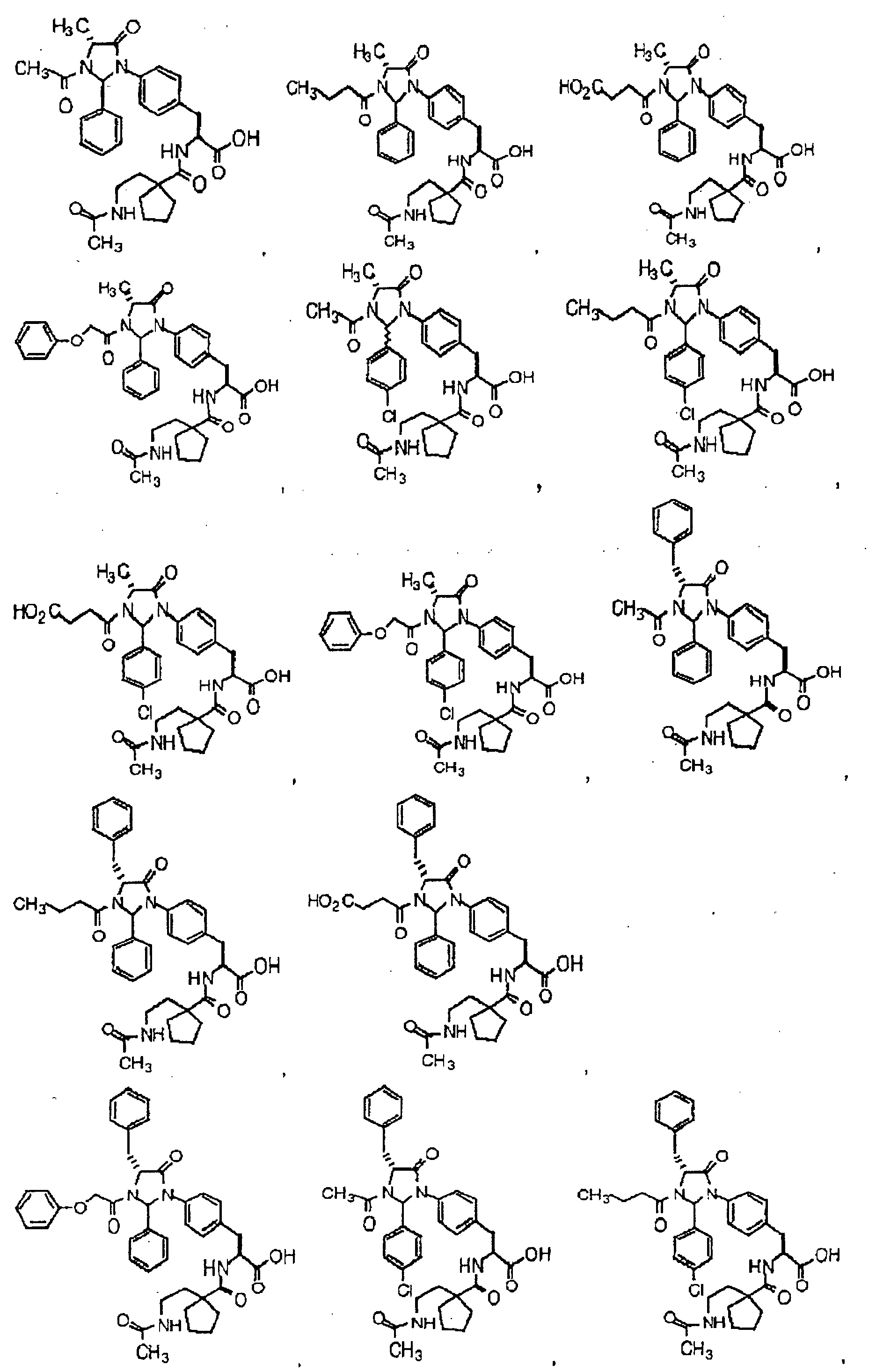

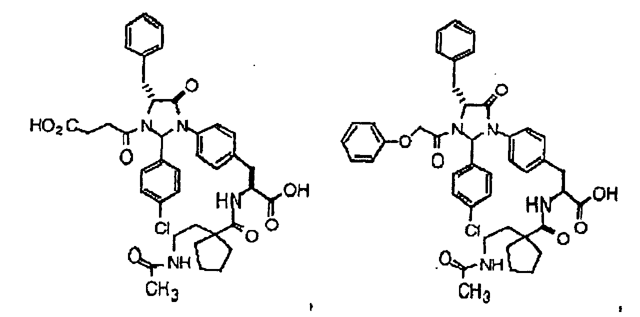

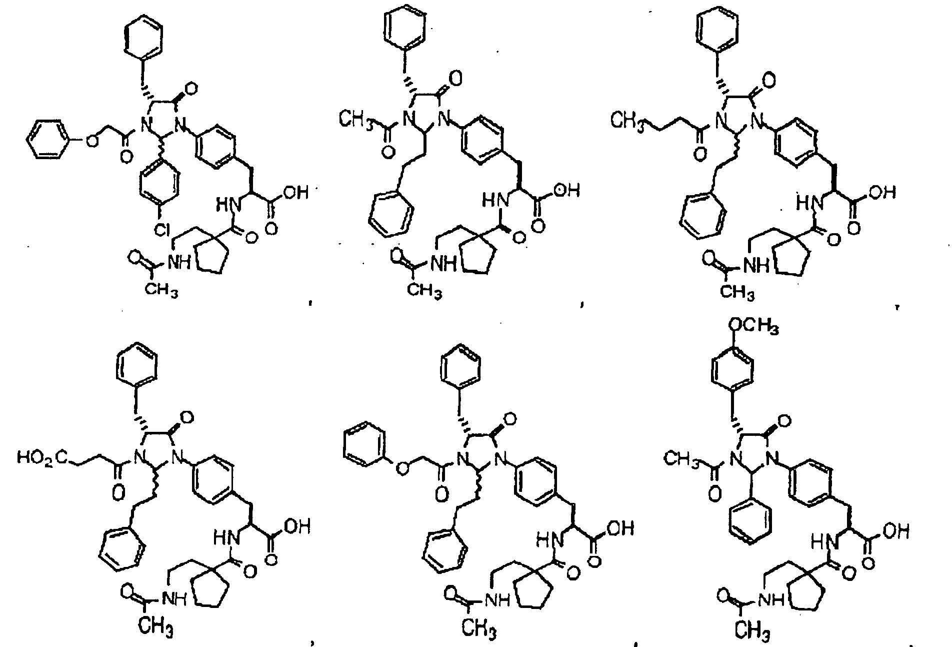

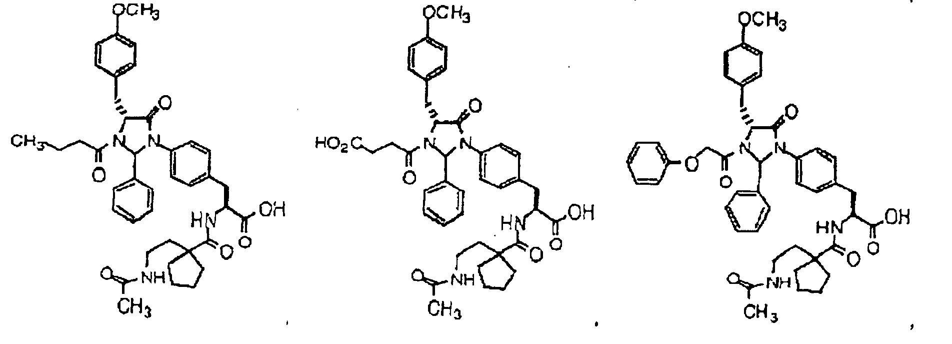

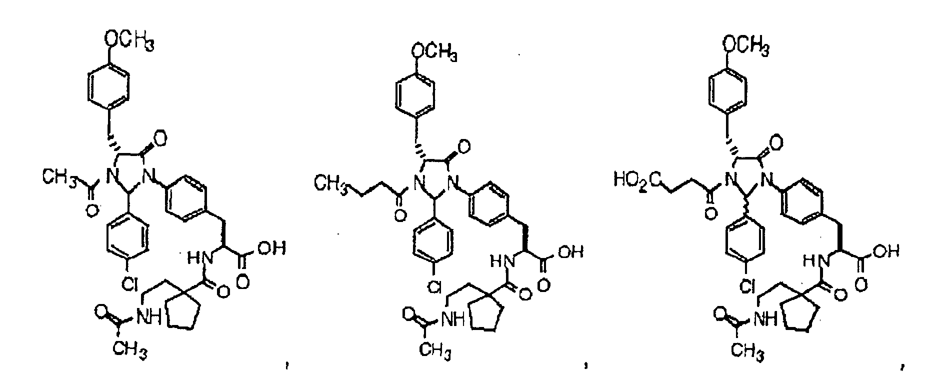

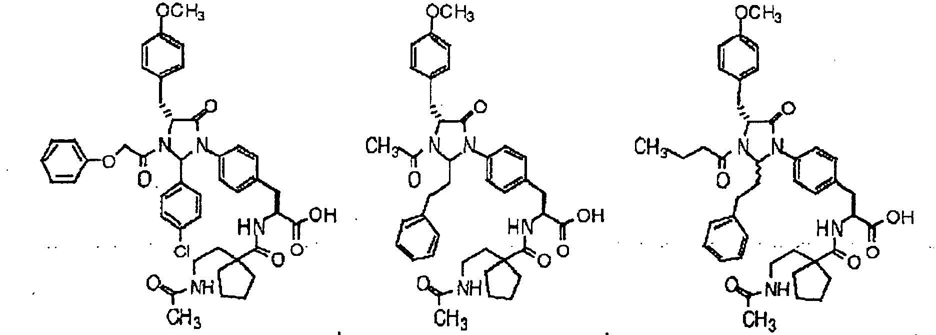

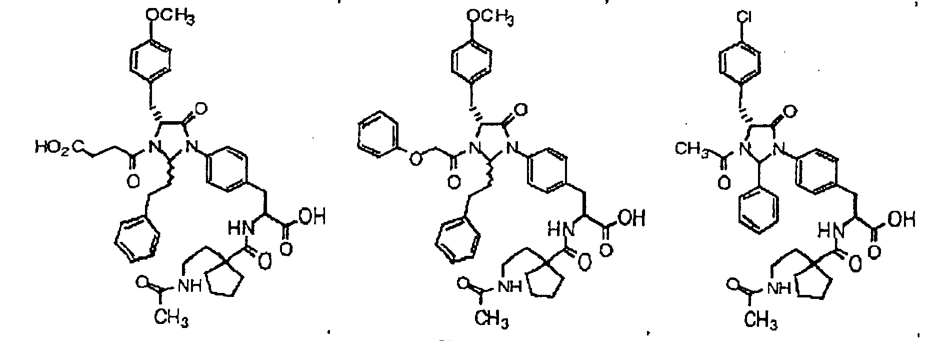

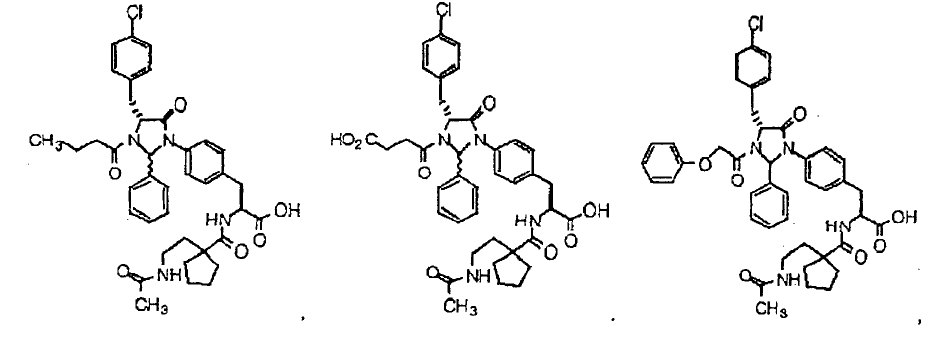

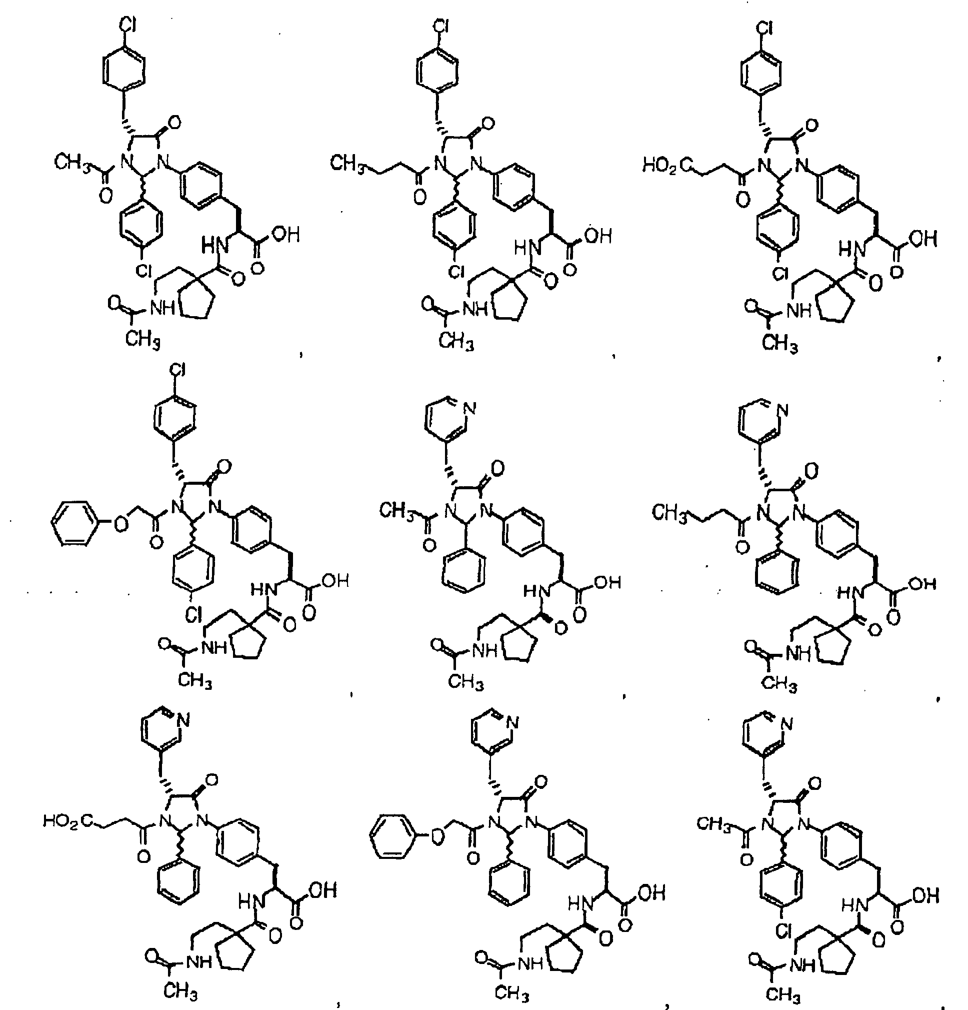

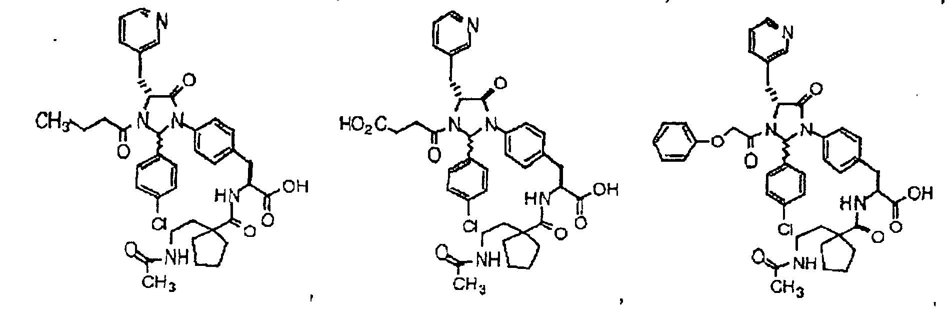

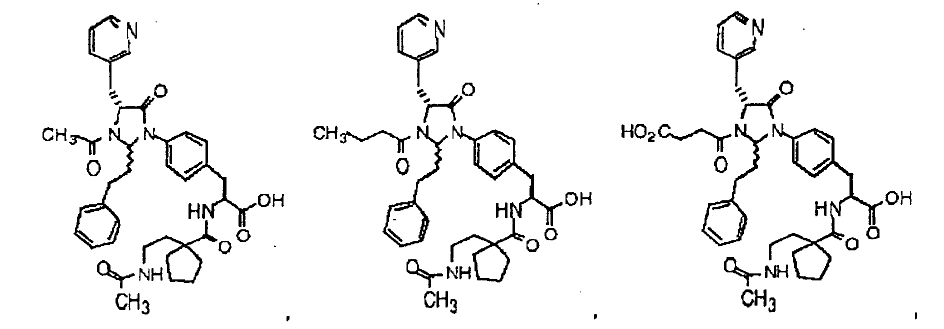

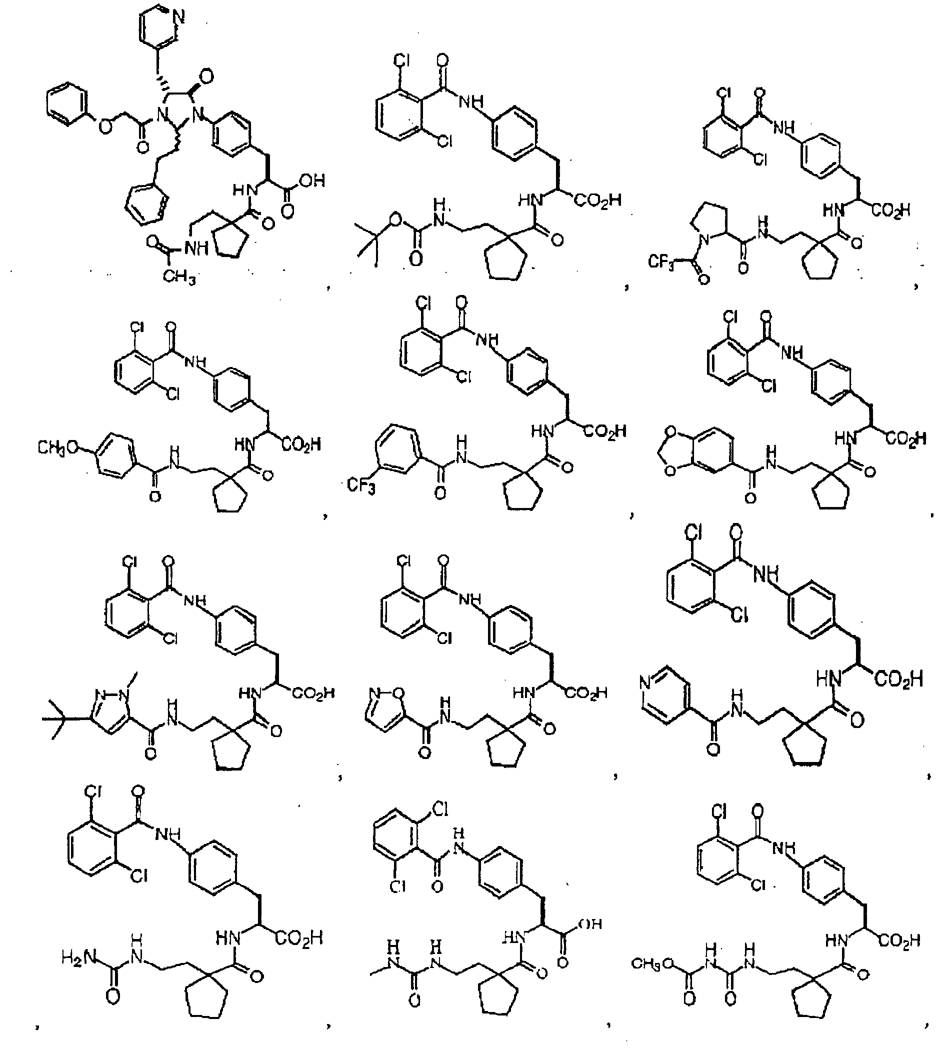

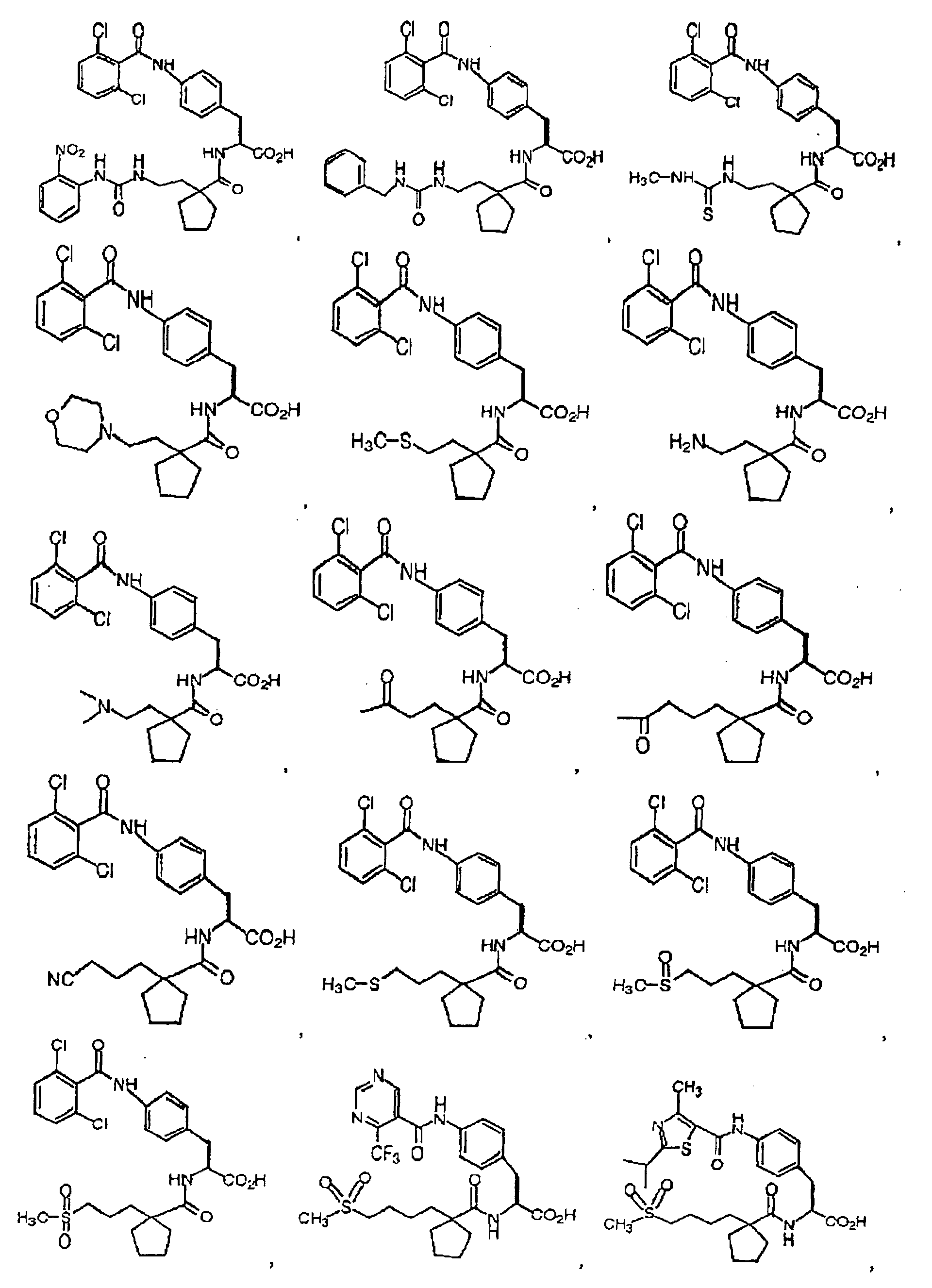

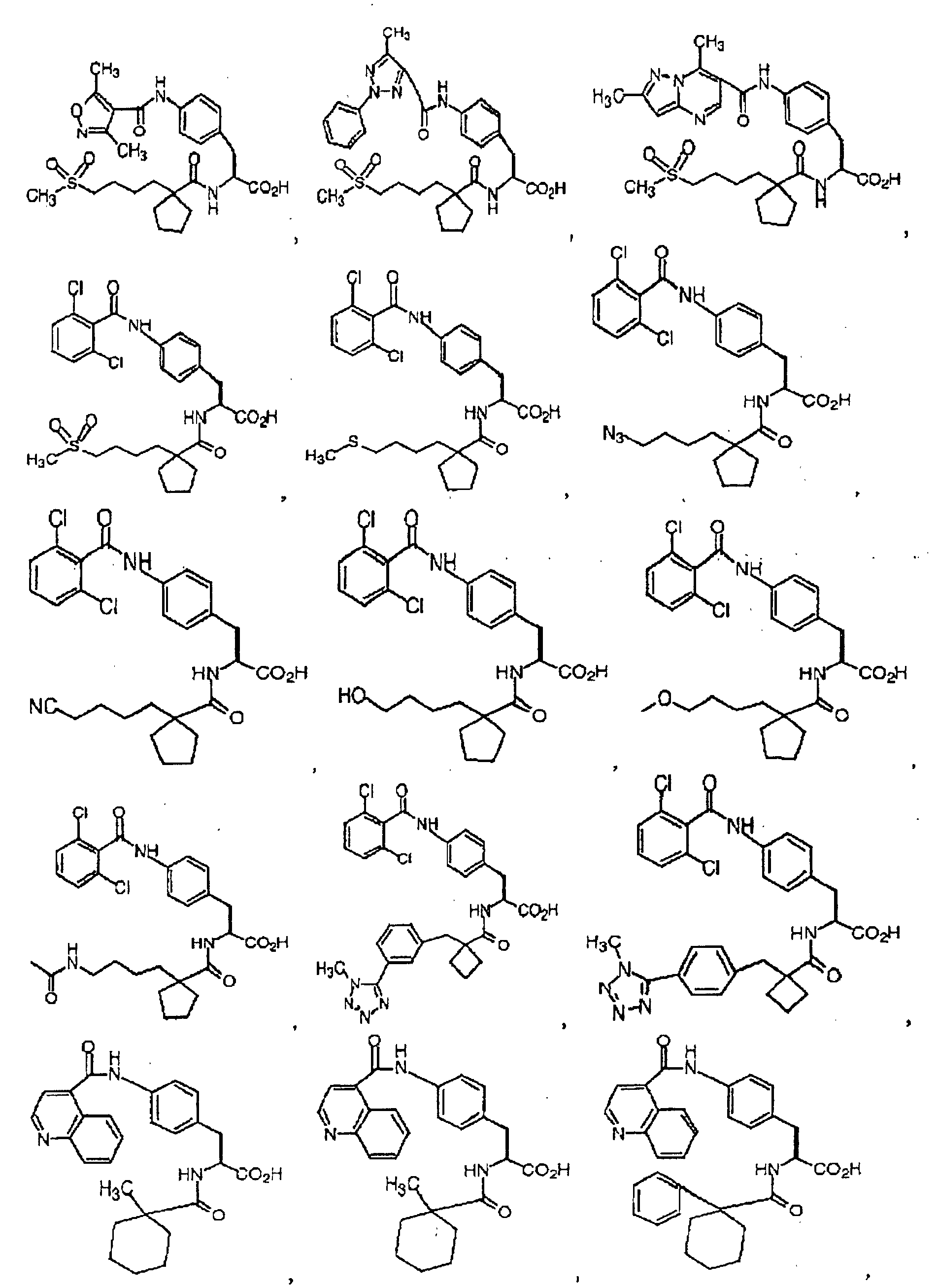

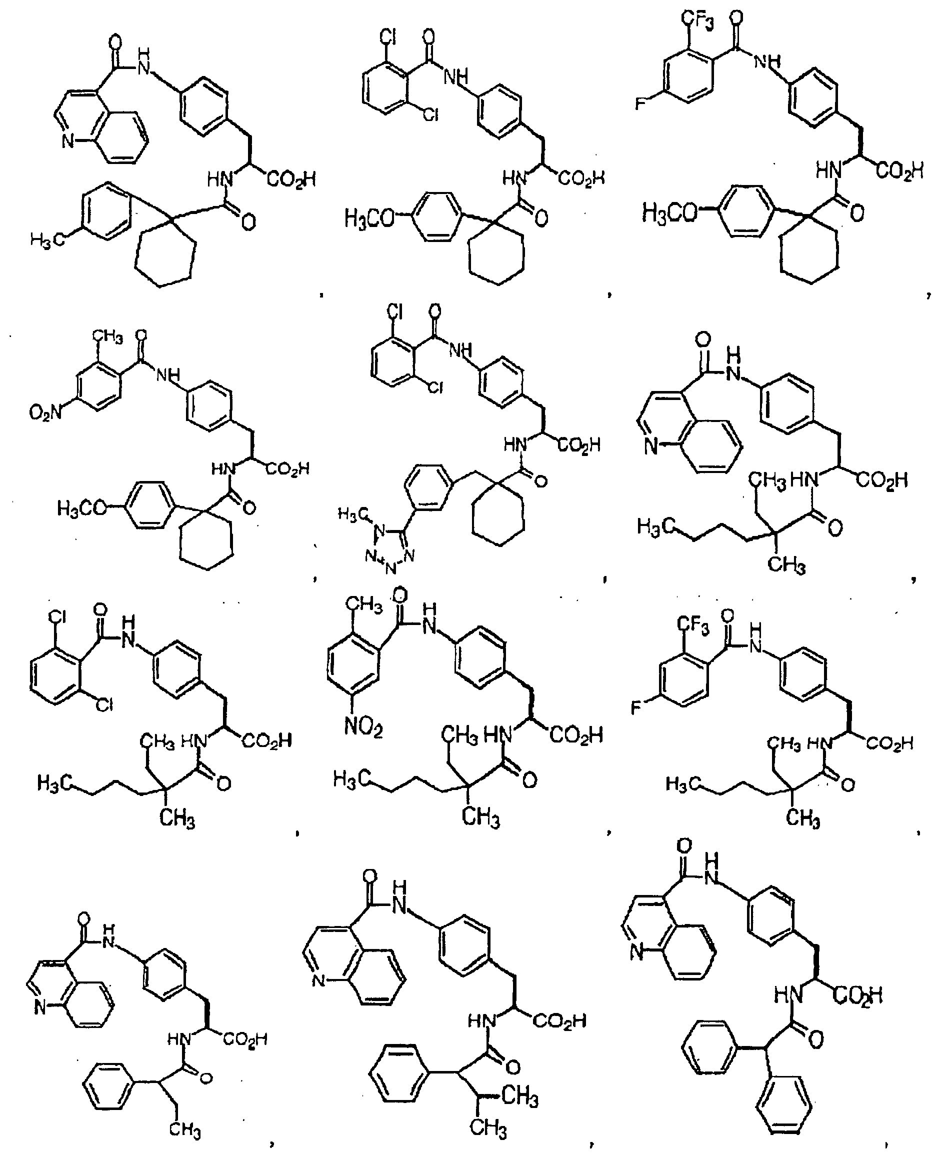

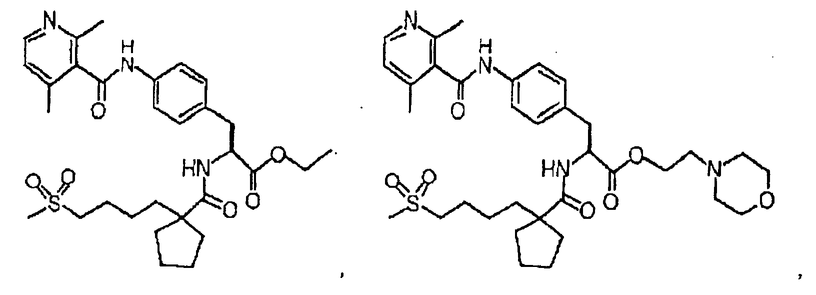

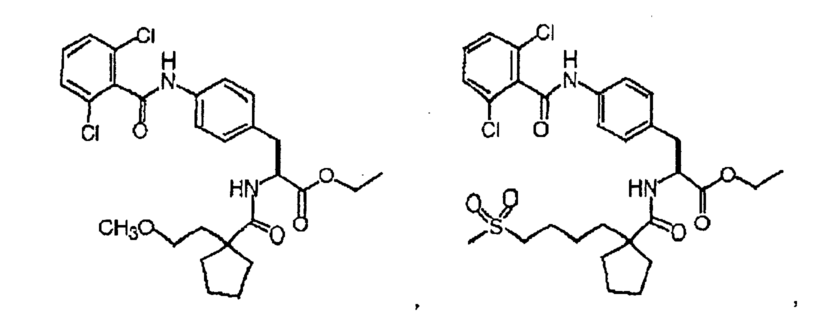

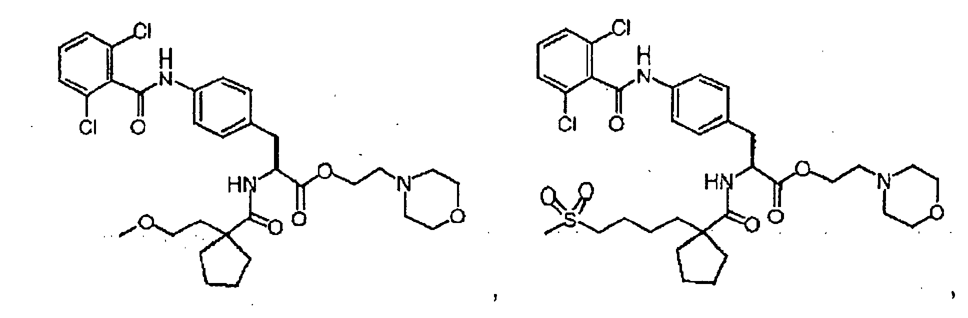

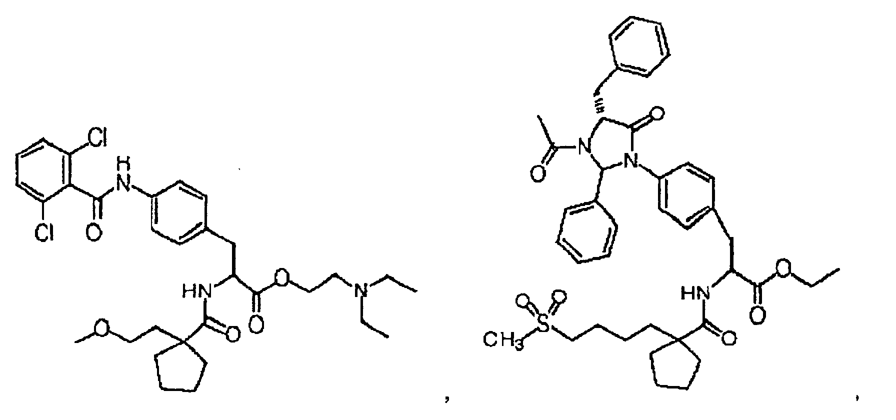

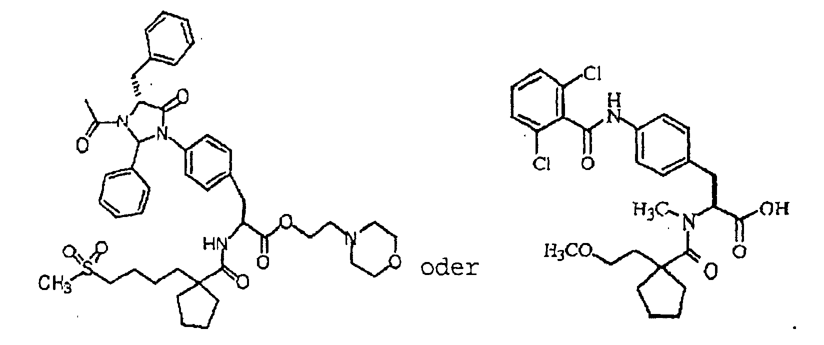

43. Verfahren zur Herstellung einer Verbindung der Formel 1 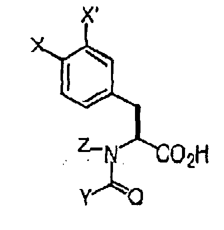 1' worin X, X', Z und Y wie in Anspruch 1 definiert sind,
**dadurch gekennzeichnet, dass** in einer Verbindung der Formel 1b 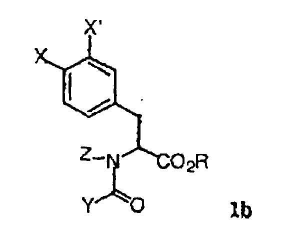 worin X, X', Z und Y wie definiert sind und R eine Schutzgruppe oder einen festen Träger darstellt,
die schutzgruppe oder der feste Träger abgespalten wird und, falls erwünscht, Umwandeln einer Verbindung der Formel 1 in ein pharmazeutisch verträgliches Salz.

44. Verbindungen nach einem der Ansprüche 1-42 zur Verwendung als ein Arzneimittel.

45. Verbindungen nach Anspruch 44 zur Verwendung als ein Arzneimittel bei der Behandlung von rheumatischer Arthritis, multipler Sklerose, entzündlicher Darmkrankheit oder Asthma.

46. Arzneimittel, enthaltend eine Verbindung nach einem der Ansprüche 1-42 und ein therapeutisch inertes Trägermaterial.

47. Arzneimittel nach Anspruch 46 für die Behandlung von rheumatischer Arthritis, multipler Sklerose, entzündlicher Darmkrankheit oder Asthma.

48. Verfahren zur Herstellung eines Arzneimittels, insbesondere für die Behandlung von rheumatischer Arthritis, multipler Sklerose, entzündlicher Darmkrankheit oder Asthma, wobei das Verfahren Bringen einer Verbindung nach einem der Ansprüche 1-42 zusammen mit einem therapeutisch inerten Trägermaterial und, falls erwünscht, einem oder mehreren zusätzlichen therapeutischen Wirkstoffen in eine galenische Verabreichungsform umfasst.

49. Verwendung einer Verbindung nach einem der Ansprüche 1-42 bei der Herstellung eines Arzneimittels für die Behandlung von rheumatischer Arthritis, multipler Sklerose, entzündlicher Darmkrankheit oder Asthma.

## Revendications

1. Composé de formule : 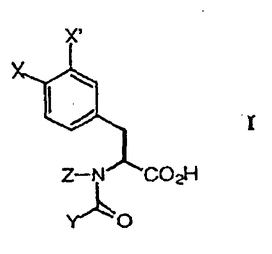 Dans laquelle :
X' est H, halogène ou alkyle en C₁₋₆ et X est un groupe de formule : 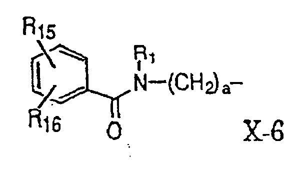
Dans laquelle :
R₁ est hydrogène ou alkyle en C₁₋₆,
R₁₅ est hydrogène, halogène, nitro, alkyle sulfonyle en C₁₋₆, cyano, alkyle en C₁₋₆, alkoxy en C₁₋₆, alkoxycarbonyle en C₁₋₆, carboxy, alkyle aminosulfonyle en C₁₋₆, perfluoroalkyle en C₁₋₆, alkylthio en C₁₋₆, hydroxy alkyle en C₁₋₆, alkoxy alkyle en C₁₋₆, alkylthio alkyle en C₁₋₆, alkylsulfinyle alkyle en C₁₋₆, alkylsulfonyle alkyle en C₁₋₆, alkylsulfinyle en C₁₋₆, alkylcarbonyle en C₁₋₆, aryloxy, aroyle, aryle ou un groupe de formule R₁₇-C≡C,
R₁₆ est H, halogène, nitro, cyano, alkyle en C₁₋₆, OH, perfluoro alkyle en C₁₋₆ ou alkylthio en C₁₋₆ et
R₁₇ est H, aryle, hétéroaryle ou alkyle en C₁₋₆ qui est substitué ou non substitué par OH, aryle ou hétéroaryle et
a est 0 ou 1 ;
ou X est un groupe de formule : 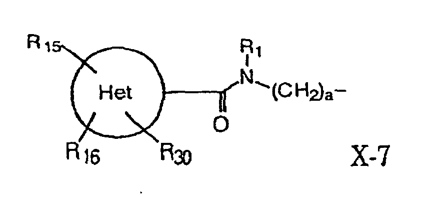
dans laquelle 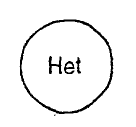 est un cycle hétéroaromatique à 5 ou 6 éléments contenant 1, 2 ou 3 hétéroatomes choisis parmi N, O et S ; ou 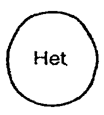 est un cycle hétéroaromatique bicyclique à 9 ou 10 éléments contenant 1, 2, 3 ou 4 hétéroatomes choisi parmi O, S et N, a, R₁, R₁₅ et R₁₆ sont comme définis dans X-6 et R₃₀ est hydrogène ou alkyle en C₁₋₆ ou est absent;
ou X est un groupe de formule : 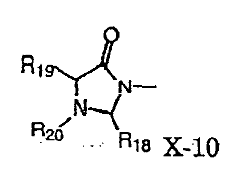 où :
R₁₈ est hydrogène, alkyle en C₁₋₆ substitué ou non substitué, aryle, hétéroaryle, aryle-alkyle ou hétéroaryle alkyle,
R₁₉ est alkyle en C₁₋₆ non substitué ou substitué, aryle, hétéroaryle, arylalkyle ou hétéroarylalkyle et
R₂₀ est alkyle en C₁₋₆ non substitué ou substitué, alkylcarbonyle en C₁₋₆ facultativement substitué par carboxyle, aroyle ou aryloxy;
Y est un groupe de formule : 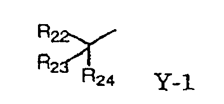
dans laquelle :
R₂₂ et R₂₃ sont indépendamment aryle, hétéroaryle ou alkyle en C₁₋₆ qui est non substitué ou substitué par un ou plusieurs parmi chloro, bromo, nitro, hydroxy, alkoxy en C₁₋₆, aryle, alkylcarbonyle en C₁₋₆, aroyle ou cyano,
R₂₄ est aryle, cyano, alkylsulfonyle ou alkyle en C₁₋₆ ou alkényle jusqu'à C₆ non substitué ou substitué par un cycle aryle ou hétéroaryle, et lorsque R₂₂ est aryle et R₂₃ est aryle ou alkyle en C₁₋₆, R₂₄ est H, et le nombre total d'atomes de carbone dans R₂₂, R₂₃ et R₂₄ est de 6 à 14 ou
Y est un cycle à 3 à 7 éléments de formule : 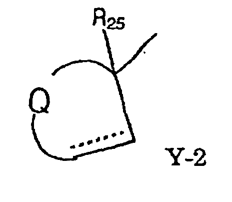
dans laquelle :
R₂₅ est alkyle en C₁₋₆, alkényle jusqu'à C₆ non substitué ou substitué avec fluorure, ou un groupe de formule R₂₆ - (CH₂)ₑ₋,
R₂₆ est aryle, hétéroaryle, azido, cyano, hydroxy, alkoxy en C₁₋₆, alkoxycarbonyle en C₁₋₆, alkylcarbonyle en C₁₋₆, alkylthio en C₁₋₆, alkylsulfonyle en C₁₋₆, alkylsulfinyle en C₁₋₆, perfluoro alkylcarbonyle en C₁₋₆, nitro ou R₂₆ est un groupe de formule NR₂₈R₂₉, dans laquelle
R₂₈ est H ou alkyle en C₁₋₆,
R₂₉ est hydrogène, alkyle en C₁₋₆, alkoxycarbonyle en C₁₋₆, aminocarbonyle facultativement substitué, alkylcarbonyle en C₁₋₆ facultativement substitué, aroyle, hétéroaroyle, hétérocycloalkylcarbonyle, alkylsulfonyle en C₁₋₆, alkylaminothiocarbonyle en C₁₋₆ ou
R₂₈ et R₂₉ pris ensemble avec l'atome d'azote auquel ils sont reliés forment un cycle hétérocyclique saturé à 4, 5 ou 6 éléments contenant un ou deux hétéroatomes avec le second hétéroatome étant 0, S ou N-R₂₇ ;
Q est -(CH₂)_{f}-O, -(CH₂)_{f}-S, -(CH₂)_{f} ou lorsque f=0, une liaison,
R₂₇ est H, alkyle en C₁₋₆, aryle, alkylcarbonyle en C₁₋₆, aroyle ou alkoxycarbonyle en C₁₋₆, les atomes de carbone dans ledit cycle sont non substitués ou substitués par alkyle en C₁₋₆ ou halogène,
e est un nombre entier compris entre 0 et 4,
f est un nombre entier compris entre 0 et 3,
et la ligne en pointillés représente une liaison qui peut être absente ou présente ; et
Z est hydrogène ou alkyle en C₁₋₆ ;
et ses sels et esters pharmaceutiquement acceptables
le terme « alkyle en C₁₋₆ substitué », s'il n'est pas défini autrement, désigne un groupe alkyle en C₁₋₆ substitué par un ou plusieurs groupes choisis indépendamment parmi cycloalkyle, nitro, aryloxy, aryle, hydroxy, halogène, cyano, alkoxy en C₁₋₆, alkylcarbonyle en C₁₋₆, alkylthio en C₁₋₆, alkylesulfinyle en C₁₋₆, alkylsulfonyle en C₁₋₆ et amino substitué ;
le terme « aryle » désigne un groupe aromatique monocyclique ou bicyclique qui est non substitué ou substitué par alkyle en C₁₋₆, alkoxy en C₁₋₆, hydroxy alkyle en C₁₋₆, hydroxy, hydroxyalkoxy, halogène, alkylthio en C₁₋₆, alkylsulfinyle en C₁₋₆, alkylsulfonyle en C₁₋₆, cyano, nitro, perfluoro-alkyle, alkanoyle, aroyle, arylalkynyle, hétéroaryl, alkynyle inférieur et alkylcarbonylamino en C₁₋₆ ;
le terme « aryle alkyle en C₁₋₆ » désigne un groupe alkyle en C₁₋₆ dans lequel un ou plusieurs atomes d'hydrogène est/sont remplacés par un groupe aryle ou hétéroaryle ;
le terme « hétéroaryl » désigne un cycle hétéroaromatique monocyclique à 5 ou 6 éléments non substitué ou substitué ou un cycle hétéroaromatique bicyclique à 9 ou 10 éléments contenant 1, 2, 3 ou 4 hétéroatomes qui sont indépendamment N, S ou O et qui peuvent être substitués par des substituants comme définis ci-dessus pour « aryle » ;
le terme « alkylcarbonyle en C₁₋₆ facultativement substitué » désigne des groupes alkyle en C₁₋₆ liés par un groupe carbonyle, ledit groupe alkyle pouvant être substitué par un ou plusieurs groupes choisis parmi hydroxycarbonyle, aroyle ou aryloxy, alkoxy en C₁₋₆, fluoro, phényle, cycloalkyle, alkoxycarbonyle en C₁₋₆, amino ou alkoxycarbonylamino en C₁₋₆;
le terme « aroyle » désigne un groupe aryle ou hétéroaryle monocyclique ou bicyclique comme défini ci-dessus, lié via un groupe carbonyle ;
le terme « cycloalkyle » désigne un cycle carbocyclique à 3 à 7 éléments non substitué ou substitué qui peut être substitué par hydroxy, halogène, cyano, alkoxy en C₁₋₆, alkylcarbonyle en C₁₋₆, alkyle en C₁₋₆, aroyle, alkylthio en C₁₋₆, alkylsulfinyle en C₁₋₆, alkylsulfonyle en C₁₋₆, aryle, hétéroaryle et amino substitué
le terme « hétérocycloalkyle » désigne un cycle carbocyclique à 5 à 6 éléments non substitué ou substitué dans lequel un ou deux des atomes de carbone a été remplacé par des hétéroatomes choisis indépendamment parmi 0, S et N.

2. Composé selon la revendication 1, dans lequel X' est hydrogène.

3. Composé selon la revendication 1 ou la revendication 2, dans lequel Z est hydrogène.

4. Composé selon l'une quelconque des revendications 1 à 3, dans lequel dans Y-1, R₂₂ et R₂₃ sont alkyle en C₁₋₆ ou phényle et R₂₄ est alkyle en C₁₋₆ sauf lorsque R₂₂ est aryle et R₂₃ est aryle ou alkyle en C₁₋₆, auquel cas R₂₄ est hydrogène.

5. Composé selon l'une quelconque des revendications 1 à 4, dans laquelle Y-1 est choisi dans le groupe comprenant :

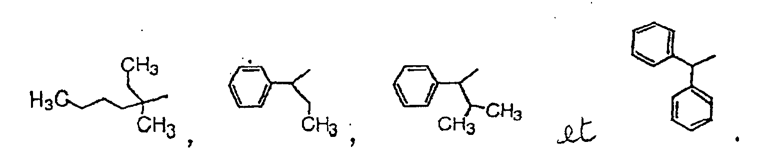

6. Composé selon l'une quelconque des revendications 1 à 3, dans lequel dans Y-2 Q est (CH₂)_{f}- ou, lorsque f=0, une liaison.

7. Composé selon la revendication 6, dans lequel f est 1, 2 ou 3.

8. Composé selon l'une quelconque des revendications 1 à 3, dans lequel dans Y-2 R₂₆ est aryle, hétéroaryle, azido, cyano, hydroxy, alkoxy en C₁₋₆, alkoxycarbonyle en C₁₋₆, alkylcarbonyle en C₁₋₆, alkylthio en C₁₋₆, alkylsulfonyle en C₁₋₆, alkylsulfinyle en C₁₋₆, nitro ou R₂₆ est un groupe de formule -NR₂₈R₂₉ dans laquelle R₂₈ est H ou alkyle en C₁₋₆, R₂₉ est hydrogène, alkyle en C₁₋₆, alkoxycarbonyle en C₁₋₆, aminocarbonyle facultativement substitué, alkylcarbonyle en C₁₋₆ facultativement substitué, aroyle, alkylsulfonyle en C₁₋₆ ou R₂₈ et R₂₉ pris ensemble avec l'azote auquel ils sont reliés forment un cycle hétérocyclique saturé à 4, 5 ou 6 éléments qui peut contenir un atome d'oxygène.

9. Composé selon l'une quelconque des revendications 1 à 3, dans lequel R₂₈ est H.

10. Composé selon la revendication 8 ou la revendication 9, dans lequel R₂₆ est aryle non substitué ou mono-substitué par halogène, alkoxy en C₁₋₆, alkyle en C₁₋₆, cyano ou tétrazolyle qui est non substitué ou substitué par méthyle ou R₂₆ est phényle disubstitué par alkoxy en C₁₋₆.

11. Composé selon la revendication 10, dans lequel R₂₆ est phényle non substitué ou monosubstitué par chloro, méthoxy ou méthyle.

12. Composé selon la revendication 8, dans lequel R₂₆ est CH₃S(O)₂-, CH₃S-, CH₃SO-, CH₃O-, CH₃CO-, NC-, N₃- ou HO-.

13. Composé selon la revendication 8, dans lequel le groupe alkyle dans le groupe alkylcarbonyle en C₁₋₆ de R₂₉ est non substitué ou substitué par alkoxy en C₁₋₆, fluoro, phényle, cycloalkyle, alkoxycarbonyle en C₁₋₆, amino ou alkoxycarbonyle amino en C₁₋₆.

14. Composé selon la revendication 13, dans lequel le groupe alkylcarbonyle en C₁₋₆ non substitué ou substitué est CH₃CO-, (CH₃)₃CCO-, CH₃(CH₂)₃CHCH₃CO-, CH₃OCH₂CO-, CF₃CO-, C₆H₅CH₂CO-, CH₃OCO(CH₂)₂CO-, cyclopentylCH₂CO-, H₂NCH₂CO ou (CH₃)₃COCONH (CH₂)₂CO-.

15. Composé selon la revendication 8, dans lequel dans R₂₉ le groupe aminocarbonyle est non substitué ou substitué par alkyle en C₁₋₆, alkoxycarbonyle en C₁₋₆, aryle monocyclique ou benzyle.

16. Composé selon la revendication 15, dans lequel le groupe aminocarbonyle non substitué ou substitué est H₂NCO-, CH₃NHCO-, CH₃OCONHCO-, C₆H₄NO₂NHCO- ou C₆H₅CH₂NHCO-.

17. Composé selon la revendication 1, dans lequel R₂₉ est (CH₃)₃COCO-, méthylaminothiocarbonyle, 4-, méthoxyphénylcarbonyle, 3-trifluorméthylphénylcarbonyle, 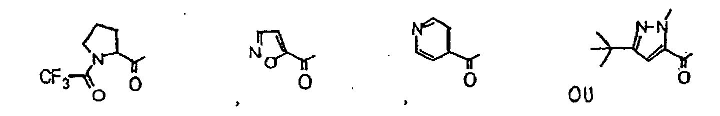 -NR₂₈R₂₉ est -NH₂ ou -N(CH₃)₂ et R₂₈ et R₂₉ pris ensemble est 4-morpholinyle.

18. Composé selon l'une quelconque des revendications 1 à 3, dans lequel Y-2 est choisi dans un groupe représenté par l'une des formules suivantes :

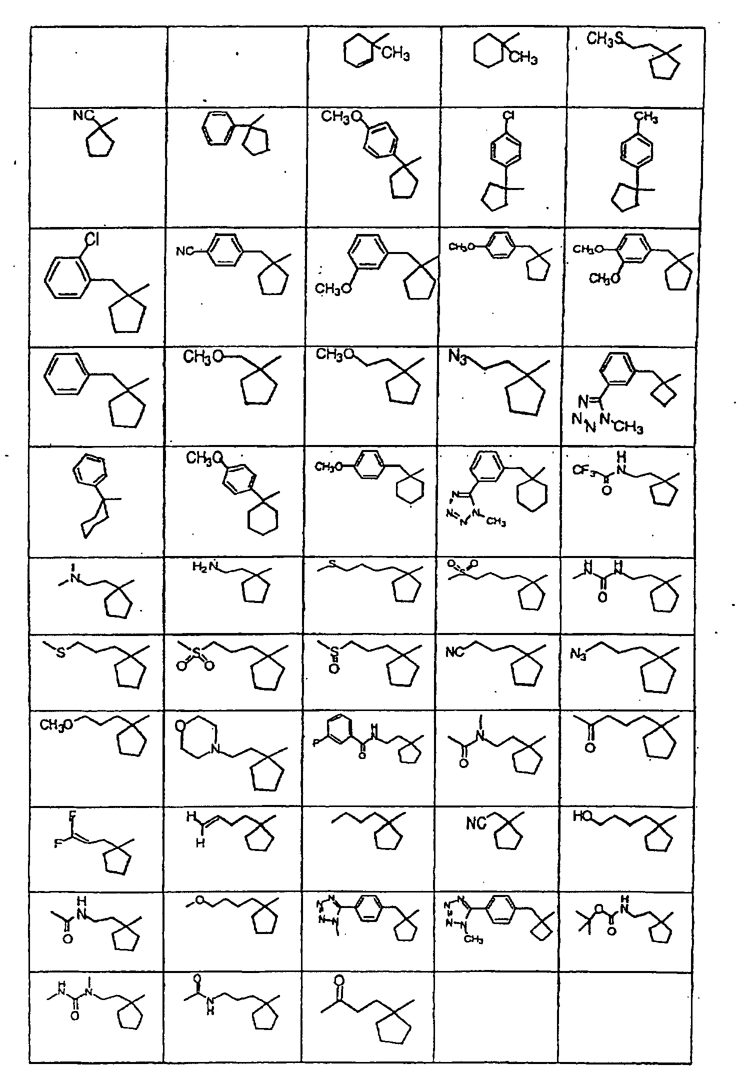

19. Composé selon l'une quelconque des revendications 1 à 3, dans lequel dans X-6 R₁₅ et R₁₆ sont indépendamment H, halogène, nitro, perfluoro alkyle en C₁₋₆, alkyle en C₁₋₆, cyano ou R₁₅ est phénoxy et R₁₆ est H.

20. Composé selon la revendication 19, dans lequel dans X-6 les groupes R₁₅ et R₁₆ sont indépendamment hydrogène, méthyle, nitro, chloro, trifluorométhyle ou cyano.

21. Composé selon l'une quelconque des revendications 1 à 3, dans lequel dans X-6 R₁ est hydrogène.

22. Composé selon l'une quelconque des revendications 1 à 3, dans lequel a dans X-6 est 0.

23. Composé selon l'une quelconque des revendications 1 à 3, dans lequel X-6 est choisi dans le groupe comprenant :

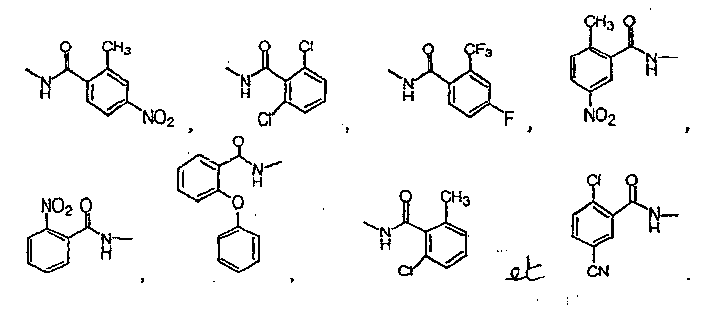

24. Composé selon l'une quelconque des revendications 1 à 3, dans lequel dans X-7 Het est un cycle hétéroaromatique monocyclique à 5 ou 6 éléments contenant 1, 2 ou 3 azotes, ou un azote et un souffre, ou un azote et un oxygène.

25. Composé selon la revendication 24, dans lequel le cycle hétéroaromatique seul est

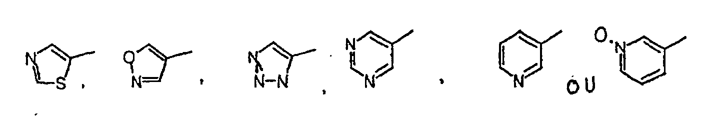

26. Composé selon l'une quelconque des revendications 1 à 3, dans lequel dans X-7 Het est un cycle hétéroaromatique bicyclique contenant 1 à 3 azotes comme hétéroatomes.

27. Composé selon la revendication 26, dans lequel le cycle hétéroaromatique bicyclique est 4-quinolinyle,
1-isoquinolinyl ou

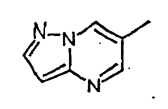

28. Composé selon l'une quelconque des revendications 1 à 3, dans lequel dans X-7 R₁₅ est hydrogène, nitro, alkylsulfonyle en C₁₋₆, cyano, alkyle en C₁₋₆, alkoxy en C₁₋₆, perfluoro alkyle en C₁₋₆, alkylthio en C₁₋₆, alkylcarbonyle en C₁₋₆ ou aryle.

29. Composé selon La revendication 28, dans lequel R₁₅ est isopropyle, méthyle ou phényle.

30. Composé selon l'une quelconque des revendications 1 à 3, dans lequel R₁₆ dans X-7 est hydrogène, halogène, nitro, cyano, alkyle en C₁₋₆ ou perfluoro alkyle en C₁₋₆.

31. Composé selon la revendication 30, dans lequel R₁₆ est méthyle ou trifluorométhyle.

32. Composé selon l'une quelconque des revendications 1 à 3, dans lequel R₃₀ dans X-7 est hydrogène ou alkyle en C₁₋₆.

33. Composé selon l'une quelconque des revendications 1 à 3, dans lequel X-7 est choisi dans le groupe comprenant 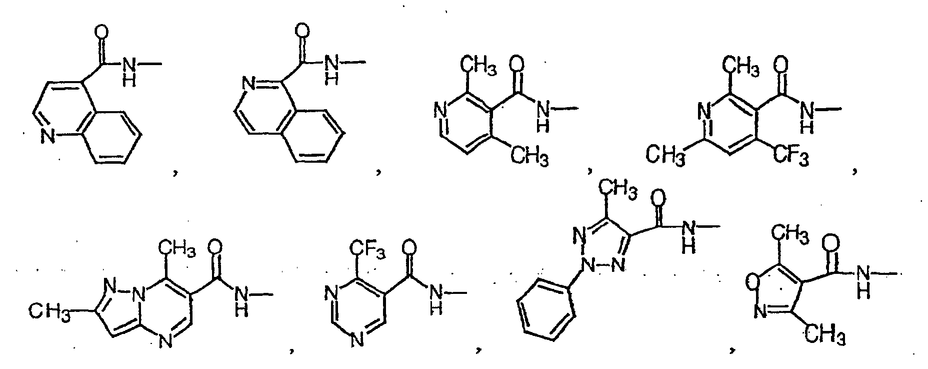 et

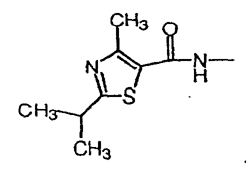

34. Composé selon l'une quelconque des revendications 1 à 3, dans lequel dans X-10 R₁₈ est phényle, le cycle phényle étant non substitué ou monosubstitué par halogène, ou est phényle alkyle en C₁₋₆.

35. Composé selon la revendication 34 dans lequel R₁₈ est phényle, chlorophényle ou phényléthyle.

36. Composé selon l'une quelconque des revendications 1 à 3, dans lequel dans X-10 R₁₉ est alkyle en C₁₋₆ qui est non substitué ou substitué par pyridyle ou phényle, le cycle phényle étant non substitué ou monosubstitué par alkoxy en C₁₋₆ ou halogène.

37. Composé selon la revendication 36 dans lequel R₁₉ est méthyle, isobutyle, benzyle, 4-chlorobenzyle, 4-méthoxybenzyle ou 2-pyridylméthyle.

38. Composé selon l'une quelconque des. revendications 1 à 3, dans lequel dans X-10 R₂₀ est alkylcarbonyle en C₁₋₆ facultativement substitué.

39. Composé selon la revendication 38, dans lequel R₂₀ est acétyle, butyryle, phénoxyacétyle, succinyle ou glutaryle.

40. Composé selon l'une quelconque des revendications 1 à 3, dans lequel X-10 est choisi dans le groupe comprenant :

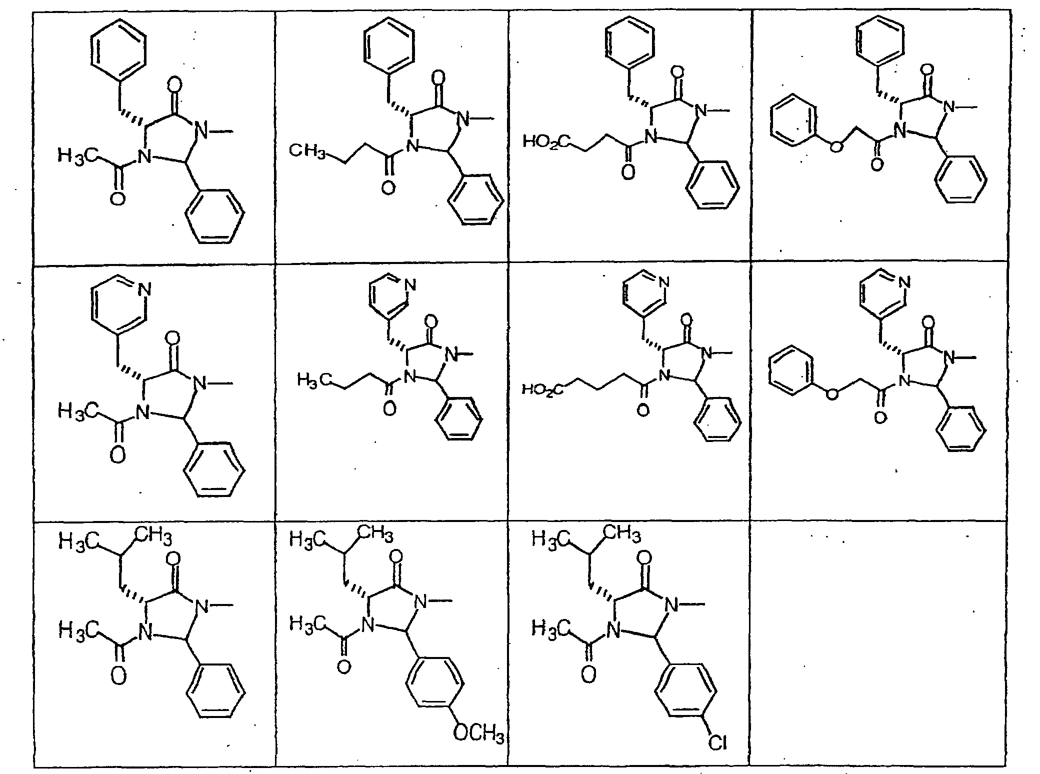

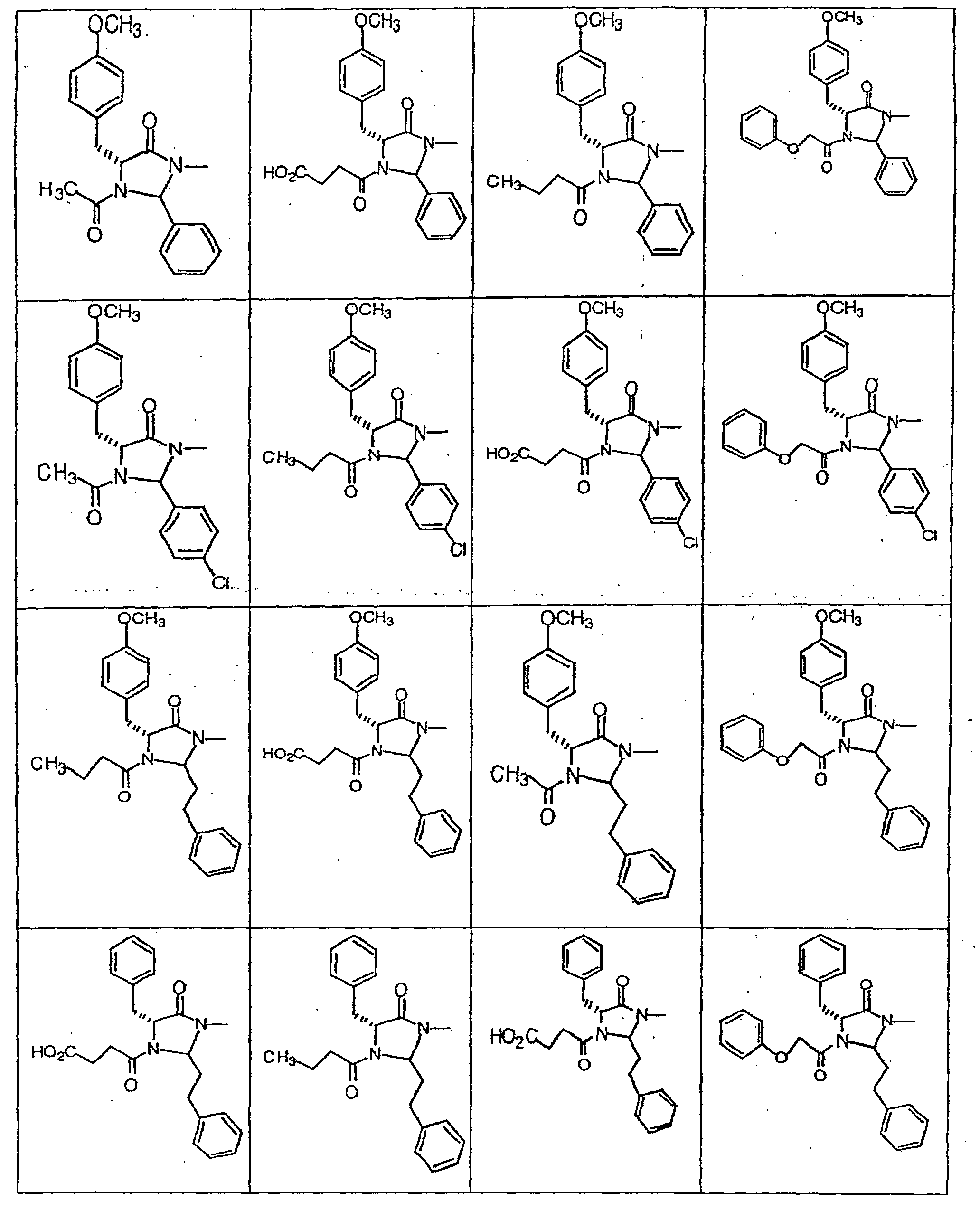

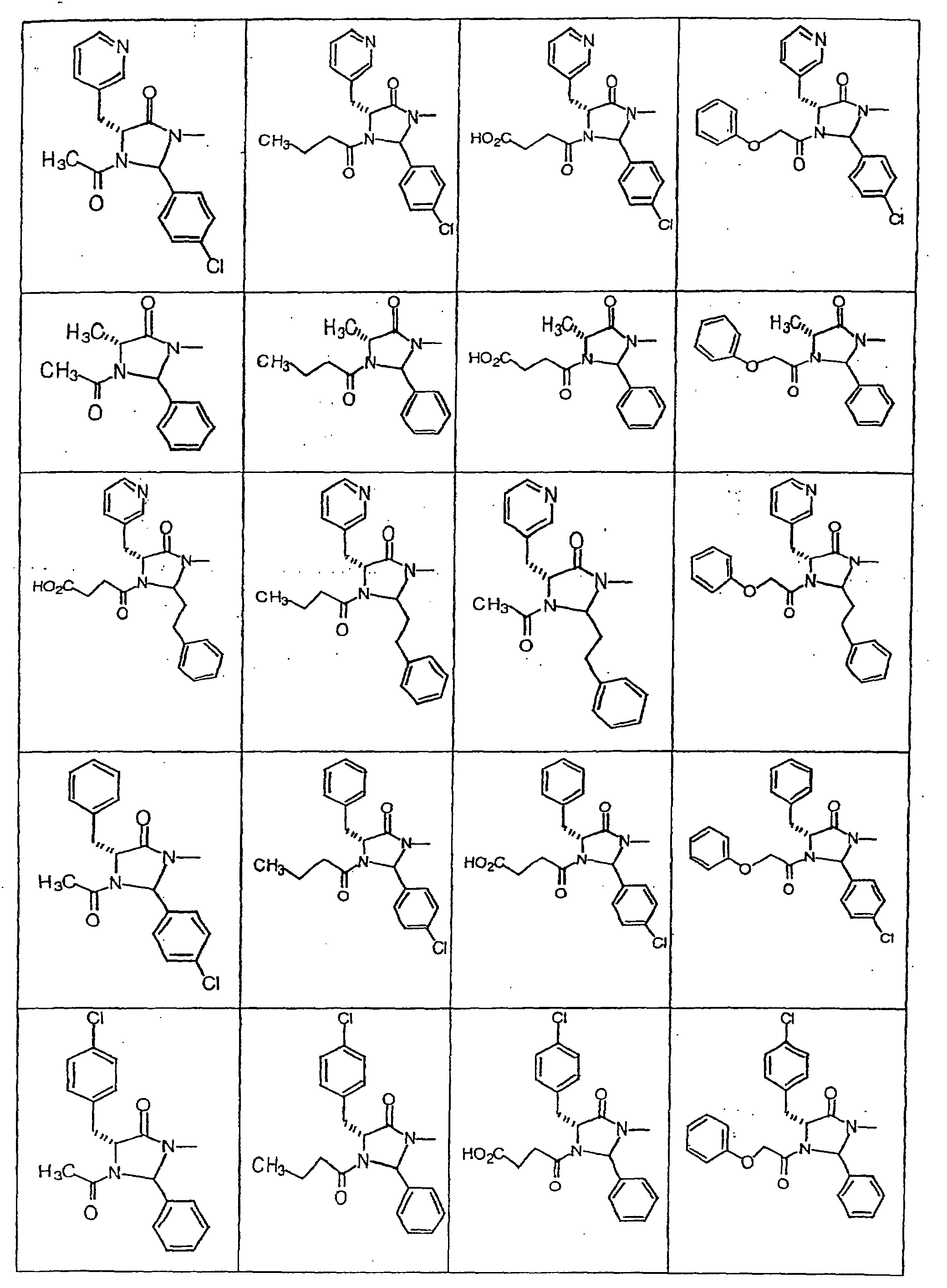

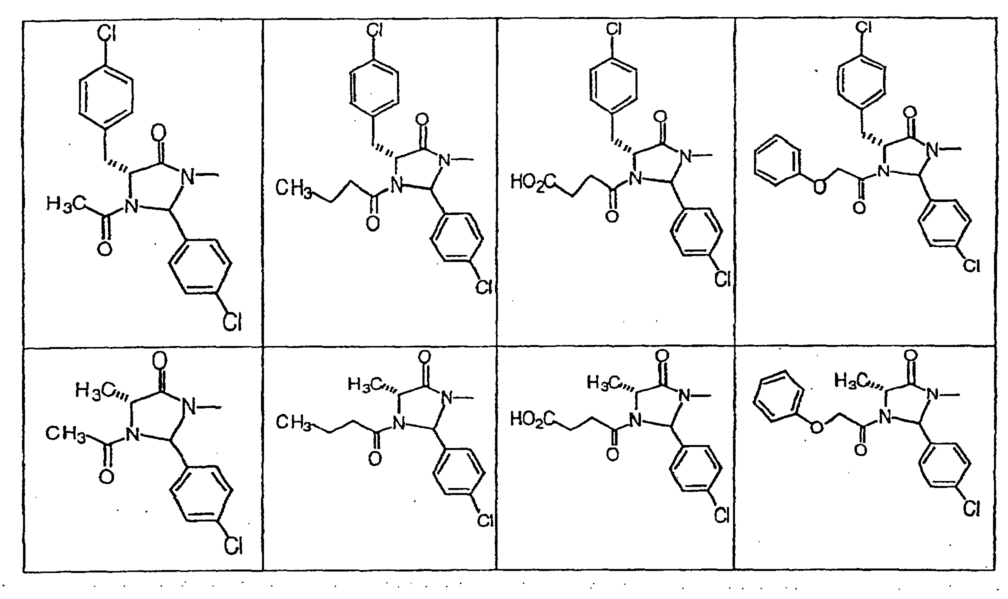

41. Composé selon la revendication 1 choisi du groupe constitué de :
4-[[(2,6-dichlorophényle)carbonyl]amino]-N-[[1-[(4-méthoxyphényle)méthyl]cyclopentyl]carbonyl]-L-phénylalanine,
N-[[1-[(4-méthoxyphényle)méthyl]cyclopentyl]carbonyl]-4-[[(2-nitrophényle)carbonyl]amino]-L-phénylalanine,
N-[[1-[(4-méthoxyphényle)méthyl]cyclopentyl]carbonyl]-4-[[(2-méthyl-5-nitrophényle)carbonyl]amino]-L-phénylalanine,
N-[[1-[(4-méthoxyphényle)méthyl]cyclopentyl]carbonyl]-4-[(4-quinolinylcarbonyl)amino]-L-phénylalanine;
N-[[1-(phénylméthyl)cyclopentyl]carbonyl]-4-[(4-quinolinylcarbonyl)amino)-L-phénylalanine,
4-[[(2,6-dichlorophényle)carbonyl]amino]-N-[[1-(phénylméthyl)cyclopentyl]carbonyl]-L-phénylalanine,
4-[[(2-nitrophényle)carbonyl]amino]-N-[[1-(phénylméthyl)cyclopentyl]carbonyl]-L-phénylalanine,
4-[[(2-méthyl-5-nitrophényle)carbonyl]amino]-N-[[1-(phénylméthyl)cyclopentyl]carbonyl]-L-phénylalanine,
4-[[(2,6-dichlorophényle)carbonyl]amino]-N-[[1-[2-[[N-(1,1-diméthyléthyle)carbonyl]amino]éthyl] cyclopentyl]carbonyl]-L-phénylalanine,
4-[[(2,6-dichlorophényle)carbonyl]amino]-N-[[1-[2-[(trifluoroacétyl)amino]éthyle]cyclopentyl]carbonyl]-L-phénylalanine,
4-[[(2,6-dichlorophényle)carbonyl]amino]-N-[[1-[2-[(2-amino-1-oxoéthyle)amino]éthyle]cyclopentyl]carbonyl]-L-phénylalanine,
4-[[(2,6-dichlorophényle)carbonyl]amino]-N- [ [1- [2-[[2-[[(1,1-diméthyléthoxy)carbonyl]amino]-1-oxoéthyle]amino]éthyle]cyclopentyl]carbonyl]-L-phénylalanine,
4-[[(2,6-dichlorophényle)carbonyl]amino]-N-[[2-[[(méthoxy)carbonyl]amino]éthyle]cyclopentyl]carbonyl] L-phénylalanine,
4-[[(2,6-dichlorophényle)carbonyl]amino]-M-[[1-[2-[(méthylsulfonyl)amino]éthyle]cyclopentyl]carbonyl]-L-phénylalanine,
4-[[(2,6-dichlorophényle)carbonyl]amino]-N-[[1-[2-[(acétyl)(méthyl)amino]éthyle]cyclopentyl]carbonyl]-L-phénylalanine,
4-[[(2,6-dichlorophényle)carbonyl]amino]-N-[[1-[2-[[(méthylamino)carbonyl](méthyl)amino]éthyle]cyclopentyl]carbonyl]-L-phénylalanine,
4-[[(2,6-dichlorophényle)carbonyl]amino]-N-[[1-[2-[(méthoxycarbonyl)-méthyl)amino]éthyle]cyclopentyl]carbonyl]-L-phénylalanine,
4-[[(2,6-dichlorophényle)carbonyl]amino]-N-[[1-(2-méthoxyéthyle)cyclopentyl]carbonyl]-L-phénylalanine,
4-[[(2,6-dichlorophényle)carbonyl]amino]N-[[1-[(2-[(méthylsulfonyl)éthyle]cyclopentyl]carbonyl]-L-phénylalanine,
4-[[(2,6-dichlorophényle)carbonyl]amino]-N-[[1-[(2-(méthylsulfinyl)éthyle]cyclopentyl]carbonyl]-L-phénylalanine,
4-[(2,6-Diméthyl-4-trifluorométhyl-3-pyridinyl)carbonyl]amino]-N-[[1-[4-(méthylsulfonyl)butyl]cyclopentyl]carbonyl]-L-phénylalanine,
4-[[(2,4-diméthylpyridin-3-yl)carbonyl]amino]-N-[[1-[4-(méthylsulfonyl)butyl]cyclopentyl]carbonyl]-L-phénylalanine,
ou 4-[[(2,6-dichlorophényle)carbonyl]amino]-N-[[1-(4-méthoxyphénylméthyl)cyclohexyl]carbonyl]-L-phénylalanine.

42. Composé selon la revendication 1 ayant la formule choisie dans le groupe comprenant :

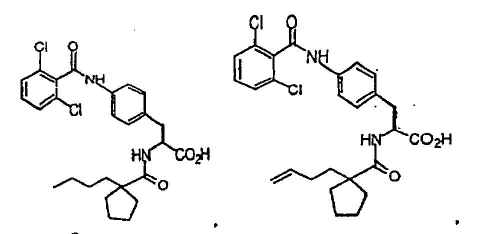

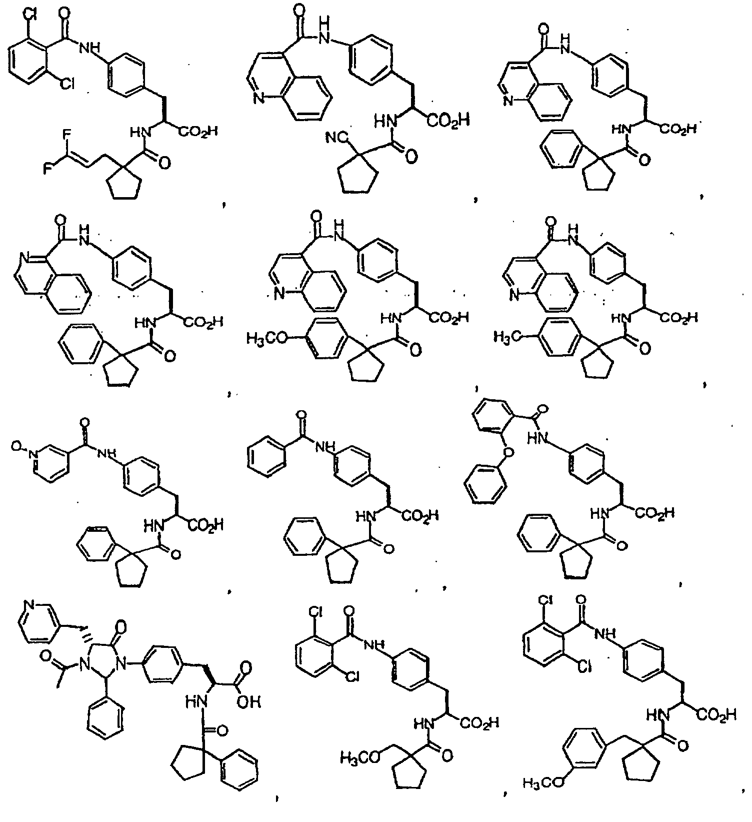

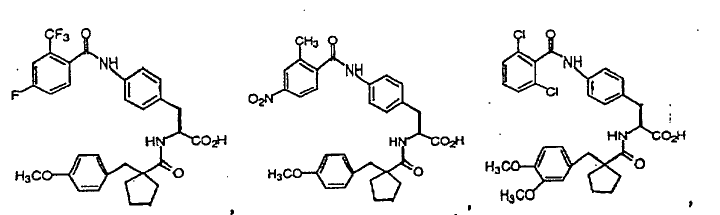

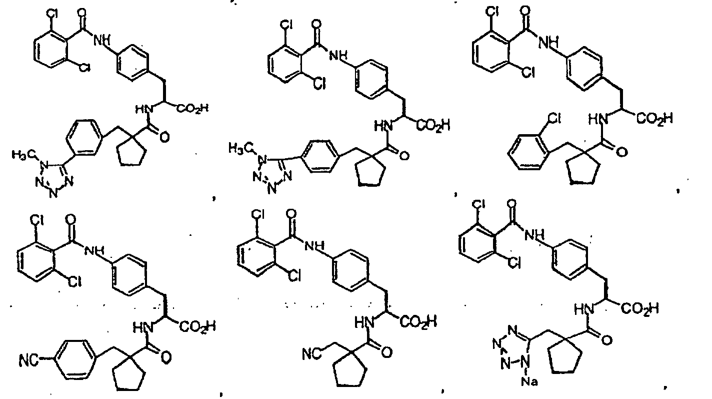

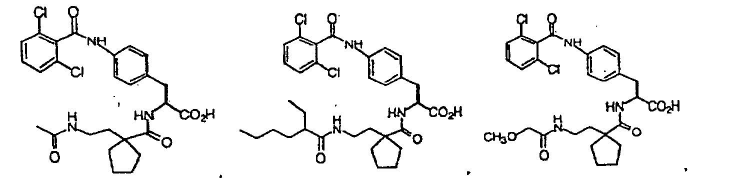

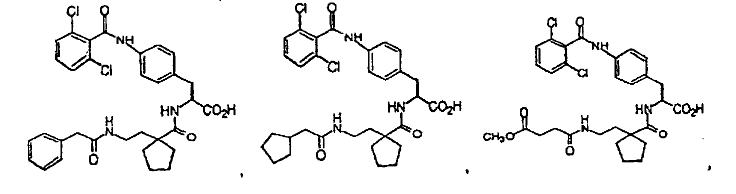

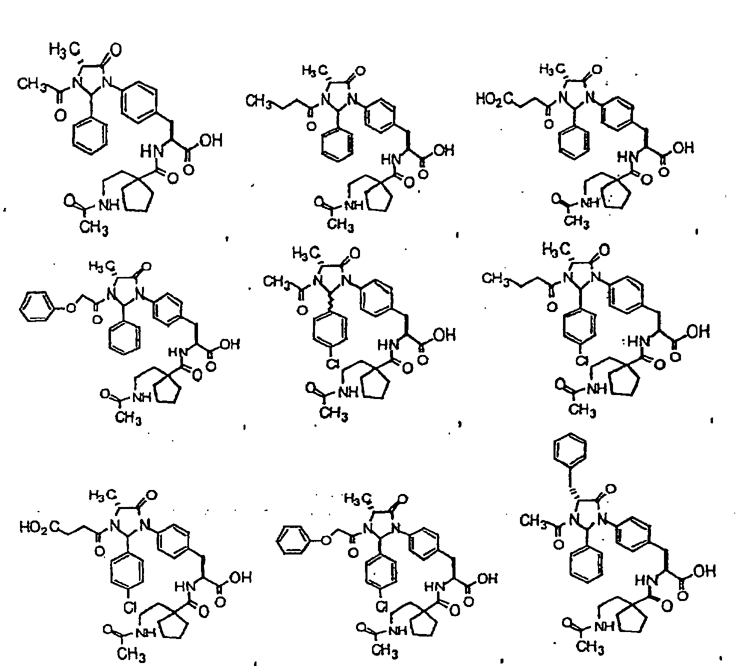

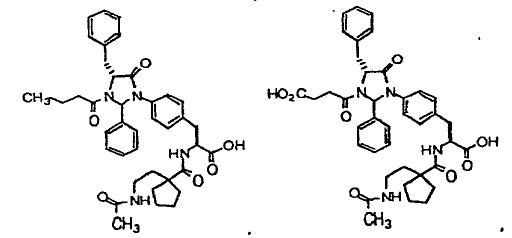

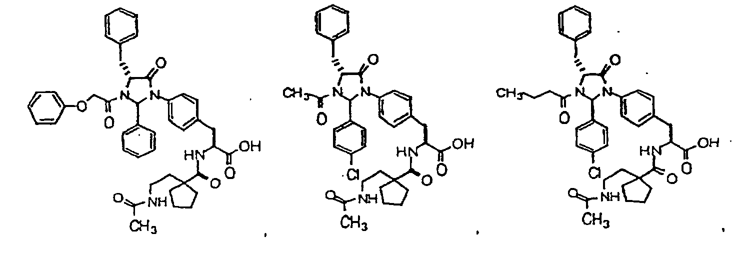

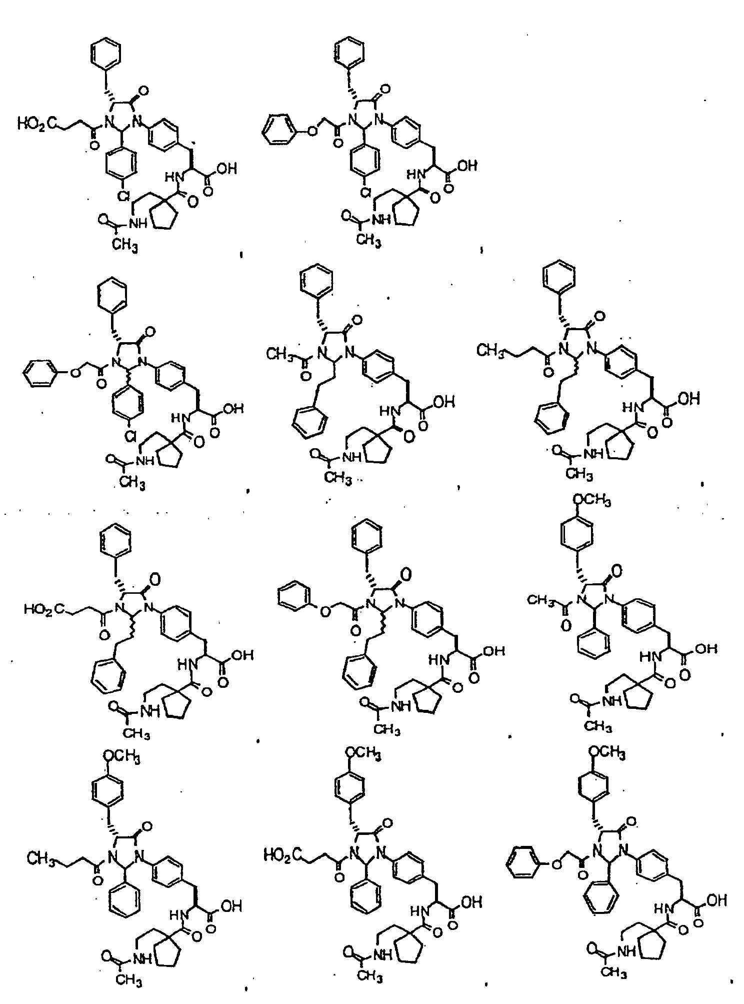

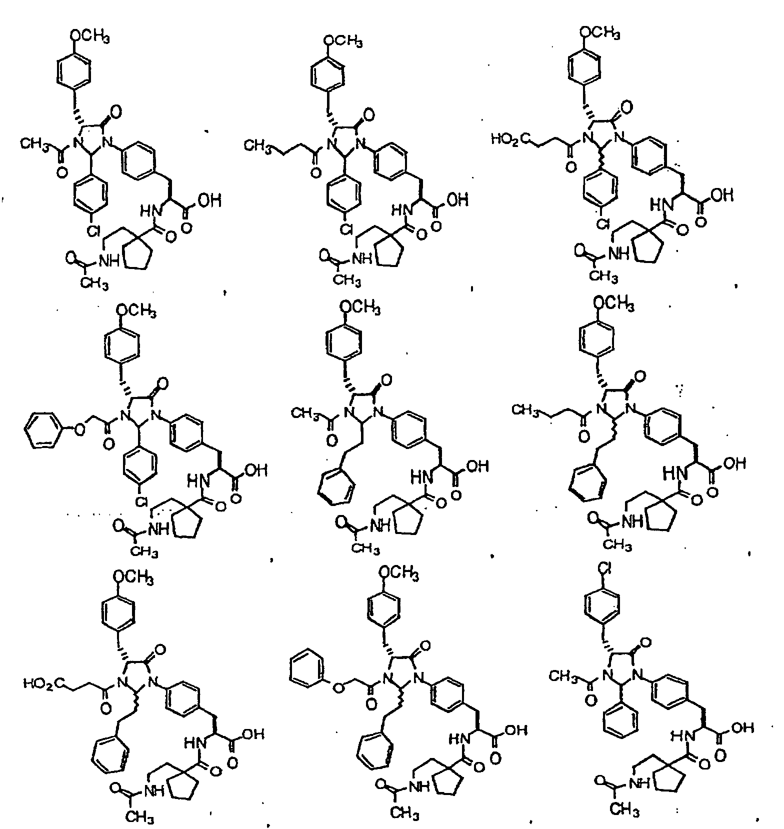

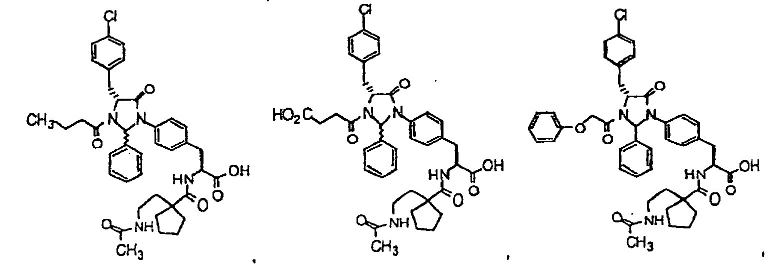

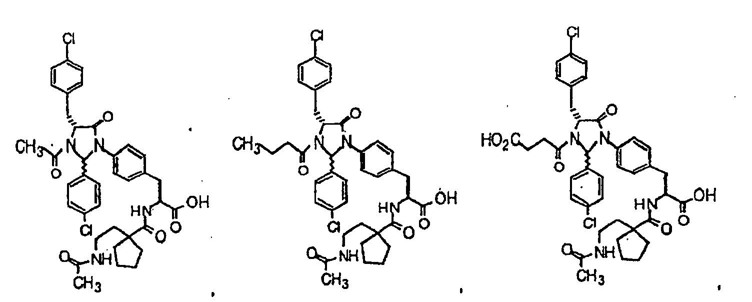

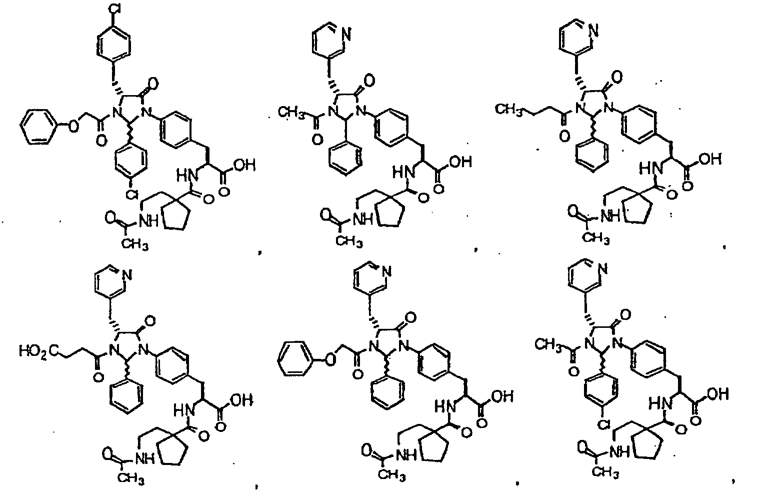

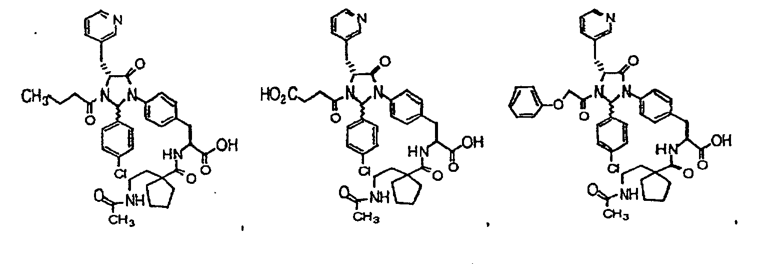

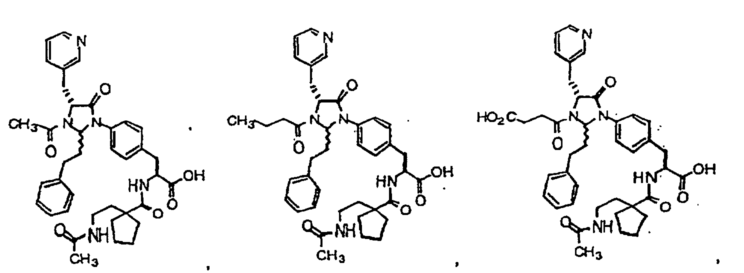

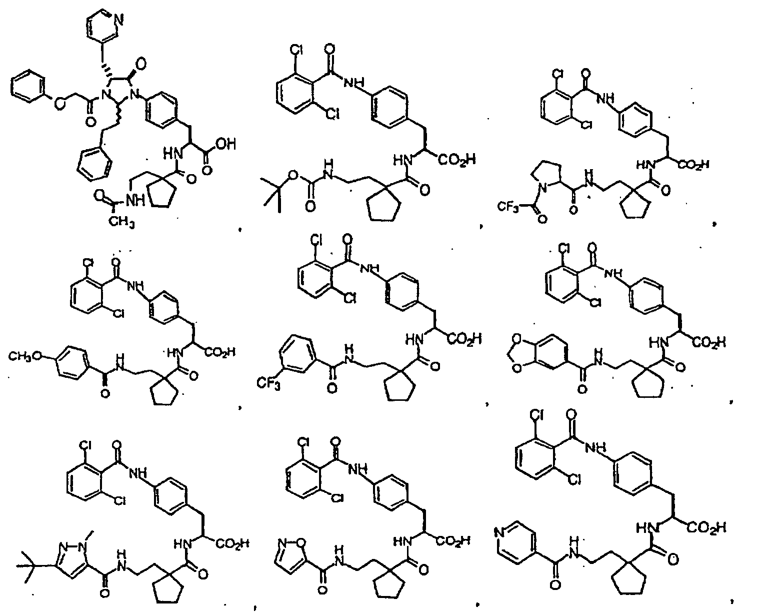

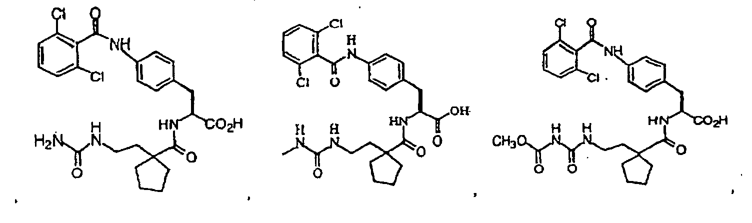

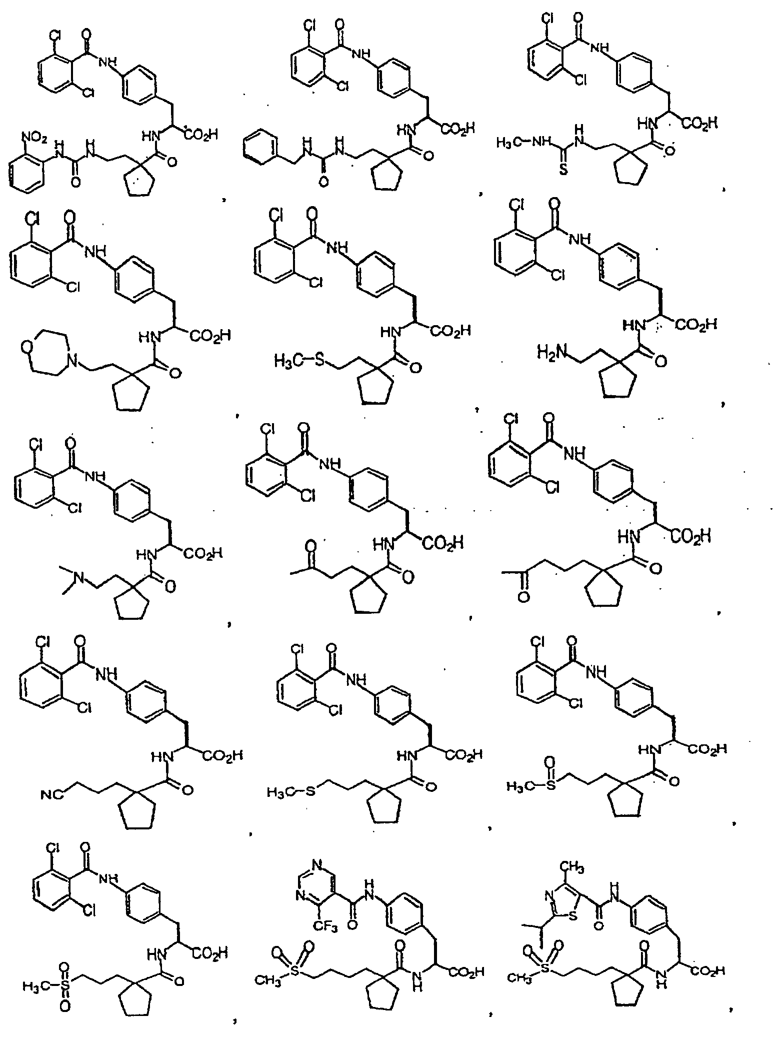

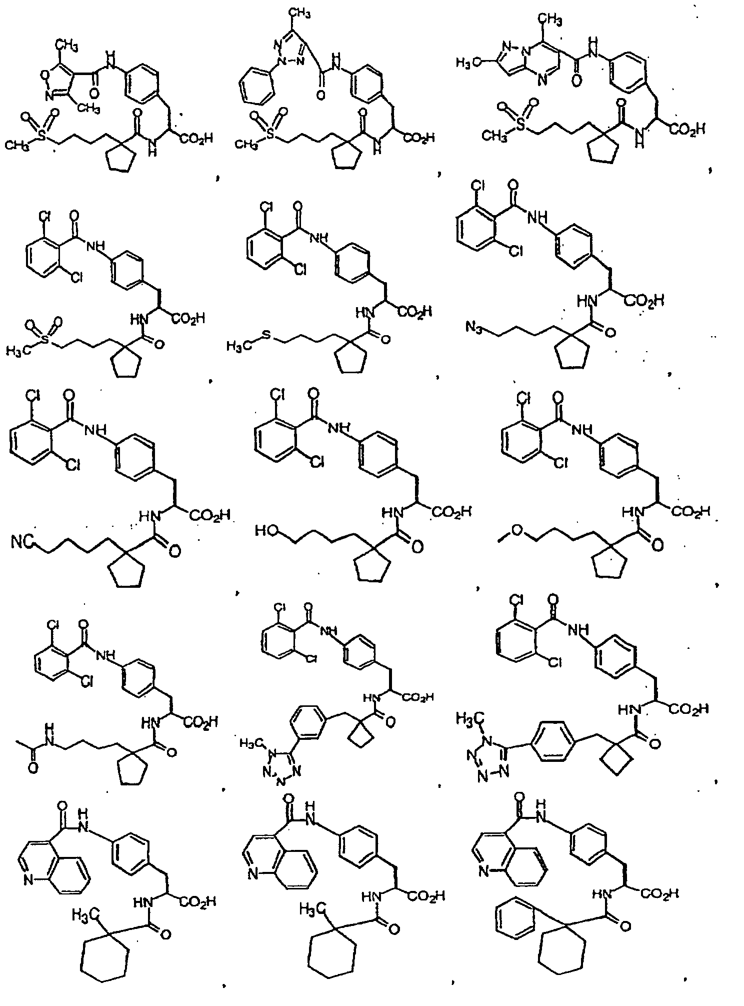

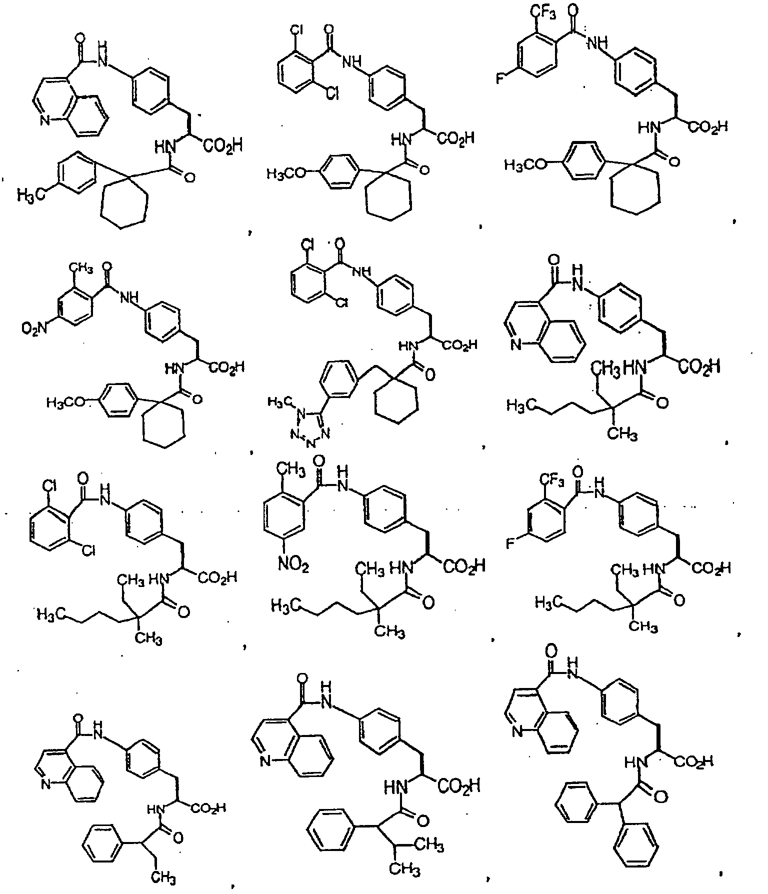

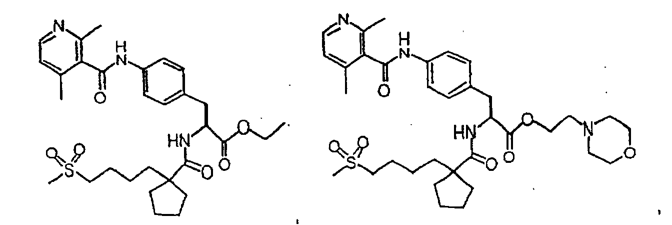

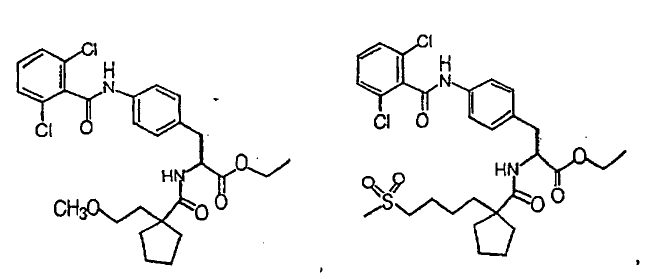

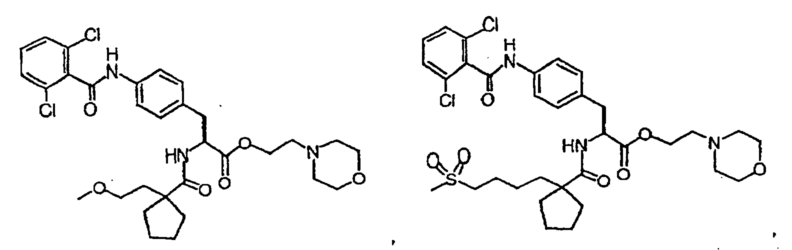

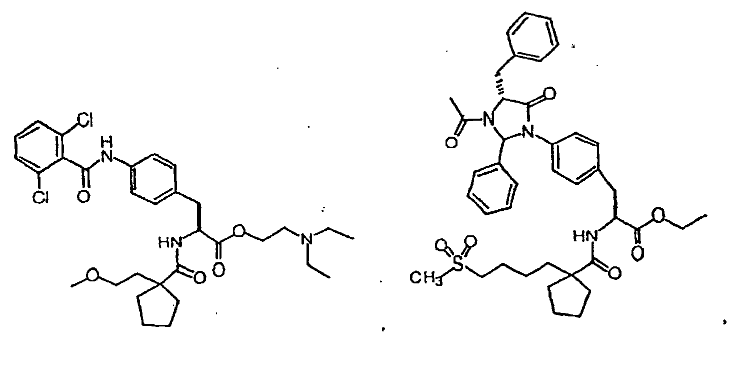

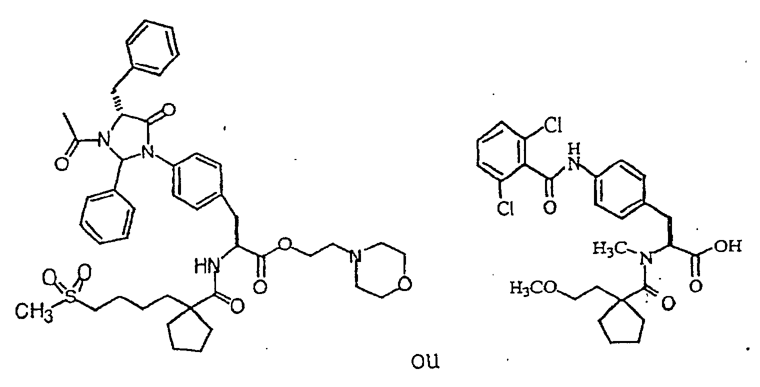

43. Procédé pour la préparation d'un composé de formule 1 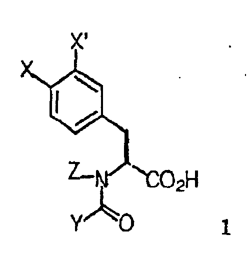 dans laquelle X, X' Z et Y sont comme définis dans la revendication 1,
**caractérisé en ce qu'**un composé de formule 1b 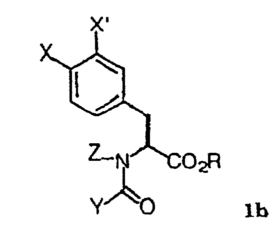 dans laquelle X, X', Z et Y sont comme définis et R est un groupe protecteur ou un support solide, le groupe protecteur ou le support solide est clivé de et, si souhaité, convertissant un composé de formule 1 en un sel pharmaceutiquement acceptable.

44. Composés selon l'une quelconque des revendications 1 à 42 pour son utilisation comme médicament.

45. Composés selon l'une quelconque des revendications 1 à 44 pour son utilisation comme médicament pour le traitement de la polyarthrite rhumatoïde, de la sclérose en plaques, de maladies intestinales inflammatoires ou de l'asthme.

46. Médicament contenant un composé selon l'une quelconque des revendications 1 à 42 et un support thérapeutiquement inerte.

47. Médicament selon la revendication 46 pour le traitement de la polyarthrite rhumatoide, de la sclérose en plaques, de maladies intestinales inflammatoires ou de l'asthme.

48. Procédé pour la production d'un médicament, en particulier pour le traitement de la polyarthrite rhumatoïde, de là sclérose en plaques, de maladies intestinales inflammatoirse ou de l'asthme, ledit procédé comprenant l'apport d'un composé selon l'une quelconque des revendications 1 à 42 dans une forme d'administration galénique avec un support thérapeutiquement inerte et, si souhaité, une ou plusieurs substances thérapeutiquement actives supplémentaires.

49. Utilisation d'un composé selon l'une quelconque des revendications 1 à 42 dans la préparation d'un médicament pour le traitement de la polyarthrite rhumatoïde, de la sclérose en plaques, de maladies inflammatoires intestinales ou de l'asthme.
